(19) Europäisches Patentamt
European Patent Office
Office européen des brevets

(11) EP 1 558 567 B1

(12) # EUROPÄISCHE PATENTSCHRIFT

(45) Veröffentlichungstag und Bekanntmachung des Hinweises auf die Patenterteilung:
**24.06.2009 Patentblatt 2009/26**

(21) Anmeldenummer: **03778292.7**

(22) Anmeldetag: **28.10.2003**

(51) Int Cl.:
***C07C 233/29*** (2006.01) ***C07C 235/24*** (2006.01)
***C07C 237/04*** (2006.01) ***C07C 255/60*** (2006.01)
***C07D 207/08*** (2006.01) ***C07D 207/20*** (2006.01)
***C07D 209/08*** (2006.01) ***C07D 211/62*** (2006.01)
***C07D 213/30*** (2006.01) ***C07D 213/56*** (2006.01)
***C07D 295/08*** (2006.01) ***C07D 295/12*** (2006.01)
***C07D 307/42*** (2006.01) ***C07D 333/16*** (2006.01)
***A61K 31/16*** (2006.01) ***A61K 31/33*** (2006.01)
***A61P 3/00*** (2006.01)

(86) Internationale Anmeldenummer:
**PCT/EP2003/011933**

(87) Internationale Veröffentlichungsnummer:
**WO 2004/039764 (13.05.2004 Gazette 2004/20)**

(54) **NEUE AMID-VERBINDUNGEN MIT MCH-ANTAGONISTISCHER WIRKUNG UND DIESE VERBINDUNGEN ENTHALTENDE ARZNEIMITTEL**

NOVEL AMIDE COMPOUNDS WITH MCH ANTAGONISTIC EFFECT AND MEDICAMENTS COMPRISING SAID COMPOUNDS

NOUVEAUX COMPOSES DE TYPE AMIDE EXER ANT UNE ACTION ANTAGONISTE SUR L'HORMONE MCH, ET MEDICAMENTS CONTENANT CES COMPOSES

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **31.10.2002 DE 10250743**

(43) Veröffentlichungstag der Anmeldung:
**03.08.2005 Patentblatt 2005/31**

(73) Patentinhaber: **Boehringer Ingelheim Pharma GmbH & Co. KG**
**55216 Ingelheim am Rhein (DE)**

(72) Erfinder:
- **STENKAMP, Dirk**
**88400 Biberach (DE)**
- **MÜLLER, Stephan, Georg**
**88447 Warthausen (DE)**
- **ROTH, Gerald, Jürgen**
**88400 Biberach (DE)**
- **LUSTENBERGER, Philipp**
**88447 Warthausen (DE)**
- **RUDOLF, Klaus**
**88447 Warthausen (DE)**
- **LEHMANN-LINTZ, Thorsten**
**88416 Ochsenhausen (DE)**
- **ARNDT, Kirsten**
**88400 Biberach (DE)**
- **LOTZ, Ralf, R., H.**
**88433 Schemmerhofen (DE)**
- **LENTER, Martin**
**89073 Ulm (DE)**
- **WIELAND, Heike-Andrea**
**65812 Bad Soden (DE)**

(56) Entgegenhaltungen:
**WO-A-00/06153** **WO-A-01/21577**
**WO-A-02/06245** **WO-A-02/057233**
**WO-A-03/035055**

- **BJURSELL M. ET AL DIABETES Bd. 55, 2006, Seiten 725 - 733**
- **PISSIOS P. ET AL DIABETES Bd. 56, 2007, Seiten 311 - 319**
- **MORENS C. ET AL BRAIN RESEARCH Bd. 1062, 2005, Seiten 32 - 38**
- **KOKKOTOU E. ET AL PNAS Bd. 105, 2008, Seiten 10613 - 10618**
- **BOROWSKY B. ET AL NATURE MEDICINE Bd. 8, 2002, Seiten 825 - 830**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 558 567 B1

• SHIMAZAKI T. ET AL CNS DRUGS Bd. 20, 2006, Seiten 801 - 811

## Beschreibung

Gegenstand der vorliegenden Erfindung sind neue Amid-Verbindungen, deren physiologisch verträglichen Salze und deren Verwendung als MCH-Antagonisten sowie deren Verwendung zur Herstellung eines Arzneimittels, welches zur Prophylaxe und/oder Behandlung von Erscheinungen und/oder Krankheiten, die durch MCH verursacht werden oder mit MCH in einem anderen kausalen Zusammenhang stehen, geeignet ist. Ein weiterer Gegenstand dieser Erfindung betrifft die Verwendung einer erfindungsgemäßen Verbindung zur Beeinflussung des Essverhaltens sowie zur Reduzierung des Körpergewichts und/ oder zum Verhindern einer Zunahme des Körpergewichts eines Säugetiers. Ferner sind Zusammensetzungen und Arzneimittel, jeweils enthaltend eine erfindungsgemäße Verbindung, sowie Verfahren zu deren Herstellung Gegenstand dieser Erfindung.

## Hintergrund der Erfindung

Die Aufnahme von Nahrung und deren Umsetzung im Körper spielt für alle Lebewesen eine existentielle Rolle im Leben. Daher führen Abweichungen bei der Aufnahme und Umsetzung der Nahrung in der Regel zu Störungen und auch Krankheiten. Die Veränderung der menschlichen Lebens- und Ernährungsgewohnheiten, insbesondere in Industrieländern, hat in den letzten Jahrzehnten Obesitas begünstigt. Obesitas führt bei den Betroffenen unmittelbar zu einer Einschränkung der Mobilität und einer Verminderung der Lebensqualität. Erschwerend kommt hinzu, dass Obesitas oft weitere Krankheiten zur Folge hat, wie beispielswiese Diabetes, Dyslipidaemie, Bluthochdruck, Arteriosklerose und koronare Herzerkrankungen. Darüber hinaus führt alleine das hohe Körpergewicht zu einer verstärkten Belastung des Stütz- und Bewegungsapparates, was zu chronischen Beschwerden und Krankheiten, wie Arthritis oder Osteoarthritis, führen kann. Somit stellt Obesitas ein schwerwiegendes gesundheitliches Problem für die Gesellschaft dar.

Der Begriff Obesitas bedeutet einen Überschuss an adipösem Gewebe. In diesem Zusammenhang ist Obesitas grundsätzlich als jeglicher erhöhter Grad an Adipositas zu sehen, der zu einem gesundheitlichen Risiko führt. Eine Abgrenzung zwischen normalen und an Obesitas leidenden Individuen ist letztlich nicht exakt möglich, jedoch steigt das mit Obesitas einhergehende gesundheitliche Risiko wahrscheinlich kontinuierlich mit zunehmender Adipositát an. Aus Gründen der Vereinfachung werden im Zusammenhang mit der vorliegenden Erfindung vorzugsweise die Individuen mit einem Körpergewichtsindex (BMI = body mass index), der als das in Kilogramm gemessene Körpergewicht geteilt durch die Körpergröße (in Metern) im Quadrat definiert ist, oberhalb des Wertes 25, insbesondere oberhalb 30, als an Obesitas leidend betrachtet.

Abgesehen von körperlicher Aktivität und Ernährungsumstellung existiert derzeit keine überzeugende Behandlungsmöglichkeit zur effektiven Reduzierung des Körpergewichts. Da Obesitas jedoch einen hohen Risikofaktor bei der Entstehung ernsthafter und sogar lebensbedrohlicher Erkrankungen darstellt, ist es umso wichtiger, pharmazeutische Wirkstoffe zur Prophylaxe und/oder Behandlung von Obesitas bereit zu stellen. Ein in neuester Zeit vorgeschlagener Ansatz ist der therapeutische Einsatz von MCH-Antagonisten (u.a. WO 01/21577, WO 01/82925).

Melanin-konzentrierendes Hormon (melanin-concentrating hormone, MCH) ist ein zyklisches Neuropeptid bestehend aus 19 Aminosäuren. Es wird in Säugetieren vorwiegend im Hypothalamus synthetisiert und erreicht von dort weitere Gehirnregionen über die Projektionen hypothalamischer Neurone. Seine biologische Aktivität wird im Menschen über zwei unterschiedliche G-Protein-gekoppelte Rezeptoren (GPCRs) aus der Familie Rhodopsin-verwandter GPCRs vermittelt, die MCH-Rezeptoren 1 und 2 (MCH-1R, MCH-2R).

Untersuchungen der Funktion von MCH in Tiermodellen ergeben gute Anhaltspunkte für eine Rolle des Peptides bei der Regulation der Energiebilanz, d.h. Veränderung metabolischer Aktivität und Futteraufnahme [1, 2]. Beispielsweise wird nach intraventrikulärer Applikation von MCH bei Ratten die Futteraufnahme im Vergleich zu Kontrolltieren gesteigert. Daneben reagieren transgene Ratten, die mehr MCH produzieren als Kontrolltiere, nach Gabe einer fettreichen Diät mit einer deutlicheren Gewichtssteigerung als Tiere mit nicht experimentell verändertem MCH-Spiegel. Auch konnte festgestellt werden, dass eine positive Korrelation zwischen Phasen gesteigerten Verlangens nach Futter und der Menge an MCH mRNA im Hypothalamus von Ratten besteht. Von besonderer Aussagekraft bezüglich der Funktion von MCH sind aber Experimente mit MCH knock out Mäusen. Ein Verlust des Neuropeptides führt zu mageren Tieren mit verminderter Fettmasse, die deutlich weniger Nahrung zu sich nehmen als Kontrolltiere.

Die anorektischen Effekte von MCH werden in Nagetieren über den $G_{\alpha s}$-gekoppelten MCH-1 R vermittelt [3-6]. Im Gegensatz zum Primaten, Frettchen und Hund, konnte bei Nagern bisher kein zweiter Rezeptor nachgewiesen werden. Nach Verlust des MCH-1 R besitzen knock out Mäuse weniger Fettmasse, einen erhöhten Energieumsatz und bei fettreicher Diät keine Gewichtssteigerung im Vergleich zu Kontrolltieren. Ein weiterer Hinweis für die Bedeutung des MCH-MCH-1 R Systems bei der Regulation der Energiebilanz stammt aus Experimenten mit einem Rezeptor-Antagonisten (SNAP-7941) [3]. In Langzeit-Versuchen verlieren die mit dem Antagonisten behandelten Tiere deutlich an Gewicht.

Neben seiner anorektischen Wirkung werden mit dem MCH-1 R-Antagonisten SNAP-7941 noch weitere anxiolytische und antidepressive Effekte in Verhaltensexperimenten mit Ratten erzielt [3]. Damit liegen deutliche Hinweise

vor, dass das MCH-MCH-1 R-System nicht nur an der Regulation der Energiebilanz sondern auch der Affektivität beteiligt ist.

Literatur:


1. Qu, D., et al., A role for melanin-concentrating hormone in the central regulation of feeding behaviour. Nature, 1996. 380(6571): p. 243-7.
2. Shimada, M., et al., Mice lacking melanin-concentrating hormone are hypophagic and lean. Nature, 1998. 396 (6712): p. 670-4.
3. Borowsky, B., et al., Antidepressant, anxiolytic and anorectic effects of a melanin-concentrating hormone-1 receptor antagonist. Nat Med, 2002. 8(8): p. 825-30.
4. Chen, Y., et al., Targeted disruption of the melanin-concentrating hormone receptor-1 results in hyperphagia and resistance to diet-induced obesity. Endocrinology, 2002. 143(7): p. 2469-77.
5. Marsh, D.J., et al., Melanin-concentrating hormone 1 receptor-deficient mice are lean, hyperactive, and hyperphagic and have altered metabolism. Proc Natl Acad Sci U S A, 2002. 99(5): p. 3240-5.
6. Takekawa, S., et al., T-226296: a novel, orally active and selective melanin-concentrating hormone receptor antagonist. Eur J Pharmacol, 2002. 438(3): p. 129-35.


In der Patentliteratur werden bestimmte Amin-Verbindungen als MCH Antagonisten vorgeschlagen. So werden in der WO 01/21577 (Takeda) Verbindungen der Formel

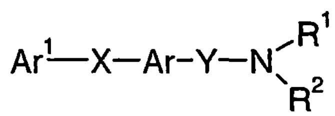

in der $Ar^1$ eine cyclische Gruppe , X einen Spacer, Y eine Bindung oder einen Spacer, Ar einen aromatischen Ring, der mit einem nicht-aromatischen Ring kondensiert sein kann, $R^1$ und $R^2$ unabhängig voneinander H oder eine Kohlenwasserstoff-Gruppe bedeuten, wobei $R^1$ und $R^2$ zusammen mit dem angrenzenden N-Atom einen N-haltigen Heteroring bilden können und $R^2$ mit Ar auch einen spirocyclischen Ring bilden kann, R zusammen mit dem angrenzenden N-Atom und Y einen N-haltigen Heteroring bilden kann, als MCH-Antagonisten zur Behandlung von u.a. Obesitas beschrieben.

Ferner werden in der WO 01/82925 (Takeda) ebenfalls Verbindungen der Formel

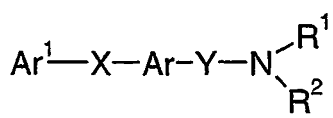

in der $Ar^1$ eine cyclische Gruppe , X und Y Spacer-Gruppen, Ar einen gegebenenfalls substituierten kondensierten polycyclischen aromatischen Ring, $R^1$ und $R^2$ unabhängig voneinander H oder eine Kohlenwasserstoff-Gruppe bedeuten, wobei $R^1$ und $R^2$ zusammen mit dem angrenzenden N-Atom einen N-haltigen heterocyclischen Ring bilden können und $R^2$ zusammen mit dem angrenzenden N-Atom und Y einen N-haltigen Heteroring bilden kann, als MCH-Antagonisten zur Behandlung von u.a. Obesitas beschrieben.

In der EP 073 016 A1 (Boehringer Ingelheim) werden 1-Aryloxy-3-alkylamino-2-propanole der allgemeinen Formel

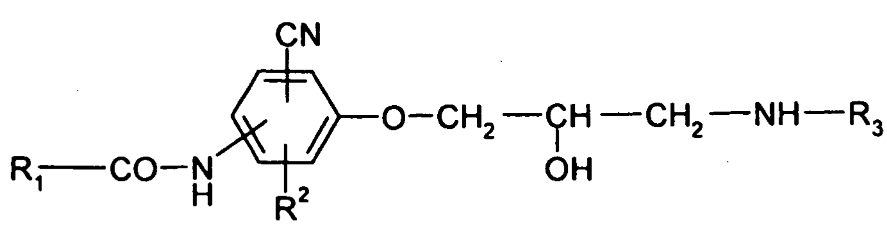

worin $R_1$ u.a. Aryloxyalkylen bedeuten kann, zur Verwendung als Herz- bzw. Coronartherapeutica oder auch zur Senkung des Blutdrucks vorgeschlagen. Eine MCH-antagonistische Aktivität dieser Verbindungen wird dagegen nicht beschrieben.

In der US 3,994,900 werden u.a. N-(4-Alkoxy-phenyl)-3-phenyl-acrylamide, N-(4-Alkylthio-phenyl)-3-phenyl-acrylamide, N-(4-Alkylsulfinyl-phenyl)-3-phenyl-acrylamide und N-(4-Alkylsulfonyl-phenyl)-3-phenyl-acrylamide als Ausgangsstoffe zur Synthese von Dihydrochinolinon-Derivaten genannt.

In der DE 1088955 werden u.a. die Verbindungen N-[4-(2-Diethylamino-ethoxy)-phenyl]-3-phenyl-acrylamid und N-[4-(2-Diethylamino-ethoxy)-phenyl]-3-phenyl-propionamid aufgeführt.

In der WO 00/06153, die Verbindungen mit CCR5-Rezeptor-Aktivität vorschlägt, wird u.a. die Verbindung 3-(3,4-Dichloro-phenyl)-N-[3-(2-diisopropylamino-ethoxy)-4-methoxy-phenyl]-acrylamid genannt.

In der FR 1176918 werden die Verbindungen (N-[4-(2-Morpholin-4-ylethoxy)-phenyl]-3-phenyl-propionamid) und N-[4-(2-Diethylamino-ethoxy)-phenyl]-3-phenyl-propionamid genannt.

In dem Artikel A. P. Tamiz et al., J. Med. Chem. 42 (17), 1999, 3412-3420 wird die Verbindung (3-(4-Chlor-phenyl)-N-{2-[4-(2-diethylamino-ethoxy)-phenyl]-ethyl}-acrylamid) angeführt.

In der DE 3016827, die Verbindungen mit einer Wirksamkeit auf das cardiovaskuläre System betrifft, werden auf der Seite 55 u.a. die Verbindungen N-{2-[3-(4-{2-[2-(4-Chlor-phenoxy)-acetylamino]-ethyl}phenoxy)-2-hydroxy-propylamino]-ethyl}-isobutyramid, Cyclopentancarbonsäure {2-[3-(4-{2-[2-(4-chlor-phenoxy)-acetylamino]-ethyl}-phenoxy)-2-hydroxy-propylamino]-ethyl}-amid und 2-(4-Chlor-phenoxy)-N-(2-{4-[2-hydroxy-3-(2-phenylacetylamino-ethylamino)-propoxy]-phenyl}-ethyl)-acetamid genannt.

In der WO 02/06245 (Synaptic) werden Verbindungen der allgemeinen Formeln

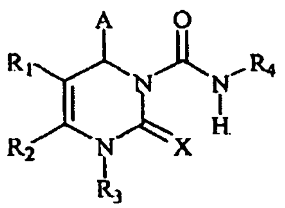
,
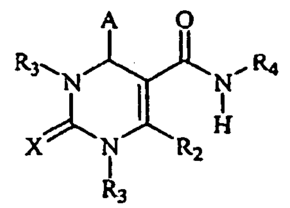
,

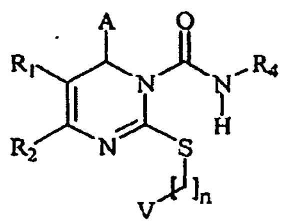
, or
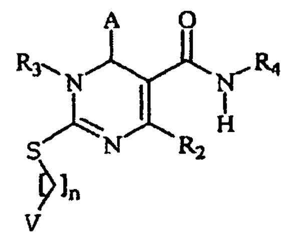

worin A eine (Hetero)Arylgruppe darstellt, als MCH Antagonisten beschrieben.

N-(4-(Aminosulfonyl)phenyl)-2-phenoxy-acetamid-Derivate werden von Fahmy et al., Archives of Pharmacal Research (2001), 24(3), 171-179 in einem Artikel (Synthesis and evaluation of the analgesic and antiinflammatory activities of O-substituted salicylamides) beschrieben.

N-substituierte Amide der Phenoxyessigsäure werden von Ariesan et al. in Farmacia (Bucharest, Romania) (1975), 23(3), 135-40 aufgeführt.

Eine hypoglykämische Aktivität von 4-(beta-Acylaminoethyl)benzolsulfonamiden wird von Khlaponina in Khimiko-Farmatsevticheskii Zhurnal (1987), 21(8), 965-8 beschrieben.

In einem Artikel von Zheng et al. in Zhongshan Daxue Xuebao, Ziran Kexueban (1989), 28(4), 124-7 zu Antitumor-Substanzen werden N-(4-Aminosulfonyl)phenyl)-2-phenoxy-acetamid-Derivate genannt.

N-(4-Aminoethyl)phenyl-2-(3-chloropehnoxy)-acetamid wird von Baker et al. in Journal of Medicinal Chemistry (1968), 11(5), 1054-9 beschrieben.

In der US 4,948,812 werden 1-Phenoxy-3-amino-2-propanol-Derivate im Zusammenhang mit der Behandlung von Herz-Kreislauferkrankungen erwähnt.

Derivate von Phenoxy-acetamiden werden in US 3,551,478 im Zusammenhang mit der Regulierung des Lipid-Stoffwechsels beschrieben.

In der US 4,146,637 werden N-Acyl-substituierte Benzamide als Verbindungen mit antiinflammatorischer Aktivität aufgeführt.

Derivate der Aminobenzoesäure werden in US 4,294,851 mit einer Aktivität auf den Lipid-Stoffwechsel beschrieben.

N-4-(2-(Diethylamino)ethoxy)phenyl)-phenylpropanamid wird von Guidicelli et al. in Compt. Rend. (1955), 241, 529-30 im Zusammenhang mit lokal anästhetischer Wirkung genannt.

**Aufgabe der Erfindung**

Der vorliegenden Erfindung liegt die Aufgabe zugrunde, neue Amid-Verbindungen aufzuzeigen, insbesondere solche, die eine Aktivität als MCH-Antagonisten besitzen.

Ebenfalls ist es eine Aufgabe dieser Erfindung, neue Amid-Verbindungen bereit zu stellen, die es erlauben, dass Essverhalten von Säugetieren zu beeinflussen und insbesondere bei Säugetieren eine Reduzierung des Körpergewichts zu erreichen und/oder eine Zunahme des Körpergewichts zu verhindern.

Ferner ist es eine Aufgabe der vorliegenden Erfindung, neue Arzneimittel bereit zu stellen, welche zur Prophylaxe und/oder Behandlung von Erscheinungen und/oder Krankheiten, die durch MCH verursacht werden oder mit MCH in einem anderen kausalen Zusammenhang stehen, geeignet sind. Insbesondere liegt dieser Erfindung die Aufgabe zugrunde, Arzneimittel zur Behandlung von metabolischen Störungen, wie Obesitas und/oder Diabetes sowie von mit Obesitas und Diabetes einhergehenden Krankheiten und/oder Störungen, zur Verfügung zu stellen. Weitere Aufgaben der vorliegenden Erfindung beziehen sich auf das Aufzeigen von vorteilhaften Verwendungen der erfindungsgemäßen Verbindungen. Ebenfalls eine Aufgabe dieser Erfindung ist es, ein Verfahren zur Herstellung der erfindungsgemäßen Amid-Verbindungen bereit zu stellen. Weitere Aufgaben der vorliegenden Erfindung ergeben sich für den Fachmann unmittelbar aus den vorhergehenden und nachfolgenden Ausführungen.

**Gegenstand der Erfindung**

Ein erster Gegenstand der vorliegenden Erfindung sind Amid-Verbindungen der allgemeinen Formel I

Amid-Verbindungen der allgemeinen Formel I

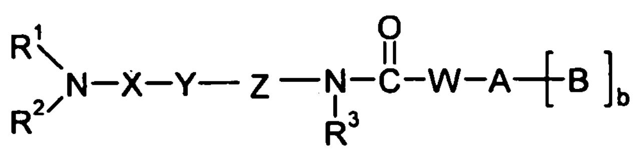

I

in der

$R^1$, $R^2$ unabhängig voneinander H, eine gegebenenfalls mit dem Rest $R^{11}$ substituierte $C_{1-8}$-Alkyl- oder $C_{3-7}$Cycloalkyl-Gruppe, wobei eine $-CH_2$-Gruppe in Position 3 oder 4 einer 5, 6 oder 7-gliedrigen Cycloalkylgruppe durch -O-, -S-, -NH-, $-N(C_{1-4}$-Alkyl)- oder $-N(CO-O-C_{1-4}$-Alkyl)- ersetzt sein kann, oder ein gegebenenfalls mit dem Rest $R^{12}$ ein- oder mehrfach und/oder mit Nitro einfach substituierter Phenyl- oder Pyridinylrest, mit der Maßgabe, dass mindestens einer der Reste $R^1$, $R^2$ eine von H verschiedene Bedeutung aufweist oder
$R^1$ und $R^2$ bilden eine $C_{2-8}$-Alkylen-Brücke, in der

- ein oder zwei $-CH_2$-Gruppen unabhängig voneinander durch -CH=N- oder -CH=CH- ersetzt sein können und/oder
- ein oder zwei $-CH_2$-Gruppen unabhängig voneinander durch -O-, -S-, -SO-, $-(SO_2)$-, $-C=N-O-R^{18}$, -CO-, $-C(=CH_2)$- oder $-NR^{13}$- derart ersetzt sein können, dass Heteroatome nicht unmittelbar miteinander verbunden sind,

wobei in der zuvor definierten Alkylen-Brücke ein oder mehrere H-Atome durch $R^{14}$ ersetzt sein können, und
wobei die zuvor definierte Alkylen-Brücke mit einer oder zwei gleichen oder verschiedenen carbo- oder heterocyclischen Gruppen Cy derart substituiert sein kann, dass die Bindung zwischen der Alkylenbrücke und der Gruppe Cy

- über eine Einfach- oder Doppelbindung,
- über ein gemeinsames C-Atom unter Ausbildung eines spirocyclischen Ringsystems,
- über zwei gemeinsame, benachbarte C- und/oder N-Atome unter Ausbildung eines kondensierten

bicyclischen Ringsystems oder
-über drei oder mehrere C- und/oder N-Atome unter Ausbildung eines verbrückten Ringsystems erfolgt,

$R^3$ H, $C_{1-6}$-Alkyl, $C_{3-7}$-Cycloalkyl oder $C_{3-7}$-Cycloalkyl-$C_{1-4}$-Alkyl-,

X eine unverzweigte $C_{1-4}$-Alkylen-Brücke und
für den Fall, dass die Gruppe Y über ein C-Atom mit X verbunden ist, auch -$CH_2$-CH=CH-, -$CH_2$-C≡C-, $C_{2-4}$-Alkylenoxy oder $C_{2-4}$-Alkylen-$NR^4$-bedeutet,
wobei die Brücke X mit $R^1$ unter Einschluss des mit $R^1$ und X verbundenen N-Atoms unter Ausbildung einer heterocyclischen Gruppe verbunden sein kann, wobei die Brücke X zusätzlich auch mit $R^2$ unter Einschluss des mit $R^2$ und X verbundenen N-Atoms unter Ausbildung einer heterocyclischen Gruppe verbunden sein kann, und
wobei zwei C-Atome oder ein C- und ein N-Atom der Alkylenbrücke durch eine zusätzliche $C_{1-4}$-Alkylen-Brücke miteinander verbunden sein können, und
wobei ein C-Atom mit $R^{10}$ und/oder ein oder zwei C-Atome jeweils mit einem oder zwei gleichen oder verschiedenen Substituenten ausgewählt aus $C_{1-6}$-Alkyl-, $C_{2-6}$-Alkenyl-, $C_{2-6}$-Alkinyl-, $C_{3-7}$-Cycloalkyl, $C_{3-7}$-Cycloalkyl-$C_{1-3}$-alkyl-, $C_{4-7}$-Cycloalkenyl und $C_{4-7}$-Cycloalkenyl-$C_{1-3}$-alkyl-substituiert sein können; wobei zwei Alkyl- und/oder Alkenyl-Substituenten unter Ausbildung eines carbocyclischen Ringsystems miteinander verbunden sein können mit der Maßgabe, dass die Gruppe X in der Bedeutung $C_{2-4}$-Alkylenoxy keinen Hydroxy-Substituenten aufweist; und

W ausgewählt ist aus der Gruppe bestehend aus -$CR^{6a}R^{6b}$-O-, -$CR^{7a}$=$CR^{7c}$-, -$CR^{6a}R^{6b}$-$NR^8$-, -$CR^{7a}R^{7b}$-$CR^{7c}R^{7d}$- und -$NR^8$-$CR^{6a}R^{6b}$-,

Z eine Einfachbindung, $C_{1-4}$-Alkylen, worin zwei benachbarte C-Atome mit einer zusätzlichen $C_{1-4}$-Alkylen-Brücke miteinander verbunden sein können,
wobei ein C-Atom der Alkylen-Brücke mit $R^{10}$ und/oder ein oder zwei C-Atome unabhängig voneinander mit einem oder zwei gleichen oder verschiedenen $C_{1-6}$-Alkyl-Resten substituiert sein können, wobei zwei Alkylreste unter Ausbildung eines carbocyclischen Rings miteinander verbunden sein können, und

Y ausgewählt ist aus der Gruppe der bivalenten cyclischen Gruppen

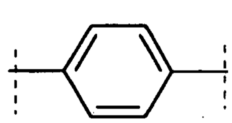 , 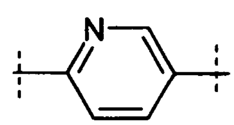 ,

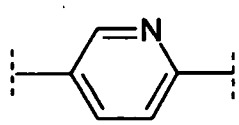 , 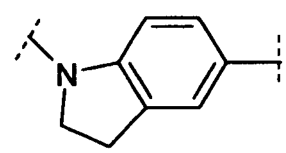 ,

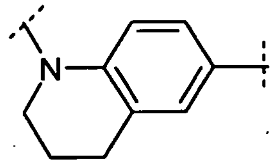 , 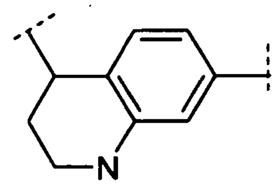 ,

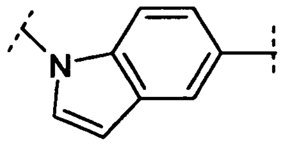 , 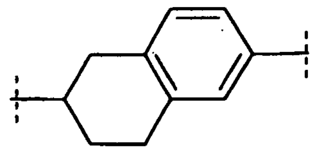 ,

wobei die zuvor genannten cyclischen Gruppen ein- oder mehrfach an ein oder mehreren C-Atomen mit $R^{20}$, im Falle einer Phenylgruppe auch zusätzlich einfach mit Nitro, und/oder eine oder mehrere NH-Gruppen mit $R^{21}$ substituiert sein können, und

A eine der für Cy angegebenen Bedeutungen,

B eine der für Cy angegebenen Bedeutungen,

b den Wert 0 oder 1,

Cy eine carbo- oder heterocyclische Gruppe ausgewählt aus einer der folgenden Bedeutungen

-eine gesättigte 3- bis 7-gliedrige carbocyclische Gruppe,
-eine ungesättigte 4- bis 7-gliedrige carbocyclische Gruppe,
-eine Phenyl-Gruppe,
-eine gesättigte 4- bis 7-gliedrige oder ungesättigte 5- bis 7-gliedrige heterocyclische Gruppe mit einem N-, O- oder S-Atom als Heteroatom,
-eine gesättigte oder ungesättigte 5- bis 7-gliedrige heterocyclische Gruppe mit zwei oder mehreren N-Atomen oder mit einem oder zwei N-Atomen und einem O- oder S-Atom als Heteroatome,
-eine aromatische heterocyclische 5- oder 6-gliedrige Gruppe mit einem oder mehreren gleichen oder verschiedenen Heteroatomen ausgewählt aus N, O und/oder S,
wobei die zuvor angeführten 4-, 5-, 6- oder 7-gliedrigen Gruppen über zwei gemeinsame, benachbarte C-Atome mit einen Phenyl- oder Pyridin-Ring kondensiert verbunden sein können, und
wobei in den zuvor genannten 5-, 6- oder 7-gliedrigen Gruppen eine oder zwei nicht benachbarte $-CH_2$-Gruppen unabhängig voneinander durch eine -CO-, $-C(=CH_2)-$, -(SO)- oder $-(SO_2)$-Gruppe ersetzt sein können, und
wobei die zuvor angeführten gesättigten 6- oder 7-gliedrigen Gruppen auch als verbrückte Ringsysteme mit einer Imino-, N-($C_{1-4}$-alkyl)-imino-, Methylen-, $C_{1-4}$-Alkyl-methylen- oder Di-($C_{1-4}$-alkyl)-methylen-Brücke vorliegen können, und
wobei die zuvor genannten cyclischen Gruppen ein- oder mehrfach an ein oder mehreren C-Atomen mit $R^{20}$, im Falle einer Phenylgruppe auch zusätzlich einfach mit Nitro, und/oder ein oder mehrere NH-Gruppen mit $R^{21}$ substituiert sein können,

$R^4$ H, $C_{1-6}$-Alkyl oder $C_{2-6}$-Alkenyl,

$R^{6a}$, $R^{6b}$ H, $C_{1-4}$-Alkyl oder $CF_3$,

$R^{7a}$, $R^{7b}$, $R^{7c}$, $R^{7d}$ H, F, $C_{1-4}$-Alkyl oder $CF_3$,

$R^8$ H, $C_{1-4}$-Alkyl, $C_{3-7}$-Cycloalkyl oder $C_{3-7}$-Cycloalkyl-$C_{1-3}$-alkyl-,

$R^{10}$ Hydroxy, ω-Hydroxy-$C_{1-3}$-alkyl, $C_{1-4}$-Alkoxy, ω-($C_{1-4}$-Alkoxy)-$C_{1-3}$-alkyl-, Carboxy, $C_{1-4}$-Alkoxycarbonyl-, Amino, $C_{1-4}$-Alkyl-amino-, Di-($C_{1-4}$-alkyl)-amino, Cyclo-$C_{3-6}$-alkylenimino-, Amino-$C_{1-3}$-alkyl-, $C_{1-4}$-Alkyl-amino-$C_{1-3}$-alkyl-, Di-($C_{1-4}$-alkyl)-amino-$C_{1-3}$-alkyl-, Cyclo-$C_{3-6}$-alkylenimino-$C_{1-3}$-alkyl-, Amino-$C_{1-3}$-alkoxy-, $C_{1-4}$-Alkyl-amino-$C_{1-3}$-alkoxy-, Di-($C_{1-4}$-alkyl)-amino-$C_{1-3}$-alkoxy-oderCyclo-$C_{3-6}$-alkylenimino-$C_{1-3}$-alkoxy-,Aminocarbonyl-,$C_{1-4}$-Alkyl-aminocarbonyl-,Di-($C_{1-4}$-alkyl)-aminocarbonyl- oder Cyclo-$C_{3-6}$-alkylenimino-carbonyl-,

$R^{11}$ $C_{2-6}$-Alkenyl, $C_{2-6}$-Alkinyl, $R^{15}$-O-, $R^{15}$-O-CO-, $R^{15}$-CO-O-, $R^{16}R^{17}$N-, $R^{18}R^{19}$N-CO- oder Cy-,

$R^{12}$ eine der für $R^{20}$ angegebenen Bedeutungen,

$R^{13}$ eine der für $R^{17}$ angegebenen Bedeutungen, ausgenommen Carboxy,

$R^{14}$ Halogen, $C_{1-6}$-Alkyl, $C_{2-6}$-Alkenyl, $C_{2-6}$-Alkinyl, $R^{15}$-O-, $R^{15}$-O-CO-, $R^{15}$-CO-, $R^{15}$-CO-O-, $R^{16}R^{17}N$-, $R^{18}R^{19}N$-CO-, $R^{15}$-O-$C_{1-3}$-alkyl-, $R^{15}$-O-CO-$C_{1-3}$-alkyl-, $R^{15}$-O-CO-NH-, $R^{15}$-$SO_2$-NH-, $R^{15}$-CO-$C_{1-3}$-alkyl-, $R^{15}$-CO-O-$C_{1-3}$-alkyl-, $R^{16}R^{17}N$-$C_{1-3}$-alkyl-, $R^{18}R^{19}N$-CO-$C_{1-3}$-alkyl- oder Cy-$C_{1-3}$-alkyl-,

$R^{15}$ H, $C_{1-4}$-Alkyl, $C_{3-7}$-Cycloalkyl, $C_{3-7}$-Cycloalkyl-$C_{1-3}$-alkyl-, Phenyl, Phenyl-$C_{1-3}$-alkyl-, Pyridinyl oder Pyridinyl-$C_{1-3}$-alkyl-,

$R^{16}$ H, $C_{1-6}$-Alkyl, $C_{3-7}$-Cycloalkyl, $C_{3-7}$-Cycloalkyl-$C_{1-3}$-alkyl-, $C_{4-7}$-Cycloalkenyl, $C_{4-7}$-Cycloalkenyl-$C_{1-3}$-alkyl-, ω-Hydroxy-$C_{2-3}$-alkyl, ω-($C_{1-4}$-Alkoxy)-$C_{2-3}$-alkyl, Amino-$C_{2-6}$-alkyl-, $C_{1-4}$-Alkyl-Amino-$C_{2-6}$-alkyl-, Di-($C_{1-4}$-$_4$-alkyl)-Amino-$C_{2-6}$-alkyl- oder Cyclo-$C_{3-6}$-alkylenimino-$_{2-6}$-alkyl-,

$R^{17}$ eine der für $R^{16}$ angegebenen Bedeutungen oder Phenyl, Phenyl-$C_{1-3}$-alkyl, Pyridinyl, Dioxolan-2-yl, -CHO, $C_{1-4}$-Alkylcarbonyl, Carboxy, Hydroxycarbonyl-$C_{1-3}$-alkyl-, $C_{1-4}$-Alkoxycarbonyl-, $C_{1-4}$-Alkoxycarbonyl-$C_{1-3}$-alkyl-, $C_{1-4}$-Alkylcarbonylamino-$C_{2-3}$-alkyl-, N-($C_{1-4}$-Alkylcarbonyl)-N-($C_{1-4}$-Alkyl)-amino-$C_{2-3}$-alkyl-, $C_{1-4}$-Alkylsulfonyl, $C_{1-4}$-Alkylsulfonylamino-$C_{2-3}$-alkyl- oder N-($C_{1-4}$-Alkylsulfonyl)-N($C_{1-4}$-Alkyl)-amino-$C_{2-3}$-alkyl-

$R^{18}$, $R^{19}$ unabhängig voneinander H oder $C_{1-6}$-Alkyl,

$R^{20}$ Halogen, Hydroxy, Cyano, $C_{1-6}$-Alkyl, $C_{2-6}$-Alkenyl, $C_{2-6}$-Alkinyl, $C_{3-7}$-Cycloalkyl, $C_{3-7}$-Cycloalkyl-$C_{1-3}$-alkyl-, Hydroxy-$C_{1-4}$-alkyl, $R^{22}$-$C_{1-3}$-alkyl- oder eine der für $R^{22}$ angegebenen Bedeutungen,

$R^{21}$ $C_{1-4}$-Alkyl, ω-Hydroxy-$C_{2-3}$-alkyl, ω-$C_{1-4}$-Alkoxy-$C_{2-6}$-alkyl, ω-$C_{1-4}$-Alkyl-amino-$C_{2-6}$-alkyl-, ω-Di-($C_{1-4}$-alkyl)-amino-$C_{2-6}$-alkyl-, ω-Cyclo-$C_{3-6}$-alkylenimino-$C_{2-6}$-alkyl-, Phenyl-$C_{1-3}$-alkyl-, $C_{1-4}$-Alkyl-carbonyl, $C_{1-4}$-Alkoxy-carbonyl oder $C_{1-4}$-Alkylsulfonyl,

$R^{22}$ Phenyl-$C_{1-3}$-alkoxy, OHC-, HO-N=HC-, $C_{1-4}$-Alkoxy-N=HC-, $C_{1-4}$-Alkoxy, $C_{1-4}$-Alkylthio, Carboxy, $C_{1-4}$-Alkylcarbonyl, $C_{1-4}$-Alkoxycarbonyl, Aminocarbonyl, $C_{1-4}$-Alkylaminocarbonyl, Di-($C_{1-4}$-alkyl)-aminocarbonyl, Cyclo-$C_{3-6}$-alkyl-amino-carbonyl-, Cyclo-$C_{3-6}$-alkylenimino-carbonyl, Cyclo-$C_{3-6}$-alkylenimino-$C_{2-4}$-alkyl-aminocarbonyl, Phenyl-amino-carbonyl, $C_{1-4}$-Alkyl-sulfonyl, $C_{1-4}$-Alkyl-sulfinyl, $C_{1-4}$-Alkyl-sulfonylamino, Amino, $C_{1-4}$-alkylamino-, Di-($C_{1-4}$-alkyl)-amino-, $C_{1-4}$-Alkyl-carbonyl-amino-, Cyclo-$C_{3-6}$-alkylenimino-, Phenyl-$C_{1-3}$-alkylamino- oder N-($C_{1-4}$-Alkyl)-phenyl-$C_{1-3}$-alkylamino-, Acetylamino-, Propionylamino-, Phenylcarbonylamino-, Phenylcarbonylmethylamino-, Hydroxyalkylaminocarbonyl, (4-Morpholinyl)carbonyl, (1-Pyrrolidinyl)carbonyl, (1-Piperidinyl)carbonyl, (Hexahydro-1-azepinyl)carbonyl, (4-Methyl-1-piperazinyl)carbonyl, Methylendioxy, Aminocarbonylamino oder Alkylaminocarbonylamino-,

wobei in den zuvor genannten Gruppen und Resten, insbesondere in insbesondere A, B, W, X, Y, Z, $R^1$ bis $R^4$, $R^{6a}$, $R^{6b}$, $R^{7a}$, $R^{7b}$, $R^{7c}$, $R^{7d}$, $R^8$, $R^{10}$ bis $R^{22}$, jeweils ein oder mehrere C-Atome zusätzlich ein- oder mehrfach mit F und/oder jeweils ein oder zwei C-Atome unabhängig voneinander zusätzlich einfach mit Cl oder Br und/oder jeweils ein oder mehrere Phenyl-Ringe unabhängig voneinander zusätzlich ein, zwei oder drei Substituenten ausgewählt aus der Gruppe F, Cl, Br, I, $C_{1-4}$-Alkyl, $C_{1-4}$-Alkoxy-, Difluormethyl-, Trifluormethyl-, Hydroxy-, Amino-, $C_{1-3}$-alkylamino-, Di-($C_{1-3}$-alkyl)-amino-, Acetylamino-, Aminocarbonyl-, Cyano, Difluormethoxy-, Trifluormethoxy-, Amino-$C_{1-3}$-alkyl-, $C_{1-3}$-Alkylamino-$C_{1-3}$-alkyl- und Di-($C_{1-3}$-Alkyl)-amino-$C_{1-3}$-alkyl- aufweisen und/oder einfach mit Nitro substituiert sein können, und
das H-Atom einer vorhandenen Carboxygruppe oder ein an ein N-Atom gebundenes H-Atom jeweils durch einen in-vivo abspaltbaren Rest ersetzt sein kann, bedeuten,
deren Tautomere, deren Diastereomere, deren Enantiomere, deren Gemische und deren Salze, mit folgenden Maßgaben (M2) und (M3)
(M2) für den Fall, wenn W -CH=CH- bedeutet und Y eine Phenylen-Gruppe und Z eine Einfachbindung ist, dann die Brücken X und Z an dem Phenylen-Ring der Gruppe Y in para-Stellung zueinander stehen und mindestens eine der folgenden Bedingungen erfüllt ist:

(a) die Gruppe Y in der Bedeutung Phenylen mindestens einfach substituiert ist,

(b) b den Wert 0 besitzt und der Rest A mindestens zweifach substituiert ist,
(c) b den Wert 1 besitzt;

(M3) dass folgende Einzelverbindungen nicht umfasst sind:

N-[4-(2-Diethylamino-ethoxy)-phenyl]-3-phenyl-propionamid,
N-[4-(2-Morpholin-4-ylethoxy)-phenyl]-3-phenyl-propionamid,
3-(4-Chlor-phenyl)-N-{2-[4-(2-diethylamino-ethoxy)-phenyl]-ethyl}-acrylamid,
N-{2-[3-(4-{2-[2-(4-Chlor-phenoxy)-acetylamino]-ethyl}-phenoxy)-2-hydroxy-propylamino]-ethyl}-isobutyramid,
Cyclopentancarbonsäure {2-[3-(4-{2-[2-(4-chlor-phenoxy)-acetylamino]-ethyl}-phenoxy)-2-hydroxy-propylamino]-ethyl}-amid,
2-(4-Chlor-phenoxy)-N-(2-{4-[2-hydroxy-3-(2-phenylacetylamino-ethylamino)-propoxy]-phenyl}-ethyl)-acetamid.

Gegenstand der Erfindung sind auch die jeweiligen Verbindungen in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren. Ebenfalls mit vom Gegenstand dieser Erfindung umfasst sind die erfindungsgemäßen Verbindungen, einschließlich deren Salze, in denen ein oder mehrere Wasserstoffatome durch Deuterium ausgetauscht sind.

Ferner sind die physiologisch verträglichen Salze der vorstehend und nachfolgend beschriebenen erfindungsgemäßen Amid-Verbindungen ebenfalls ein Gegenstand dieser Erfindung.

Ebenfalls eine Gegenstand dieser Erfindung sind Zusammensetzungen, enthaltend mindestens eine erfindungsgemäße Amid-Verbindung und/ oder ein erfindungsgemäßes Salz neben gegebenenfalls einem oder mehreren physiologisch verträglichen Hilfsstoffen.

Weiterhin sind Arzneimittel, enthaltend mindestens eine erfindungsgemäße Amid-Verbindung und/ oder ein erfindungsgemäßes Salz neben gegebenenfalls einem oder mehreren inerten Trägerstoffen und/oder Verdünnungsmitteln Gegenstand der vorliegenden Erfindung.

Ebenfalls ein Gegenstand dieser Erfindung ist die nicht-therapeutische Verwendung mindestens einer erfindungsgemäßen Amid-Verbindung und/ oder eines erfindungsgemäßen Salzes, einschließlich der durch die Maßgaben (M2) und (M3) ausgeschlossenen Verbindungen, zur Beeinflussung des Essverhaltens eines Säugetiers durch Reduzierung des Hungers, Zügeln des Appetits, Kontrollieren des Essverhaltens und/oder Hervorrufen eines Sättigungsgefühls.

Weiterhin ist die nicht-therapeutische Verwendung mindestens einer erfindungsgemäßen Amid-Verbindung und/ oder eines erfindungsgemäßen Salzes, einschließlich der durch die Maßgaben (M2) und (M3) ausgeschlossenen Verbindungen, zur Reduzierung des Körpergewichts und/ oder zum Verhindern einer Zunahme des Körpergewichts eines Säugetiers ein Gegenstand dieser Erfindung.

Ebenfalls ein Gegenstand der vorliegenden Erfindung ist die Verwendung mindestens einer erfindungsgemäßen Amid-Verbindung und/ oder eines erfindungsgemäßen Salzes, einschließlich der durch die Maßgaben (M2) und (M3) ausgeschlossenen Verbindungen, zur Herstellung eines Arzneimittels mit MCH-Rezeptor antagonistischer Aktivität, insbesondere mit MCH-1 Rezeptor antagonistischer Aktivität.

Darüber hinaus ist ein Gegenstand dieser Erfindung die Verwendung mindestens einer efindungsgemäßen Amid-Verbindung und/ oder eines erfindungsgemäßen Salzes, einschließlich der durch die Maßgaben (M2) und (M3) ausgeschlossenen Verbindungen, zur Herstellung eines Arzneimittels, welches zur Prophylaxe und/oder Behandlung von Erscheinungen und/oder Krankheiten, die durch MCH verursacht werden oder mit MCH in einem anderen kausalen Zusammenhang stehen, geeignet ist.

Ein weiterer Gegenstand dieser Erfindung ist die Verwendung mindestens einer erfindungsgemäßen Amid-Verbindung und/ oder eines erfindungsgemäßen Salzes, einschließlich der durch die Maßgaben (M2) und (M3) ausgeschlossenen Verbindungen, zur Herstellung eines Arzneimittels, welches zur Prophylaxe und/oder Behandlung von metabolischen Störungen und/oder Essstörungen, insbesondere von Obesitas, Bulimie, Bulimie nervosa, Cachexia, Anorexie, Anorexie nervosa und Hyperphagia, geeignet ist.

Ebenfalls ein Gegenstand dieser Erfindung liegt in der Verwendung mindestens einer erfindungsgemäßen Amid-Verbindung und/ oder eines erfindungsgemäßen Salzes, einschließlich der durch die Maßgaben (M2) und (M3) ausgeschlossenen Verbindungen, zur Herstellung eines Arzneimittels, welches zur Prophylaxe und/oder Behandlung von mit Obesitas einhergehenden Krankheiten und/oder Störungen, insbesondere von Diabetes, besonders Typ II Diabetes, diabetischen Komplikationen, einschließlich diabetischer Retinopathie, diabetischer Neuropathie, diabetischer Nephropathie, Insulin-Resistenz, pathologischer Glukosetoleranz, Herzinsuffizienz, Herzkreislauferkrankungen, insbesondere Arteriosklerose und Bluthochdruck, Arthritis und Gonitis geeignet ist.

Darüber hinaus hat die vorliegende Erfindung die Verwendung mindestens einer erfindungsgemäßen Amid-Verbindung und/ oder eines erfindungsgemäßen Salzes, einschließlich der durch die Maßgaben (M2) und (M3) ausgeschlossenen Verbindungen, zur Herstellung eines Arzneimittels, welches zur Prophylaxe und/oder Behandlung von Hyperlipidämie, Fettakkumulation, emotionalen Störungen, Affektivitätsstörungen, Depressionen und Angstzuständen geeignet ist, zum Gegenstand.

Ein weiterer Gegenstand dieser Erfindung ist ein Arzneimittel, enthaltend einen ersten Wirkstoff, der aus den erfindungsgemäßen Amid-Verbindungen und/ oder den entsprechenden Salzen, einschließlich der durch die Maßgaben (M2) und (M3) ausgeschlossenen Verbindungen, ausgewählt ist, sowie einen zweiten Wirkstoff, der aus der Gruppe ausgewählt ist bestehend aus Wirkstoffen zur Behandlung von Diabetes, Wirkstoffen zur Behandlung diabetischer Komplikationen, Wirkstoffen zur Behandlung von Obesitas, vorzugsweise anderen als MCH-Antagonisten, Wirkstoffen zur Behandlung von Bluthochdruck, Wirkstoffen zur Behandlung von Hyperlipidemia, einschließlich Arteriosklerose, Wirkstoffen zur Behandlung,von Arthritis, Wirkstoffen zur Behandlung von Angstzuständen und Wirkstoffen zur Behandlung von Depressionen, neben gegebenenfalls einem oder mehreren inerten Trägerstoffen und/oder Verdünnungsmitteln.

## Detailierte Beschreibung der Erfindung

Sofern nicht anders angegeben besitzen die Gruppen, Reste und Substituenten, insbesondere A, B, W, X, Y, Z, $R^1$ bis $R^4$, $R^{6a}$, $R^{6b}$, $R^{7a}$, $R^{7b}$, $R^{7c}$, $R^{7d}$, $R^8$, $R^{10}$ bis $R^{22}$, sowie der Index b die zuvor angegebenen Bedeutungen.

Gemäß einer Ausführungsform besitzen die Gruppen $R^1$, $R^2$, X, Z, b, $R^{10}$, $R^{14}$, $R^{15}$, $R^{17}$, $R^{20}$, $R^{21}$, $R^{22}$ folgende Bedeutungen:

$R^1$, $R^2$ unabhängig voneinander H, eine gegebenenfalls mit dem Rest $R^{11}$ substituierte $C_{1-8}$-Alkyl- oder $C_{3-7}$-Cycloalkyl-Gruppe, oder ein gegebenenfalls mit dem Rest $R^{12}$ ein- oder mehrfach und/oder mit Nitro einfach substituierter Phenylrest, mit der Maßgabe, dass mindestens einer der Reste $R^1$, $R^2$ eine von H verschiedene Bedeutung aufweist, oder
$R^1$ und $R^2$ bilden eine $C_{2-8}$-Alkylen-Brücke, in der

- ein oder zwei $-CH_2$-Gruppen unabhängig voneinander durch -CH=N- oder -CH=CH- ersetzt sein können und/ oder .
- ein oder zwei $-CH_2$-Gruppen unabhängig voneinander durch -O-, -S-, -CO-, $-C(=CH_2)$- oder $-NR^{13}$- derart ersetzt sein können, dass Heteroatome nicht unmittelbar miteinander verbunden sind,

wobei in der zuvor definierten Alkylen-Brücke ein oder mehrere H-Atome durch $R^{14}$ ersetzt sein können, und wobei die zuvor definierte Alkylen-Brücke mit einer oder zwei gleichen oder verschiedenen carbo- oder heterocyclischen Gruppen Cy derart substituiert sein kann, dass die Bindung zwischen der Alkylenbrücke und der Gruppe Cy

- über eine Einfach- oder Doppelbindung,
- über ein gemeinsames C-Atom unter Ausbildung eines spirocyclischen Ringsystems,
- über zwei gemeinsame, benachbarte C- und/oder N-Atome unter Ausbildung eines kondensierten bicyclischen Ringsystems oder
- über drei oder mehrere C- und/oder N-Atome unter Ausbildung eines verbrückten Ringsystems erfolgt,

X eine unverzweigte $C_{1-4}$-Alkylen-Brücke und
für den Fall, dass die Gruppe Y über ein C-Atom mit X verbunden ist, auch $-CH_2-CH=CH-$, $-CH_2-C\equiv C-$, $C_{2-4}$-Alkylenoxy oder $C_{2-4}$-Alkylen-$NR^4$-bedeutet,
wobei die Brücke X mit $R^1$ unter Einschluss des mit $R^1$ und X verbundenen N-Atoms unter Ausbildung einer heterocyclischen Gruppe verbunden sein kann, und
wobei zwei C-Atome oder ein C- und ein N-Atom der Alkylenbrücke durch eine zusätzliche $C_{1-4}$-Alkylen-Brücke miteinander verbunden sein können, und
wobei ein C-Atom mit $R^{10}$ und/oder ein oder zwei C-Atome jeweils mit einem oder zwei gleichen oder verschiedenen $C_{1-6}$-Alkyl-Resten substituiert sein können, mit der Maßgabe, dass die Gruppe X in der Bedeutung $C_{2-4}$-Alkylenoxy keinen Hydroxy-Substituenten aufweist und

Z eine Einfachbindung, $C_{1-4}$-Alkylen, worin zwei benachbarte C-Atome mit einer zusätzlichen $C_{1-4}$-Alkylen-Brücke miteinander verbunden sein können,
wobei ein C-Atom der Alkylen-Brücke mit $R^{10}$ und/oder ein oder zwei C-Atome unabhängig voneinander mit einem oder zwei gleichen oder verschiedenen $C_{1-6}$-Alkyl-Resten substituiert sein können, und

b den Wert 0 besitzt,

$R^{10}$ Hydroxy, ω-Hydroxy-$C_{1-3}$-alkyl, $C_{1-4}$-Alkoxy, ω-($C_{1-4}$-Alkoxy)-$C_{1-3}$-alkyl-, Amino, $C_{1-4}$-Alkyl-amino-, Di-($C_{1-4}$-alkyl)-amino-, Cyclo-$C_{3-6}$-alkylenimino-, Amino-$C_{1-3}$-alkyl-, $C_{1-4}$-Alkyl-amino-$C_{1-3}$-alkyl-, Di-($C_{1-4}$-alkyl)-amino-$C_{1-3}$-alkyl-,Cyclo-$C_{3-6}$-alkylenimino-$C_{1-3}$-alkyl-,Amino-$C_{1-3}$-alkoxy-,$C_{1-4}$-Alkyl-amino-$C_{1-3}$-alkoxy-,Di-($C_{1-4}$-alkyl)-amino-$C_{1-3}$-alkoxy- oder Cyclo-$C_{3-6}$-alkylenimino-$C_{1-3}$-alkoxy-,

$R^{14}$ Halogen, $C_{1-6}$-Alkyl, $R^{15}$-O-, $R^{15}$-O-CO-, $R^{15}$-CO-, $R^{15}$-CO-O-, $R^{16}R^{17}$N-, $R^{18}R^{19}$N-CO-, $R^{15}$-O-$C_{1-3}$-alkyl- , $R^{15}$-O-CO-$C_{1-3}$-alkyl-, $R^{15}$-CO-$C_{1-3}$-alkyl-, $R^{15}$-CO-O-$C_{1-3}$-alkyl-, $R^{16}R^{17}$N-$C_{1-3}$-alkyl-, $R^{18}R^{19}$N-CO-$C_{1-3}$-alkyl- oder Cy-$C_{1-3}$-alkyl-,

$R^{15}$ H, $C_{1-4}$-Alkyl, $C_{3-7}$-Cycloalkyl, $C_{3-7}$-Cycloalkyl-$C_{1-3}$-alkyl-, Phenyl oder Phenyl-$C_{1-3}$-alkyl-,

$R^{17}$ eine der für $R^{16}$ angegebenen Bedeutungen oder Phenyl, Phenyl-$C_{1-3}$-alkyl-, $C_{1-4}$-Alkylcarbonyl, Hydroxycarbonyl-$C_{1-3}$-alkyl-, $C_{1-4}$-Alkylcarbonylamino-$C_{2-3}$-alkyl-, N-($C_{1-4}$-Alkylcarbonyl)-N-($C_{1-4}$-Alkyl)-amino-$C_{2-3}$-alkyl-, $C_{1-4}$-Alkylsulfonyl, $C_{1-4}$-Alkylsulfonylamino-$C_{2-3}$-alkyl- oder N-($C_{1-4}$-Alkylsulfonyl)-N($C_{1-4}$-Alkyl)-amino-$C_{2-3}$-alkyl-

$R^{20}$ Halogen, Hydroxy, Cyano, $C_{1-6}$-Alkyl, $C_{3-7}$-Cycloalkyl, $C_{3-7}$-Cycloalkyl- $C_{1-3}$-alkyl-, Hydroxy-$C_{1-4}$-alkyl-, $R^{22}$-$C_{1-3}$-alkyl- oder eine der für $R^{22}$ angegebenen Bedeutungen,

$R^{21}$ $C_{1-4}$-Alkyl, ω-Hydroxy-$C_{2-3}$-alkyl, ω-$C_{1-4}$-Alkoxy-$C_{2-6}$-alk-yl, ω-$C_{1-4}$-Alkyl-amino-$C_{2-6}$-alkyl-, ω-Di-($C_{1-4}$-alkyl)-amino-$C_{2-6}$-alkyl-, ω-Cyclo-$C_{3-6}$-alkylenimino-$C_{2-6}$-alkyl-, Phenyl, Phenyl-$C_{1-3}$-älkyl-, $C_{1-4}$-Alkyl-carbonyl, Carboxy, $C_{1-4}$-Alkoxy-carbonyl oder $C_{1-4}$-Alkylsulfonyl,

$R^{22}$ Phenyl, Phenyl-$C_{1-3}$-alkoxy, $C_{1-4}$-Alkoxy, $C_{1-4}$-Alkylthio, Carboxy, $C_{1-4}$-Alkylcarbonyl, $C_{1-4}$-Alkoxycarbonyl, Aminocarbonyl, $C_{1-4}$-Alkylaminocarbonyl, Di-($C_{1-4}$-alkyl)-aminocarbonyl, Cyclo-$C_{3-6}$-alkylenimino-carbonyl, $C_{1-4}$-Alkyl-sulfonyl, $C_{1-4}$-Alkyl-sulfinyl, $C_{1-4}$-Alkyl-sulfonylamino-, Amino-, $C_{1-4}$-alkylamino-, Di-($C_{1-4}$-alkyl)-amino-, Cyclo-$C_{3-6}$-alkylenimino-, Phenyl-$C_{1-3}$-alkylamino- oder N-($C_{1-4}$-Alkyl)-phenyl-$C_{1-3}$-alkylamino-, Acetylamino-, Propionylamino-, Phenylcarbonylamino-, Phenylcarbonylmethylamino-, Hydroxyalkylaminocarbonyl, (4-Morpholinyl)carbonyl, (1-Pyrrolidinyl)carbonyl, (1-Piperidinyl)carbonyl, (Hexahydro-1-azepinyl)carbonyl, (4-Methyl-1-piperazinyl)carbonyl, Methylendioxy, Aminocarbonylamino- oder Alkylaminocarbonylamino-

wobei die Gruppen und Reste $R^3$, $R^4$, $R^{6a}$, $R^{6b}$, $R^{7a}$, $R^{7b}$, $R^{7c}$, $R^{7d}$, $R^8$, $R^{11}$, $R^{12}$, $R^{16}$, $R^{18}$, $R^{19}$, W, Y, A, Cy die zuvor angegebene Bedeutung besitzen.

Vorzugsweise besitzt der Rest $R^3$ die Bedeutung H oder $C_{1-4}$-Alkyl, besonders bevorzugt H oder Methyl, insbesondere H.

Sind $R^1$ und $R^2$ nicht über eine Alkylenbrücke miteinander verbunden, so bedeuten $R^1$ und $R^2$ unabhängig voneinander vorzugsweise eine gegebenenfalls mit dem Rest $R^{11}$ substituierte $C_{1-8}$-Alkyl- oder $C_{3-7}$-Cycloalkyl-Gruppe, wobei eine -$CH_2$-Gruppe in Position 3 oder 4 einer 5, 6 oder 7-gliedrigen Cycloalkylgruppe durch -O-, -S- oder -NH-, -N($C_{1-4}$-Alkyl)- oder -N(CO-O-$C_{1-4}$-Alkyl)- ersetzt sein kann, oder ein gegebenenfalls mit dem Rest $R^{12}$ ein- oder mehrfach und/oder mit Nitro einfach substituierter Phenyl- oder Pyridinylrest, und wobei einer der Reste $R^1$ und $R^2$ auch H bedeuten kann.

Bevorzugt bedeuten die Reste $R^1$, $R^2$ unabhängig voneinander H, $C_{1-6}$-Alkyl, $C_{3-7}$-Cycloalkyl, $C_{3-7}$-Cycloalkyl-$C_{1-3}$-alkyl-, ω-Hydroxy-$C_{2-3}$-alkyl-, ω-($C_{1-4}$-Alkoxy)-$C_{2-3}$-alkyl-, $C_{1-4}$-Alkoxy-carbonyl-$C_{1-4}$-alkyl-, Carboxyl-$C_{1-4}$-alkyl-, Amino-$C_{2-4}$-alkyl-, $C_{1-4}$-Alkyl-amino-$C_{2-4}$-alkyl-, Di-($C_{1-4}$-alkyl)-amino-$C_{2-4}$-alkyl-, Cyclo-$C_{3-6}$-alkylenimino-$C_{2-4}$-alkyl-, Pyrrolidin-3-yl, N-($C_{1-4}$-alkyl)-pyrrolidinyl, Pyrrolidinyl-$C_{1-3}$-alkyl-, N-($C_{1-4}$-alkyl)-pyrrolidinyl-$_{1-3}$-alkyl, Piperidinyl, N-($C_{1-4}$-alkyl)-piperidinyl, Piperidinyl-$C_{1-3}$-alkyl-, N-($C_{1-4}$-alkyl)-piperidinyl-$C_{1-3}$-alkyl-, Phenyl, Phenyl-$C_{1-3}$-alkyl, Pyridyl oder Pyridyl-$C_{1-3}$-alkyl-, wobei in den zuvor angegebenen Gruppen und Resten ein oder mehrere C-Atome ein- oder mehrfach mit F und/oder ein oder zwei C-Atome unabhängig voneinander einfach mit Cl oder Br substituiert sein können, und wobei der Phenyl- oder Pyridylrest ein- oder mehrfach mit dem zuvor definierten Rest $R^{12}$ und/oder einfach mit Nitro substituiert sein kann mit der Maßgabe, dass mindestens einer der Reste $R^1$, $R^2$ eine von H verschiedene Bedeutung aufweist. Bevorzugte Substituenten der zuvor genannten Phenyl- oder Pyridylreste sind ausgewählt aus der Gruppe F, Cl, Br, I, Cyano, $C_{1-4}$-alkyl, $C_{1-4}$-alkoxy-, Difluormethyl-, Trifluormethyl-, Hydroxy-, Amino-, $C_{1-3}$-alkylamino-, Di-($C_{1-3}$-alkyl)-amino-, Acetylamino-, Aminocarbonyl-, Difluormethoxy-, Trifluormethoxy-, Amino-$C_{1-3}$-alkyl-, $C_{1-3}$-alkylamino-$C_{1-3}$-alkyl- und Di-($C_{1-3}$-Alkyl)-amino-$C_{1-3}$-alkyl-, wobei ein Phenylrest auch einfach mit Nitro substituiert sein kann.

Besonders bevorzugte Bedeutungen der Reste $R^1$ und/oder $R^2$ sind ausgewählt aus der Gruppe bestehend aus $C_{1-6}$-Alkyl, $C_{3-7}$-Cycloalkyl, $C_{3-7}$-Cycloalkyl-$C_{1-3}$-alkyl-, ω-Hydroxy-$C_{2-3}$-alkyl, ω-($C_{1-4}$-Alkoxy)-$C_{2-3}$-alkyl, $C_{1-4}$-Alk-

oxy-carbonyl-$C_{1-4}$-alkyl-, Carboxyl-$C_{1-4}$-alkyl, wobei einer der Reste $R^1$ und $R^2$ auch H bedeuten kann.

Besonders bevorzugt weisen beide Reste $R^1$, $R^2$, eine von H verschiedene Bedeutung auf.

Bilden $R^1$ und $R^2$ eine Alkylen-Brücke, so handelt es sich hierbei bevorzugt um eine $C_{3-7}$-Alkylen-Brücke, in der

- eine nicht mit dem N-Atom der $R^1R^2N$-Gruppe benachbarte $-CH_2$-Gruppe durch
- CH=N- oder -CH=CH- ersetzt sein kann und/oder
- eine $-CH_2$-Gruppe, die vorzugsweise nicht mit dem N-Atom der $R^1R^2N$-Gruppe benachbart ist, durch -O-, -S-, -C(=N-O-$R^{18}$)-, -CO-, -C(=$CH_2$)- oder -$NR^{13}$-, besonders bevorzugt durch durch -O-, -S- oder -$NR^{13}$-, derart ersetzt sein kann,

  dass Heteroatome nicht unmittelbar miteinander verbunden sind,

wobei in der zuvor definierten Alkylen-Brücke ein oder mehrere H-Atome durch $R^{14}$ ersetzt sein können, und wobei die zuvor definierte Alkylen-Brücke mit einer carbo- oder heterocyclischen Gruppe Cy derart substituiert sein kann, dass die Bindung zwischen der Alkylenbrücke und der Gruppe Cy

- über eine Einfach- oder Doppelbindung,
- über ein gemeinsames C-Atom unter Ausbildung eines spirocyclischen Ringsystems,
- über zwei gemeinsame, benachbarte C- und/oder N-Atome unter Ausbildung eines kondensierten bicyclischen Ringsystems oder
- über drei oder mehrere C- und/oder N-Atome unter Ausbildung eines verbrückten Ringsystems erfolgt.

Weiterhin bevorzugt bilden $R^1$ und $R^2$ derart eine Alkylen-Brücke, dass $R^1R^2N$- eine Gruppe ausgewählt aus Azetidin, Pyrrolidin, Piperidin, Azepan, 2,5-Dihydro-1H-pyrrol, 1,2,3,6-Tetrahydro-pyridin, 2,3,4,7-Tetrahydro-1H-azepin, 2,3,6,7-Tetrahydro-1H-azepin, Piperazin, worin die freie Imin-Funktion mit $R^{13}$ substituiert ist, Piperidin-4-on, Piperidin-4-on-oxim, Piperidin-4-on-O-$C_{1-4}$-alkyl-oxim, Morpholin und Thiomorpholin bedeutet, wobei gemäß der allgemeinen Definition von $R^1$ und $R^2$ ein- oder mehrere H-Atome durch $R^{14}$ ersetzt sein können, und/ oder die zuvor genannten Gruppen in einer gemäß der allgemeinen Definition von $R^1$ und $R^2$ angegebenen Weise mit einer oder zwei gleichen oder verschiedenen carbo- oder heterocyclischen Gruppen Cy substituiert sein kann. Hierbei besonders bevorzugte Gruppen Cy sind $C_{3-7}$-Cycloalkyl, Aza-$C_{4-7}$-cycloalkyl-, insbesondere Cyclo-$C_{3-6}$-alkylenimino-, sowie 1-$C_{1-4}$-Alkyl-aza-$C_{4-7}$-cycloalkyl-.

Die von $R^1$ und $R^2$ gebildete $C_{2-8}$-Alkylen-Brücke, in der wie angegeben $-CH_2$-Gruppen ersetzt sein können, kann, wie beschrieben, mit einer oder zwei gleichen oder verschiedenen carbo- oder heterocyclischen Gruppen Cy substituiert sein.

Für den Fall, dass die Alkylenbrücke mit einer Gruppe Cy über ein Einfachbindung verbunden ist, ist Cy bevorzugt ausgewählt aus der Gruppe bestehend aus $C_{3-7}$-Cycloalkyl, Cyclo-$C_{3-6}$-alkylenimino-, 1H-Imidazol, Thienyl und Phenyl.

Für den Fall, dass die Alkylenbrücke mit einer Gruppe Cy über ein gemeinsames C-Atom unter Ausbildung eines spirocyclischen Ringsystems verbunden ist, ist Cy bevorzugt ausgewählt aus der Gruppe bestehend aus $C_{3-7}$-Cycloalkyl, Aza-$C_{4-8}$-cycloalky-, Oxa-$C_{4-8}$-cycloalkyl-, 2,3-Dihydro-1H-chinazolin-4-on.

Für den Fall, dass die Alkylenbrücke mit einer Gruppe Cy über zwei gemeinsame, benachbarte C- und/oder N-Atome unter Ausbildung eines kondensierten bicyclischen Ringsystems verbunden ist, ist Cy bevorzugt ausgewählt aus der Gruppe bestehend aus $C_{4-7}$-Cycloalkyl, Phenyl, Thienyl.

Für den Fall, dass die Alkylenbrücke mit einer Gruppe Cy über drei oder mehrere C- und/oder N-Atome unter Ausbildung eines verbrückten Ringsystems verbunden ist, bedeutet Cy bevorzugt $C_{4-8}$-Cycloalkyl oder Aza-$C_{4-8}$-cycloalkyl.

Besonders bevorzugt besitzt die Gruppe

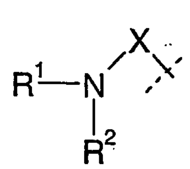

eine Bedeutung gemäß einer der folgenden Teilformeln

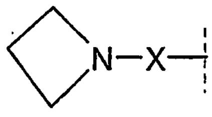 , 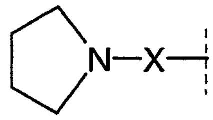 , 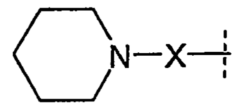 ,

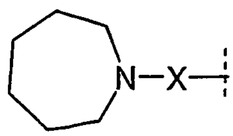 , 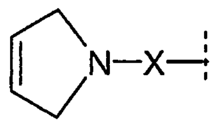 , 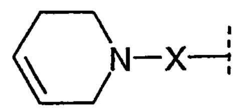 ,

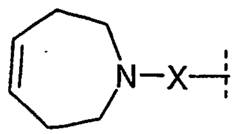 , 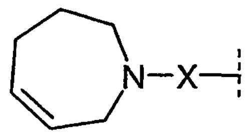 , 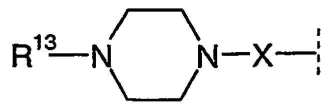

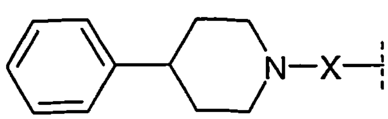 , 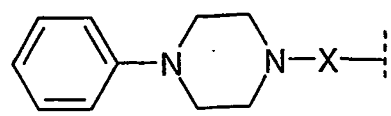 ,

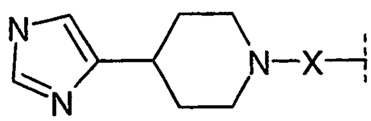 , 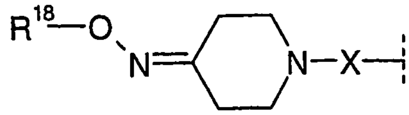 ,

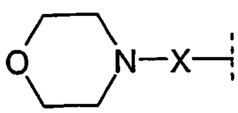 , 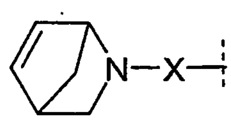 , 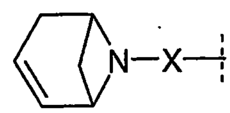 ,

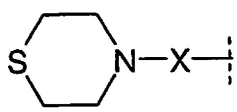 , 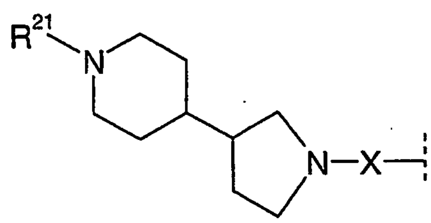 ,

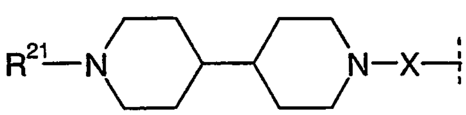 , 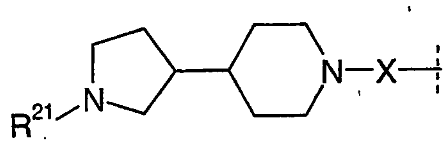 ,

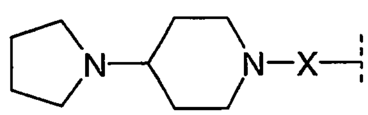 , 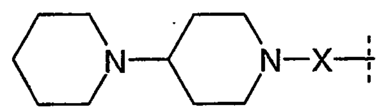 ,

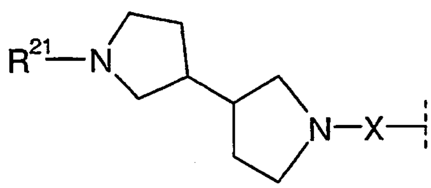 , 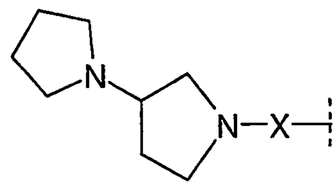 ,

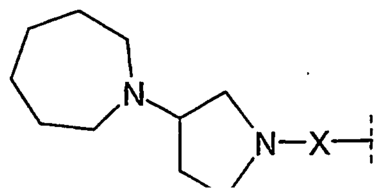 ,

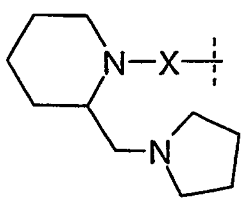 , 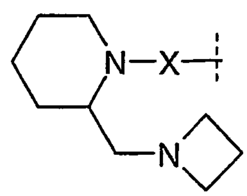 ,

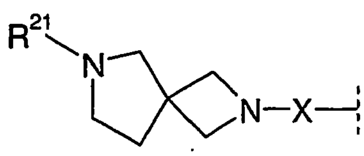 , ,

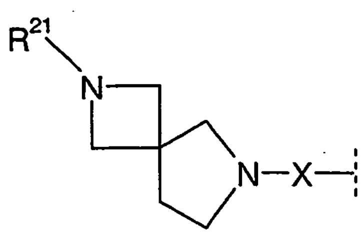 , 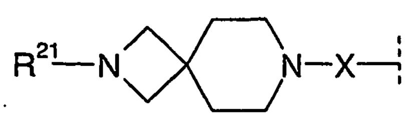 ,,

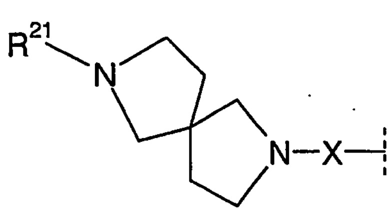 , 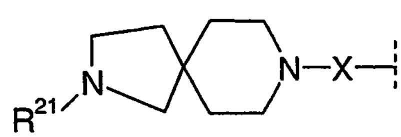 ,

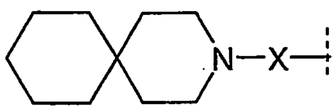 , 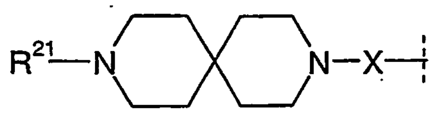 ,

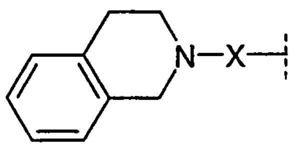 , 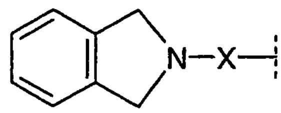 , 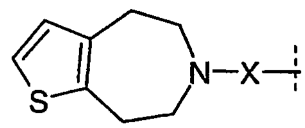 ,

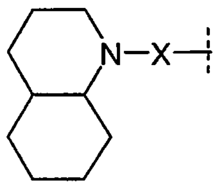 , 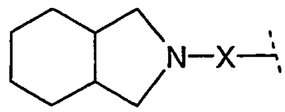 , 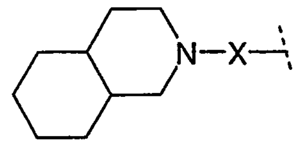

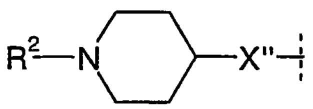 , 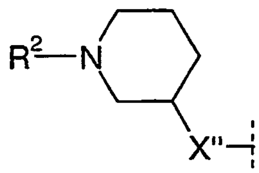 , 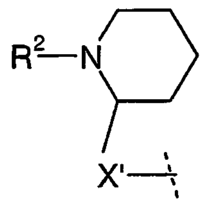 ,

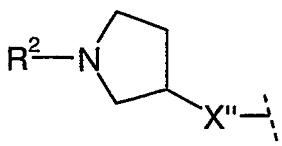 , 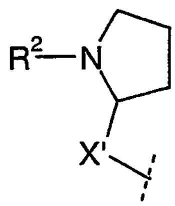 ,

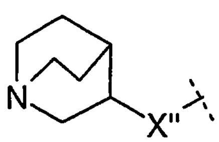 , 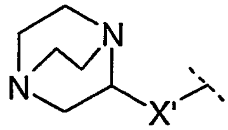 , 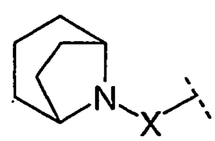 ,

worin ein- oder mehrere H-Atome des durch die Gruppe $R^1R^2N$- gebildeten Heterocyclus durch $R^{14}$ ersetzt sein können und der mit dem durch die Gruppe $R^1R^2N$-gebildeten Heterocyclus verbundene Ring ein- oder mehrfach an einem oder mehreren C-Atomen mit $R^{20}$, im Falle eines Phenyl-Rings auch zusätzlich einfach mit Nitro substituiert sein kann und

X', X" unabhängig voneinander eine Einfachbindung oder $C_{1-3}$-Alkylen und für den Fall, dass die Gruppe Y über ein C-Atom mit X' bzw. X" verbunden ist, auch -$C_{1-3}$-Alkylen-O-, -$C_{1-3}$-Alkylen-NH- oder -$C_{1-3}$-Alkylen-N($C_{1-3}$-alkyl)-, und wobei in den zuvor für X', X" genannten Bedeutungen jeweils ein C-Atom mit $R^{10}$, vorzugsweise mit einem Hydroxy-, ω-Hydroxy-$C_{1-3}$-alkyl-, ω-($C_{1-4}$-Alkoxy)-$C_{1-3}$-alkyl- und/oder $C_{1-4}$-Alkoxy-Rest, und/oder ein oder zwei

C-Atome jeweils mit einem oder zwei gleichen oder verschiedenen Substituenten ausgewählt aus $C_{1-6}$-Alkyl-, $C_{2-6}$-Alkenyl-, $C_{2-6}$-Alkinyl-, $C_{3-7}$-Cycloalkyl, $C_{3-7}$-Cycloalkyl-$C_{1-3}$-alkyl, $C_{4-7}$-Cycloalkenyl und $C_{4-7}$-Cycloalkenyl-$C_{1-3}$-alkyl substituiert sein können, wobei zwei Alkyl- und/oder Alkenyl-Substituenten unter Ausbildung eines carbocyclischen Ringsystems miteinander verbunden sein können, und
wobei in X', X" unabhängig voneinander jeweils ein oder mehrere C-Atome ein- oder mehrfach mit F und/oder jeweils ein oder zwei C-Atome unabhängig voneinander einfach mit Cl oder Br substituiert sein können und

worin $R^2$, $R^{10}$, $R^{13}$, $R^{14}$, $R^{18}$, $R^{20}$, $R^{21}$ und X die zuvor und nachstehend angegebenen Bedeutungen besitzen.

In den zuvor aufgeführten bevorzugten und besonders bevorzugten Bedeutungen von $R^1R^2N$ sind folgende Definitionen des Substituenten $R^{14}$ bevorzugt: $C_{1-4}$-Alkyl, $C_{2-4}$-Alkenyl, $C_{2-4}$-Alkinyl, $C_{3-7}$-Cycloalkyl, $C_{3-7}$-Cycloalkyl-$C_{1-3}$-alkyl-, Hydroxy, ω-Hydroxy-$C_{1-3}$-alkyl-, $C_{1-4}$-Alkoxy, ω-($C_{1-4}$-Alkoxy)-$C_{1-3}$-alkyl-, $C_{1-4}$-Alkyl-carbonyl-, Carboxy, $C_{1-4}$-Alkoxycarbonyl-, Hydroxy-carbonyl-$C_{1-3}$-alkyl-, $C_{1-4}$-Alkoxycarbonyl-$C_{1-3}$-alkyl-, $C_{1-4}$-Alkoxy-carbonylamino-, $C_{1-4}$-Alkoxy-carbonylamino-$C_{1-3}$-alkyl,Amino-,$C_{1-4}$-Alkyl-amino-,$C_{3-7}$-Cycloalkyl-amino-,N-($C_{3-7}$-Cycloalkyl)-N-($C_{1-4}$-alkyl)-amino-, Di-($C_{1-4}$-alkyl)-amino-, Amino-$C_{1-3}$-alkyl-, $C_{1-4}$-Alkyl-amino-$C_{1-3}$-alkyl-, $C_{3-7}$-Cycloalkyl-amino-$C_{1-3}$-alkyl-, N-($C_{3-7}$-Cycloalkyl)-N-($C_{1-4}$-alkyl)-amino-$C_{1-3}$-alkyl-, Di-($C_{1-4}$-alkyl)-amino-$C_{1-3}$-alkyl-, Cyclo-$C_{3-6}$-alkylenimino-$C_{1-3}$-alkyl-,Aminocarbonyl-,$C_{1-4}$-Alkyl-amino-carbonyl-,$C_{3-7}$-Cycloalkyl-amino-carbonyl-,N-($C_{3-7}$-Cycloalkyl)-N-($C_{1-4}$-alkyl)-amino-carbonyl-, Di-($C_{1-4}$-alkyl)-amino-carbonyl-, Pyridinyl-oxy-, Pyridinyl-amino-, Pyridinyl-$C_{1-3}$-alkyl-amino-.

Ganz besonders bevorzugten Bedeutungen des Substituenten $R^{14}$ sind $C_{1-4}$-Alkyl, Hydroxy, ω-Hydroxy-$C_{1-3}$-alkyl, $C_{1-4}$-Alkoxy, ω-($C_{1-4}$-Alkoxy)-$C_{1-3}$-alkyl, Amino-$C_{1-3}$-alkyl-, $C_{1-4}$-Alkyl-amino-$C_{1-3}$-alkyl-, $C_{3-7}$-Cycloalkyl-amino-$C_{1-3}$-alkyl-, N-($C_{3-7}$-Cycloalkyl)-N-($C_{1-4}$-alkyl)-amino-$C_{1-3}$-alkyl-, Di-($C_{1-4}$-alkyl)-amino-$C_{1-3}$-alkyl- und Cyclo-$C_{3-6}$-alkylenimino-$C_{1-3}$-alkyl-.

Vorzugsweise bedeutet X eine $C_{1-6}$-Alkylen-Brücke, in der

- eine nicht mit dem N-Atom der $R^1R^2N$-Gruppe benachbarte $-CH_2$-Gruppe durch
- CH=CH- oder -C≡C- ersetzt sein kann und/oder
- eine nicht mit dem N-Atom der $R^1R^2N$-Gruppe benachbarte $-CH_2$-Gruppe durch
- O-, -S-, -CO- oder $-NR^4$-, besonders bevorzugt durch -O-, -S- oder $-NR^4$-, derart ersetzt sein kann, dass jeweils zwei O-, S- oder N-Atome oder ein O- mit einem S-Atom nicht unmittelbar miteinander verbunden sind,

wobei $R^4$ mit Y unter Ausbildung eines heterocyclischen Ringsystems miteinander verbunden sein kann,
wobei die Brücke X mit $R^1$ unter Einschluss des mit $R^1$ und X verbundenen N-Atoms unter Ausbildung einer heterocyclischen Gruppe verbunden sein kann, wobei die Brücke X zusätzlich auch mit $R^2$ unter Einschluss des mit $R^2$ und X verbundenen N-Atoms unter Ausbildung einer heterocyclischen Gruppe verbunden sein kann, und
wobei zwei C-Atome oder ein C- und ein N-Atom der Alkylenbrücke durch eine zusätzliche $C_{1-4}$-Alkylen-Brücke miteinander verbunden sein können, und
wobei ein C-Atom mit $R^{10}$ und/oder ein oder zwei C-Atome jeweils mit einem oder zwei gleichen oder verschiedenen Substituenten ausgewählt aus $C_{1-6}$-Alkyl-, $C_{2-6}$-Alkenyl-, $C_{2-6}$-Alkinyl-, $C_{3-7}$-Cycloalkyl, $C_{3-7}$-Cycloalkyl-$C_{1-3}$-alkyl-, $C_{4-7}$-Cycloalkenyl und $C_{4-7}$-Cycloalkenyl-$C_{1-3}$-alkyl- substituiert sein können, wobei zwei Alkyl- und/oder Alkenyl-Substituenten unter Ausbildung eines carbocyclischen Ringsystems, insbesondere einer Cyclopropyl-, Cyclobutyl- oder Cyclopentylgruppe, miteinander verbunden sein können.

Ist in der Gruppe X eine $-CH_2$-Gruppe der Alkylen-Brücke erfindungsgemäß ersetzt, so ist diese $-CH_2$-Gruppe vorzugsweise nicht mit einem Heteroatom, einer Doppel- oder Dreifachbindung unmittelbar verbunden.

Vorzugsweise weist die Alkylen-Brücke X, X' oder X" keine oder maximal eine Imino-Gruppe auf. Die Position der Imino-Gruppe innerhalb der Alkylenbrücke X, X' oder X" ist vorzugsweise derart gewählt, dass zusammen mit der Aminogruppe $NR^1R^2$ oder einer anderen benachbarten Aminogruppe keine Aminalfunktion gebildet wird oder zwei N-Atome nicht miteinander benachbart sind.

Bevorzugt bedeutet X eine unverzweigte $C_{1-4}$-Alkylen-Brücke und
für den Fall, dass die Gruppe Y über ein C-Atom mit X verbunden ist, auch $-CH_2$-CH=CH-, $-CH_2$-C≡C-, $C_{2-4}$-Alkylenoxy oder $C_{2-4}$-Alkylen-$NR^4$-,
wobei $R^4$ mit Y unter Ausbildung eines heterocyclischen Ringsystems miteinander verbunden sein kann,
wobei die Brücke X mit $R^1$ unter Einschluss des mit $R^1$ und X verbunden N-Atoms unter Ausbildung einer heterocyclischen Gruppe verbunden sein kann, und
wobei in X ein C-Atom mit $R^{10}$ und/oder ein oder zwei C-Atome jeweils mit einem oder zwei gleichen oder verschiedenen Substituenten ausgewählt aus $C_{1-6}$-Alkyl-, $C_{2-6}$-Alkenyl-, $C_{2-6}$-Alkinyl-, $C_{3-7}$-Cycloalkyl, $C_{3-7}$-Cycloalkyl-$C_{1-3}$-alkyl-, $C_{4-7}$-Cycloalkenyl und $C_{4-7}$-Cycloalkenyl-$C_{1-3}$-alkyl- substituiert sein können, wobei zwei Alkyl- und/oder Alkenyl-Substituenten unter Ausbildung eines carbocyclischen Ringsystems miteinander verbunden sein können, und
wobei in den zuvor angegebenen Gruppen und Resten ein oder mehrere C-Atome ein- oder mehrfach mit F und/oder ein oder zwei C-Atome unabhängig voneinander einfach mit Cl oder Br substituiert sein können und

worin $R^1$, $R^4$ und $R^{10}$ wie zuvor und nachstehend definiert sind.

Besonders bevorzugt bedeutet X $-CH_2-$, $-CH_2-CH_2-$ oder $-CH_2-CH_2-CH_2-$ und für den Fall, dass die Gruppe Y über ein C-Atom mit X verbunden ist, auch $-CH_2-CH=CH-$, $-CH_2-C{\equiv}C-$, $-CH_2-CH_2-O-$, $-CH_2-CH_2-CH_2-O-$ oder $-CH_2-CH_2-NR^4-$ oder $-CH_2-CH_2-CH_z-NR^4-$, wobei $R^4$ mit Y unter Ausbildung eines heterocyclischen Ringsystems miteinander verbunden sein kann, wobei die Brücke X mit $R^1$ unter Einschluss des mit $R^1$ und X verbundenen N-Atoms unter Ausbildung einer heterocyclischen Gruppe verbunden sein kann, und wobei in X ein C-Atom mit $R^{10}$, vorzugsweise einem Hydroxy-, ω-Hydroxy-$C_{1-3}$-alkyl-, ω-($C_{1-4}$-Alkoxy)-$C_{1-3}$-alkyl- und/oder $C_{1-4}$-Alkoxy-Rest, und/oder ein oder zwei C-Atome unabhängig voneinander jeweils mit einem oder zwei gleichen oder verschiedenen $C_{1-4}$-Alkyl-Resten substituiert sein können, wobei zwei Alkyl-Reste unter Ausbildung eines carbocyclischen Ringsystems miteinander verbunden sein können, und wobei jeweils ein oder mehrere C-Atome ein- oder mehrfach mit F und/oder jeweils ein oder zwei C-Atome unabhängig voneinander einfach mit Cl oder Br substituiert sein können.

Ganz besonders bevorzugt bedeutet X für den Fall, dass die Gruppe Y über ein C-Atom mit X verbunden ist, $-CH_2-CH_2-O-$, das wie angegeben substituiert sein kann.

Für den Fall, das $R^4$ mit Y unter Ausbildung eines heterocyclischen Ringsystems miteinander verbunden ist, besitzt Y vorzugsweise die Bedeutung Phenyl und $R^4$ vorzugsweise die Bedeutung $C_{2-6}$-Alkyl oder $C_{2-6}$-Alkenyl. Hierbei bevorzugte heterocyclische Ringsysteme sind Indol, Dihydroindol, Dihydrochinolin und Tetrahydrochinolin.

Der Rest $R^4$ weist vorzugsweise nur dann die Bedeutung Vinyl auf, wenn $R^4$ mit Y unter Ausbidlung eines heterocyclischen Ringsystems verbunden ist.

Die Gruppe X in der Bedeutung $C_{2-4}$-Alkylenoxy, insbesondere $-CH_2-CH_2-CH_2-O-$, weist keinen Hydroxy-Substituenten auf.

Ist in X, X' oder X" ein C-Atom substituiert, so sind bevorzugte Substituenten ausgewählt aus der Gruppe der $C_{1-4}$-Alkyl-, $C_{2-4}$-Alkenyl-, $C_{2-4}$-Alkinyl-, $C_{3-7}$-Cycloalkyl, $C_{3-7}$-Cycloalkyl-$C_{1-3}$-alkyl, Hydroxy-, ω-Hydroxy-$C_{1-3}$-alkyl-, ω-($C_{1-4}$-Alkoxy)-$C_{1-3}$-alkyl- und $C_{1-4}$-Alkoxy-Reste. Des weiteren können in X, X' oder X" ein C-Atom zweifach und/oder ein oder zwei C-Atome ein- oder zweifach substituiert sein, wobei bevorzugte Substituenten ausgewählt sind aus der Gruppe $C_{1-4}$-Alkyl-, $C_{2-4}$-Alkenyl-, $C_{2-4}$-Alkinyl-, $C_{3-7}$-Cycloalkyl und $C_{3-7}$-Cycloalkyl-$C_{1-3}$-alkyl, und zwei $C_{1-4}$-Alkyl- und/oder $C_{2-4}$-Alkenyl-Substituenten auch unter Ausbildung eines gesättigten oder einfach ungesättigten carbocyclischen Rings miteinander verbunden sein können.

Ganz besonders bevorzugte Substituenten ein oder zweier C-Atome in X, X' oder X" sind ausgewählt aus Methyl, Ethyl, n-Propyl, i-Propyl, Cyclopropyl, Cyclopropylmethyl, wobei zwei Alkylsubstituenten an einem C-Atom auch unter Ausbildung eines carbocyclischen Rings miteinander verbunden sein können.

Bedeutet Y ein kondensiertes bicyclisches Ringsystem, so ist eine bevorzugte Bedeutung der Gruppe X $-CH_2-$, $-CH_2-CH_2-$ und $-CH_2-CH_2-CH_2-$, insbesondere $-CH_2-$ oder $-CH_2-CH_2-$, die wie angegeben substituiert sein können.

Sind in der Gruppe X, X' oder X" ein oder mehrere C-Atome mit einem Hydroxy- und/oder $C_{1-4}$-Alkoxy-Rest substituiert, so ist das substituierte C-Atom vorzugsweise nicht unmittelbar mit einem weiteren Heteroatom benachbart.

Bevorzugt ist Z eine Einfachbindung, $-CH_2-$ oder $-CH_2-CH_2-$, wobei ein oder zwei C-Atome unabhängig voneinander ein- oder zweifach mit F, $CH_3$ oder $CF_3$ und/oder einfach mit Cl substituiert sein können.

Besonders bevorzugte Bedeutungen der Gruppe Z sind Einfachbindung, $-CH_2-$ oder $-CH_2-CH_2-$, insbesondere eine Einfachbindung.

Gemäß einer ersten Ausführungsform weisen die erfindungsgemäßen Verbindungen eine Brücke W auf, die ausgewählt aus der Gruppe $-CR^{6a}R^{6b}-O-$, $-CR^{6a}R^{6b}-NR^8-$, $-CR^{7a}R^{7b}-CR^{7c}R^{7d}-$ und $-NR^8-CR^{6a}R^{6b}-$.

Gemäß einer zweiten Ausführungsform weisen die erfindungsgemäßen Verbindungen eine Brücke W auf, die $-CR^{7a}=CR^{7c}-$ bedeutet.

W bedeutet vorzugsweise $-CH_2-O-$, $-CH_2-NR^8-$, $-CH_2-CH_2-$ oder $-CH=CH-$, wobei in den beiden zuletzt genannten Bedeutungen jeweils ein oder zwei C-Atome unabhängig voneinander mit F, Cl, $CH_3$ oder $CF_3$ substituiert sein können. In den zuvor genannten Bedeutungen $-CH_2-O-$ und $-CH_2-NR^8-$ ist die Gruppe A vorteilhaft über ein C-Atom mit der Brücke W verbunden.

Bevorzugte Bedeutungen des Substituenten $R^8$ sind H und Methyl.

Besonders bevorzugte Bedeutungen der Gruppe W sind $-CH_2-O-$, $-CH_2-NH-$, $-CH_2-NCH_3-$ und $-CH_2-CH_2-$, insbesondere $-CH_2-O-$.

Besitzt die Gruppe W die zuvor angegebene Bedeutung einer gegebenenfalls substituierten -CH=CH-Brücke, so ist die Gruppe Z vorzugsweise eine Einfachbindung.

Bevorzugte Ausführungsformen dieser Erfindung umfassen daher Verbindungen, die jeweils durch die nachfolgenden Formeln Ia, Ib, Ic und Id beschrieben werden können:

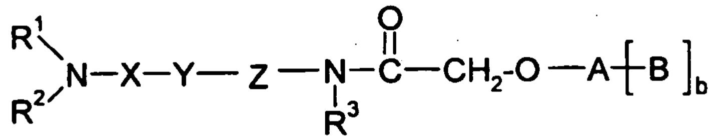

Ia

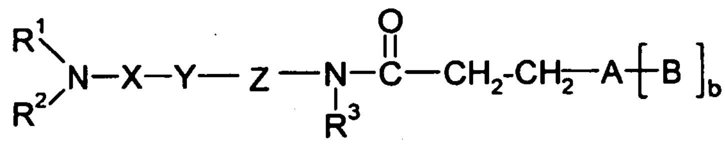

Ib

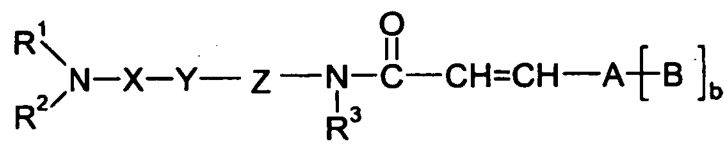

Ic

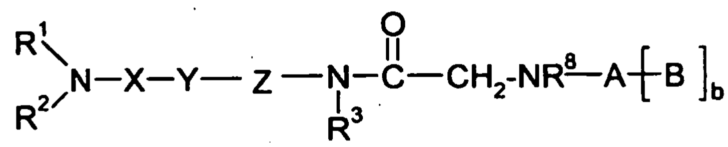

Id

worin $R^1$, $R^2$, X, Y, Z, $R^3$, $R^8$, A, B und b die zuvor und nachfolgend angegebenen Bedeutungen, insbesondere die als bevorzugt angegebenen Bedeutungen besitzen, wobei $R^8$ vorzugsweise H oder Methyl bedeutet.

Besonders bevorzugt ist eine Bedeutung der Gruppe Y ausgewählt aus der Gruppe der bivalenten cyclischen Gruppen

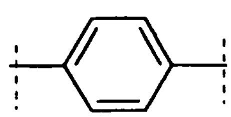 , 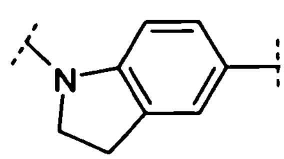 ,

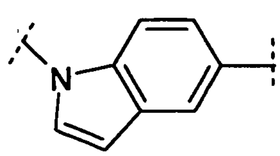 und 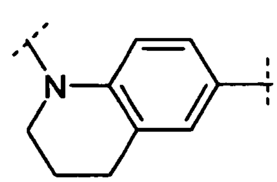 ,

wobei die vorstehend aufgeführten cyclischen Gruppen ein- oder mehrfach an ein oder mehreren C-Atomen mit $R^{20}$ substituiert sein können, im Falle einer Phenylgruppe auch zusätzlich einfach mit Nitro, und/oder eine oder mehrere NH-Gruppen mit $R^{21}$ substituiert sein.

Die Gruppe Y ist vorzugsweise unsubstituiert oder ein- oder zweifach substituiert.

Besonders bevorzugte Substituenten $R^{20}$ der Gruppe Y sind ausgewählt aus der Gruppe bestehend aus Fluor,

Chlor, Brom, Cyano, Nitro, $C_{1-4}$-Alkyl, $C_{2-6}$-Alkenyl, Hydroxy, ω-Hydroxy-$C_{1-3}$-alkyl, $C_{1-4}$-Alkoxy, Trifluormethyl, Trifluormethoxy, $C_{2-4}$-Alkinyl, $C_{1-4}$-Alkoxycarbonyl-, ω-($C_{1-4}$-Alkoxy)-$C_{1-3}$-alkyl-, $C_{1-4}$-Alkoxy-carbonylamino-, Amino-, $C_{1-4}$-Alkyl-amino-, Di-($C_{1-4}$-alkyl)-amino-, Aminocarbonyl-, $C_{1-4}$-Alkyl-amino-carbonyl-, Di-($C_{1-4}$-alkyl)-amino-carbonyl-, -CH=N-OH und -CH=N-O-$C_{1-4}$-alkyl.

Ganz besonders bevorzugte Substituenten $R^{20}$ der Gruppe Y sind ausgewählt aus der Gruppe bestehend aus Fluor, Chlor, Brom, Cyano, $C_{1-3}$-Alkyl, $C_{1-3}$-Alkoxy, Trifluormethyl, Trifluormethoxy, im Falle eines Phenylrings auch Nitro.

Ist Y eine Phenyl- oder Pyridinyl-Gruppe, so ist, insbesondere für den Fall, dass die Gruppe W gegebenenfalls substituiertes -CH=CH- oder -$CH_2$-$CH_2$- bedeutet, die Phenyl- bzw. Pyridinyl-Gruppe mindestens einfach substituiert.

Ganz besonders bevorzugt bedeutet die Gruppe Y substituiertes Phenylen der Teilformel

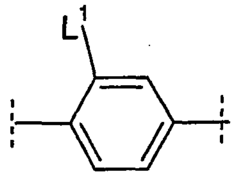

,

worin $L^1$ eine der zuvor für $R^{20}$ angegebenen Bedeutungen, vorzugsweise F, Cl, Br, I, $CH_3$, $CF_3$, $OCH_3$, $OCF_3$, CN oder $NO_2$, besitzt oder H bedeutet.

Die Gruppe Y weist bevorzugt die Bedeutung monosubstituiertes Phenylen gemäß obiger Teilformel auf, wenn die Brücke W die Bedeutung -CH=CH- besitzt.

Eine bevorzugte Bedeutung der Gruppe A ist Aryl oder Heteroaryl.

Vorzugsweise ist die Gruppe A ausgewählt aus der Gruppe der cyclischen Gruppen Phenyl, Pyridinyl oder Naphthyl, die ein- oder mehrfach an ein oder mehreren C-Atomen mit $R^{20}$, im Falle eines Phenylrings auch zusätzlich einfach mit Nitro, substituiert sein können.

Besitzt b den Wert 0, so ist die Gruppe A vorzugsweise ein-, zwei- oder dreifach substituiert.

Besitzt b den Wert 1, so ist die Gruppe A vorzugsweise unsubstituiert oder ein- oder zweifach substituiert.

Ganz besonders bevorzugt ist A eine der nachfolgend aufgeführten Gruppen

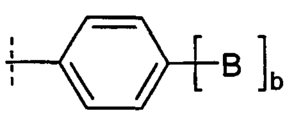

,

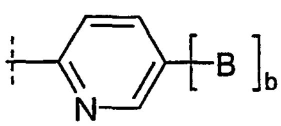

,

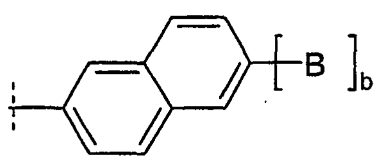

,

wobei die aufgeführten Gruppen wie angegeben mit $R^{20}$ ein- oder mehrfach substituiert sein können. Die angegebenen Bedeutungen der Gruppe A Phenyl und Pyridyl sind in dem Fall bevorzugt, dass b den Wert 1 besitzt.

Besonders bevorzugte Substituenten $R^{20}$ der Gruppe A sind ausgewählt aus der Gruppe bestehend aus Fluor, Chlor, Brom, Cyano, $C_{1-4}$-Alkyl, $C_{2-6}$-Alkenyl, -CHO, Hydroxy, ω-Hydroxy-$C_{1-3}$-alkyl, $C_{1-4}$-Alkoxy, Trifluormethyl, Trifluormethoxy, $C_{2-4}$-Alkinyl, Carboxy, $C_{1-4}$-Alkoxycarbonyl-, ω-($C_{1-4}$-Alkoxy)-$C_{1-3}$-alkyl, $C_{1-4}$-Alkoxycarbonylamino-, Amino-, $C_{1-4}$-Alkyl-amino-, Di-($C_{1-4}$-alkyl)-amino-, Cyclo-$C_{3-6}$-alkylenimino-, Aminocarbonyl-, $C_{1-4}$-Alkyl-amino-carbonyl-, Di-($C_{1-4}$-alkyl)-amino-carbonyl-, -CH=N-OH und -CH=N-O-$C_{1-4}$-alkyl.

Ganz besonders bevorzugte Substituenten $R^{20}$ der Gruppe A sind ausgewählt aus der Gruppe bestehend aus Fluor, Chlor, Brom, Cyano, $C_{1-4}$-Alkyl, $C_{1-4}$-Alkoxy Trifluormethyl, Trifluormethoxy, Carboxy, $C_{1-4}$-Alkoxycarbonyl-, $C_{1-4}$-Alkyl-amino-, Di-($C_{1-4}$-alkyl)-amino-,

Für den Fall, dass b den Wert 0 besitzt, ist eine besonders bevorzugte Bedeutung der Gruppe A substituiertes Phenyl der Teilformel

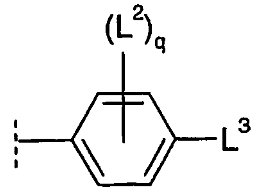

worin

$L^2$ eine der für $R^{20}$ angegebenen Bedeutungen besitzt oder H, vorzugsweise F, Cl, Br, I, $CH_3$, $CF_3$, $OCH_3$, $OCF_3$, CN oder $NO_2$ bedeutet,

$L^3$ eine der für $R^{20}$ angegebenen Bedeutungen besitzt oder H, vorzugsweise F, Cl, Br, I, $CF_3$, $OCF_3$, CN, $NO_2$, $C_{1-4}$-Alkyl, $C_{3-7}$-Cycloalkyl, $C_{3-7}$-Cycloalkyl-$C_{1-3}$-alkyl, $C_{1-4}$-Alkoxy, $C_{3-7}$-Cycloalkyl-O-, $C_{3-7}$-Cycloalkyl-$C_{1-3}$-alkoxy, -COO-$C_{1-4}$-alkyl oder -COOH bedeutet,

q den Wert 0, 1 oder 2 besitzt.

mit der Maßgabe, dass die Phenyl- und Naphthyl-Gruppe lediglich maximal einfach mit Nitro substituiert sein kann.

Besonders bevorzugt ist A substituiertes Phenyl gemäß obiger Teilformel, in der q 1 oder 2 bedeutet und/oder mindestens ein Substituent $L^2$ zum Substituenten $L^3$ in meta-Stellung steht.

Die Gruppe A weist bevorzugt die Bedeutung substituiertes Phenyl gemäß obiger Teilformel auf, wobei q den Wert 1 oder 2 besitzt, wenn die Brücke W die Bedeutung -CH=CH-, die Gruppe Y die Bedeutung Phenyl und b den Wert 0 besitzt.

Weiterhin eine bevorzugte Teilformel für A, insbesondere für den Fall, dass b den Wert 0 besitzt, ist

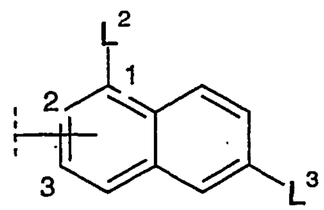
,

wobei die Bindung zur Gruppe W über das C-Atom mit der Positionsnummer 2 oder 3 erfolgt.

Für den Fall, dass b den Wert 1 besitzt, ist eine bevorzugte Bedeutung der Gruppe B Aryl oder Heteroaryl, das wie angegeben substituiert sein kann.

Bevorzugte Bedeutungen der Gruppe B sind ausgewählt aus der Gruppe bestehend aus Phenyl, Pyridyl, Thienyl und Furanyl. Besonders bevorzugt bedeutet die Gruppe B Phenyl. Die Gruppe B in den angegebenen Bedeutungen kann ein- oder mehrfach mit $R^{20}$, eine Phenylgruppe zusätzlich auch einfach mit Nitro substituiert sein. Vorzugsweise ist die Gruppe B ein-, zwei- oder dreifach, insbesondere ein- oder zweifach substituiert. Im Falle einer Einfachsubstitution ist der Substituent vorzugsweise in para-Position zur Gruppe A.

Besonders bevorzugte Substituenten $R^{20}$ der Gruppe B sind ausgewählt aus der Gruppe bestehend aus Fluor, Chlor, Brom, Cyano, Nitro, $C_{1-4}$-Alkyl, Hydroxy, ω-Hydroxy-$C_{1-3}$-alkyl, $C_{1-4}$-Alkoxy, Trifluormethyl, Trifluormethoxy, $C_{2-4}$-Alkinyl, Carboxy, $C_{1-4}$-Alkoxycarbonyl-, ω-($C_{1-4}$-Alkoxy)-$C_{1-3}$-alkyl-, $C_{1-4}$-Alkoxy-carbonylamino-, Amino-, $C_{1-4}$-Alkyl-amino-, Di-($C_{1-4}$-alkyl)-amino-, Cyclo-$C_{3-6}$-alkylenimino-, Aminocarbonyl-, $C_{1-4}$-Alkyl-amino-carbonyl- und Di-($C_{1-4}$-alkyl)-amino-carbonyl-.

Ganz besonders bevorzugte Substituenten $R^{20}$ der Gruppe B sind ausgewählt aus der Gruppe bestehend aus Fluor, Chlor, Brom, Cyano, $CF_3$, $C_{1-3}$-Alkyl, $C_{1-4}$-Alkoxy und Trifluormethoxy.

Die Gruppe B weist bevorzugt die Bedeutung eines mindestens einfach substituierten Phenyl-Rings auf, wenn die Brücke W die Bedeutung -CH=CH-, die Gruppe Y die Bedeutung Phenyl und b den Wert 1 besitzt.

$R^4$ weist eine der für $R^{17}$, vorzugsweise eine der für $R^{16}$, angegebenen Bedeutungen auf.

Besonders bevorzugte Bedeutungen des Substituenten $R^4$ sind H, $C_{1-6}$-Alkyl und $C_{3-6}$-Alkenyl. Ist $R^4$ mit Y unter Ausbildung eines heterocyclischen Ringsystems verbunden, so sind besonders bevorzugte Bedeutungen von $R^4$

$C_{2-6}$-Alkyl und $C_{2-6}$-Alkenyl.

Die Reste $R^{6a}$, $R^{6b}$ bedeuten H, $C_{1-4}$-Alkyl oder $CF_3$, vorzugsweise H oder Methyl, insbesondere H.

Die Reste $R^{7a}$, $R^{7b}$, $R^{7c}$, $R^{7d}$ bedeuten H, F, $C_{1-4}$-Alkyl oder $CF_3$, vorzugsweise H oder Methyl, insbesondere H.

Der Rest $R^8$ bedeutet vorzugsweise H oder Methyl.

Ist $R^{11}$ eine $C_{2-6}$-Alkenyl- oder $C_{2-6}$-Alkinyl-Gruppe, so sind die Bedeutungen $-CH=CH_2$, $-CH=CH(CH_3)$, $-CH=C(CH_3)_2$ sowie $-C\equiv CH$, $-C\equiv C-CH_3$ bevorzugt.

Der Substituent $R^{20}$ weist vorzugsweise keines der folgenden Strukturelemente auf:

a) -CO-Aryl oder -CO-Heteroaryl, insbesondere -CO-Phenyl, wobei Heteroaryl, Aryl und Phenyl substituiert sein können,
b) -C(=NH)-NH-, wobei die H-Atome substituiert sein können und/oder
c) -NH-CO-NH-, wobei die H-Atome substituiert sein können.

Bevorzugte Bedeutungen der Gruppe $R^{20}$ sind Halogen, Hydroxy, Cyano, $C_{1-4}$-Alkyl, $C_{3-7}$Cycloalkyl und $C_{1-4}$-Alkoxy. Besonders bevorzugt bedeutet $R^{20}$ F, Cl, Br, I, OH, Cyano, Methyl, Difluormethyl, Trifluormethyl, Ethyl, n-Propyl, iso-Propyl, Methoxy, Difluormethoxy, Trifluormethoxy, Ethoxy, n-Propoxy oder iso-Propoxy.

Bevorzugte Bedeutungen der Gruppe $R^{21}$ sind $C_{1-4}$-Alkyl, $C_{1-4}$-Alkylcarbonyl, $C_{1-4}$-Alkylsulfonyl-, $-SO_2-NH_2$, $-SO_2-NH-C_{1-3}$-alkyl, $-SO_2-N(C_{1-3}\text{-alkyl})_2$ und Cyclo-$C_{3-6}$-alkylenimino-sulfonyl-.

Cy bedeutet vorzugsweise eine $C_{3-7}$Cycloalkyl-, insbesondere eine $C_{5-7}$-CycloalkylGruppe, eine $C_{5-7}$Cycloalkenyl-Gruppe, Pyrrolidinyl, Piperidinyl, Piperazinyl, Morpholinyl, Thiomorpholinyl, Aryl oder Heteroaryl, wobei Aryl oder Heteroaryl vorzugsweise ein monocyclisches oder kondensiert bicyclisches Ringsystem darstellt, und wobei die zuvor genannten cyclischen Gruppen ein- oder mehrfach an ein oder mehreren C-Atomen mit $R^{20}$, im Falle einer Phenylgruppe auch zusätzlich einfach mit Nitro, und/oder ein oder mehrere NH-Gruppen mit $R^{21}$ substituiert sein können.

Diejenigen erfindungsgemäßen Verbindungen sind bevorzugt, in denen eine oder mehrere der Gruppen, Reste, Substituenten und/oder Indizes eine der zuvor als bevorzugt angegebenen Bedeutungen aufweisen.

Insbesondere sind diejenigen erfindungsgemäßen Verbindungen bevorzugt, in denen

Y Phenyl, 1 H-Indolyl, 2,3-Dihydro-1H-indolyl oder 1,2,3,4-Tetrahydrochinolin gemäß der vorstehend als bevorzugt beschriebenen Bedeutung, insbesondere mit $L^1$ substituiertes Phenyl gemäß zuvor angegebener Teilformel und/oder

A mit $L^2$ und $L^3$ substituiertes Phenyl gemäß der zuvor angegebener Teilformel bedeutet.

Ganz besonders sind diejenigen erfindungsgemäßen Verbindungen bevorzugt, in denen A, X, Y, Z, $R^1$, $R^2$, $R^3$ und W unabhängig voneinander eine oder mehrere der vorstehend genannten bevorzugten Bedeutungen aufweisen.

Bevorzugte Gruppen von Verbindungen gemäß dieser Erfindung lassen sich durch folgende Formeln beschreiben

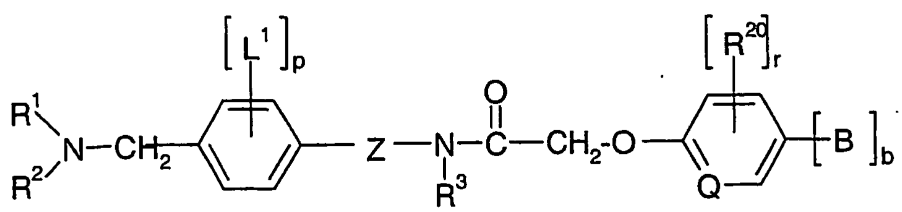

Ia.1

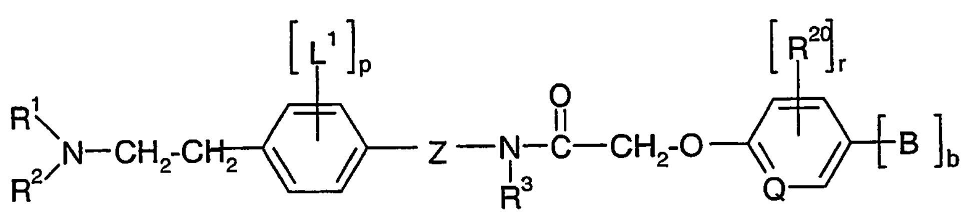

Ia.2

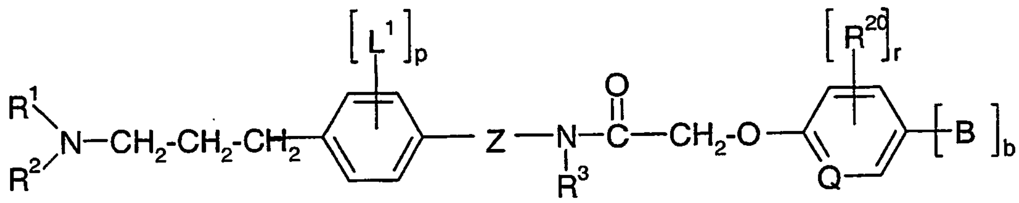

Ia.3

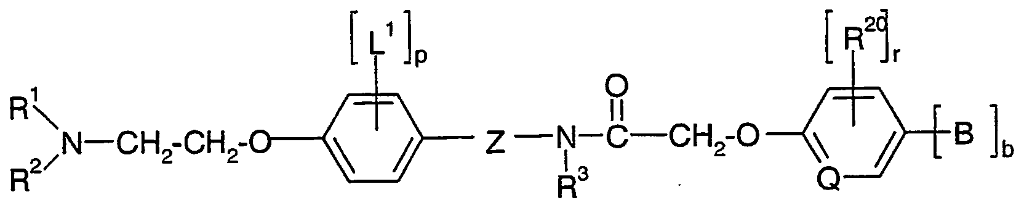

Ia.4

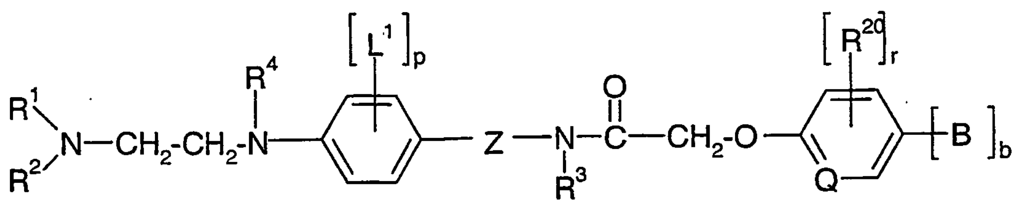

Ia.5

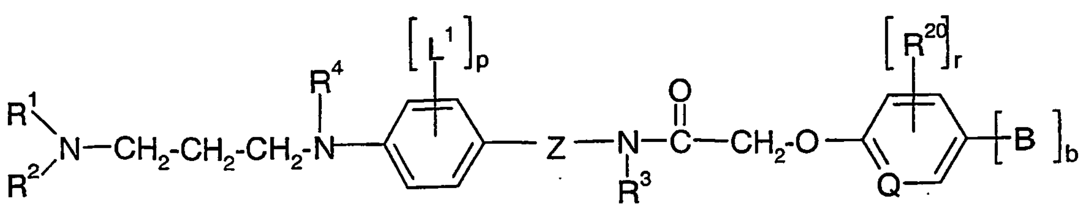

Ia.6

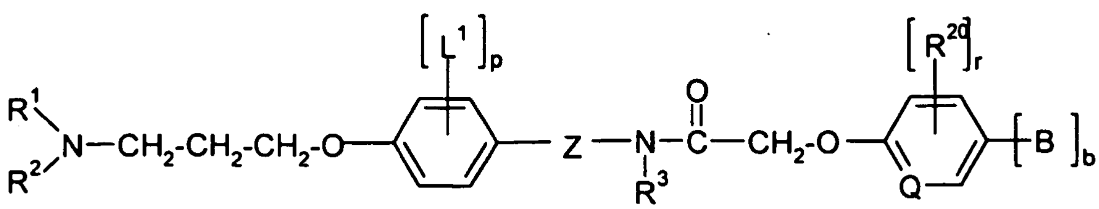

Ia.7

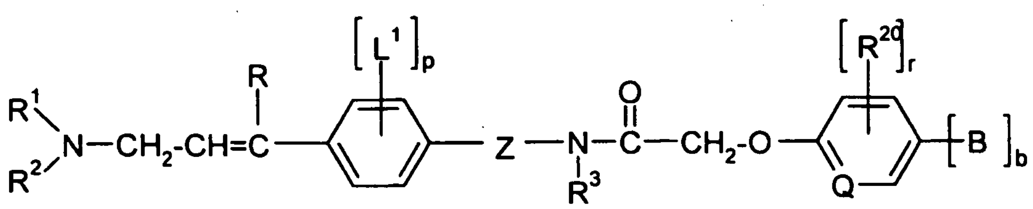

Ia.9

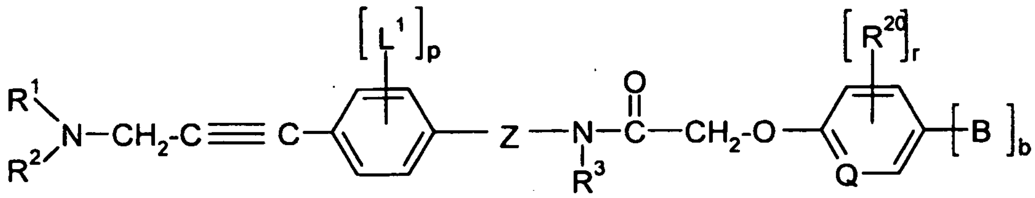

Ia.10

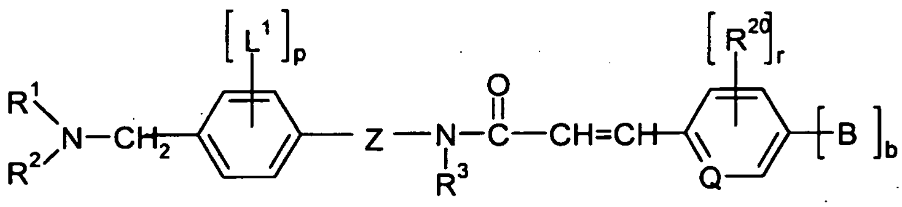

Ia.11

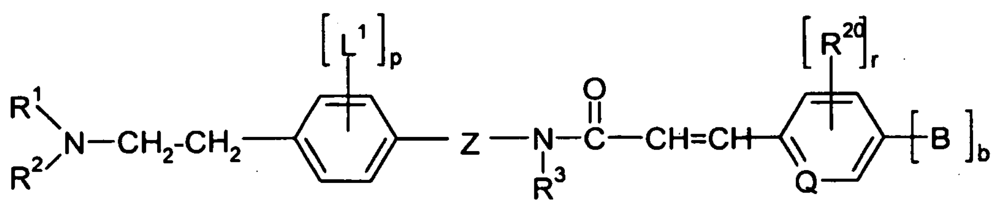

Ia.12

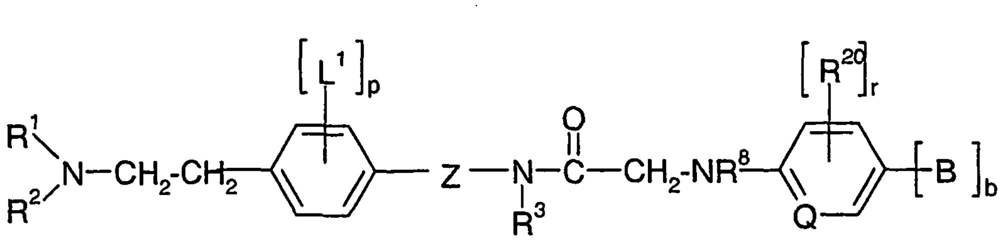

Ia.13

in denen $R^1$, $R^2$, Z, $R^3$, $R^4$, $R^8$, $R^{20}$, B und b die zuvor angegebenen Bedeutungen aufweisen und

Z vorzugsweise eine Einfachbindung oder $-CH_2-CH_2-$ bedeutet,

$R^3$ vorzugsweise H oder Methyl bedeutet,

$R^4$ vorzugsweise H, $C_{1-6}$-Alkyl oder $C_{2-6}$-Alkenyl bedeutet, wobei $R^4$ mit Y unter Ausbildung eines heterocyclischen Ringsystems, besonders bevorzugt unter Ausbildung einer Indol, Dihydroindol, Dihydrochinolin oder Tetrahydrochinolin-Gruppe, verbunden sein kann,

Q CH oder N bedeutet, wobei CH durch $R^{20}$ substituiert sein kann,

B vorzugsweise Aryl oder Heteroaryl, besonders bevorzugt Phenyl, Pyridyl, Furanyl oder Thienyl bedeutet, wobei B ein- oder mehrfach durch $R^{20}$ substituiert sein kann,

$L^1$ vorzugsweise Fluor, Chlor, Brom, Cyano, Nitro, $C_{1-3}$-Alkyl, $C_{1-3}$-Alkoxy, Trifluormethyl, Trifluormethoxy oder Nitro bedeutet,

p den Wert 0 oder 1 besitzt,

$R^{20}$ vorzugsweise Fluor, Chlor, Brom, Cyano, $C_{1-4}$-Alkyl, $C_{2-6}$-Alkenyl, -CHO, Hydroxy, ω-Hydroxy-$C_{1-3}$-alkyl, $C_{1-4}$-Alkoxy, Trifluormethyl, Trifluormethoxy, $C_{2-4}$-Alkinyl, Carboxy, $C_{1-4}$-Alkoxycarbonyl-, ω-($C_{1-4}$-Alkoxy)-$C_{1-3}$-alkyl, $C_{1-4}$-Alkoxycarbonylamino-, Amino-, $C_{1-4}$-Alkyl-amino-, Di-($C_{1-4}$-alkyl)-amino-, Cyclo-$C_{3-6}$-alkylenimino-, Aminocarbonyl-, $C_{1-4}$-Alkyl-amino-carbonyl-, Di-($C_{1-4}$-alkyl)-amino-carbonyl-, -CH=N-OH und -CH=N-O-$C_{1-4}$-alkyl bedeutet,

r den Wert 1, 2 oder 3 und für den Fall, dass b den Wert 1 besitzt, auch 0 und

wobei die Verbindungen gemäß der Maßgaben (M1), (M2) und (M3) nicht umfasst sind.

Besonders bevorzugt sind die folgenden Einzelverbindungen:

(1)

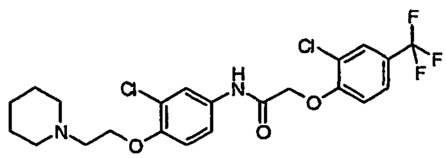

N-[3-Chlor-4-(2-piperidin-1-yl-ethoxy)-phenyl]-2-(2-chlor-4-trifiuormethyl-phenoxy)-acetamid

(2)

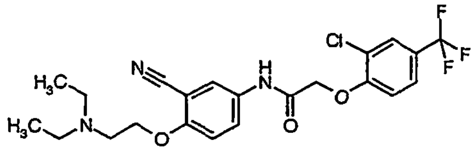

2-(2-Chlor-4-trifluormethyl-phenoxy)-N-[3-cyano-4-(2-diethylamino-ethoxy)-phenyl]-acetamid

(3)

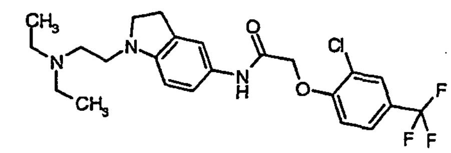

2-(2-Chlor-4-trifluormethyl-phenoxy)-N-[1-(2-diethylamino-ethyl)-2,3-dihydro-1H-indol-5-yl]-acetamid

(4)

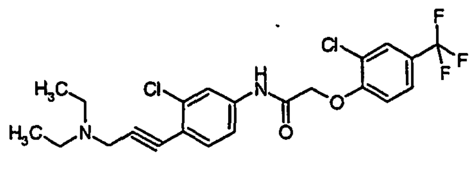

N-[3-Chlor-4-(3-diethylamino-prop-1-ynyl)-phenyl]-2-(2-chlor-4-trifluormethyl-phenoxy)-acetamid

(5)

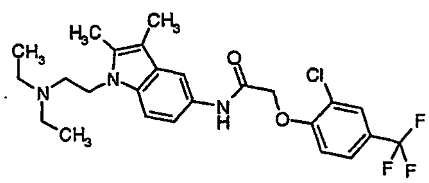

2-(2-Chlor-4-trifluormethyl-phenoxy)-N-[1-(2-diethylamino-ethyl)-2,3-dimethyl-1 H-indol-5-yl]-acetamid

(6)

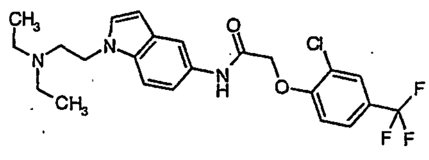

2-(2-Chlor-4-trifluormethyl-phenoxy)-N-[1-(2-diethylamino-ethyl)-1 H-indol-5-yl]-acetamid

(7)

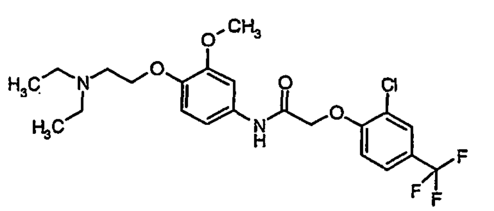

2-(2-Chlor-4-trifluormethyl-phenoxy)-N-[4-(2-diethylamino-ethoxy)-3-methoxy-phenyl]-acetamid

(8)

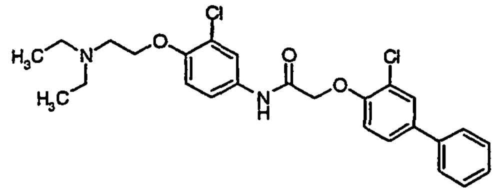

2-(3-Chlor-biphenyl-4-yloxy)-N-[3-chlor-4-(2-diethylamino-ethoxy)-phenyl]-acetamid

(9)

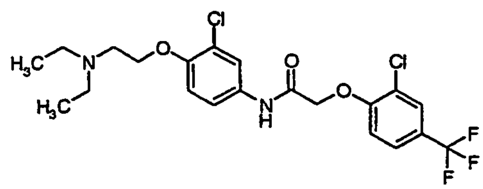

N-[3-Chlor-4-(2-diethylamino-ethoxy)-phenyl]-2-(2-chlor-4-trifluormethyl-phenoxy)-acetamid

(10)

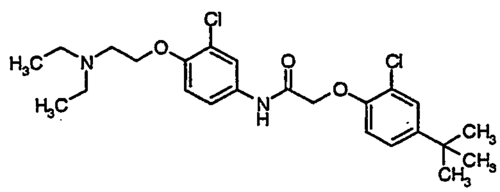

2-(4-tert.-Butyl-2-chlor-phenoxy)-N-[3-chlor-4-(2-diethylamino-ethoxy)-phenyl]-acetamid

(11)

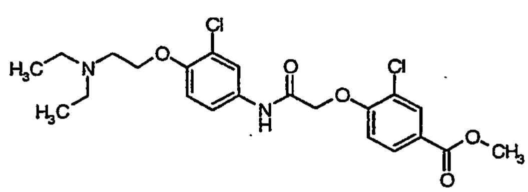

3-Chlor-4-{[3-chlor-4-(2-diethylamino-ethoxy)-phenylcarbamoyl]-methoxy}-benzoesäure-methylester

(12)

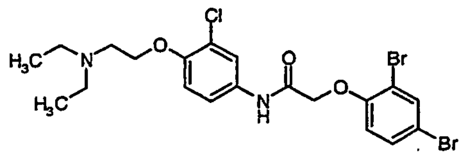

N-[3-Chlor-4-(2-diethylamino-ethoxy)-phenyl]-2-(2,4-dibromo-phenoxy)-acetamid

(13)

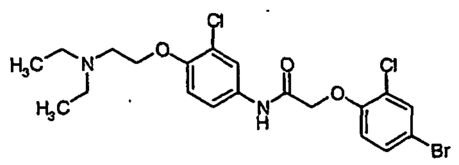

2-(4-Brom-2-chlor-phenoxy)-N-[3-chlor-4-(2-diethylamino-ethoxy)-phenyl]-acetamid

(14)

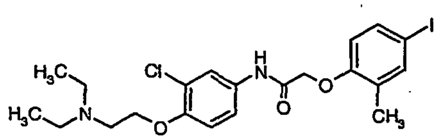

N-[3-Chlor-4-(2-diethylamino-ethoxy)-phenyl]-2-(4-iod-2-methyl-phenoxy)-acetamid

(15)

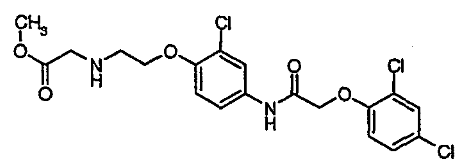

(2-{2-Chlor-4-[2-(2,4-dichlor-phenoxy)-acetylamino]-phenoxy}-ethylamino)-essigsäure-methylester

(16)

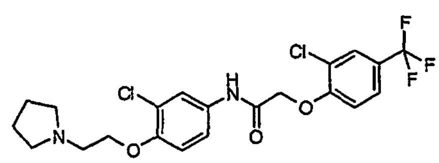

N-[3-Chlor-4-(2-pyrrolidin-1-yl-ethoxy)-phenyl]-2-(2-chlor-4-trifluormethyl-phenoxy)-acetamid

(17)

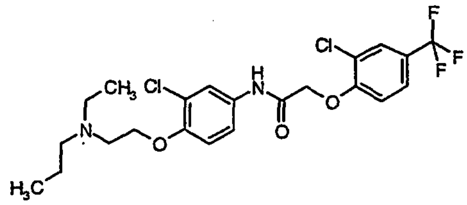

N-{3-Chlor-4-[2-(ethyl-propyl-amino)-ethoxy]-phenyl}-2-(2-chlor-4-trifluormethyl-phenoxy)-acetamid

(18)

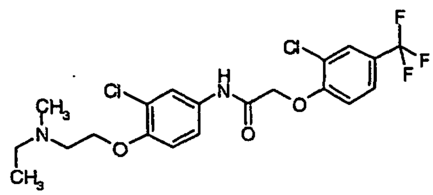

N-{3-Chlor-4-[2-(ethyl-methyl-amino)-ethoxy]-phenyl}-2-(2-chlor-4-trifluormethyl-phenoxy)-acetamid

(19)

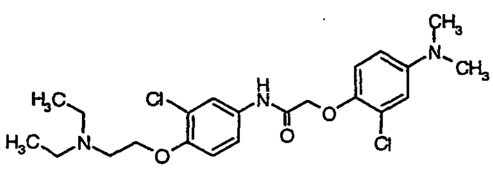

N-[3-Chlor-4-(2-diethylamino-ethoxy)-phenyl]-2-(2-chlor-4-dimethylamino-phenoxy)-acetamid

(20)

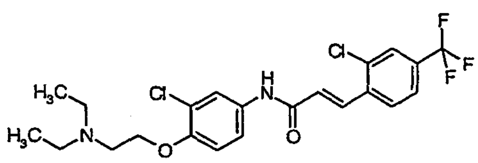

(E)-N-[3-Chlor-4-(2-diethylamino-ethoxy)-phenyl]-3-(2-chlor-4-trifluormethyl-phenyl)-acrylamid

(21)

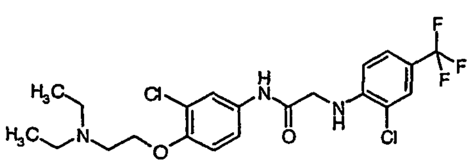

N-[3-Chlor-4-(2-diethylamino-ethoxy)-phenyl]-2-(2-chlor-4-trifluormethyl-phenylamino)-acetamid

(22)

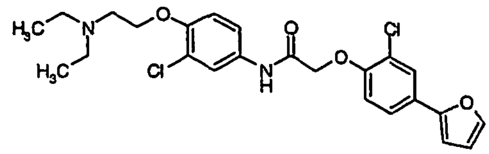

N-[3-Chlor-4-(2-diethylamino-ethoxy)-phenyl]-2-(2-chlor-4-furan-2-yl-phenoxy)-acetamid

(23)

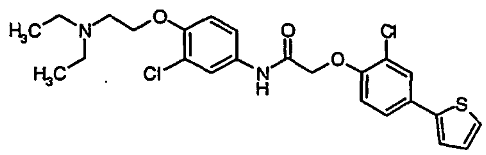

N-[3-Chlor-4-(2-diethylamino-ethoxy)-phenyl]-2-(2-chlor-4-thiophen-2-yl-phenoxy)-acetamid

(24)

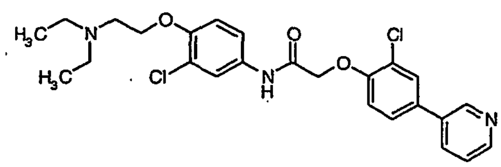

N-[3-Chlor-4-(2-diethylamino-ethoxy)-phenyl]-2-(2-chlor-4-pyridin-3-yl-phenoxy)-acetamid

(25)

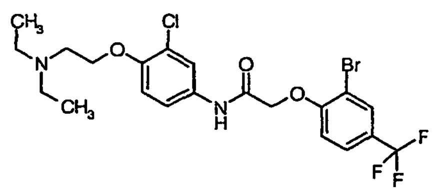

2-(2-Brom-4-trifluormethyl-phenoxy)-N-[3-chlor-4-(2-diethylamino-ethoxy)-phenyl]-acetamid

(26)

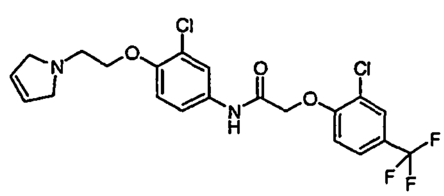

N-{3-Chlor-4-[2-(2,5-dihydro-pyrrol-1-yl)-ethoxy]-phenyl}-2-(2-chlor-4-trifluormethyl-phenoxy)-acetamid

(27)

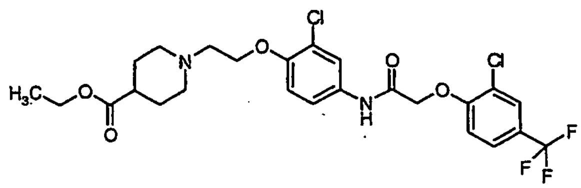

1-(2-{2-Chlor-4-[2-(2-chlor-4-trifluormethyl-phenoxy)-acetylamino]-phenoxy}-ethyl)-piperidin-4-carbonsäure-ethylester

(28)

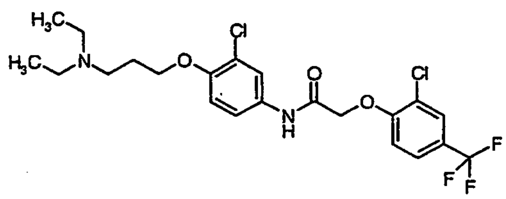

N-[3-Chlor-4-(3-diethylamino-propoxy)-phenyl]-2-(2-chlor-4-trifluormethyl-phenoxy)-acetamid

(29)

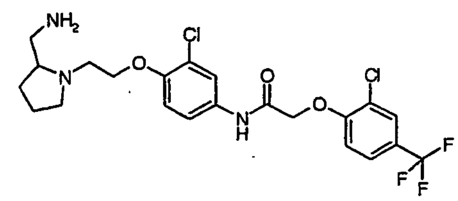

N-{4-[2-(2-Aminomethyl-pyrrolidin-1-yl)-ethoxy]-3-chlor-phenyl}-2-(2-chlor-4-trifluormethyl-phenoxy)-acetamid

(30)

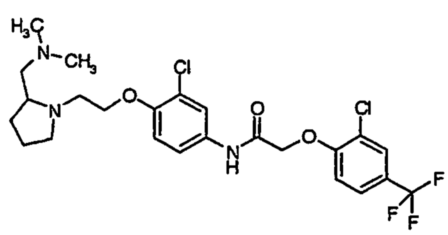

N-{3-Chlor-4-[2-(2-dimethylaminomethyl-pyrrolidin-1-yl)-ethoxy]-phenyl}-2-(2-chlor-4-trifluormethyl-phenoxy)-acetamid

(31)

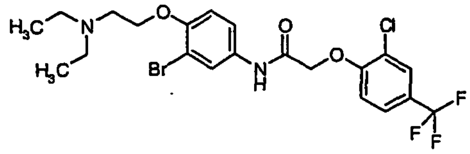

N-[3-Brom-4-(2-diethylamino-ethoxy)-phenyo-2-(2-chlor-4-trifluormethyl-phenoxy)-acetamid

(32)

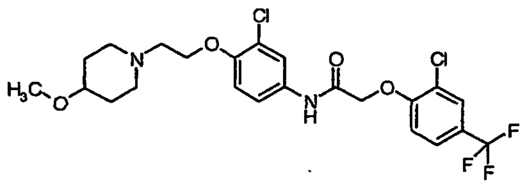

N-{3-Chlor-4-[2-(4-methoxy-piperidin-1-yl)-ethoxy]-phenyl}-2-(2-chlor-4-trifluormethyl-phenoxy)-acetamid

(33)

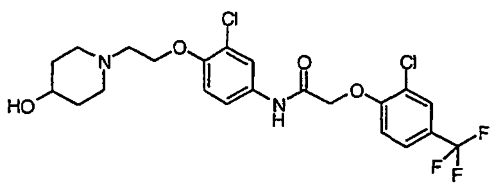

N-{3-Chlor-4-[2-(4-hydroxy-piperidin-1-yl)-ethoxy]-phenyl}-2-(2-chlor-4-trifluormethyl-phenoxy)-acetamid

(34)

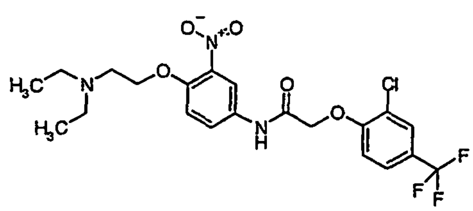

2-(2-Chlor-4-trifluormethyl-phenoxy)-N-[4-(2-diethylamino-ethoxy)-3-nitro-phenyl]-acetamid

(35)

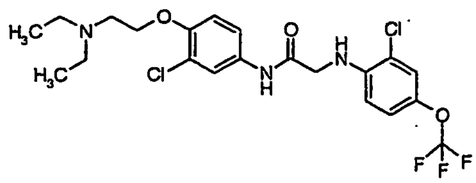

N-[3-Chlor-4-(2-diethylamino-ethoxy)-phenyl]-2-(2-chlor-4-trifluormethoxy-phenylamino)-acetamid

(36)

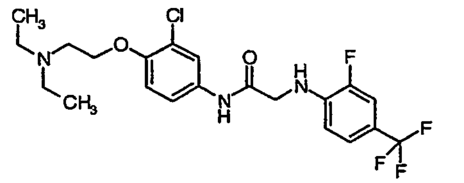

N-[3-Chlor-4-(2-diethylamino-ethoxy)-phenyl]-2-(2-fluor-4-trifluormethylphenylamino)-acetamid

(37)

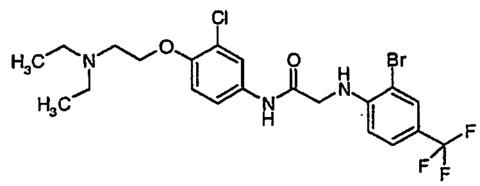

2-(2-Brom-4-trifluormethyl-phenylamino)-N-[3-chlor-4-(2-diethylamino-ethoxy)-phenyl]-acetamid

(38)

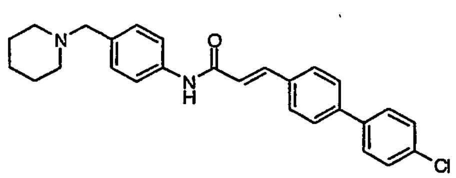

(E)-3-(4'-Chlor-biphenyl-4-yl)-N-(4-piperidin-1-ylmethyl-phenyl)-acrylamid

(39)

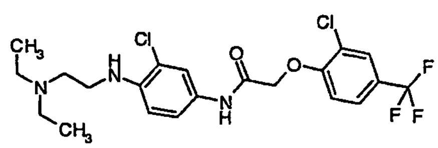

N-[3-Chlor-4-(2-diethylamino-ethylamino)-phenyl]-2-(2-chlor-4-trifluormethyl-phenoxy)-acetamid

(40)

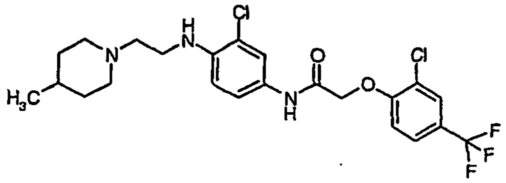

N-{3-Chlor-4-[2-(4-methyl-piperidin-1-yl)-ethylamino]-phenyl}-2-(2-chlor-4-trifluormethyl-phenoxy)-acetamid

(41)

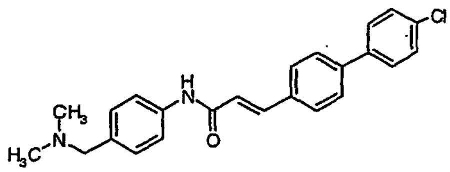

(E)-3-(4'-Chlor-biphenyl-4-yl)-N-(4-dimethylaminomethyl-phenyl)-acrylamid

(42)

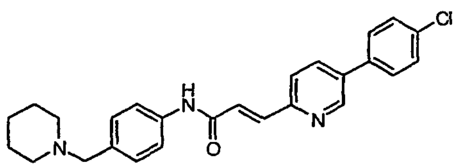

(E)-3-[5-(4-Chlor-phenyl)-pyridin-2-yl]-N-(4-piperidin-1-ylmethyl-phenyl)-acrylamid

(43)

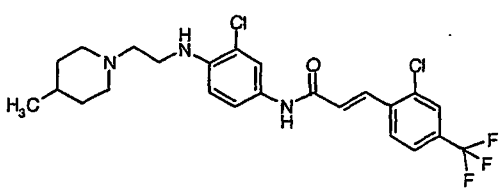

(E)-N-{3-Chlor-4-[2-(4-methyl-piperidin-1-yl)-ethylamino]-phenyl}-3-(2-chlor-4-trifluormethyl-phenyl)-acrylamid

(44)

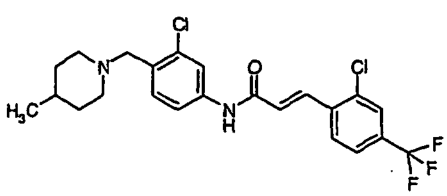

(E)-N-[3-Chlor-4-(4-methyl-piperidin-1-ylmethyl)-phenyl]-3-(2-chlor-4-trifluormethyl-phenyl)-acrylamid

(45)

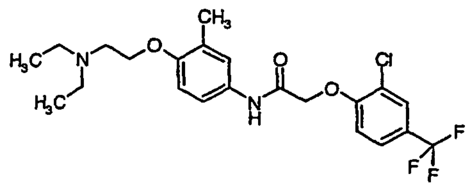

2-(2-Chlor-4-trifluormethyl-phenoxy)-N-[4-(2-diethylamino-ethoxy)-3-methyl-phenyl]-acetamid

(46)

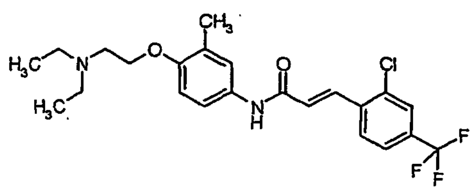

(E)-3-(2-Chlor-4-trifluormethyl-phenyl)-N-[4-(2-diethylamino-ethoxy)-3-methyl-phenyl]-acrylamid

(47)

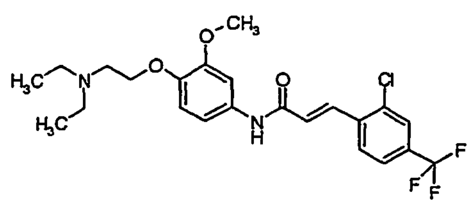

(E)-3-(2-Chlor-4-trifluormethyl-phenyl)-N-[4-(2-diethylamino-ethoxy)-3-methoxy-phenyl]-acrylamid

(48)

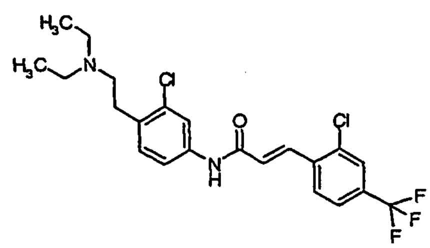

(E)-N-[3-Chlor-4-(2-diethylamino-ethyl)-phenyl]-3-(2-chlor-4-trifluormethyl-phenyl)-acrylamid

(49)

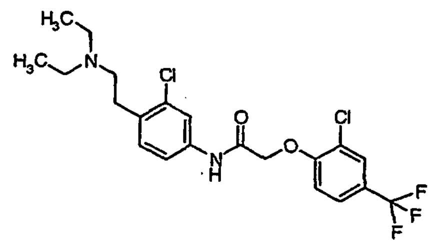

N-[3-Chlor-4-(2-diethylamino-ethyl)-phenyl]-2-(2-chlor-4-trifluormethyl-phenoxy)-acetamid

(50)

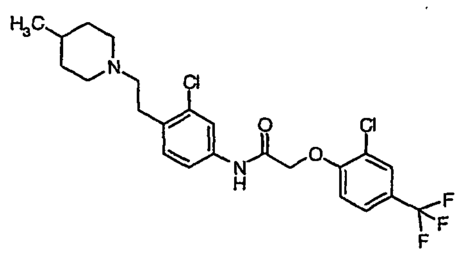

N-{3-Chlor-4-[2-(4-methyl-piperidin-1-yl)-ethyl]-phenyl}-2-(2-chlor-4-trifluormethyl-phenoxy)-acetamid

einschließlich deren Salze.

Im folgenden werden Begriffe, die zuvor und nachfolgend zur Beschreibung der erfindungsgemäßen Verbindungen verwendet werden, näher definiert.

Die Bezeichnung Halogen bezeichnet ein Atom ausgewählt aus der Gruppe bestehend aus F, Cl, Br und I, insbesondere F, Cl und Br.

Die Bezeichnung $C_{1-n}$-Alkyl, wobei n einen Wert von 3 bis 8 besitzt, bedeutet eine gesättigte, verzweigte oder unverzweigte Kohlenwasserstoffgruppe mit 1 bis n C-Atomen. Beispiele solcher Gruppen umfassen Methyl, Ethyl, n-Propyl, iso-Propyl, Butyl, iso-Butyl, sec-Butyl, tert-Butyl, n-Pentyl, iso-Pentyl, neo-Pentyl, tert-Pentyl, n-Hexyl, iso-Hexyl, etc..

Die Bezeichnung $C_{1-n}$-Alkylen, wobei n einen Wert von 1 bis 8 besitzen kann, bedeutet eine gesättigte, verzweigte oder unverzweigte Kohlenwasserstoffbrücke mit 1 bis n C-Atomen. Beispiele solcher Gruppen umfassen Methylen ($-CH_2-$), Ethylen ($-CH_2-CH_2-$), 1-Methyl-ethylen ($-CH(CH_3)-CH_2-$), 1,1-Dimethyl-ethylen ($-C(CH_3)_2-CH_2-$), n-Prop-1,3-ylen ($-CH_2-CH_2-CH_2-$), 1-Methylprop-1,3-ylen ($-CH(CH_3)-CH_2-CH_2-$), 2-Methylprop-1,3-ylen ($-CH_2-CH(CH_3)-CH_2-$), etc., sowie die entsprechenden spiegelbildlichen Formen.

Der Begriff $C_{2-n}$-Alkenyl, wobei n einen Wert von 3 bis 6 besitzt, bezeichnet eine verzweigte oder unverzweigte Kohlenwasserstoffgruppe mit 2 bis n C-Atomen und einer C=C-Doppelbindung. Beispiele solcher Gruppen umfassen Vinyl, 1-Propenyl, 2-Propenyl, iso-Propenyl, 1-Butenyl, 2-Butenyl, 3-Butenyl, 2-Methyl-1-propenyl, 1-Pentenyl, 2-Pentenyl, 3-Pentenyl, 4-Pentenyl, 3-Methyl-2-butenyl, 1-Hexenyl, 2-Hexenyl, 3-Hexenyl, 4-Hexenyl-, 5-Hexenyl etc..

Der Begriff $C_{2-n}$-Alkinyl, wobei n einen Wert von 3 bis 6 besitzt, bezeichnet eine verzweigte oder unverzweigte Kohlenwasserstoffgruppe mit 2 bis n C-Atomen und einer C≡C-Dreifachbindung. Beispiele solcher Gruppen umfassen Ethinyl, 1-Propinyl, 2-Propinyl, iso-Propinyl, 1-Butinyl, 2-Butinyl, 3-Butinyl, 2-Methyl-1-propinyl, 1-Pentinyl, 2-Pentinyl, 3-Pentinyl, 4-Pentinyl, 3-Methyl-2-butinyl, 1-Hexinyl, 2-Hexinyl, 3-Hexinyl, 4-Hexinyl-, 5-Hexinyl etc..

Der Begriff $C_{1-n}$-Alkoxy bezeichnet eine $C_{1-n}$-Alkyl-O-Gruppe, worin $C_{1-n}$-Alkyl wie oben definiert ist. Beispiele solcher Gruppen umfassen Methoxy, Ethoxy, n-Propoxy, iso-Propoxy, n-Butoxy, iso-Butoxy, sec-Butoxy, tert-Butoxy, n-Pentoxy, iso-Pentoxy, neo-Pentoxy, tert-Pentoxy, n-Hexoxy, iso-Hexoxy etc..

Der Begriff $C_{1-n}$-Alkylthio bezeichnet eine $C_{1-n}$-Alkyl-S-Gruppe, worin $C_{1-n}$-Alkyl wie oben definiert ist. Beispiele solcher Gruppen umfassen Methylthio, Ethylthio, n-Propylthio, iso-Propylthio, n-Butylthio, iso-Butylthio, sec-Butylthio, tert-Butylthio, n-Pentylthio, iso-Pentylthio, neo-Pentylthio, tert-Pentylthio, n-Hexylthio, iso-Hexylthio, etc..

Der Begriff $C_{1-n}$-Alkylcarbonyl bezeichnet eine $C_{1-n}$-Alkyl-C(=O)-Gruppe, worin $C_{1-n}$-Alkyl wie oben definiert ist. Beispiele solcher Gruppen umfassen Methylcarbonyl, Ethylcarbonyl, n-Propylcarbonyl, iso-Propylcarbonyl, n-Butylcarbonyl, iso-Butylcarbonyl, sec-Butylcarbonyl, tert-Butylcarbonyl, n-Pentylcarbonyl, iso-Pentylcarbonyl, neo-Pentylcarbonyl, tert-Pentylcarbonyl, n-Hexylcarbonyl, iso-Hexylcarbonyl, etc..

Der Begriff $C_{3-n}$-Cycloalkyl bezeichnet eine gesättigte mono-, bi-, tri- oder spirocarbocyclische Gruppe mit 3 bis n C-Atomen. Beispiele solcher Gruppen umfassen Cyclopropyl, Cyclobutyl, Cyclopentyl, Cyclohexyl, Cycloheptyl, Cyclooctyl, Cyclononyl, Cyclododecyl, Bicyclo[3.2.1.]octyl, Spiro[4.5]decyl, Norpinyl, Norbonyl, Norcaryl, Adamantyl, etc..

Der Begriff $C_{5-n}$-Cycloalkenyl bezeichnet eine einfach ungesättigte mono-, bi-, tri- oder spirocarbocyclische Gruppe mit 5 bis n C-Atomen. Beispiele solcher Gruppen umfassen Cyclopentenyl, Cyclohexenyl, Cycloheptenyl, Cyclooctenyl, Cyclononenyl, etc..

Der Begriff $C_{3-n}$-Cycloalkylcarbonyl bezeichnet eine $C_{3-n}$-Cycloalkyl-C(=O)-Gruppe, worin $C_{3-n}$-Cycloalkyl wie oben definiert ist.

Der Begriff Aryl bezeichnet ein carbocyclisches, aromatisches Ringsystem, wie beispielsweise Phenyl, Biphenyl, Naphthyl, Anthracenyl, Phenanthrenyl, Fluorenyl, Indenyl, Pentalenyl, Azulenyl, Biphenylenyl, etc.. Eine besonders bevorzugte Bedeutung von "Aryl" ist Phenyl.

Der Begriff Cyclo-$C_{3-7}$-alkylenimino- bezeichnet einen 4- bis 7-gliedrigen Ring, der 3 bis 7 Methylen-Einheiten sowie eine Imino-Gruppe aufweist, wobei die Bindung zum Rest des Moleküls über die Imino-Gruppe erfolgt.

Der Begriff Cyclo-$C_{3-7}$-alkylenimino-carbonyl bezeichnet einen zuvor definierten Cyclo-$C_{3-7}$-alkylenimino-Ring, der über die Imino-Gruppe mit einer Carbonyl-Gruppe verbunden ist.

Der in dieser Anmeldung verwendete Begriff Heteroaryl bezeichnet ein heterocyclisches, aromatisches Ringsystem, das neben mindestens einem C-Atom ein oder mehrere Heteroatome ausgewählt aus N, O und/oder S umfasst. Beispiele solcher Gruppen sind Furanyl, Thiophenyl, Pyrrolyl, Oxazolyl, Thiazolyl, Imidazolyl, Isoxazolyl, Isothiazolyl, 1,2,3-Triazolyl, 1,3,5-Triazolyl, Pyranyl, Pyridyl, Pyridazinyl, Pyrimidinyl, Pyrazinyl, 1,2,3-Triazinyl, 1,2,4-Triazinyl, 1,3,5-Triazinyl, 1,2,3-Oxadiazolyl, 1,2,4-Oxadiazolyl, 1,2,5-Oxadiazolyl, 1,3,4-Oxadiazolyl, 1,2,3-Thiadiazolyl, 1,2,4-Thiadiazolyl, 1,2,5-Thiadiazolyl, 1,3,4-Thiadiazolyl, Tetrazolyl, Thiadiazinyl, Indolyl, Isoindolyl, Benzofuranyl, Benzothiophenyl (Thianaphthenyl), Indazolyl, Benzimidazolyl, Benzthiazolyl, Benzisothiazolyl, Benzoxazolyl, Benzisoxazolyl, Purinyl, Chinazolinyl, Chinozilinyl, Chinolinyl, Isochinolinyl, Chinoxalinyl, Naphthyridinyl, Pteridinyl, Carbazolyl, Azepinyl, Diazepinyl, Acridinyl, etc.. Der Begriff Heteroaryl umfasst auch die partiell hydrierten Vertreter heterocyclischer, aromatischer Ringsysteme, insbesondere der oben aufgeführten Ringsysteme. Beispiele solcher partiell hydrierten Heterocyclen sind 2,3-Dihydrobenzofuranyl, Pyrolinyl, Pyrazolinyl, Indolinyl, Oxazolidinyl, Oxazolinyl, Oxazepinyl, etc.. Besonders bevorzugt bedeutet Heteroaryl ein heteroaromatisches mono- oder bicyclisches Ringsystem.

Begriffe, wie Aryl-$C_{1-n}$-alkyl, Heteroaryl-$C_{1-n}$-alkyl, etc. bezeichnen $C_{1-n}$-Alkyl, wie oben definiert, das mit einer Aryl- oder Heteroaryl-Gruppe substituiert ist.

Manche der zuvor angeführten Begriffe können mehrfach in der Definition einer Formel oder Gruppe verwendet werden und besitzen jeweils unabhängig voneinander eine der angegebenen Bedeutungen.

Der Begriff "ungesättigt", beispielsweise in "ungesättigte carbocyclische Gruppe" oder "ungesättigte heterocyclische Gruppe", wie er insbesondere in der Definition der Gruppe Cy verwendet wird, umfasst neben den einfach oder mehrfach ungesättigten Gruppen auch die entsprechenden vollständig ungesättigten Gruppen, insbesondere jedoch die ein- und zweifach ungesättigten Gruppen.

Der in dieser Anmeldung verwendete Begriff "gegebenenfalls substituiert" bedeutet, dass die so bezeichnete Gruppe entweder unsubstituiert oder ein- oder mehrfach mit den angegebenen Substituenten substituiert ist. Falls die betreffende Gruppe mehrfach substituiert ist, so können die Substituenten gleich oder verschieden sein.

Das H-Atom einer vorhandenen Carboxygruppe oder ein an ein N-Atom gebundenes H-Atom (Imino- oder Amino-Gruppe) kann jeweils durch einen in-vivo abspaltbaren Rest ersetzt sein. Unter einem von einem N-Atom in-vivo abspaltbaren Rest versteht man beispielsweise eine Hydroxygruppe, eine Acylgruppe wie die Benzoyl- oder Pyridinoylgruppe oder eine $C_{1-16}$-Alkanoylgruppe wie die Formyl-, Acetyl-, Propionyl-, Butanoyl-, Pentanoyl- oder Hexanoylgruppe, eine Allyloxycarbonylgruppe, eine $C_{1-16}$-Alkoxycarbonylgruppe wie die Methoxycarbonyl-, Ethoxycarbonyl-, Propoxycarbonyl-, Isopropoxycarbonyl-, Butoxycarbonyl-, tert.Butoxycarbonyl-, Pentoxycarbonyl-, Hexyloxycarbonyl-, Octyloxycarbonyl-, Nonyloxycarbonyl-, Decyloxycarbonyl-, Undecyloxycarbonyl-, Dodecyloxycarbonyl- oder Hexadecyloxycarbonylgruppe, eine Phenyl-$C_{1-6}$-alkoxycarbonylgruppe wie die Benzyloxycarbonyl-, Phenylethoxycarbonyl- oder Phenylpropoxycarbonylgruppe, eine $C_{1-3}$-Alkylsulfonyl-$C_{2-4}$-alkoxycarbonyl-, $C_{1-3}$-Alkoxy-$C_{2-4}$-alkoxy-$C_{2-4}$-alkoxycarbonyl- oder $R_eCO$-O-($R_fCR_g$)-O-CO-Gruppe, in der

$R_e$ eine $C_{1-8}$-Alkyl-, $C_{5-7}$-Cycloalkyl-, Phenyl- oder Phenyl- $C_{1-3}$-alkylgruppe,

$R_f$ ein Wasserstoffatom, eine $C_{1-3}$-Alkyl-, $C_{5-7}$-Cycloalkyl- oder Phenylgruppe und

$R_g$ ein Wasserstoffatom, eine $C_{1-3}$-Alkyl- oder $R_eCO$-O-($R_fCR_g$)-O-Gruppe, in der

$R_e$ bis $R_g$ wie vorstehend erwähnt definiert sind, darstellen,

wobei zusätzlich für eine Aminogruppe die Phthalimidogruppe in Betracht kommt, wobei die vorstehend erwähnten Esterreste ebenfalls als in-vivo in eine Carboxygruppe überführbare Gruppe verwendet werden können.

Die zuvor beschriebenen Reste und Substituenten können in der beschriebenen Weise ein- oder mehrfach mit Fluor substituiert sein. Bevorzugte fluorierte Alkylreste sind Fluormethyl, Difluormethyl und Trifluormethyl. Bevorzugte

fluorierte Alkoxyreste sind Fluormethoxy, Difluormethoxy und Trifluormethoxy. Bevorzugte fluorierte Alkylsulfinyl- und Alkylsulfonylgruppen sind Trifluormethylsulfinyl und Trifluormethylsulfonyl.

Die erfindungsgemäßen Verbindungen der allgemeinen Formel I können Säuregruppen besitzen, hauptsächlich Carboxylgruppen, und/oder basische Gruppen wie z.B. Aminofunktionen. Verbindungen der allgemeinen Formel I können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Die erfindungsgemäßen Verbindungen sind unter Anwendung im Prinzip bekannter Syntheseverfahren erhältlich. Bevorzugt werden die Verbindungen in analoger Anwendung zu den nachfolgend näher erläuterten Herstellungsverfahren erhalten, die ebenfalls Gegenstand dieser Erfindung darstellen. Nachfolgend verwendete Abkürzungen sind in der Einleitung zum experimentellen Teil definiert oder dem Fachmann als solche geläufig. Der in den Reaktionsschemata A, B und C verwendete Begriff Alkyl bedeutet $C_{1-4}$-Alkyl, sofern nicht anders angegeben.

Enthalten nachfolgend aufgeführte Ausgangsstoffe oder Zwischenprodukte Reste $R^1$, $R^2$, $R^3$, X, Y, Z, A oder B mit Aminfunktionen, so werden diese vorzugsweise in geschützter Form, beispielsweise mit einer Boc-, Fmoc- oder Cbz-Schutzgruppe, eingesetzt und am Ende der Reaktionen nach Standardmethoden freigesetzt.

Gemäß dem allgemeinen Reaktionsschema A wird ein Halogenessigsäure-alkylester (A-1), bevorzugt gegebenenfalls substituierter Bromessigsäure-ethylester, mit einer Hydroxy-verbindung (A-2), beispielsweise einem Phenol, in einem molaren Verhältnis von 1:1 bis 2:1 in einem geeigneten Lösungsmittel in Anwesenheit einer geeigneten Base umgesetzt. Geeignete Basen sind insbesondere tertiäre Amine wie Triethylamin oder Hünigbase sowie Alkalicarbonate, beispielsweise Kaliumcarbonat. Die Reaktionen erfolgen in einem geeigneten Lösungsmittel, wobei bevorzugt DMF verwendet wird.

Die Umsetzung erfolgt in einem Zeitraum von 4 bis 24 Stunden in einem Temperaturbereich von RT bis 120 °C, bevorzugt 60 °C bis 100 °C.

Die so erhaltenen Aryloxy-essigsäure-alkylester (A-3) werden nach Aufreinigung zur entsprechenden Aryloxy-essigsäuren (A-4) hydrolysiert. Die Reaktion wird bevorzugt in Ethanol-Wasser-Gemischen in Anwesenheit von einem Überschuss Alkalihydroxiden, beispielsweise 2-5 äquiv. Natriumhydroxid, durchgeführt. Die Umsetzung erfolgt in einem Zeitraum von 1 bis 4 Stunden in einem Temperaturbereich von RT bis 80 °C.

Die Aryloxy-essigsäuren (A-4) werden nach der Aufreinigung mit einem Anilin (A-5) zum Amid (A-6) gekuppelt. Die dazu notwendige Aktivierung der Essigsäure erfolgt bevorzugt über ein gemischtes Anhydrid oder mit Hilfe von Kupplungsreagenzien. Das gemischte Anhydrid der jeweiligen Aryloxy-essigsäure (A-4) wird bevorzugt durch Reaktion der Essigsäure mit einem Überschuss Chlorameisensäure-alkylester, bevorzugt Chlorameisensäure-isopropylester in einem molaren Verhältnis von 1:1 bis 1:1.2, hergestellt. Als Basen werden bevorzugt tertiäre Amine, beispielsweise N-Methylmorpholin, verwendet, die äquimolar zum jeweiligen Chlorameisensäure-alkylester eingesetzt werden.

Die Umsetzung wird in einem geeigneten Lösungsmittel wie THF bei Temperaturen zwischen -10 °C und -5 °C durchgeführt und erfolgt in einem Zeitraum von 10 bis 30 Minuten.

Das so erhaltene gemischte Anhydrid wird bevorzugt ohne weitere Reinigung mit einer Amin-Verbindung (A-5), beispielsweise einem Anilin, umgesetzt. Das Anilin wird im Überschuss zur jeweiligen Essigsäure (A-4), bevorzugt 5-10 mol%, eingesetzt. Die Umsetzung wird beispielsweise bei RT in einem Zeitraum von 1 bis 4 Stunden durchgeführt. Eine weitere bevorzugte Umsetzung liefert das Amid (A-6) durch Kupplung des Anilins (A-5) mit der entsprechenden Aryloxy-essigsäure (A-4) mit Hilfe von Peptidkupplungsreagentien in einem geeigneten Lösungsmittel unter Verwendung einer geeigneten Base. Die Aryloxy-essigsäure (A-4) und ein Anilin (A-5) werden bevorzugt in einem molaren Verhältnis von 1.5:1 bis 1:1.5 eingesetzt. Als Peptidkupplungsreagens wird beispielsweise TBTU verwendet, wobei dieses äquimolar oder im Überschuss, bevorzugt äquimolar bis 50 mol% Überschuss, eingesetzt wird. Alternativ kann die Umsetzung auch in Gegenwart einer zum TBTU äquimolaren Menge an HOBt durchgeführt werden. Bevorzugte Lösungsmittel sind THF und DMF in einem Temperaturbereich von RT bis 80 °C, bevorzugt RT bis 40 °C. Als Basen werden bevorzugt tertiäre Amine wie Triethylamin oder Hünigbase eingesetzt.

Reaktionsschema A: (W in der Bedeutung $-CH_2-O-$)

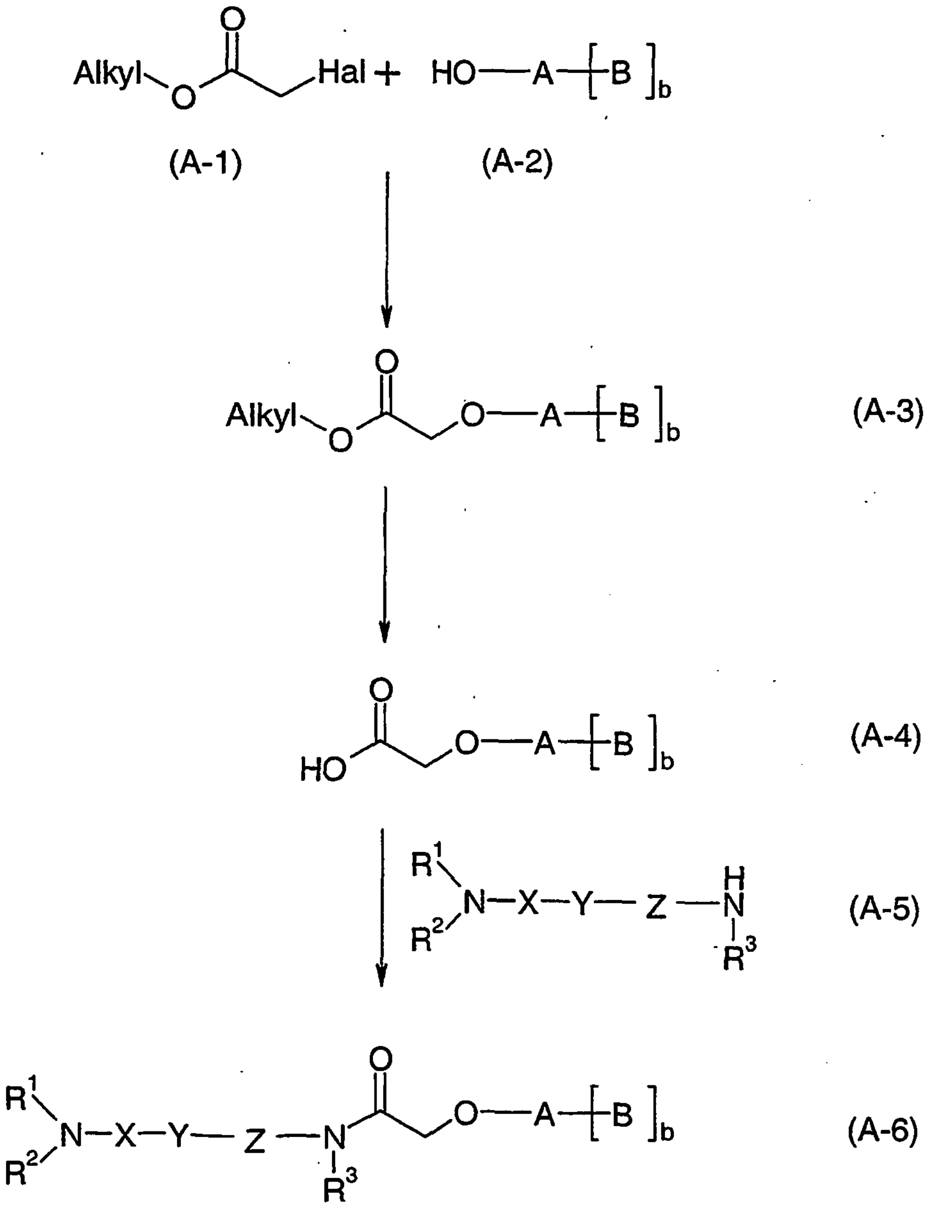

(A-1) (A-2) (A-3) (A-4) (A-5) (A-6)

Gemäß dem allgemeinen Reaktionsschema B wird ein Halogenessigsäure-alkylester (B-1), bevorzugt Bromessigsäure-ethylester, mit einer Amin-Verbindung (B-2), beispielsweise einem Anilin, im Überschuss, bevorzugt in einem molaren Verhältnis von 1:1 bis 1.2:1, in einem geeigneten Lösungsmittel in Anwesenheit einer geeigneten Base umgesetzt. Geeignete Basen sind insbesondere tertiäre Amine wie Hünigbase. Als Lösungsmittel wird bevorzugt Hünigbase, DMF oder deren Gemische verwendet. Die Umsetzung erfolgt in einem Zeitraum von 4 bis 48 Stunden in einem Temperaturbereich von 90 °C und 130 °C. Die weitere Umsetzung des Arylamino-essigsäure-ethylesters (B-3) erfolgte analog zum allgemeinen Reaktionsschema A durch Hydrolyse zur Arylamino-essigsäure (B-4). Das Amid (B-6) wird bevorzugt aus der Essigsäure (B-4) und dem Anilin (B-5) unter Verwendung von TBTU und einer geeigneten Base in einem geeigneten Lösungsmittel hergestellt. Die Aryloxy-essigsäure (B-4) und das Anilin (B-5) werden bevorzugt in einem molaren Verhältnis von 1.5:1 bis 1:1.5 eingesetzt. Als Peptidkupplungsreagens wird beispielsweise TBTU verwendet, wobei dieses äquimolar oder im Überschuss, bevorzugt äquimolar bis 50 mol% Überschuss, eingesetzt wird. Bevorzugtes Lösungsmittel ist DMF in einem Temperaturbereich von RT bis 80 °C, bevorzugt RT bis 40 °C. Als Basen werden bevorzugt tertiäre Amine wie Hünigbase eingesetzt.

Reaktionsschema B: (W in der Bedeutung $-CH_2-NR^8-$ am Beispiel $R^8 = H$)

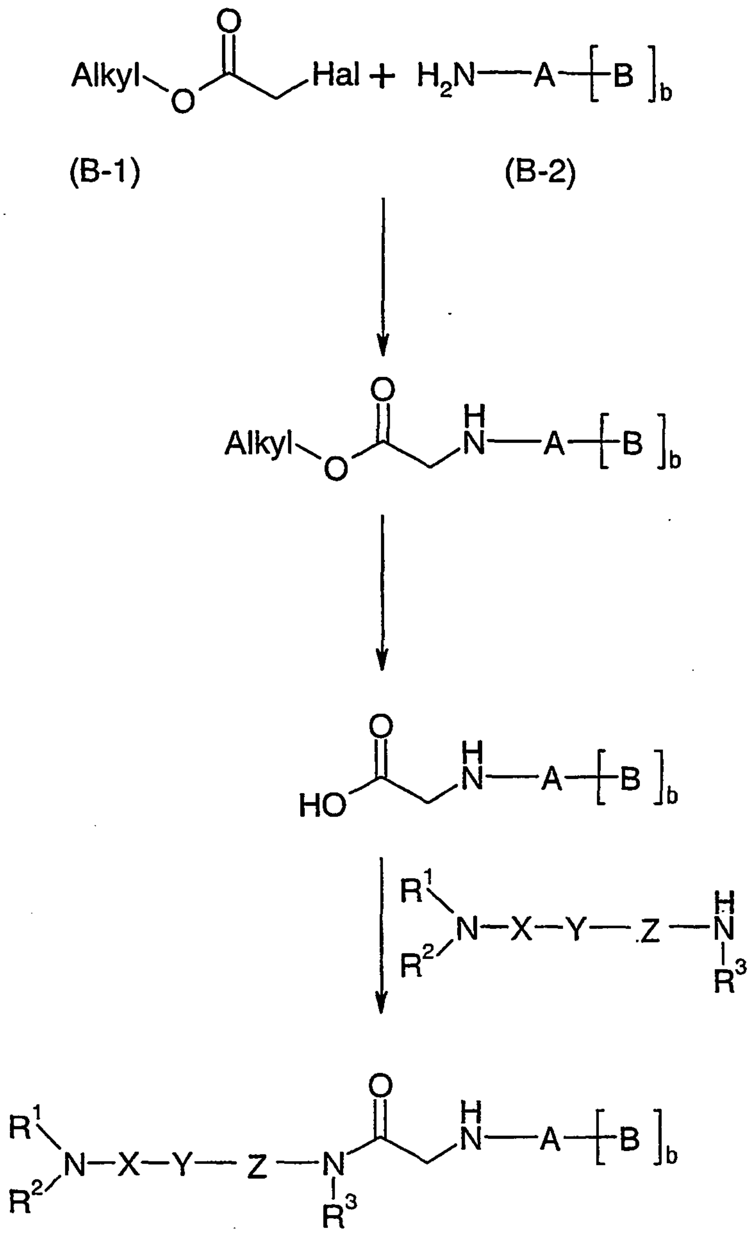

(B-1) (B-2)

(B-3)

(B-4)

(B-5)

(B-6)

Gemäß Reaktionsschema C wird ein Acrylsäureester (C-3), beispielweise ein gegebenenfalls substituierter Phenylacrylsäure-ethylester, durch Palladiumkatalysierte Umsetzung eines Acrylsäure-alkylesters (C-1) und einem (Hetero)Arylhalogenid (C-2) hergestellt. Bevorzugt werden (Hetero)Arylbromide und (Hetero)Aryliodide (C-2) im Überschuss zur Acrylsäure, bevorzugt in einem molaren Verhältnis von 1:1 bis 1:1.5, in die Reaktion eingesetzt. Als Lösungsmittel kann beispielsweise Acetonitril bei 80 °C verwendet werden. Als Palladiumkatalysator wird bevorzugt Palladium(II)acetat (1 mol%) in Kombination mit Tri-o-tolylphosphin (3 bis 4 mol%) verwendet. Geeignete Basen sind tertiäre Amine wie Triethylamin.

Der Acrylsäureester (C-3) wird nach Aufreinigung zur entsprechenden Acrylsäure (C-4) hydrolysiert. Die Reaktion wird bevorzugt in Ethanol-Wasser-Gemischen in Anwesenheit von Alkalihydroxiden wie Natriumhydroxid (200-300 mol%) durchgeführt. Die Umsetzung erfolgt in einem Zeitraum von 1 bis 4 Stunden bei Raumtemperatur.

Die Acrylsäure-Verbindung (C-4) wird nach der Aufreinigung mit einer Amin-Verbindung (C-5), beispielsweise einem Anilin, zum Acrylamid (C-6) gekuppelt. Die dazu notwendige Aktivierung der Acrylsäure (C-4) erfolgt analog zum

allgemeinen Reaktionsschema A bevorzugt über ein gemischtes Anhydrid oder mit Hilfe von Kupplungsreagenzien wie TBTU oder TBTU in Kombination mit HOBt.

**<u>Reaktionsschema C:</u> (W in der Bedeutung -CH=CH-)**

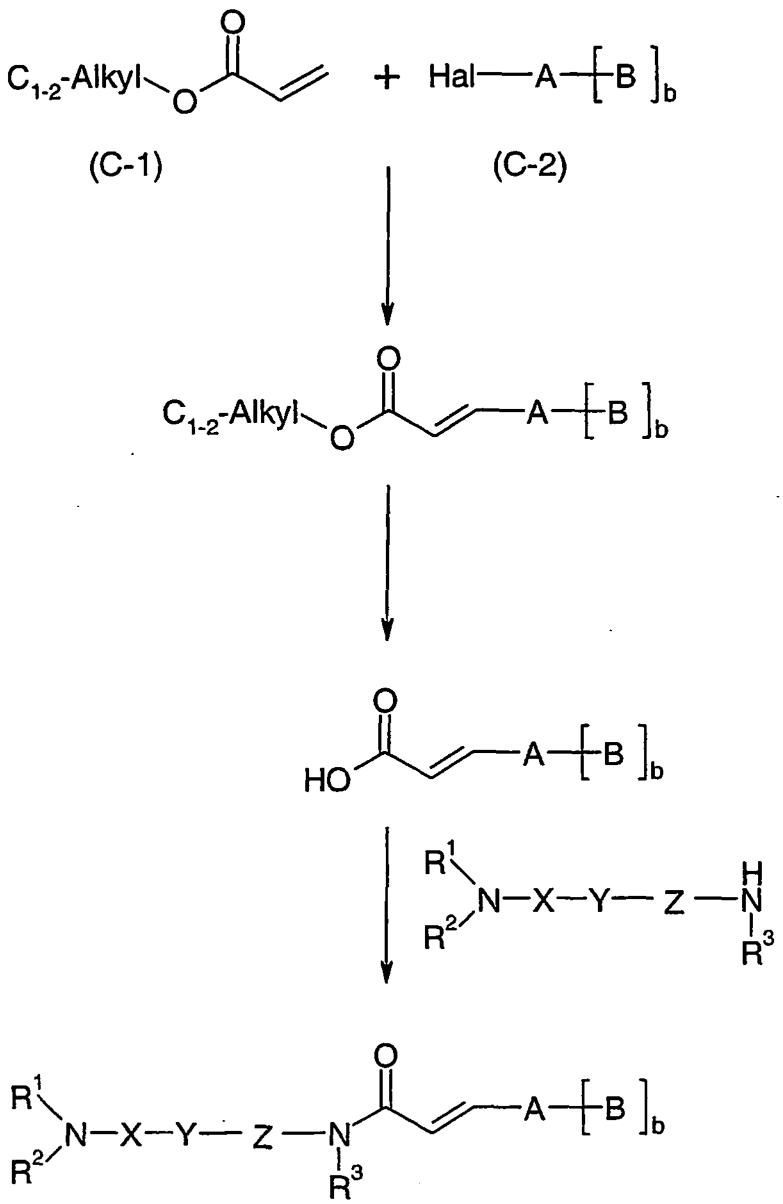

(C-1) (C-2)

(C-3)

(C-4)

(C-5)

(C-6)

Die erfindungsgemäßen Verbindungen sind vorteilhaft auch nach den in den nachfolgenden Beispielen beschriebenen Verfahren zugänglich, wobei diese hierzu auch mit dem Fachmann beispielsweise aus der Literatur bekannten Verfahren kombiniert werden können.

Stereoisomere Verbindungen der Formel (I) lassen sich prinzipiell nach üblichen Methoden trennen. Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel (I) fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit

einer optisch aktiven Säure, beispielsweise (+)- oder (-)-Weinsäure, (+)- oder (-)-Diacetylweinsäure, (+)- oder (-)-Monomethyltartrat oder (+)-Camphersulfonsäure, bzw. einer optisch aktiven Base, beispielsweise mit (*R*)-(+)-1-Phenylethylamin, (*S*)-(-)-1-Phenylethylamin oder (*S*)-Brucin, entstehen.

Nach einem üblichen Verfahren zur Isomerentrennung wird das Racemat einer Verbindung der allgemeinen Formel (I) mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (*R*)- oder (*S*)-Enantiomer bzw. ein Gemisch zweier optisch aktiver, unter die allgemeine Formel (I) fallender diastereomerer Verbindungen wird auch dadurch erhalten, dass man die oben beschriebenen Synthesen mit jeweils einer geeigneten (*R*)- bzw. (*S*)-konfigurierten Reaktionskomponente durchführt.

Wie vorstehend genannt, können die Verbindungen der Formel (I) in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel (I) mit anorganischen oder organischen Säuren vorliegen. Andererseits kann die Verbindung der Formel (I) im Falle von acidisch gebundenem Wasserstoff durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure, Ethansulfonsäure, Toluolsulfonsäure, Benzolsulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel (I) mit acidisch gebundenem Wasserstoff kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Die Verbindungen gemäß der vorliegenden Erfindung, einschließlich der physiologisch verträglichen Salze, besitzen eine Wirkung als Antagonisten des MCH-Rezeptors, insbesondere des MCH-1 Rezeptors, und zeigen gute Affinitäten in MCH-Rezeptorbindungsstudien. Pharmakologische Testsysteme für MCH-antagonistische Eigenschaften werden im nachfolgenden experimentellen Teil beschrieben.

Als Antagonisten des MCH-Rezeptors sind die erfindungsgemäßen Verbindungen vorteilhaft als pharmazeutische Wirkstoffe zur Prophylaxe und/oder Behandlung von Erscheinungen und/oder Krankheiten geeignet, die durch MCH verursacht werden oder mit MCH in einem anderen kausalen Zusammenhang stehen. Generell weisen die erfindungsgemäßen Verbindungen eine geringe Toxizität, eine gute orale Absorbierbarkeit und intracerebrale Transitivität, insbesondere Hirngängigkeit, auf.

Daher sind MCH-Antagonisten, die mindestens eine erfindungsgemäße verbindung aufweisen, besonders bei Säugetieren, wie beispielsweise Ratten, Mäusen, Meerschweinchen, Hasen, Hunden, Katzen, Schafen, Pferden, Schweinen, Rindern, Affen sowie Menschen, zur Behandlung und/oder Prophylaxe von Erscheinungen und/oder krankheiten, die durch MCH verursacht werden oder mit MCH in einem anderen kausalen Zusammenhang stehen, geeignet.

Krankheiten, die durch MCH verursacht werden oder mit MCH in einem anderen kausalen Zusammenhang stehen, sind insbesondere metabolische Störungen, wie beispielsweise Obesitas, und Essstörungen, wie beispielsweise Bulimie, einschließlich Bulimie nervosa. Die Indikation Obesitas umfasst vorallem exogener Obesitas, hyperinsulinärer Obesitas, hyperplasmischer Obesitas, hyperphysealer Adipositas, hypoplasmischer Obesitas, hypothyroider Obesitas, hypothalamischer Obesitas, symptomatischer Obesitas, infantiler Obesitas, Oberkörperobesitas, alimentärer Obesitas, hypogonadaler Obesitas, zentraler Obesitas. Des weiteren sind in diesem Indikationsumfeld auch Cachexia, Anorexie und Hyperphagia zu nennen.

Erfindungsgemäße Verbindungen können insbesondere geeignet sein, den Hunger zu reduzieren, Appetit zu zügeln, das Essverhalten zu kontrollieren und/oder ein Sättigungsgefühl hervorzurufen.

Darüber hinaus können zu den Krankheiten, die durch MCH verursacht werden oder mit MCH in einem anderen kausalen Zusammenhang stehen, auch Hyperlipidämie, Fettakkumulation, emotionale Störungen, Affektivitätsstörungen, Depressionen und Angstzuständen geeignet ist.

Erfindungsgemäße Verbindungen sind auch als Wirkstoffe zur Prophylaxe und/oder Behandlung weiterer Krankheiten und/oder Störungen, insbesondere solcher die mit Obesitas einhergehen, wie beispielsweise von Diabetes, Diabetes mellitus, insbesondere Typ II Diabetes, Hyperglykämie, Insbesondere chronischer Hyperglykämie, diabetischen Komplikationen, einschließlich diabetischer Retinopathie, diabetischer Neuropathie, diabetischer Nephropathie, etc., Insulin-Resistenz, pathologischer Glukosetoleranz, Herzinsuffizienz, Herzkreislauferkrankungen, insbesondere Arteriosklerose und Bluthochdruck, Arthritis und Gonitis geeignet ist.

Erfindungsgemäße MCH Antagonisten und Formulierungen können vorteilhaft in Kombination mit einer alimentären Therapie, wie beispielsweise einer alimentären Diabetes-Therapie, und Übung eingesetzt werden.

Die zur Erzielung einer entsprechenden Wirkung erforderliche Dosierung beträgt zweckmäßigerweise bei intravenöser oder subcutaner Gabe 0,001 bis 30 mg/kg Körpergewicht, vorzugsweise 0,01 bis 5 mg/kg Körpergewicht, und bei oraler, nasaler oder inhalativer Gabe 0,01 bis 50 mg/kg Körpergewicht, vorzugsweise 0,1 bis 30 mg/kg Körpergewicht, jeweils einmal bis dreimal täglich.

Hierzu lassen sich die erfindungsgemäß hergestellten Verbindungen der allgemeinen Formel I, gegebenenfalls in Kombination mit anderen Wirksubstanzen, wie sie nachfolgend näher beschrieben werden, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Oblaten, Pulver, Granulate, Lösungen, Emulsionen, Sirupe, Inhalationsaerosole, Salben, Suppositorien einarbeiten.

Neben Arzneimitteln umfasst die Erfindung auch Zusammensetzungen, enthaltend mindestens eine erfindungsgemäße Amid-Verbindung und/ oder ein erfindungsgemäßes Salz neben gegebenenfalls einem oder mehreren physiologisch verträglichen Hilfsstoffen. Solche Zusammensetzungen können beispielsweise auch Lebensmittel, die fest oder flüssig sein können, sein, in die die erfindungsgemäße Verbindung eingearbeitet ist.

Für die oben erwähnten Kombinationen kommen als weitere Wirksubstanzen insbesondere solche in Betracht, die beispielsweise die therapeutische Wirksamkeit eines erfindungsgemäßen MCH-Antagonisten im Hinblick auf eine der genannten Indikationen verstärken und/oder die eine Reduzierung der Dosierung eines erfindungsgemäßen MCH-Antagonisten erlauben. Vorzugsweise sind ein oder mehrere weiteren Wirksubstanzen ausgewählt aus der Gruppe bestehend aus

- Wirkstoffe zur Behandlung von Diabetes,
- Wirkstoffe zur Behandlung diabetischer Komplikationen,
- Wirkstoffe zur Behandlung von Obesitas, vorzugsweise andere als MCH-Antagonisten,
- Wirkstoffe zur Behandlung von Bluthochdruck,
- Wirkstoffe zur Behandlung von Hyperlipidemia, einschließlich Arteriosklerose,
- Wirkstoffe zur Behandlung von Arthritis,
- Wirkstoffe zur Behandlung von Angstzuständen,
- Wirkstoffe zur Behandlung von Depressionen.

Nachfolgend werden die zuvor genannten Wirkstoffklassen anhand von Beispielen näher erläutert.

Beispiele von Wirkstoffen zur Behandlung von Diabetes sind Insulin Sensibilisatoren, Insulin Sekretionsbeschleuniger, Biguanide, Insuline, α-Glucosidase Inhibitoren, β3 Adreno Rezeptor Agonisten.

Insulin Sensibilisatoren umfassen Pioglitazone und seine Salze (vorzugsweise Hydrochloride), Troglitazone, Rosiglitazone und seine Salze (vorzugsweise Maleate), JTT-501, GI-262570, MCC-555, YM-440, DRF-2593, BM-13-1258, KRP-297, R-119702, GW-1929.

Insulin Sekretionsbeschleuniger umfassen Sulfonylharnstoffe, wie beispielsweise Tolbutamide, Chlorpropamide, Tolzamide, Acetohexamide, Glyclopyramide und seine Ammonium-Salze, Glibenclamide, Gliclazide, Glimepiride. Weitere Beispiele von Insulin Sektretionsbeschleunigern sind Repaglinide, Nateglinide, Mitiglinide (KAD-1229), JTT-608.

Biguanide umfassen Metformin, Buformin, Phenformin.

Insuline umfassen aus Tieren, insbesondere Rindern oder Schweinen, gewonnene Insuline, halbsynthetische Human-Insuline, die enzymatisch aus tierisch gewonnenem Insulin synthetisiert werden, Human-Insulin, das gentechnologisch, beispielsweise aus Escherichia coli oder Hefen, erhalten wird.

Ferner wird als Insulin Insulin-Zink (enthaltend 0,45 bis 0,9 Gewichtsprozent Zink) und Protamin-Insulin-Zink erhältlich aus Zinkchlorid, Protaminsulfat und Insulin, verstanden. Darüber hinaus kann Insulin aus Insulin-Fragmenten oder Derivaten (beispielsweise INS-1, etc.) erhalten werden.

Insulin kann auch unterschiedliche Arten umfassen, beispielsweise bezüglich der Eintrittszeit und Dauer der Wirkung ("ultra immediate action type", "immediate action type", "two phase type", "intermediate type", "prolonged action type", etc.), die in Abhängigkeit vom pathologischen Zustand der Patienten ausgewählt werden.

α-Glucosidase Inhibitoren umfassen Acarbose, Voglibose, Miglitol, Emiglitate.

β3 Adreno Rezeptor Agonisten umfassen AJ-9677, BMS-196085, SB-226552, AZ40140.

Andere als die zuvor genannten Wirkstoffe zur Behandlung von Diabetes umfassen Ergoset, Pramlintide, Leptin, BAY-27-9955 sowie Glykogen Phosphorylase Inhibitoren, Sorbitol Dehydrogenase Inhibitoren, Protein Tyrosin Phosphatase 1 B Inhibitoren, Dipeptidyl Protease Inhibitoren, Glipizid, Glyburide.

Wirkstoffe zur Behandlung diabetischer Komplikationen umfassen beispielsweise Aldose Reduktase Inhibitoren, Glykations Inhibitoren, Protein Kinase C Inhibitoren.
Aldose Reduktase Inhibitoren sind beispielsweise Tolrestat, Epalrestat, Imirestat, Zenarestat, SNK-860, Zopolrestat, ARI-50i, AS-3201.
Ein Beispiel eines Glykations Inhibitors ist Pimagedine.
Protein Kinase C Inhibitoren sind beispielsweise NGF, LY-333531.
Andere als die zuvor genannten Wirkstoffe zur Behandlung diabetischer Komplikationen umfassen Alprostadil, Thiapride Hydrochlorid, Cilostazol, Mexiletine Hydrochlorid, Ethyl eicosapentate, Memantine, Pimagedine (ALT-711).

Wirkstoffe zur Behandlung von Obesitas, vorzugsweise andere als MCH-Antagonisten, umfassen Lipase Inhibitoren und Anorektika.
Ein bevorzugtes Beispiel eines Lipase Inhibitors ist Orlistat.
Beispiele bevorzugter Anorektika sind Phentermin, Mazindol, Dexfenfluramine, Fluoxetine, Sibutramine, Baiamine, (S)-Sibutramine, SR-141716, NGD-95-1.
Andere als die zuvor genannten Wirkstoffe zur Behandlung von Obesitas umfassen Lipstatin.
Ferner werden für die Zwecke dieser Anmeldung zu der Wirkstoffgruppe der AntiObesitas-Wirkstoffe auch die Anorektika gezählt, wobei die $\beta_3$ Agonisten, thyromimetische Wirkstoffe und NPY Antagonisten hervorzuheben sind. Der Umfang der hierbei als bevorzugte Antiobesitas oder anorektische Wirkstoffe in Frage kommenden Substanzen wird durch folgende weitere Liste beispielhaft angegeben: Phenylpropanolamin, Ephedrin, Pseudoephedrin, Phentermin, ein Cholecystokinin-A (nachfolgend als CCK-A bezeichnet) Agonist, ein Monoamin Wiederaufnahme (reuptake)-Inhibitor (wie beispielsweise Sibutramine), ein sympathomimetischer Wirkstoff, ein serotonerger Wirkstoff (wie beispielsweise Dexfenfluramine oder Fenfluramine), ein Dopamin-Antagonist (wie beispielsweise Bromocriptine), ein Melanocyten-stimulierender Hormonrezeptor Agonist oder Mimetikum, ein Analog zum Melanocyten-stimulierenden Hormon, ein Cannabinoid-Rezeptor Antagonist, ein MCH Antagonist, das OB Protein (nachfolgend als Leptin bezeichnet), ein Leptin Analog, ein Leptin Rezeptor Agonist, ein Galanin Antagonist, ein GI Lipase Inhibitor oder Verminderer (wie beispielsweise Orlistat). Weitere Anorektika umfassen Bombesin Agonisten, Dehydroepiandrosteron oder seine Analoga, Glucocorticoid Rezeptor Agonisten und Antagonisten, Orexin Rezeptor Antagonisten, Urocortin Bindungsprotein Antagonisten, Agonisten des Glukogon ähnlichen Peptid-1 Rezeptors, wie beispielsweise Exendin und ciliäre neurotrophe Faktoren, wie beispielsweise Axokine.

Wirkstoffe zur Behandlung von Bluthochdruck umfassen Inhibitoren des Angiotensin umwandelnden Enzyms, Kalzium Antagonisten, Kalium-Kanal Öffner, Angiotensin II Antagonisten.
Inhibitoren des Angiotensin umwandelnden Enzyms umfassen Captopril, Enalapril, Alacepril, Delapril (Hydrochloride), Lisinopril, Imidapril, Benazepril, Cilazapril, Temocapril, Trandolapril, Manidipine (Hydrochloride).
Beispiele von Kalzium Antagonisten sind Nifedipine, Amlodipine, Efonidipine, Nicardipine.
Kalium-Kanal Öffner umfassen Levcromakalim, L-27152, AL0671, NIP-121.
Angiotensin II Antagonisten umfassen Telmisartan, Losartan, Candesartan Cilexetil, Valsartan, Irbesartan, CS-866, E4177.

Wirkstoffe zur Behandlung von Hyperlipidemia, einschließlich Arteriosklerose, umfassen HMG-CoA Reduktase Inhibitoren, Fibrat-Verbindungen.
HMG-CoA Reduktase Inhibitoren umfassen Pravastatin, Simvastatin, Lovastatin, Atorvastatin, Fluvastatin, Lipantil, Cerivastatin, Itavastatin, ZD-4522 und deren Salze.
Fibrat-Verbindungen umfassen Bezafibrate, Clinofibrate, Clofibrate, Simfibrate.

Wirkstoffe zur Behandlung von Arthritis umfassen Ibuprofen.

Wirkstoffe zur Behandlung von Angstzuständen umfassen Chlordiazepoxide, Diazepam, Oxazolam, Medazepam, Cloxazolam, Bromazepam, Lorazepam, Alprazolam, Fludiazepam.

Wirkstoffe zur Behandlung von Depressionen umfassen Fluoxetine, Fluvoxamine, Imipramine, Paroxetine, Sertraline.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung.

In einer weiteren Ausführungsform betrifft die Erfindung auch die nicht-therapeutische Verwendung mindestens einer erfindungsgemäßen Amid-Verbindung und/ oder eines erfindungsgemäßen Salzes zur Beeinflussung des Essverhaltens eines Säugetiers. Diese Verwendung beruht insbesondere darauf, dass erfindungsgemäße Verbindungen geeignet sein können, den Hunger zu reduzieren, Appetit zu zügeln, das Essverhalten zu kontrollieren und/oder ein Sättigungsgefühl hervorzurufen. Das Essverhalten wird vorteilhaft dahingehend beeinflusst, dass die Nahrungsaufnahme reduziert wird. Daher finden die erfindungsgemäßen Verbindungen vorteilhaft Anwendung zur Reduzierung des Körpergewichts. Eine weitere erfindungsgemäße Verwendung ist das Verhindern einer Zunahme des Körpergewichts, beispielsweise in Menschen, die zuvor Maßnahmen zur Gewichtsreduzierung ergriffen hatten und anschließend an einer Beibehaltung des reduzierten Körpergewichts interessiert sind. Gemäß dieser Ausführungsform handelt es sich vorzugsweise um eine nicht-therapeutische Verwendung. Solch eine nicht-therapeutische Verwendung kann eine kosmetische Anwendung, beispielsweise zur Veränderung der äußeren Erscheinung, oder eine Anwendung zur Verbesserung der Allgemeinbefindens sein. Die erfindungsgemäßen Verbindungen werden vorzugsweise für Säugetiere, insbesondere Menschen, nicht-therapeutisch verwendet, die keine diagnostizierten Störungen des Essverhaltens, keine diagnostizierte Obesitas, Bulimie, Diabetes und/ oder keine diagnostizierten Miktionsstörungen, insbesondere Harninkontinenz aufweisen.

Bevorzugt sind die erfindungsgemäßen Verbindungen zur nicht-therapeutischen Verwendung für Menschen geeignet, deren Körpergewichtsindex (BMI = body mass index), der als das in Kilogramm gemessene Körpergewicht geteilt durch die Körpergröße (in Metern) im Quadrat definiert ist, unterhalb des Wertes 30, insbesondere unterhalb 25, liegt.

Die nachfolgenden Beispiele sollen die Erfindung näher erläutern:

<u>Vorbemerkungen:</u>

Für hergestellte Verbindungen liegen in der Regel IR-, $^1$H-NMR und/oder Massenspektren vor. Wenn nicht anders angegeben, werden $R_f$-Werte unter Verwendung von DC-Fertigplatten Kieselgel 60 $F_{254}$ (E. Merck, Darmstadt, Artikel-Nr. 1.05714) ohne Kammersättigung bestimmt. Die unter der Bezeichnung Alox ermittelten $R_f$-Werte werden unter Verwendung von DC-Fertigplatten Aluminiumoxid 60 $F_{254}$ (E. Merck, Darmstadt, Artikel-Nr. 1.05713) ohne Kammersättigung bestimmt. Die bei den Fliessmitteln angegebenen Verhältnisse beziehen sich auf Volumeneinheiten der jeweiligen Lösungsmittel. Die angegebenen Volumeneinheiten bei $NH_3$ beziehen sich auf eine konzentrierte Lösung von $NH_3$ in Wasser. Zu chromatographischen Reinigungen wird Kieselgel der Firma Millipore (MATREX™, 35-70 my) verwendet. Zu chromatographischen Reinigungen wird Alox (E. Merck, Darmstadt, Aluminiumoxid 90 standardisiert, 63-200 μm, Artikel-Nr: 1.01097.9050) verwendet. Die angegebenen HPLC-Daten werden unter nachstehend angeführten Parametern gemessen:

Analytische Säulen: Zorbax-Säule (Agilent Technologies), SB (Stable Bond) - C18; 3.5 μm; 4.6 x 75 mm; Säulentemperatur: 30°C; Fluss: 0.8 mL / min; Injektionsvolumen: 5 μL; Detektion bei 254 nm (Methoden A und B)
Symmetry 300 (Waters), 3.5 μm; 4.6 x 75 mm; Säulentemperatur: 30°C; Fluss: 0.8 mL / min; Injektionsvolumen: 5 μL; Detektion bei 254 nm (Methode C)
Methode A: Wasser:Acetonitril:Ameisensäure 9:1:0.01 nach 1:9:0.01 über 9 min
Methode B: Wasser:Acetonitril:Ameisensäure 9:1:0.01 nach 1:9:0.01 über 4 min, dann 6 min 1:9:0.01
Methode C: Wasser:Acetonitril:Ameisensäure 9:1:0.01 nach 1:9:0.01 über 4 min, dann 6 min 1:9:0.01
Präparative Säule: Zorbax-Säule (Agilent Technologies), SB (Stable Bond) - C18; 3.5 μm; 30 x 100 mm; Säulentemperatur: Raumtemperatur; Fluss: 30 mL / min; Detektion bei 254 nm.

Bei präparativen HPLC-Reinigungen werden in der Regel die gleichen Gradienten verwendet, die bei der Erhebung der analytischen HPLC-Daten benutzt wurden.
Die Sammlung der Produkte erfolgt massengesteuert, die Produkt enthaltenden Fraktionen werden vereinigt und gefriergetrocknet.

Falls nähere Angaben zur Konfiguration fehlen, bleibt offen, ob es sich um reine Enantiomere handelt oder ob partielle oder gar völlige Racemisierung eingetreten ist.

Vorstehend und nachfolgend werden die folgenden Abkürzungen verwendet:

| | |
|---|---|
| abs. | absolutiert |
| Boc | tert-Butoxycarbonyl |
| Cbz | Benzyloxycarbonyl |
| CDI | N,N'-Carbonyldiimidazol |
| CDT | 1,1'-Carbonyldi(1,2,4-triazol) |
| DMF | N,N-Dimethylformamid |
| Ether | Diethylether |
| EtOAc | Essigsäureethylester |
| EtOH | Ethanol |
| Fmoc | 9-Fluorenylmethoxycarbonyl |
| ges. | gesättigt |
| halbkonz. | halbkonzentriert |
| HCl | Salzsäure |
| HOAc | Essigsäure |
| HOBt | 1-Hydroxybenzotriazol-hydrat |
| Hünigbase | N-Ethyldiisopropylamin |

| | |
|---|---|
| HV | Hochvakuum |
| i. vac. | in vacuo (im Vakuum) |
| KOH | Kaliumhydroxid |
| konz. | konzentriert |
| MeOH | Methanol |
| MTBE | Methyl-tert-butylether |
| NaCl | Natriumchlorid |
| NaOH | Natriumhydroxid |
| org. | organisch |
| Ph | Phenyl |
| RT | Raumtemperatur |
| TBTU | 2-(1H-Benzotriazol-1-yl)-1,1,3,3-tetramethyluronium-tetrafluorborat |
| TEBAC | Triethylbenzylammoniumchlorid |
| TFA | Trifluoressigsäure |
| THF | Tetrahydrofuran |
| →* | kennzeichnet die Bindungsstelle eines Rests |

Synthese von Zwischenprodukten

Zwischenprodukt 1:

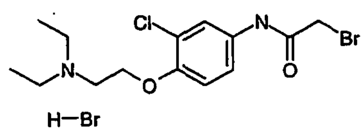

Z1a) [2-(2-Chlor-4-nitro-phenoxy)-ethyl]-diethyl-amin-hydrobromid

Zu einer Lösung von 50.00 g (0.288 mol) 2-Chlor-4-nitro-phenol und 60.23 g (0.350 mol) (2-Chlor-ethyl)-diethylamin in 700 mL DMF wurde 40.00 g (1.00 mol) Kaliumcarbonat zugegeben und das Gemisch 16 Stunden bei 80 °C gerührt. Das Reaktionsgemisch wurde i. vac. eingeengt, der Rückstand mit Wasser versetzt und die wässrige Phase mit EtOAc erschöpfend extrahiert. Die vereinigten org. Extrakte wurden mit Wasser gewaschen, über Magnesiumsulfat getrocknet und i. vac. eingeengt. Das Rohprodukt wurde aus EtOAc umkristallisiert und die Mutterlauge i. vac. eingeengt. Reinigung des Rückstands mittels Säulenchromatographie (Kieselgel, Gradient Dichlormethan / MeOH 10:0 → 9:1) ergab das gewünschte Produkt. Ausbeute: 29.00 g (37 % der Theorie)
$C_{12}H_{17}ClN_2O_3$ (M= 272.734)
Ber. Molpeak $(M+H)^+$: 273/275
gef.: Molpeak $(M+H)^+$: 273/275 (CI)

Z1b) 3-Chlor-4-(2-diethylamino-ethoxy)-phenylamin

Zu einer Suspension von 20.00 g (358 mmol) Eisenpulver und 20 g (73.33 mmol) [2-(2-Chlor-4-nitro-phenoxy)-ethyl]-diethyl-amin in 200 mL EtOH wurde eine Lösung von 100 mL konz. wässriger HCl in 100 mL EtOH zugetropft, wobei die Temperatur durch Eiskühlung unter 20 °C gehalten wurde. Das Reaktionsgemisch wurde 30 Minuten gerührt, mit 10% wässriger Natriumbicarbonat-Lösung neutralisiert und mit EtOAc erschöpfend extrahiert. Die vereinigten org. Extrakte wurden über Magnesiumsulfat getrocknet und i. vac. eingeengt. Das Produkt wurde unter Stickstoffatmosphäre gelagert.
Ausbeute: 17.40 g (98 % der Theorie)
$C_{12}H_{19}ClN_2O$(M= 242.751)
Ber. Molpeak $(M+H)^+$: 243/245
gef.: Molpeak $(M+H)^+$: 243/245 (CI)
$R_f$-Wert: 0.6 (Kieselgel, Dichlormethan / MeOH / konz. wässriger Ammoniak 90:10:1)

Z1c) 2-Brom-N-[3-chlor-4-(2-diethylamino-ethoxy)-phenyl]-acetamid-hydrobromid

Zu einer Lösung von 5.00 g (21.00 mmol) 3-Chlor-4-(2-diethylamino-ethoxy)-phenylamin in 100 mL Dichlormethan wurde bei 0 ˚C eine Lösung von 1.86 mL (21.00 mmol) Bromacetylbromid in 10 mL Dichlormethan zugetropft und das Gemisch wurde 20 Minuten bei 0 ˚C gerührt. Der gebildete Niederschlag wurde abfiltriert, mit Dichlormethan und MTBE gewaschen und bei 40 ˚C i. vac. getrocknet.
Ausbeute: 8.20 g (89 % der Theorie)
$C_{14}H_{21}BrClN_2O_2$ * Br (M= 444.597)
Ber. Molpeak $(M+H)^+$: 363/365/367
gef.: Molpeak $(M+H)^+$: 363/365/367 (BrCl)
HPLC-MS: 4.25 Min. (Stable Bond C18; 3.5 μm; Wasser:Acetonitril:Ameisensäure 9:1:0.01 → 1:9:0.01 über 9 min)

Zwischenprodukt 2:

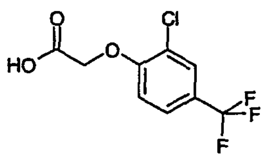

Z2a) (2-Chlor-4-trifluormethyl-phenoxy)-essigsäure-ethylester

Zu einer Lösung von 20.00 g (0.102 mol) 2-Chlor-4-trifluormethyl-phenol und 11.36 mL (0.102 mol) Bromessigsäure-ethylester in 300 mL DMF wurde 28.19 g (0.204 mol) Kaliumcarbonat zugegeben und das Gemisch 7 Stunden bei 60 ˚C und 16 Stunden bei RT gerührt. Das Reaktionsgemisch wurde i. vac. eingeengt und der Rückstand mit EtOAc versetzt. Die org. Phase wurde mit Wasser gewaschen, über Magnesiumsulfat getrocknet und i. vac. eingeengt.
Ausbeute: 23.79 g (83% der Theorie)
$C_{11}H_{10}ClF_3O_3$ (M= 282.649)
Ber. Molpeak $(M+Na)^+$: 305/307
gef.: Molpeak $(M+Na)^+$: 305/307 (Cl)
$R_f$-Wert: 0.58 (Kieselgel, Petrolether / EtOAc 4:1)

Z2b) (2-Chlor-4-trifluormethyl-phenoxy)-essigsäure

Zu einer Lösung von 23.97 g (0.084 mol) (2-Chlor-4-trifluormethyl-phenoxy)-essigsäure-ethylester in 200 mL EtOH wurde 84 mL 2 M wässrige NaOH zugegeben und das Gemisch 1 Stunde unter Rückfluss erhitzt. EtOH wurde i. vac. eingedampft, der Rückstand mit Eiswasser verdünnt und mit 2 M wässriger HCl angesäuert. Der gebildete Niederschlag wurde abfiltriert, mit Wasser gewaschen und bei 70 ˚C i. vac. getrocknet.
Ausbeute: 12.33 g (58 % der Theorie)
$C_9H_6ClF_3O_3$ (M= 254.595)
Ber. Molpeak $(M-H)^-$: 253/255
gef.: Molpeak $(M-H)^-$: 253/255 (Cl)
$R_f$-Wert: 0.04 (Kieselgel, Petrolether /EtOAc 3:2)

Zwischenprodukt 3:

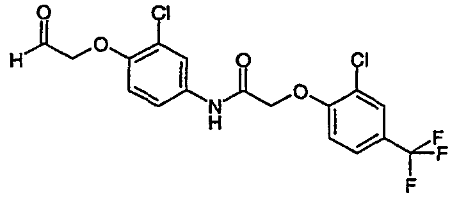

Z3a) 2-Chlor-1-(2,2-diethoxy-ethoxy)-4-nitro-benzol

Zu einer Suspension von 22.80 g (0.165 mol) Kaliumcarbonat in 250 mL DMF wurde 26.56 g (0.150 mol) 2-Chlor-4-nitrophenol und 24.25 mL (0.150 mol) 2-Brom-1,1-diethoxy-ethan zugegeben und das Gemisch 24 Stunden auf 140 ˚C erhitzt. Das Reaktionsgemisch wurde mit 1 L Wasser verdünnt und mit MTBE erschöpfend extrahiert. Die vereinigten org. Extrakte wurden mit Wasser gewaschen, über Magnesiumsulfat getrocknet und i. vac. eingeengt.
Ausbeute: 32.10 g (74 % der Theorie)
$C_{12}H_{16}ClNO_5$ (M= 289.718)
$R_f$-Wert: 0.7 (Kieselgel, Dichlormethan / Cyclohexan / EtOAc 1:4:1)

Z3b) 3-Chlor-4-(2,2-diethoxy-ethoxy)-phenylamin

Zu einer Suspension von 1.50 g Pd/C (10 %) in 500 mL EtOAc wurde 30 g (0.104 mol) 2-Chlor-1-(2,2-diethoxy-ethoxy)-4-nitro-benzol zugegeben und das Gemisch wurde 2 Stunden bei 20 psi hydriert. Der Katalysator wurde abfiltriert und das Filtrat i. vac. eingeengt.
Ausbeute: 27.00 g (quantitative Ausbeute)
$C_{12}H_{18}ClNO_3$ (M= 259.735)
Ber. Molpeak $(M+H)^+$: 260/262
gef.: Molpeak $(M+H)^+$: 260/262 (Cl)
$R_f$-Wert: 0.75 (Kieselgel, Dichlormethan / MeOH 9:1)

Z3c) N-[3-Chlor-4-(2,2-diethoxy-ethoxy)-phenyl]-2-(2-chlor-4-trifluormethyl-phenoxy)-acetamid

Zu einer Lösung von 6.365 g (0.025 mol) (2-Chlor-4-trifluormethyl-phenoxy)-essigsäure (Zwischenprodukt 2b) in 100 mL abs. THF wurde 4.495 g (0.028 mol) CDI zugegeben und das Gemisch 30 Minuten bei 50 ˚C gerührt. 6.494 g (0.025 mol) 3-Chlor-4-(2,2-diethoxy-ethoxy)-phenylamin wurde zugegeben und das Gemisch 16 Stunden bei RT gerührt. Das Reaktionsgemisch wurde in Eiswasser gegossen und 1 Stunde gerührt. Der gebildete Niederschlag wurde abfiltriert, mit Wasser gewaschen und bei 50 ˚C getrocknet.
Ausbeute: 11.40 g (92 % der Theorie)
$C_{21}H_{22}Cl_2F_3NO_5$ (M= 496.314)
Ber. Molpeak $(M-H)^-$: 494/496/498
gef.: Molpeak $(M-H)^-$: 494/496/498 ($Cl_2$)
$R_f$-Wert: 0.73 (Kieselgel, Petrolether /EtOAc 3:2)

Z3d) N-[3-Chlor-4-(2-oxo-ethoxy)-phenyl]-2-(2-chlor-4-trifluormethyl-phenoxy)-acetamid

Zu einer Lösung von 11.40 g (0.023 mol) N-[3-Chlor-4-(2,2-diethoxy-ethoxy)-phenyl]-2-(2-chlor-4-trifluormethyl-phenoxy)-acetamid in 130 mL Chloroform wurde bei 0 ˚C 40 mL Wasser und 130 mL TFA zugegeben und das Gemisch 3.5 Stunden bei 0 ˚C und 48 Stunden bei RT gerührt. Das Reaktionsgemisch wurde mit ges. wässriger Natriumcarbonat-Lösung neutralisiert und mit Dichlormethan erschöpfend extrahiert. Die vereinigten org. Extrakte wurden mit Wasser gewaschen, über Magnesiumsulfat getrocknet und i. vac. eingeengt.
Ausbeute: 8.40g (86 % der Theorie)
$C_{17}H_{12}Cl_2F_3NO_4$(M= 422.191)
Ber. Molpeak $(M-H)^-$: 421/423/425
gef.: Molpeak $(M-H)^-$: 421/423/425 ($Cl_2$)
$R_f$-Wert: 0.14 (Kieselgel, Petrolether /EtOAc 3:2)

Zwischenprodukt 4:

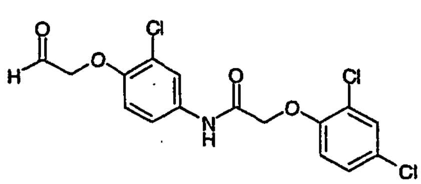

Z4a) N-[3-Chlor-4-(2,2-diethoxy-ethoxy)-phenyl]-2-(2,4-dichlor-phenoxy)-acetamid

Zu einer Lösung von 0.50 g (2.16 mmol) 3-Chlor-4-(2,2-diethoxy-ethoxy)-phenylamin (Zwischenprodukt Z3b) und 0.74 mL (4.32 mmol) Ethyl-diisopropylamin in 10 mL Dichlormethan wurde bei 0 ˚C eine Lösung von 0.57 g (2.38 mmol) (2,4-Dichlor-phenoxy)-acetylchlorid in 4 mL Dichlormethan zugetropft und das Gemisch 1 Stunde bei 0 ˚C gerührt. MeOH wurde zugegeben und das ausgefallene Produkt abfiltriert. Das Produkt wurde mit MeOH gewaschen und i. vac. getrocknet.
Ausbeute: 0.74 g (79% der Theorie)
$C_{18}H_{18}Cl_3NO_5$ (M= 434.70)
Ber. Molpeak $(M-H)^-$: 432/434/436
gef.: Molpeak $(M-H)^-$: 432/434/436 ($Cl_3$)
HPLC-MS: 5.00 Min. (Devosil RPAqueous; 30-100% Wasser / Acetonitril 70:30 → 0:100 in 5 Min.)

Z4b) N-[3-Chlor-4-(2-oxo-ethoxy)-phenyl]-2-(2,4-dichlor-phenoxy)-acetamid

Zu einer Lösung von 50 mg (0.011 mmol) N-[3-Chlor-4-(2,2-diethoxy-ethoxy)-phenyl]-2-(2,4-dichlor-phenoxy)-acetamid in 2 mL Dichlormethan wurde bei 0 ˚C 2 mL TFA und 0.15 mL Wasser zugegeben und das Gemisch 3.5 Stunden gerührt. 200 mL 2 M wässrige Natriumcarbonat-Lösung wurde zugegeben und mit Dichlormethan erschöpfend extrahiert. Die vereinigten org. Extrakte wurden über Magnesiumsulfat getrocknet, i. vac. eingeengt und der Rückstand mittels Säulenchromatographie (Kieselgel, EtOAc / Hexan 1:1) gereinigt.
Ausbeute: 40 mg (89 % der Theorie)
$C_{16}H_{12}Cl_3NO_4$ (M= 388.63)
Ber. Molpeak $(M-H)^-$: 386/388/390
gef.: Molpeak $(M-H)^-$: 386/388/390($Cl_3$)
$R_f$-Wert: 0.25 (Kieselgel, Hexan / EtOAc 3:2)
HPLC-MS: 4.56 Min. (Devosil RPAqueous; 5-100% Wasser / Acetonitril 70:30 → 0:100 in 5 Min.)

Zwischenprodukt 5:

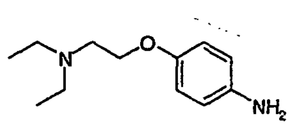

Z5a) Diethyl-[2-(4-nitro-phenoxy)-ethyl]-amin

Zu einer Lösung von 1.04 g (7.5 mmol) 4-Nitrophenol in 20 mL DMF wurde unter Argonatmosphäre 2.07 g (15.0 mmol) Kaliumcarbonat zugegeben und das Gemisch 20 Minuten bei 80 ˚C gerührt. 1.72 g (10.0 mmol) (2-Chlor-ethyl)-diethyl-amin-hydrochlorid wurde zugegeben und das Gemisch 8 Stunden bei 90 ˚C gerührt. 100 mL 2 M wässrige Natriumcarbonat-Lösung wurde zugegeben und mit Ether erschöpfend extrahiert. Die vereinigten org. Extrakte wurden über Magnesiumsulfat getrocknet und i. vac. eingeengt. Das Rohprodukt wurde ohne weitere Reinigung im nachfolgenden Reaktionsschritt eingesetzt.
Ausbeute: 1.59 g (89% der Theorie)
$C_{12}H_{18}N_2O_3$ (M= 238.28)
Ber. Molpeak $(M+H)^+$: 239
gef.: Molpeak $(M+H)^+$: 239
$R_f$-Wert: 0.2 (Kieselgel, EtOAc)

Z5b) 4-(2-Diethylamino-ethoxy)-phenylamin

Zu einer Suspension von 130 mg Pd/C (10 %) in 20 mL MeOH wurde 2.6 g (10.9 mmol) Diethyl-[2-(4-nitro-phenoxy)-ethyl]-amin zugegeben und das Gemisch wurde 4 Stunden hydriert. Der Katalysator wurde abfiltriert und das Filtrat i. vac. eingeengt.
Ausbeute: 2.19 g (96% der Theorie)
$C_{12}H_{20}N_2O$ (M= 208.30)

Ber. Molpeak $(M+H)^+$: 209
gef.: Molpeak $(M+H)^+$: 209
$R_f$-Wert: 0.2 (Kieselgel, Dichlormethan / MeOH 9:1)

Zwischenprodukt 6:

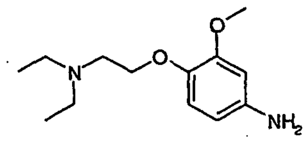

Z6a) Diethyl-[2-(2-methoxy-4-nitro-phenoxy)-ethyl]-amin

Das Produkt wurde analog zu Zwischenprodukt Z5a ausgehend von 1.27 g (7.5 mmol) 2-Methoxy-4-nitro-phenol und 1.72 g (10.0 mmol) (2-Chlor-ethyl)-diethyl-amin-hydrochlorid erhalten.
Ausbeute: 1.01 g (50 % der Theorie)
$C_{13}H_{20}N_2O_4$ (M= 268.31)
Ber. Molpeak $(M+H)^+$: 269
gef.: Molpeak $(M+H)^+$: 269
$R_f$-Wert: 0.2 (Kieselgel, EtOAc)

Z6b) 4-(2-Diethylamino-ethoxy)-3-methoxy-phenylamin

Zu einer Suspension von 1.00 g (17.9 mmol) Eisenpulver in 7 mL EtOH wurde 0.77 g (2.87 mmol) Diethyl-[2-(2-methoxy-4-nitro-phenoxy)-ethyl]-amin zugegeben und das Gemisch 10 Minuten bei RT gerührt. 6.6 mL konz. wässrige HCl wurde innerhalb von 15 Minuten zugetropft und das Gemisch 1 Stunde gerührt. 100 mL 2 M Natriumcarbonat-Lösung wurde zugegeben und mit EtOAc erschöpfend extrahiert. Die vereinigten org. Extrakte wurden über Magnesiumsulfat getrocknet und i. vac. eingeengt.
Ausbeute: 0.62 g (92 % der Theorie)
$C_{13}H_{22}N_2O_2$ (M= 238.33)
Ber. Molpeak $(M+H)^+$: 269
gef.: Molpeak $(M+H)^+$: 269
$R_f$-Wert: 0.05 (Kieselgel, Dichlormethan / MeOH 9:1)

Zwischenprodukt 7:

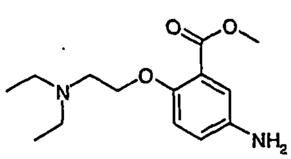

Z7a) 2-(2-Diethylamino-ethoxy)-5-nitro-benzoesäure-methylester

Das Produkt wurde analog zu Zwischenprodukt Z5a ausgehend von 1.48 g (7.5 mmol) 2-Hydroxy-5-nitro-benzoesäure-methylester und 1.72 g (10.0 mmol) (2-Chlor-ethyl)-diethyl-amin-hydrochlorid erhalten.
Ausbeute: 0.81 g (40 % der Theorie)
$C_{14}H_{20}N_2O_5$ (M= 296.32)
Ber. Molpeak $(M+H)^+$: 297
gef.: Molpeak $(M+H)^+$: 297
$R_f$-Wert: 0.1 (Kieselgel, EtOAc / MeOH 9:1)

Z7b) 5-Amino-2-(2-diethylamino-ethoxy)-benzoesäure-methylester

Das Produkt wurde analog zu Zwischenprodukt Z5b ausgehend von 400 mg (1.35 mmol) 2-(2-Diethylamino-ethoxy)-5-nitro-benzoesäure-methylester erhalten.
Ausbeute: 0.35 g (97 % der Theorie)
$C_{14}H_{22}N_2O_3$ (M= 266.34)
Ber. Molpeak $(M+H)^+$: 267
gef.: Molpeak $(M+H)^+$: 267
$R_f$-Wert: 0.2 (Kieselgel, EtOAc / MeOH 9:1)

Zwischenprodukt 8:

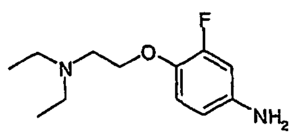

Z8a) Diethyl-[2-(2-fluor-4-nitro-phenoxy)-ethyl]-amin

Das Produkt wurde analog zu Zwischenprodukt Z5a ausgehend von 1.18 g (7.5 mmol) 2-Fluor-4-nitro-phenol und 1.72 g (10.0 mmol) (2-Chlor-ethyl)-diethyl-amin-hydrochlorid erhalten.
Ausbeute: 1.65 g (86 % der Theorie)
$C_{12}H_{17}FN_2O_3$ (M= 256.27)
Ber. Molpeak $(M+H)^+$: 257
gef.: Molpeak $(M+H)^+$: 257
$R_f$-Wert: 0.1 (Kieselgel, EtOAc)

Z8b) 4-(2-Diethylamino-ethoxy)-3-fluor-phenylamin

Das Produkt wurde analog zu Zwischenprodukt Z6b ausgehend von 0.68 g (2.65 mmol) Diethyl-[2-(2-fluor-4-nitro-phenoxy)-ethyl]-amin erhalten.
Ausbeute: 0.60 g (quantitative Ausbeute)
$C_{12}H_{19}FN_2O$ (M= 226.29)
Ber. Molpeak $(M+H)^-$: 227
gef.: Molpeak $(M+H)^+$: 227
$R_f$-Wert: 0.1 (Kieselgel, Dichlormethan / MeOH 9:1)

Zwischenprodukt 9:

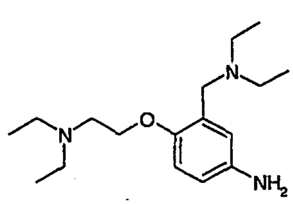

Z9a) (3-Diethylaminomethyl-4-hydroxy-phenyl)-carbaminsäure-tert-butylester

Zu einer Lösung von 1.00 g (3.74 mmol) 4-Amino-2-diethylaminomethyl-phenol und 0.52 mL (3.74 mmol) Triethylamin in 20 mL abs. THF wurde bei 80 ˚C eine Lösung von 0.90 g (4.11 mmol) Boc-Anhydrid in 20 mL THF

zugegeben und das Gemisch 24 Stunden unter Rückfluss erhitzt. 100 mL 2 M wässrige Natriumcarbonat-Lösung wurde zugegeben und mit Ether erschöpfend extrahiert. Die vereinigten org. Extrakte wurden über Magnesiumsulfat getrocknet, i. vac. eingeengt und der Rückstand mittels Säulenchromatographie (Kieselgel, EtOAc) gereinigt.
Ausbeute: 1.03 g (94 % der Theorie)
$C_{16}H_{26}N_2O_3$ (M= 294.39)
Ber. Molpeak $(M+H)^-$: 295
gef.: Molpeak $(M+H)^+$: 295
$R_f$-Wert: 0.3 (Kieselgel, EtOAc)

Z9b) [4-(2-Diethylamino-ethoxy)-3-diethylaminomethyl-phenyl]-carbaminsäure-tert-butylester

Das Produkt wurde analog zu Zwischenprodukt Z5a ausgehend von 2.21 g (7.5 mmol) (3-Diethylaminomethyl-4-hydroxy-phenyl)-carbaminsäure-tert-butylester und 1.72 g (10.0 mmol) (2-Chlor-ethyl)-diethyl-amin-hydrochlorid erhalten.
Ausbeute: 0.88 g (30 % der Theorie)
$C_{22}H_{99}N_3O_3$ (M= 393.56)
Ber. Molpeak $(M+H)^+$: 394
gef.: Molpeak $(M+H)^+$: 394
$R_f$-Wert: 0.05 (Kieselgel, Dichlormethan / MeOH 9:1)

Z9c) 4-(2-Diethylamino-ethoxy)-3-diethylaminomethyl-phenylamin

Zu einer Lösung von 0.18 g (0.457 mmol) [4-(2-Diethylamino-ethoxy)-3-diethylaminomethyl-phenyl]-carbaminsäure-tert-butylester in 5 mL Chloroform wurde 5 mL TFA zugegeben und das Gemisch 1 Stunde bei RT gerührt. 100 mL 2 M wässrige Natriumcarbonat-Lösung wurde zugegeben und mit Ether erschöpfend extrahiert. Die vereinigten org. Extrakte wurden über Magnesiumsulfat getrocknet und i. vac. eingeengt.
Ausbeute: 0.13 g (97 % der Theorie)
$C_{17}H_{31}N_3O$ (M= 293.45)
Ber. Molpeak $(M+H)^+$: 294
gef.: Molpeak $(M+H)^+$: 294
$R_f$-Wert: 0.05 (Kieselgel, Dichlormethan / MeOH 4:1)

Zwischenprodukt 10:

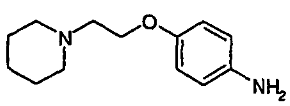

Z10) 4-(2-Piperidin-1-yl-ethoxy)-phenylamin

Zu einer Lösung von 4.0 g (27.86 mmol) 4-Amino-2-chlorphenol und 5.1 g (27.86 mmol) 1-(2-Chlor-ethyl)-piperidin in 50 mL Acetonitril wurde 15.4 g (111.00 mmol) Kaliumcarbonat zugegeben und das Gemisch wurde 48 Stunden bei RT gerührt. Das Lösungsmittel wurde i. vac. abgedampft, der Rückstand mit Wasser versetzt und die wässriger Phase mit EtOAc erschöpfend extrahiert. Die vereinigten org. Extrakte wurden mit Wasser gewaschen, über Magnesiumsulfat getrocknet, i. vac. eingeengt und der Rückstand mittels Säulenchromatographie (Kieselgel, Dichlormethan / MeOH / konz. wässriger Ammoniak 90:10:1) gereinigt.
Ausbeute: 77 mg (61 % der Theorie)
$C_{13}H_{19}ClN_2O$ (M= 254.762)
Ber. Molpeak $(M+H)^+$: 255/257
gef.: Molpeak $(M+H)^+$: 255/257 (Cl)

Zwischenprodukt 11:

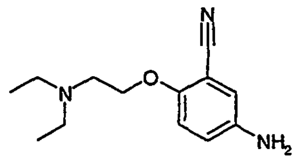

Z11a) 2-Hydroxy-5-nitro-benzonitril

Zu einer Lösung von 50 g (0.416 mol) 2-Hydroxy-benzonitril in 150 mL konz. Essigsäure wurde bei 45-50 ˚C eine Lösung von 36.0 mL 65% wässriger Salpetersäure in 50 mL konz. Essigsäure zugetropft und das Gemisch 1 Stunde bei 50 ˚C gerührt. Das Reaktionsgemisch wurde auf RT gekühlt, mit 400 mL Wasser verdünnt und der gebildete Niederschlag abfiltriert (Gemisch aus o- und p-substituiertem Produkt). Die Mutterlauge wurde mit 1 L Eiswasser verdünnte und der gebildete Niederschlag abfiltriert (Produkt). Das Produkt-Gemisch wurde in Dichlormethan / MeOH gelöst und mittels Säulenchromatographie (Kieselgel, Gradient Dichlormethan / MeOH 10:0 → 4:1) gereinigt.
Ausbeute: 25.22 g (37 % der Theorie)
$C_7H_4N_2O_3$ (M=164.122)
Ber. Molpeak $(M-H)^-$: 163
gef.: Molpeak $(M-H)^-$: 163
$R_f$-Wert: 0.35 (Kieselgel, Dichlormethan / MeOH 9:1)

Z11b) 5-Amino-2-hydroxy-benzonitril

Zu einer Suspension von 0.45 g Pd/C (10 %) in 45 mL EtOAc wurde 4.50 g (27.00 mmol) 2-Hydroxy-5-nitrobenzonitril zugegeben und das Gemisch wurde 1.5 Stunden bei 3 bar $H_2$-Atmosphäre hydriert. Der Katalysator wurde abfiltriert und der Rückstand i. vac. getrocknet.
Ausbeute: 3.40 g (94 % der Theorie)
$C_7H_6N_2O$ (M= 134.139)
Ber. Molpeak $(M-H)^-$: 133
gef.: Molpeak $(M-H)^-$: 133
$R_f$-Wert: 0.3 (Kieselgel, Dichlormethan / MeOH 9:1)

Z11c) 5-Amino-2-(2-diethylamino-ethoxy)-benzonitril

Zu einer Lösung von 2.683 g (0.020 mol) 5-Amino-2-hydroxy-benzonitril und 3.786 g (0.022 mol) N,N-Diethylamino-ethylchorid-hydrochlorid in 100 mL abs. Acetonitril wurde 11.06 g (0.080 mol) Kaliumcarbonat zugegeben und das Gemisch 48 Stunden bei RT gerührt. Das Lösungsmittel wurde i. vac. abgedampft und der Rückstand mit Wasser versetzt. Die wässrige Phase wurde mit EtOAc erschöpfend extrahiert, die vereinigten org. Extrakte mit Wasser gewaschen, über Magnesiumsulfat getrocknet und i. vac. eingeengt. Der Rückstand wurde mittels Säulenchromatographie (Kieselgel, Dichlormethan / MeOH / konz. wässriger Ammoniak 90:10:1) gereinigt.
Ausbeute: 0.80 g (17 % der Theorie)
$C_{13}H_{19}N_3O$ (M= 233.316)
Ber. Molpeak $(M+H)^+$: 234
gef.: Molpeak $(M+H)^+$: 234
$R_f$-Wert: 0.15 (Kieselgel, Dichlormethan / MeOH / konz. wässriger Ammoniak 90:10:1)

Zwischenprodukt 12:

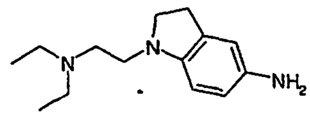

Z12a) Diethyl-[2-(5-nitro-2,3-dihydro-indol-1-yl)-ethyl]-amin

Zu einer Lösung von 0.477 g (2.905 mmol) 5-Nitro-2,3-dihydro-1H-indol und 0.500 g (2.905 mmol) N,N-Diethylamino-ethylchorid-hydrochlorid in 5 mL DMF wurde 1.00 g (7.262 mmol) Kaliumcarbonat zugegeben und das Gemisch 16 Stunden bei 90 ˚C gerührt. Das Reaktionsgemisch wurde mit Wasser verdünnt und mit EtOAc erschöpfend extrahiert. Die vereinigten org. Phasen wurden über Magnesiumsulfat getrocknet und i. vac. eingeengt. Der Rückstand wurde mittels Säulenchromatographie (Kieselgel, EtOAc) gereinigt.
Ausbeute: 0.14 g (18 % der Theorie)
$C_{14}H_{21}N_3O_2$ (M= 263.342)
Ber. Molpeak $(M+H)^+$: 264
gef.: Molpeak $(M+H)^+$: 264
$R_f$-Wert: 0.26 (Kieselgel, EtOAc / MeOH 9:1)

Z12b) 1-(2-Diethylamino-ethyl)-2,3-dihydro-1H-indol-5-ylamin

Zu einer Suspension von 50 mg Raney-Ni in 5 mL MeOH wurde 140 mg (0.532 mmol) Diethyl-[2-(5-nitro-2,3-dihydro-indol-1-yl)-ethyl]-amin zugegeben und das Gemisch 1 Stunde bei RT und 20 psi $H_2$-Atmosphäre hydriert. Der Katalysator wurde abfiltriert und das Filtrat i. vac. eingeengt. Das Rohprodukt wurde ohne weitere Reinigung sofort umgesetzt (siehe Beispiel 12). Ausbeute: 80 mg (64 % der Theorie)

Zwischenprodukt 13:

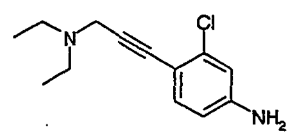

Z13a) [3-(2-Chlor-4-nitro-phenyl)-prop-2-ynyl]-diethyl-amin

Unter Stickstoffatmosphäre wurde zu 25.00 g (0.106 mol) 4-Brom-3-chlor-nitrobenzol, 43.7 mL (0.315 mol) Triethylamin, 10.40 g (0.009 mol) Tetrakis[triphenylphosphin]-palladium(II) und 1.71 g (0.009 mol) Kupfer(I)-iodid in 250 mL Acetonitril 12.5 mL (0.090 mol) 3-N,N-Diethylamino-propin zugegeben und das Gemisch 18 Stunden unter Rückfluss erhitzt. Das Reaktionsgemisch wurde i. vac. eingeengt, mit EtOAc versetzt und die organische Phase mit Wasser gewaschen. Die org. Phase wurde i. vac. eingeengt und der Rückstand mittels Säulenchromatographie (Kieselgel, Petrolether / EtOAc 10:0 → 4:1) gefolgt von einer Säulenchromatographie (Kieselgel, Dichlormethan) gereinigt.
Ausbeute: 15.0 g (62 % der Theorie)
$C_{13}H_{15}ClN_2O_2$ (M= 266.730)
Ber. Molpeak $(M+H)^+$: 267/269
gef: Molpeak $(M+H)^+$: 267/269 (C)

Z13b) 3-Chlor-4-(3-diethylamino-prop-1-ynyl)-phenylamin

Zu einer Suspension von 4.189 g (75.00 mmol) Eisenpulver und 2.00 g (7.50 mmol) [3-(2-Chlor-4-nitro-phenyl)-prop-2-ynyl]-diethyl-amin in 20 mL EtOH wurde unter starkem Rühren eine Lösung von 15 mL konz. wässrige HCl in 15 mL EtOH zugegeben und das Gemisch 30 Minuten gerührt. Das Reaktionsgemisch wurde mit 200 mL 10 % wässriger Natriumcarbonat-Lösung neutralisiert und mit EtOAc erschöpfend extrahiert. Die vereinigten org. Phasen wurden über Magnesiumsulfat getrocknet, i. vac. eingeengt und der Rückstand mittels Säulenchromatographie (Kieselgel, Gradient Dichlormethan / 10 % konz. wässriger Ammoniak in MeOH 100:0 → 5:95) gereinigt. Ausbeute: 0.45 g (25 % der Theorie)
$C_{13}H_{17}ClN_2$ (M= 236.747)
Ber. Molpeak $(M+H)^+$: 237/239
gef.: Molpeak $(M+H)^+$: 237/239 (Cl)

Zwischenprodukt 14:

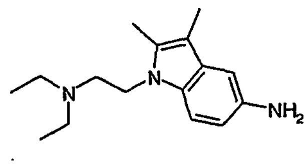

Z14a) [2-(2,3-Dimethyl-5-nitro-indol-1-yl)-ethyl]-diethyl-amin

Zu einer Lösung von 0.553 g (2.905 mmol) 2,3-Dimethyl-5-nitro-1H-indol und 0.500 g (2.905 mmol) N,N-Diethylamino-ethylchorid-hydrochlorid in 5 mL DMF wurde 1.00 g (7.262 mmol) Kaliumcarbonat zugegeben und das Gemisch 16 Stunden bei 90 ˚C gerührt. Das Reaktionsgemisch wurde mit Wasser verdünnt und mit EtOAc erschöpfend extrahiert. Die vereinigten org. Phasen wurden über Magnesiumsulfat getrocknet und i. vac. eingeengt. Der Rückstand wurde mittels Säulenchromatographie (Kieselgel, EtOAc / MeOH 9:1) gereinigt.
Ausbeute: 0.15 g (18 % der Theorie)
$C_{16}H_{23}N_3O_2$ (M= 289.3812)
Ber. Molpeak $(M+H)^+$: 290
gef.: Molpeak $(M+H)^+$: 290
$R_f$-Wert: 0.54 (Kieselgel, EtOAc / MeOH 9:1)

Z14b) 1-(2-Diethylamino-ethyl)-2,3-dimethyl-1H-indol-5-ylamin

Zu einer Suspension von 100 mg Raney-Ni in 5 mL MeOH wurde 150 mg (0.518 mmol) [2-(2,3-Dimethyl-5-nitro-indol-1-yl)-ethyl]-diethyl-amin zugegeben und das Gemisch 1 Stunde bei RT und 20 psi $H_2$-Atmosphäre hydriert. Der Katalysator wurde abfiltriert und das Filtrat i. vac. eingeengt. Das Rohprodukt wurde ohne weitere Reinigung sofort umgesetzt (siehe Beispiel 5).
Ausbeute: 100 mg (74 % der Theorie)

Zwischenprodukt 15:

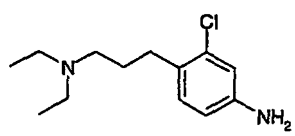

Z15) 3-Chlor-4-(3-diethylamino-propyl)-phenylamin

Zu einer Suspension von 0.50 g Raney-Ni in 50 mL abs. MeOH wurde 2.00 g (7.498 mmol) 3-Chlor-4-(3-diethylamino-prop-1-ynyl)-phenylamin (Zwischenprodukt Z13b) zugegeben und das Gemisch 2.5 Stunden bei RT und 50 psi $H_2$-Atmosphäre hydriert. Der Katalysator wurde abfiltriert, das Filtrat i. vac. eingeengt und der Rückstand mittels Säulenchromatographie (Kieselgel, Gradient Dichlormethan / 10 % konz. wässriger Ammoniak in MeOH 100:0 → 5:95) gereinigt.
Ausbeute: 0.90 g (50 % der Theorie)

$C_{13}H_{21}ClN$ (M= 240.779)
Ber. Molpeak $(M+H)^+$: 241/243
gef.: Molpeak $(M+H)^+$: 241/243 (Cl)

Zwischenprodukt 16:

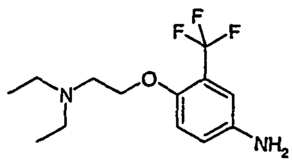

Z16a) Diethyl-[2-(4-nitro-2-trifluormethyl-phenoxy)-ethyl]-amin

Zu einer Lösung von 4.10 g (20.00 mmol) 4-Nitro-2-trifluormethyl-phenol (J. Org. Chem. 1962, 27, 4660-4662.) in 40 mL DMF wurde 5.60 g (40.00 mmol) Kaliumcarbonat zugegeben und das Gemisch auf 80 ˚C erhitzt. Eine Lösung von 3.5 g (20.00 mmol) N,N-Diethylamino-ethylchorid-hydrochlorid in 10 mL DMF wurde zugetropft und das Gemisch weitere 3 Stunden bei 80 ˚C gerührt. Das Reaktionsgemisch wurde mit 100 mL ges. wässriger NaCl-Lösung verdünnt und mit EtOAc erschöpfend extrahiert. Die vereinigten org. Extrakte wurden mit 10 % wässriger Natriumcarbonat-Lösung gewaschen, über Magnesiumsulfat getrocknet und i. vac. eingeengt.
Ausbeute: 7.5 g (80 % der Theorie)
$C_{13}H_{17}F_3N_2O_3$ (M= 306.287)
Ber. Molpeak $(M+H)^+$: 307
gef.: Molpeak $(M+H)^+$: 307

Z16b) 4-(2-Diethylamino-ethoxy)-3-trifluormethyl-phenylamin

Zu einer Suspension von 0.50 g Pd/C (10 %) in EtOAc wurde 7.0 g (22.854 mmol) 4-(2-Diethylamino-ethoxy)-3-trifluormethyl-phenylamin zugegeben und das Gemisch 6 Stunden bei 50 ˚C und 50 psi $H_2$-Atmosphäre hydriert. Der Katalysator wurde abfiltriert und das Filtrat i. vac. eingeengt. MTBE wurde zugegeben und die org. Phase mehrmals mit Wasser gewaschen, über Magnesiumsulfat getrocknet, über Aktivkohle filtriert und i. vac. eingeengt. Ausbeute: 4.40 g (70 % der Theorie)
$C_{13}H_{19}F_3N_2O$ (M= 276.304)
Ber. Molpeak $(M+H)^+$: 277
gef.: Molpeak $(M+H)^+$: 277

Zwischenprodukt 17:

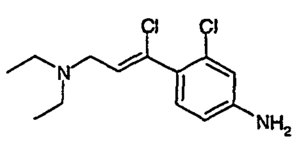

Z17) 3-Chlor-4-((Z)-1-chlor-3-diethylamino-propenyl)-phenylamin

Zu einer Suspension von 2.20 g (75.00 mmol) Eisenpulver und 2.20 g (8.25 mmol) [3-(2-Chlor-4-nitro-phenyl)-prop-2-ynyl]-diethyl-amin (Zwischenprodukt Z13a) in 20 mL EtOH wurde unter starkem Rühren eine Lösung von 15 mL konz. wässrige HCl in 15 mL EtOH zugegeben und das Gemisch 2 Stunden bei 80 ˚C gerührt. Das Reaktionsgemisch wurde mit 200 mL 10 % wässriger Natriumcarbonat-Lösung neutralisiert und mit EtOAc erschöpfend extrahiert. Die vereinigten org. Phasen wurden über Magnesiumsulfat getrocknet und i. vac. eingeengt.

Ausbeute: 1.70 g (75 % der Theorie)
$C_{13}H_{18}Cl_2N_2$ (M= 273.208)
Ber. Molpeak $(M+H)^+$: 273/275/277
gef.: Molpeak $(M+H)^+$: 273/275/277 ($Cl_2$)
$R_f$-Wert: 0.71 (Kieselgel, EtOAc / MeOH / konz. wässriger Ammoniak 90:10:1)

Zwischenprodukt 18:

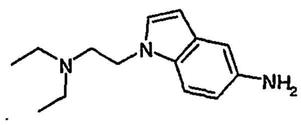

Z18a) Diethyl-[2-(5-nitro-indol-1-yl)-ethyl]-amin

Zu einer Lösung von 0.47 g (2.905 mmol) 5-Nitro-1H-indol und 0.50 g (2.905 mmol) N,N-Diethylamino-ethylchorid-hydrochlorid in 5 mL DMF wurde 1.00 g (7.262 mmol) Kaliumcarbonat zugegeben und das Gemisch 3 Stunden bei 80 ˚C gerührt. Das Reaktionsgemisch wurde mit Wasser verdünnt und mit EtOAc erschöpfend extrahiert. Die vereinigten org. Phasen wurden über Magnesiumsulfat getrocknet und i. vac. eingeengt.
Ausbeute: 0.65g (86 % der Theorie)
$C_{14}H_{19}N_3O_2$ (M= 261.326)
Ber. Molpeak $(M+H)^+$: 262
gef.: Molpeak $(M+H)^+$: 264

Z18b) 1-(2-Diethylamino-ethyl)-1H-indol-5-ylamin

Zu einer Suspension von 200 mg Raney-Ni in 10 mL MeOH wurde 650 mg (2.487 mmol) Diethyl-[2-(5-nitroindol-1-yl)-ethyl]-amin zugegeben und das Gemisch 2 Stunden bei RT und 20 psi $H_2$-Atmosphäre hydriert. Der Katalysator wurde abfiltriert und das Filtrat i. vac. eingeengt.
Ausbeute: 520 mg (90 % der Theorie)
$C_{14}H_{21}N_3$ (M= 231.344)
Ber. Molpeak $(M+H)^+$: 232 gef.: Molpeak $(M+H)^+$: 232

Zwischenprodukt 19:

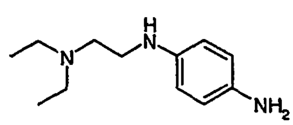

Z19a) N'-(2-Chlor-4-nitro-phenyl)-N,N-diethyl-ethane-1,2-diamin

Zu einer Lösung von 1.00 g (5.795 mmol) 2-Chlor-4-nitro-phenylamin, 2.995 g (17.384 mmol) (2-Chlorethyl)-diethyl-amin und 0.66 g (2.898 mmol) TEBAC in 50 mL Toluol wurde 25 mL 50 % wässrige KOH-Lösung zugegeben und das Gemisch 5 Tage unter Rückfluss erhitzt. Das Reaktionsgemisch wurde auf RT gekühlt und mit EtOAc erschöpfend extrahiert. Die vereinigten org. Phasen wurden über Magnesiumsulfat getrocknet, i. vac. eingeengt und der Rückstand mittels Säulenchromatographie (Kieselgel, Dichlormethan / MeOH 4:1) gereinigt.
Ausbeute: 1.2 g (76 % der Theorie)
$C_{12}H_{18}ClN_3O_2$ (M= 271.749)
Ber. Molpeak $(M+H)^+$: 272/274 gef.: Molpeak $(M+H)^+$: 272/274 (Cl)

Z19b) N-(2-Diethylamino-ethyl)-benzene-1,4-diamin

Zu einer Suspension von 200 mg Raney-Ni in 20 mL MeOH wurde 1.20 mg (4.416 mmol) N'-(2-Chlor-4-nitrophenyl)-N,N-diethyl-ethane-1,2-diamin zugegeben und das Gemisch 2 Stunden bei RT und 20 psi $H_2$-Atmosphäre hydriert. Der Katalysator wurde abfiltriert und das Filtrat i. vac. eingeengt.
Ausbeute: 800 mg (87 % der Theorie)
$C_{12}H_{21}N_3$ (M= 207.321)
Ber. Molpeak $(M+H)^+$: 207 gef.: Molpeak $(M+H)^+$: 207

Zwischenprodukt 20:

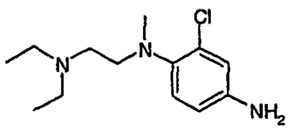

Z20a) N-(2-Chlor-4-nitro-phenyl)-N',N'-diethyl-N-methyl-ethane-1,2-diamin

Zu einer Lösung von 1.085 g (6.181 mmol) 2-Chlor-1-fluor-4-nitro-benzol und 1.03 mL (7.417 mmol) Triethylamin in 20 mL THF wurde 1.00 mL (6.181 mmol) N,N-Diethyl-N'-methyl-ethane-1,2-diamin zugegeben und das Gemisch 48 Stunden bei RT gerührt. Das Reaktionsgemisch wurde mit ges. wässriger Natriumbicarbonat-Lösung versetzt und mit EtOAc erschöpfend extrahiert. Die vereinigten org. Extrakte wurden über Magnesiumsulfat getrocknet und i. vac. eingeengt.
Ausbeute: 1.60 mg (91 % der Theorie)
$C_{13}H_{20}ClN_3O_2$ (M= 285.776)
Ber. Molpeak $(M+H)^+$: 286/288
gef.: Molpeak $(M+H)^+$: 286/288 (Cl)

Z20b) 2-Chlor-N'-(2-diethylamino-ethyl)-N'-methyl-benzene-1 ,4-diamin

Zu einer Suspension von 200 mg Raney-Ni in 20 mL MeOH wurde 1.60 mg (5.599 mmol) N-(2-Chlor-4-nitrophenyl)-N',N'-diethyl-N-methyl-ethane-1,2-diamin zugegeben und das Gemisch 2 Stunden bei RT und 20 psi $H_2$-Atmosphäre hydriert. Der Katalysator wurde abfiltriert und das Filtrat i. vac. eingeengt.
Ausbeute: 1.30 mg (91 % der Theorie)
$C_{13}H_{22}ClN_3$ (M= 255.793)
Ber. Molpeak $(M+H)^+$: 256/258
gef.: Molpeak $(M+H)^+$: 256/258

Zwischenprodukt 21:

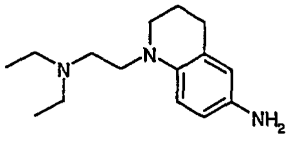

Z21a) N-[1-(2-Diethylamino-ethyl)-1,2,3,4-tetrahydro-quinolin-6-yl]-2,2,2-trifluor-acetamid

Zu einer Lösung von 3.00 g (12.284 mmol) 6-Nitro-1,2,3,4-tetrahydro-quinolin, 6.342 g (36.852 mmol) (2-Chlorethyl)-diethyl-amin und 1.68 g (7.370 mmol) TEBAC in 100 mL Toluol wurde 50 mL 50 % wässrige KOH-Lösung zugegeben und das Gemisch 1 Stunde bei 80 °C gerührt. Das Reaktionsgemisch wurde auf RT gekühlt und mit EtOAc

erschöpfend extrahiert. Die vereinigten org. Phasen wurden über Magnesiumsulfat getrocknet, i. vac. eingeengt und der Rückstand mittels Säulenchromatographie (Kieselgel, EtOAc / MeOH 9:1) gereinigt.
Ausbeute: 0.75 g (18 % der Theorie)
$C_{17}H_{24}F_3N_3O$ (M= 343.396)
Ber. Molpeak $(M+H)^+$: 344
gef.: Molpeak $(M+H)^+$: 344

Z21b) 1-(2-Diethylamino-ethyl)-1,2,3,4-tetrahydro-quinolin-6-ylamin

Zu einer Lösung von 0.75 g (2.184 mmol) N-[1-(2-Diethylamino-ethyl)-1,2,3,4-tetrahydro-quinolin-6-yl]-2,2,2-trifluor-acetamid in 5 mL MeOH wurde bei 0 °C 1.1 mL 6 M wässrige NaOH-Lösung zugegeben und das Gemisch 15 Minuten bei 0 °C und 1 Stunde bei RT gerührt.. Das Reaktionsgemisch wurde i. vac. eingeengt, ges. wässrige Natriumbicarbonat-Lösung zugegeben und mit EtOAc erschöpfend extrahiert. Die vereinigten org. Extrakte wurden über Magnesiumsulfat getrocknet, i. vac. eingeengt und der Rückstand mittels Säulenchromatographie (Kieselgel, Dichlormethan / MeOH / konz. wässriger Ammoniak 90:10:1) gereinigt.
Ausbeute: 220 mg (41 % der Theorie)
$C_{15}H_{25}N_3$ (M= 247.387)
Ber. Molpeak $(M+H)^+$: 248
gef.: Molpeak $(M+H)^+$: 248

Zwischenprodukt 22:

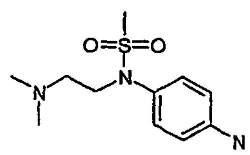

Z22a) N-(4-Nitro-phenyl)-methansulfonsäureamid

27.60 g (0.20 mol) 4-Nitroanilin wurde in 100 mL Pyridin gelöst. Bei 0°C wurden 16.3 mL (0.21 mol) Methansulfonsäurechlorid so zugetropft, dass die Reaktionstemperatur 20-25°C nicht überstieg. Anschließend wurde 2.5 Stunden bei RT gerührt. Das Reaktionsgemisch wurde unter Rühren auf 800 mL Eiswasser gegeben und 30 Minuten gerührt. Der ausgefallene Feststoff wurde abfiltriert, mit 500 mL Wasser und 100 mL EtOH gewaschen und getrocknet.
Ausbeute: 41.00 g (95 % der Theorie)
Schmelzpunkt: 183-184°C
$R_f$-Wert: 0.50 (Kieselgel, Dichlormethan / EtOAc = 90:10)

Z22b) N-(2-Dimethylamino-ethyl)-N-(4-nitro-phenyl)-methansulfonsäureamid

36.00 g (0.166 mol) N-(4-Nitro-phenyl)-methansulfonsäureamid wurde in 2000 mL Aceton gelöst. Die Lösung wurde mit 47.8 g (0.332 mol) 1-Chlor-2-dimethylaminoethan * HCl, 68.8 g (0.498 mol) Kaliumcarbonat, 5.0 g (0.033 mol) Natriumiodid und 50 mL Wasser versetzt. Es wurde 16 Stunden unter Rühren refluxiert. Nach Zugabe von weiteren 23.9 g (0.166 mol) 1-Chlor-2-dimethylaminoethan * HCl, 45.9 g (0.332 mol) Kaliumcarbonat und 5.0 g (0.033 mol) Natriumiodid wurde 5 Stunden unter Rühren refluxiert. Bei RT wurden die anorganischen Salze abfiltriert. Das Filtrat wurde i. vac. eingeengt und der Rückstand in EtOAc gelöst. Die org. Phase wurde 2x mit halbges. wässriger Natriumchlorid-Lösung gewaschen, über Magnesiumsulfat getrocknet, filtriert und i. vac. eingeengt.
Ausbeute: 30.57 g (64 % der Theorie)
$C_{11}H_{17}N_3O_4S$ (M= 287.340)
Ber. Molpeak $(M+H)^+$: 288
gef.: Molpeak $(M+H)^+$: 288
$R_f$-Wert: 0.60 (Kieselgel, Dichlormethan / MeOH / konz. wässriger Ammoniak = 90:10:1)

Z22c) N-(4-Amino-phenyl)-N-(2-dimethylamino-ethyl)-methansulfonsäureamid

9.00 g (31.3 mmol) N-(2-Dimethylamino-ethyl)-N-(4-nitro-phenyl)-methansulfonsäureamid wurden in 120 mL MeOH gelöst. Nach Zugabe von 1.0 g Palladium/Kohle 10% wurde bei RT und 50 psi $H_2$-Atmosphäre 1 Stunde hydriert. Das Reaktionsgemisch wurde filtriert und das Filtrat i. vac. eingeengt. Der Rückstand wurde mit Ether/Petrolether =1: 1 verrührt. Der Feststoff wurde abfiltriert, mit Ether/Petrolether = 1:1 gewaschen und getrocknet.
Ausbeute: 7.65 g (95 % der Theorie)
Schmelzpunkt: 151-152˚C
$R_f$-Wert: 0.40 (Kieselgel, Dichlormethan / MeOH / konz. wässriger Ammoniak = 90:10:1)

Zwischenprodukt 23:

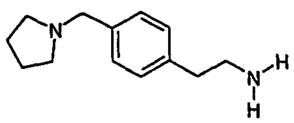

Z23a) 4-Cyanomethyl-benzoesäureethylester

Zu einer Lösung aus 147,5 g (2,263 mol) Kaliumcyanid in 250 mL heißem Wasser wird tropfenweise eine Lösung von 500 g (2,057 mol) 4-Brommethyl-benzoesäureethylester in 1000 mL Ethanol zugesetzt. Die Reaktionsmischung wird eine Stunde zum Rückfluss erhitzt und 12 Stunden bei RT gerührt. Es werden weitere 73,7 g (0,5 mol) Kaliumcyanid zugegeben und zwei Stunden zum Rückfluss erhitzt. Der in der Reaktionsmischung vorhandene Feststoff wird abfiltriert und das Filtrat über ein Gemisch aus Kieselgel und Aktivkohle filtriert. Das erhaltene Filtrat wird eingeengt und der Rückstand auf 1000 mL Wasser gegossen. Die wässrige Lösung wird mit MTBE extrahiert und die organische Phase dreimal mit Wasser extrahiert. Anschließend wird die organische Phase über Magnesiumsulfat getrocknet und das Solvens am Rotationsverdampfer abdestilliert. Die Reinigung erfolgt durch Säulenchromatographie an Kieselgel (Petrolether/ Essigsäuerethylester 8:2).
Ausbeute: 164,46 g (42,2 % der Theorie)
$C_{11}H_{11}NO_2$ (M= 189,216)
ber.: Molpeak $(M+H)^+$: 190
gef.: Molpeak $(M+H)^+$: 190
$R_f$-Wert: 0.3 (Kieselgel, Petrolether/EtOAc 8:2)

Z23b) 4-Cyanomethyl-benzoesäure

Eine Lösung von 10 g (53 mol) 4-Cyanomethyl-benzoesäureethylester und 2,02 mL einer 1 M Natronlaugelösung in 100 mL Ethanol wird eine Stunde zum Rückfluss erhitzt. Anschließend wird die Reaktionslösung eingeengt und der Rückstand mit Eiswasser versetzt. Es wird solange konzentrierte Salzsäure zur Reaktionslösung zugetropft bis kein Niederschlag mehr entsteht. Der Niederschlag wird abfiltriert, zweimal mit Wasser gewaschen und getrocknet. Ausbeute: 4,7 g (55 % der Theorie)
$C_9H_7NO_2$ (M= 161,162)
ber.: Molpeak $(M-H)^-$: 160
gef.: Molpeak $(M-H)^-$: 160

Z23c) (4-Hydroxymethyl-phenyl)-acetonitril

Zu einer Lösung von 4,7 g (29 mol) 4-Cyanomethyl-benzoesäure in 250 mL Tetrahydrofuran werden 5,17 g (32 mol) CDI gegeben und bis zum Ende der Gasentwicklung gerührt. Diese Reaktionsmischung wird zu einer Lösung von 3,29 g (87 mol) Natriumborhydrid in 200 mL Wasser derart zugetropft, dass die Temperatur 30˚C nicht übersteigt. Es wird zwei Stunden nachgerührt und die Reaktionsmischung mit Kaliumhydrogensulfat-Lösung auf pH 3-4 eingestellt. Anschließend wird mit EtOAc extrahiert, die organische Phase über Magnesiumsulfat getrocknet und das Solvens am Rotationsverdampfer abgetrennt.
Ausbeute: 2,6 g (60,9 % der Theorie)

$C_9H_9NO$ (M= 147,178)
ber.: Molpeak $(M-H)^-$: 146
gef.: Molpeak $(M-H)^-$: 146

Z23d) (4-Brommethyl-phenyl)-acetonitril

Zu einer Lösung von 2,6 g (17,66 mmol) (4-Hydroxymethyl-phenyl)-acetonitril in 25 mL MTBE werden bei 0°C 0,86 mL (9 mmol) Phosphortribromid getropft. Nach Beendigung der Reaktion wird die Reaktionsmischung bei RT mit Wasser versetzt, die organische Phase abgetrennt und diese nacheinander mit Natriumhydrogencarbonat-Lösung und Wasser extrahiert. Die organische Phase wird über Magnesiumsulfat getrocknet und das Solvens am Rotationsverdampfer abdestilliert.
Ausbeute: 2,9 g (78,1 % der Theorie)
$C_9H_8BrN$ (M= 210,075)
ber.: Molpeak $(M+H)^+$: 209/211
gef.: Molpeak $(M+H)^+$: 209/211

Z23e) (4-Pyrrolidin-1-ylmethyl-phenyl)-acetonitril

0,446 mL (5,44 mmol) Pyrrolidin und 1,366 g (9,882 mmol) Kaliumcarbonat werden in 20 mL Dimethylformamid eingetragen. Unter Rühren werden 1,038 g (4,941 mmol) (4-Brommethyl-phenyl)-acetonitril zugegeben und 12 Stunden bei RT gerührt. Die Reaktionsmischung wird am Rotationsverdampfer eingeengt und der Rückstand mit EtOAc und Wasser extrahiert. Die organische Phase wird über Magnesiumsulfat getrocknet und das Solvens am Rotationsverdampfer abgezogen.
Ausbeute: 0,732 g (74 % der Theorie)
$C_{13}H_{16}N_2$ (M= 200,286)
ber.: Molpeak $(M+H)^+$: 201
gef.: Molpeak $(M+H)^+$: 201
$R_f$-Wert: 0.5 (Kieselgel, Dichlormethan / MeOH / konz. wässriger Ammoniak 90:10:1)

Z23f) 2-(4-Pyrrolidin-1-ylmethyl-phenyl)-ethylamin

Eine Reaktionsmischung aus 0,73 g (3,66 mmol) (4-Pyrrolidin-1-ylmethyl-phenyl)-acetonitril und 0,1 g Raney-Nickel in 25 mL methanolischer Ammoniaklösung wird 9 Stunden bei 50°C und 3 bar Wasserstoff hydriert.
Ausbeute: 0,72 g (96,4 % der Theorie)
$C_{13}H_{20}N_2$ (M= 204,31)
ber.: Molpeak $(M+H)^+$: 205
gef.: Molpeak $(M+H)^+$: 205
$R_f$-Wert: 0.23 (Kieselgel, Dichlormethan / MeOH / konz. wässriger Ammoniak 90:10:1)

Zwischenprodukt 24

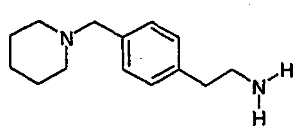

Z24a) (4-Piperidin-1-ylmethyl-phenyl)-acetonitril

Hergestellt analog Beispiel Z23e aus Piperidin und (4-Brommethyl-phenyl)-acetonitril. Ausbeute: 1,6 g (39 % der Theorie)
$C_{14}H_{18}N_2$(M= 214,31)
ber.: Molpeak $(M+H)^+$: 215
gef.: Molpeak $(M+H)^+$: 215
$R_f$-Wert: 0.4 (Kieselgel, Cyclohexan/EtOAc 1:1)

Z24b) 2-(4-Piperidin-1-ylmethyl-phenyl)-ethylamin

Hergestellt analog Beispiel Z23f aus (4-Piperidin-1-ylmethyl-phenyl)-acetonitril Ausbeute: 1,4 g (85,9 % der Theorie)
$C_{14}H_{22}N_2$ (M= 218,34)
ber.: Molpeak $(M+H)^+$: 219
gef.: Molpeak $(M+H)^+$: 219
$R_f$-Wert: 0.2 (Kieselgel, Dichlormethan/Ethanol/Ammoniak 20:1:0.1)

Zwischenprodukt 25:

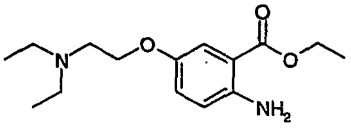

Z25a) 5-Hydroxy-2-nitrobenzoesäure-ethylester

Eine Lösung von 5.00 g (27.304 mmol) 5-Hydroxy-2-nitrobenzoesäure wurden in 200 mL ethanolischer HCl 5 h unter Rückfluss erhitzt und anschließend 48 h bei RT gerührt. Das Reaktionsgemisch wurde i. vac. eingeengt, mit EtOAc verdünnt. Die org. Phase mit Wasser gewaschen, über Magnesiumsulfat getrocknet und i. vac. eingeengt. Das Rohprodukt wurde ohne weitere Reinigung im nachfolgenden Reaktionsschritt eingesetzt.
Ausbeute: 5,00 g (87% der Theorie)
$C_9H_9NO_5$ (M= 211,176)
ber.: Molpeak $(M-H)^-$: 210
gef.: Molpeak $(M-H)^-$: 210

Z25b) 2-Nitro-5-(2-diethylamino-ethoxy)-benzoesäure-ethylester

Hergestellt analog Zwischenprodukt Z5a aus 5-Hydroxy-2-nitrobenzoesäure-ethylester und (2-Chlorethyl)-diethyl-amin-hydrochlorid
Ausbeute: 6,30 g (85% der Theorie)
$C_{15}H_{22}N_2O_5$ (M= 310,353)
ber.: Molpeak $(M+H)^+$: 311
gef.: Molpeak $(M+H)^+$: 311

Z25c) 2-Amino-5-(2-diethylamino-ethoxy)-benzoesäure-ethylester

Hergestellt analog Zwischenprodukt Z5b aus 2-Nitro-5-(2-diethylamino-ethoxy)-benzoesäure-ethylester. Säulenchromatographie (Kieselgel, EtOAc/MeOH/Ammoniak 90:10:1) ergab das Produkt.
Ausbeute: 4,00 g (71% der Theorie)
$C_{15}H_{24}N_2O_3$ (M= 280,370)
ber.: Molpeak $(M+H)^+$: 281
gef.: Molpeak $(M+H)^+$: 281

Zwischenprodukt 26: (4-Brom-2-chlor-phenoxy)-essigsäure-ethylester

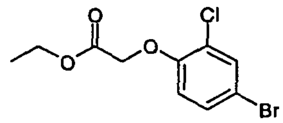

Z26) Zu einer Lösung von 7.800 g (37.222 mmol) 4-Brom-2-chlorphenol und 4.70 mL (41.537 mmol) Bromessigsäure-ethylester in 100 mL DMF wurde 14.5 mL (83.242 mmol) Hünigbase zugegeben und das Gemisch 4 h bei 100 ˚C gerührt. Das Reaktionsgemisch wurde mit Wasser versetzt und mit EtOAc erschöpfend extrahiert. Die vereinigten org. Phasen wurden mit ges. wässr. Natriumbicarbonat, Wasser und ges. wässr. NaCl gewaschen, über Natriumsulfat getrocknet und i. vac. eingeengt. Das Rohprodukt wurde ohne weitere Reinigung in den nachfolgenden Reaktionsschritt eingesetzt.
Ausbeute: 11.36 g (quant. Ausbeute)
$C_{10}H_{10}BrClO_3$ (M= 293.546)
$R_f$-Wert: 0.65 (Kieselgel, Dichlormethan)

Zwischenprodukt 27:

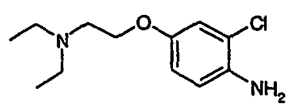

Z27a) [2-(3-Chlor-4-nitro-phenoxy)-ethyl]-diethyl-amin

Hergestellt analog zu Zwischenprodukt Z5a ausgehend von 3-Chlor-4-nitro-phenol und (2-Chlor-ethyl)-diethylamin.
Ausbeute: 1.25 g (79% der Theorie)
$C_{12}H_{17}ClN_2O_3$ (M= 272.734)
ber.: Molpeak $(M+H)^+$: 273/275
gef.: Molpeak $(M+H)^+$: 273/275 (Cl)
$R_f$-Wert: 0.44 (Kieselgel, Dichlormethan / MeOH 9:1).
Z27b) 2-Chlor-4-(2-diethylamino-ethoxy)-phenylaminEine Suspension von 1.24 g (4.547 mmol) [2-(3-Chlor-4-nitro-phenoxy)-ethyl]diethyl-amin (Z27a) und 300 mg Raney-Nickel in EtOAc wurde bei RT und 3 bar hydriert. Der Katalysator wurde abfiltriert und das Filtrat i. vac. eingeengt.
Ausbeute: 1.10 g (quant. Ausbeute)
$C_{12}H_{19}ClN_2O$ (M= 242.751)
ber.: Molpeak $(M+H)^+$: 243/245
gef.: Molpeak $(M+H)^+$: 243/245 (Cl)
$R_f$-Wert: 0.41 (Kieselgel, Dichlormethan / MeOH 9:1).

Zwischenprodukt 28:

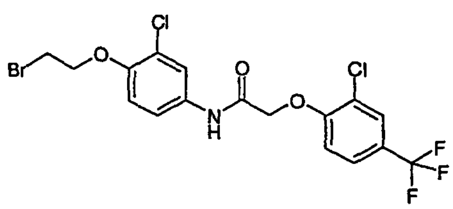

Z28a) N-(3-Chlor-4-hydroxy-phenyl)-2-(2-chlor-4-trifluormethyl-phenoxy)-acetamid

Zu einer Lösung von 7.600 g (29.850 mmol) (2-Chlor-4-trifluormethyl-phenoxy)-essigsäure (Z2b), 4.370 g (29.850 mmol) 4-Amino-2-chlor-phenol und 10.56 g (32.840 mmol) TBTU in 550 mL as. THF wurde bei RT 17.31 mL (98.510 mmol) Hünigbase zugegeben und das Gemisch 48 h gerührt. Das Reaktionsgemisch wurde i. vac. eingeengt und mit EtOAc verdünnt. Die org. Phase wurde mit Wasser, ges. wässr. Natriumbicarbonat und ges. wässr. NaCl gewaschen, über Natriumsulfat getrocknet und i. vac. eingeengt. Säulenchromatographie (Kieselgel, Gradient Dichlormethan / MeOH 99:1 → 19:1) ergab das Produkt.
Ausbeute: 4.200 g (37% der Theorie)
$C_{15}H_{10}Cl_2F_3NO_3$ (M= 380.153)
ber.: Molpeak $(M+H)^+$: 380/382/384
gef.: Molpeak $(M+H)^+$: 380/382/384 ($Cl_2$)
$R_f$-Wert: 0.58 (Kieselgel, Dichlormethan / MeOH 19:1).

Z28b) N-[4-(2-Brom-ethoxy)-3-chlor-phenyl]-2-(2-chlor-4-trifluormethyl-phenoxy)-acetamid

Zu einer Suspension von 1.580 g (4.156 mmol) N-(3-Chlor-4-hydroxy-phenyl)-2-(2-chlor-4-trifluormethyl-phenoxy)-acetamid und 2.880 g (8.310 mmol) Kaliumcarbonat in 20 mL DMF wurde bei RT eine Lösung von 3.66 mL (41.56 mmol) 1,2-Dibrom-ethan in 5 mL DMF langsam zugegeben und das Gemisch 2.5 h gerührt. Das Reaktionsgemisch wurde i. vac. eingeengt und mit EtOAc verdünnt. Die org. Phase wurde mit Wasser und ges. wässr. NaCl gewaschen, über Magnesiumsulfat getrocknet und i. vac. eingeengt. Säulenchromatographie (Kieselgel, Petrolether → Dichlormethan) ergab das Produkt.
Ausbeute: 1.120 g (55% der Theorie)
$C_{17}H_{13}BrCl_2F_3NO_3$ (M= 487.103)
ber.: Molpeak $(M+H)^+$: 486/488/490/492
gef.: Molpeak $(M+H)^+$: 486/488/490/492 ($BrCl_2$)
$R_f$-Wert: 0.72 (Kieselgel, Dichlormethan / MeOH 49:1).

Zwischenprodukt 29:

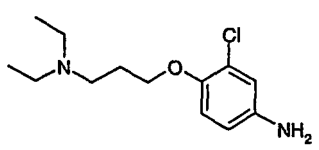

Z29a) [2-(2-Chloro-4-nitro-phenoxy)-propyl]-diethyl-amin

Zu einer Lösung von 5.30 g (30.000 mmol) 3-Chlor-4-fluor-nitrobenzol und 4.30 g (33.000 mmol) 3-Diethylamino-propanol in 50 mL abs. DMF wurde bei 0 ˚C 1.60 g (33.000 mmol) NaH (50% in Öl) zugegeben und das Gemisch 2 h bei 0 ˚C und 1 h bei RT gerührt. Das Reaktionsgemisch wurde i. vac. eingeengt und mit EtOAc verdünnt. Die org. Phase wurde mit Wasser gewaschen, über Natriumsulfat getrocknet und i. vac. eingeengt. Säulenchromatographie (Kieselgel, Dichlormethan / EtOH / konz. wässr. Ammoniak 90:10:0.1) ergab das Produkt.
Ausbeute: 8.00 g (93% der Theorie)
$C_{13}H_{19}ClN_2O_3$ (M= 286.761)
$R_f$-Wert: 0.30 (Kieselgel, Dichlormethan / MeOH / konz. wässr. Ammoniak 90:10:0.1).

Z29b) 3-Chlor-4-(3-diethylamino-propoxy)-phenylamin

Eine Suspension von 8.00 g (27.900 mmol) [2-(2-Chloro-4-nitro-phenoxy)-propyl]-diethyl-amin (Z29a) und 0.80 g Raney-Nickel in 170 mL MeOH wurde 8 h bei RT und 50 psi hydriert. Der Katalysator wurde abfiltriert und das Filtrat i. vac. eingeengt.
Ausbeute: 6.70 g (93% der Theorie)
$C_{13}H_{21}ClN_2O$ (M= 256.778)
ber.: Molpeak $(M+H)^+$: 257/259

gef.: Molpeak $(M+H)^+$: 257/259 (Cl)
$R_f$-Wert: 0.40 (Kieselgel, Dichlormethan / MeOH / konz. wässr. Ammoniak 50:10:0.1).

Zwischenprodukt 30:

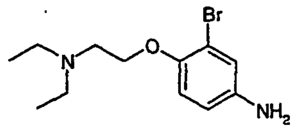

Z30a) [2-(2-Brom-4-nitro-phenoxy)-ethyl]-diethyl-amin

Hergestellt analog Zwischenprodukt Z29a ausgehend von 2-Brom-1-fluor-4-nitrobenzol und 2-Diethylamino-ethanol.
Ausbeute: 0.790 g (83% der Theorie)
$C_{12}H_{17}BrN_2O_3$ (M= 317.185)
ber.: Molpeak $(M+H)^+$: 317/319
gef.: Molpeak $(M+H)^+$: 317/319 (Br)
$R_f$-Wert: 0.48 (Kieselgel, Dichlormethan / MeOH 9:1).

Z30b) 3-Brom-4-(2-diethylamino-ethoxy)-phenylamin

Hergestellt analog Zwischenprodukt Z29b ausgehend von [2-(2-Brom-4-nitro-phenoxy)-ethyl]-diethyl-amin (Z30a).
Ausbeute: 0.670 g (96% der Theorie)
$C_{12}H_{19}BrN_2O$ (M= 287.202)
ber.: Molpeak $(M+H)^+$: 287/289
gef.: Molpeak $(M+H)^+$: 287/289 (Br) $R_f$-Wert: 0.30 (Kieselgel, Dichlormethan / MeOH 9:1).

Zwischenprodukt 31:

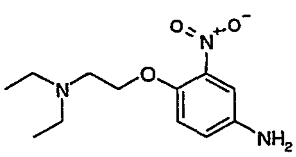

Z31a) N-[4-(2-Diethylamino-ethoxy)-3-nitro-phenyl]-acetamid

Zu einer Lösung von 1.520 g (6.072 mmol) N-[4-(2-Diethylamino-ethoxy)-phenyl]-acetamid in 25 mL konz. Schwefelsäure wurde bei -10 ˚C portionsweise 0.737 g (7.286 mmol) Kaliumnitrat zugegeben und das Gemisch 1 h bei -10 ˚C gerührt. Das Reaktionsgemisch wurde auf ein Gemisch aus Eis und konz. wässr. Ammoniak gegossen und die wässr. Phase mit EtOAc erschöpfend extrahiert. Die vereinigten org. Phasen wurde über Natriumsulfat getrocknet und i. vac. eingeengt.
Ausbeute: 1.8 g (quant. Ausbeute)
$C_{14}H_{21}N_3O_4$ (M= 295.341)
ber.: Molpeak $(M+H)^+$: 296
gef.: Molpeak $(M+H)^+$: 296 $R_f$-Wert: 0.51 (Alox, Dichlormethan / MeOH 39:1).

Z31b) 4-(2-Diethylamino-ethoxy)-3-nitro-phenylamin

Eine Lösung von 1.85 g (6.264 mmol) N-[4-(2-Diethylamino-ethoxy)-3-nitro-phenyl]-acetamid (Z31a) in halbkonz. wässr. HCl wurde 2 h bei 100 ˚C gerührt, auf RT gekühlt, mit Eis und konz. wässr. Ammoniak basisch gestellt und die wässr. Phase wurde mit EtOAc erschöpfend extrahiert. Die vereinigten org. Phasen wurden mit Wasser gewaschen und über Natriumsulfat getrocknet.
Ausbeute: 1.38 g (87% der Theorie)
$C_{12}H_{19}N_3O_3$ (M= 253.304)
ber.: Molpeak $(M+H)^+$: 254
gef.: Molpeak $(M+H)^+$: 254 $R_f$-Wert: 0.68 (Alox, Dichlormethan / MeOH 39:1).

Zwischenprodukt 32:

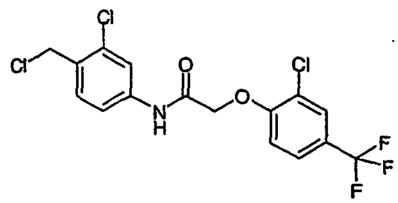

Z32a) N-(3-Chlor-4-hydroxymethyl-phenyl)-2-(2-chlor-4-trifluormethyl-phenoxy)-acetamid

Zu einer Lösung von 1.018 g (4.000 mmol) aus (2-Chlor-4-trifluormethyl-phenoxy)-essigsäure (Z2b) und 0.46 mL (4.200 mmol) N-Methylmorpholin in 5 mL abs. DMF wurde -5 ˚C langsam 0.55 mL (4.200 mmol) Chlorameisensäure-iso-propylester zugetropft und das Gemisch weiter 5 Min. gerührt. 0.662 g (4.200 mmol) (4-Amino-2-chlor-phenyl)-methanol wurde bei -5 ˚C zugegeben, das Gemisch 2 h bei RT gerührt und dann das Reaktionsgemisch auf Eiswasser gegossen. Der Niederschlag wurde abfiltriert, mit Wasser gewaschen und im HV getrocknet.
Ausbeute: 1.360 g (83% der Theorie)
$C_{16}H_{12}Cl_2F_3NO_3$ (M= 394.180)
$R_f$-Wert: 0.50 (Kieselgel, Dichlormethan / MeOH 19:1).

Z32b) N-(3-Chlor-4-chlormethyl-phenyl)-2-(2-chlor-4-trifluormethyl-phenoxy)-acetamid

Zu einer Lösung von 1.620 g (4.110 mmol) N-(3-Chlor-4-hydroxymethyl-phenyl)-2-(2-chlor-4-trifluormethyl-phenoxy)-acetamid (Z32a) in 30 mL Toluol wurde 1.12 mL (16.440 mmol) Thionylchlorid zugegeben und das Gemisch 2 h bei 80 ˚C gerührt. Das Reaktionsgemisch wurde i. vac. eingeengt und der Rückstand aus Ether/Petrolether umkristallisiert. Der Niederschlag wurde abfiltriert, mit Petrolether gewaschen und im HV getrocknet.Ausbeute: 1.100 g (65% der Theorie)
$C_{16}H_{11}Cl_3F_3NO_2$(M= 412.626)
ber.: Molpeak $(M+H)^+$: 412/414/416/418
gef.: Molpeak $(M+H)^+$: 412/414/416/418 ($Cl_3$)
$R_f$-Wert: 0.69 (Kieselgel, Petrolether / EtOAc 3:1).

Zwischenprodukt 33:

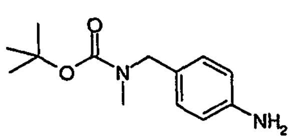

Z33a) Methyl-(4-nitro-benzyl)-carbaminsäure-tert-butylester

Zu einer Lösung von 13.40 g (81.00 mmol) Methyl-(4-nitro-benzyl)-amin in 25 mL EtOAc wurde bei 0 °C langsam 17.68 g (81.00 mmol) Boc-Anhydrid zugegeben und das Gemisch 3 h bei RT gerührt. Die org. Phase wurde mit Wasser gewaschen, über Natriumsulfat getrocknet und i. vac. eingeengt.
Ausbeute: 21.36 g (99% der Theorie)
$C_{13}H_{18}N_2O_4$ (M= 266.299)
$R_f$-Wert: 0.60 (Kieselgel, Petrolether / EtOAc 3:7).

Z33b) (4-Amino-benzyl)-methyl-carbaminsäure-tert-butylester

Eine Suspension von 23.00 g (86.00 mmol) Methyl-(4-nitro-benzyl)-carbaminsäure-tert-butylester (Zwischenprodukt Z33a) und 2.30 g Raney-Nickel in 460 mL EtOH/EtOAc (1:1) wurde bei RT und 3 bar hydriert. Der Katalysator wurde abfiltriert, das Filtrat i. vac. eingeengt und der Rückstand mittels Säulenchromatographie (Kieselgel, Petrolether / EtOAc 1:1) gereinigt.
Ausbeute: 9.23 g (45% der Theorie)
$C_{13}H_{20}N_2O_2$ (M= 236.317)
ber.: Molpeak $(M+H)^+$: 237
gef.: Molpeak $(M+H)^+$: 237
$R_f$-Wert: 0.40 (Kieselgel, Petrolether / EtOAc 1:1).

Zwischenprodukt 34:

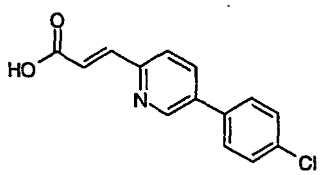

Z34a) (E)-3-[5-(4-Chlor-phenyl)-pyridin-2-yl]-acrylsäure-ethylester

Zu einer Lösung von 1.100 g (5.050 mmol) 5-(4-Chlor-phenyl)-pyridine-2-carbaldehyd in 50 mL abs. THF wurde bei RT 2.000 g (5.560 mmol) (Ethoxycarbonylmethylen)-triphenylphosphoran zugegeben und das Gemisch 4h bei RT gerührt. Das Reaktionsgemisch wurde i. vac. eingeengt und der Rückstand mittels Säulenchromatographie (Alox, neutral, Akt. II-III, Petrolether / EtOAc 5:1) gereinigt. Umkristallisation aus Petrolether ergab das Produkt.
Ausbeute: 1.200 g (83% der Theorie)
$C_{16}H_{14}ClNO_2$ (M= 287.748)
ber.: Molpeak $(M+H)^+$: 288/290
gef.: Molpeak $(M+H)^+$: 288/290 (Cl)
$R_f$-Wert: 0.60 (Kieselgel, Petrolether / EtOAc 5:1).

Z34b) (E)-3-[5-(4-Chlor-phenyl)-pyridin-2-yl]-acrylsäure

Zu einer Suspension von 1.200 g (4.200 mmol) (E)-3-[5-(4-Chlor-phenyl)-pyridin-2-yl]-acrylsäure-ethylester (Z34a) in 50 mL EtOH wurde bei RT 12.6 mL wässr. NaOH (1 M) zugegeben und das Gemisch 1 h gerührt. 12.6 mL wässr. HCl (1 M) wurde bei 0 °C zugegeben. Der gebildete Niederschlag wurde abfiltriert, mit Wasser gewaschen und i. vac. bei 100 °C getrocknet.
Ausbeute: 1.000 g (92% der Theorie)
$C_{14}H_{10}ClNO_2$ (M= 259.694)
ber.: Molpeak $(M+H)^+$: 260/262
gef.: Molpeak $(M+H)^+$: 260/262 (Cl)
$R_f$-Wert: 0.40 (Kieselgel, Dichlormethan / MeOH 9:1).
Zwischenprodukt 35:

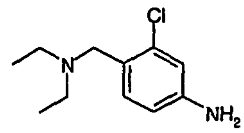

Z35a) (2-Chlor-4-nitro-benzyl)-diethyl-amin

Zu einer Lösung von 2.60 mL (25.000 mmol) Diethylamin in 50 mL THF wurde 0.80 g (3.883 mmol) 2-Chlor-1-chlormethyl-4-nitro-benzol zugegeben und das Gemisch 8 h unter Rückfluss erhitzt. Das Reaktionsgemisch wurde i. vac. eingeengt und der Rückstand in EtOAc aufgenommen. Die org. Phase wurde mit Wasser gewaschen, über Natriumsulfat getrocknet und i. vac. eingeengt.
Ausbeute: 0.750 g (80% der Theorie)
$C_{11}H_{15}ClN_2O_2$ (M= 242.707)
ber.: Molpeak $(M+H)^+$: 243/245
gef.: Molpeak $(M+H)^+$: 243/245 (Cl)
$R_f$-Wert: 0.50 (Alox, Petrolether).

Z35b) 3-Chlor-4-diethylaminomethyl-phenylamin

Eine Suspension von 0.700 g (2.884 mmol) (2-Chlor-4-nitro-benzyl)-diethyl-amin (Z35a) und 0.400 g Raney-Nickel in 20 mL THF wurde 7.5 h bei RT und 25 psi hydriert. Der Katalysator wurde abfiltriert und das Filtrat i. vac. eingeengt. Das Rohprodukt wurde mittels Säulenchromatographie (Alox, neutral, Akt. II-III, Petrolether / EtOAc 4:1) gereinigt.
Ausbeute: 0.510 g (83% der Theorie)
$C_{11}H_{17}ClN_2$(M= 212.725)
ber.: Molpeak $(M+H)^+$: 213/215
gef.: Molpeak $(M+H)^+$: 213/215 (Cl)
$R_f$-Wert: 0.58 (Alox, Petrolether / EtOAc 3:1).

Zwischenprodukt 36:

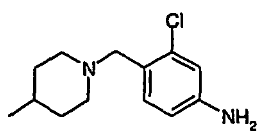

Z36a) 1-(2-Chlor-4-nitro-benzyl)-4-methyl-piperidin

Zu 2.00 mL (16.223 mmol) 4-Methylpiperidin wurde bei RT langsam 1.00 g (4.854 mmol) 2-Chlor-1-chlormethyl-4-nitro-benzol zugegeben und das Gemisch weitere 15 Min. gerührt. EtOAc wurde zugegeben, die org. Phase mit Wasser gewaschen, über Natriumsulfat getrocknet und i. vac. eingeengt. $C_{13}H_{17}ClN_2O_2$(M= 268.746)
ber.: Molpeak $(M+H)^+$: 269/271
gef.: Molpeak $(M+H)^+$: 269/271 (Cl)
$R_f$-Wert: 0.40 (Alox, Petrolether).

Z36b) 3-Chlor-4-(4-methyl-piperidin-1-ylmethyl)-phenylamin

Hergestellt analog Zwischenprodukt Z35b ausgehend von 1-(2-Chlor-4-nitro-benzyl)-4-methyl-piperidin (Z36a). Das Rohprodukt wurde mittels Säulenchromatographie (Alox, neutral, Akt. II-III, Gradient Petrolether / EtOAc 6:1 → 3:1) gereinigt.
Ausbeute: 0.930 g (80% der Theorie)
$C_{13}H_{19}ClN_2$ (M= 238.763)

ber.: Molpeak $(M+H)^+$: 239/241
gef.: Molpeak $(M+H)^+$: 239/241 (Cl)
$R_f$-Wert: 0.58 (Alox, Petrolether / EtOAc 3:1).

Zwischenprodukt 37:

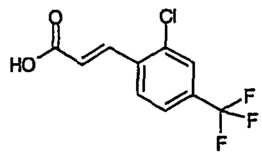

Z37a) (E)-3-(2-Chlor-4-trifluormethyl-phenyl)-acrylsäure-ethylester

Zu 21.48 g (68.69 mmol) 2-Chlor-1-iod-4-trifluormethyl-benzol und 10.45 mL (96.17 mmol) Acrylsäure-ethylester in 100 mL Acetonitril wurde 0.157 g (0.686 mmol) Palladium(II)acetat, 0.800 g (2.550 mmol) Tri-o-tolylphosphin und 23.94 mL (171.73 mmol) Triethylamin zugegeben und das Gemisch 6 h unter Rückfluss erhitzt. Das Reaktionsgemisch wurde i. vac. eingeengt und der Rückstand mit EtOAc verrieben. Der Niederschlag wurde abfiltriert und im HV getrocknet. Das Rohprodukt wurde ohne weitere Reinigung in den nachfolgenden Reaktionsschritt eingesetzt.
Ausbeute: 19.1 g (quant. Ausbeute)
$C_{12}H_{10}ClF_3O_2$ (M= 278.661)
$R_f$-Wert: 0.65 (Kieselgel, Petrolether / EtOAc 6:1).

Z37b) (E)-3-(2-Chlor-4-trifluormethyl-phenyl)-acrylsäure

Zu einer Lösung von 19.1 g (68.61 mmol) (E)-3-(2-Chlor-4-trifluormethyl-phenyl)-acrylsäure-ethylester (Z37a) in 100 mL EtOH wurde langsam eine Lösung von 5.80 g (145.00 mmol) NaOH in 30 mL Wasser zugegeben und das Gemisch 1 h bei RT gerührt. EtOH wurde i. vac. eingedampft, die wässr. Phase mit EtOAc gewaschen und mit halbkonz. wässr. HCl auf pH 1 angesäuert. Der Niederschlag wurde abfiltriert, mit Wasser gewaschen und im HV getrocknet.
Ausbeute: 15.80 g (92% der Theorie)
$C_{10}H_6ClF_3O_2$(M= 250.606)
ber.: Molpeak $(M+H)^+$: 249/250
gef.: Molpeak $(M+H)^+$: 249/250 (Cl)
$R_f$-Wert: 0.50 (Kieselgel, Petrolether / EtOAc 3:1).

Zwischenprodukt 38:

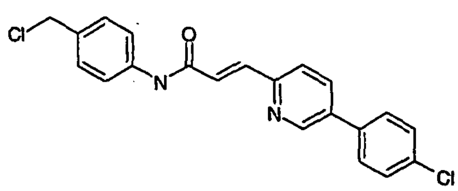

Z38a) (E)-3-[5-(4-Chlor-phenyl)-pyridin-2-yl]-*N*-(4-hydroxymethyl-phenyl)-acrylamid

Zu einer Lösung von 0.180 g (0.690 mmol) (E)-3-[5-(4-Chlor-phenyl)-pyridin-2-yl]-acrylsäure (Z34b) und 80 µL (0.720 mmol) N-Methylmorpholin in 5 mL abs. DMF wurde bei -6 °C 90 µL (0.720 mmol) Chlorameisensäure-isobutylester zugetropft und das Gemisch 5 Min. gerührt. 90 mg (0.720 mmol) 4-Amino-benzylalkohol wurde zugegeben und das Gemisch 2 h gerührt. Das Reaktionsgemisch wurde auf Wasser gegossen und der Niederschlag abfiltriert. Der

Niederschlag wurde in Toluol suspendiert, i. vac. eingedampft, anschliessend mit Ether verrührt und i. vac. bei 80 ˚C getrocknet.
Ausbeute: 0.210 g (83% der Theorie)
$C_{21}H_{17}ClN_2O_2$(M= 364.835)
ber.: Molpeak $(M+H)^+$: 365/367
gef.: Molpeak $(M+H)^+$: 365/367 (Cl)
$R_f$-Wert: 0.30 (Kieselgel, Dichlormethan / MeOH / HOAC 90:10:0.1).

Z38b) (E)-*N*-(4-Chlormethyl-phenyl)-3-[5-(4-chlor-phenyl)-pyridin-2-yl]-acrylamid

Zu einer Suspension von 0.190 g (0.520 mmol) (E)-3-[5-(4-Chlor-phenyl)-pyridin-2-yl]-N-(4-hydroxymethyl-phenyl)-acrylamid (Z38a) in 20 mL Dichlormethan wurde 50 μL (0.680 mmol) Thionylchlorid zugegeben und das Gemisch 2 h bei RT gerührt. Weiter 50 μL Thionylchlorid wurden zugegeben und erneut 3 h gerührt. Das Lösungsmittel wurde i. vac. eingedampft, der Rückstand zweimal in Toluol aufgenommen und i. vac. eingedampft. Das Rohprodukt wurde als HCl-Salz erhalten und ohne weiter Reinigung in den nachfolgenden Reaktionsschritt eingesetzt.
Ausbeute: 0.132 g (60% der Theorie)
$C_{21}H_{16}Cl_2N_2O$ * HCl (M= 419.741)
ber.: Molpeak $(M+H)^+$: 383/385/387
gef.: Molpeak $(M+H)^+$: 383/385/387 ($Cl_2$)
$R_f$-Wert: 0.90 (Kieselgel, Dichlormethan / MeOH / konz. wässr. Ammoniak 90:10:0.1).

Zwischenprodukt 39:

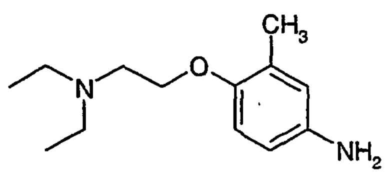

Z39a) Diethyl-[2-(2-methyl-4-nitro-phenoxy)-ethyl]-amin

Zu einer Lösung von 2.70 g (17.4 mmol) 2-Fluor-5-nitro-toluol und 2.54 mL (19.2 mmol) 2-Diethylaminoethanol in 50 mL DMF wurde unter Argonatmosphäre bei 0 ˚C 0.92 g (19.2 mmol) Natriumhydrid (50-prozentig in Öl) zugegeben und das Gemisch 2 Stunden bei 0 ˚C und 1 Stunde bei Raumtemperatur gerührt. Das Lösungsmittel wurde abgezogen, der Rückstand in Essigester aufgenommen und mit Wasser extrahiert. Die organische Phase wurde über Natriumsulfat getrocknet und i. vac. eingeengt. Anschließend wurde durch Kieselgel-Säulenchromatographie mit Dichlormethan/Methanol 9:1 als Laufmittel aufgereinigt.
Ausbeute: 3,1 g (71 % der Theorie)
$C_{13}H_{20}N_2O_3$ (M= 252.31)
Ber. Molpeak $(M+H)^+$: 253
Gef.: Molpeak $(M+H)^+$: 253
$R_f$-Wert: 0.60 (Kieselgel, Dichlormethan / MeOH 9:1)

Z39b) 4-(2-Diethylamino-ethoxy)-3-methyl-phenylamin

3.10 g (12.3 mmol) Diethyl-[2-(2-methyl-4-nitro-phenoxy)-ethyl]-amin wurden in 250 mL Essigester gelöst, 0.55 g Raney-Nickel zugegeben und das Gemisch wurde 36 Stunden bei 50 psi und Raumtemperatur hydriert. Der Katalysator wurde abfiltriert und das Filtrat i. vac. eingeengt.
Ausbeute: 2.70 g (99% der Theorie)
$C_{13}H_{22}N_2O$ (M= 222.33)
Ber. Molpeak $(M+H)^+$: 223
Gef.: Molpeak $(M+H)^+$: 223
$R_f$-Wert: 0.35 (Kieselgel, Dichlormethan/Methanol 9:1)

Zwischenprodukt 40:

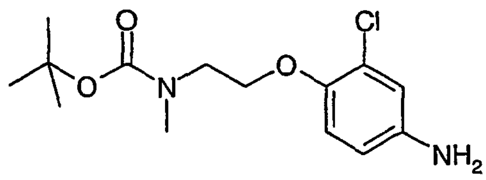

Z40a) N-tert.Butoxycarbonyl-N-methyl-[2-(2-chlor-4-nitro-phenoxy)-ethyl]-amin

Das Produkt wurde analog zu Zwischenprodukt 29a ausgehend von 2.00 g (11.4 mmol) 3-Chlor-4-fluor-nitrobenzol, 2.10 g (12.0 mmol) N-tert.Butoxycarbonyl-N-methyl-aminoethanol und 820 mg (17.1 mmol) Natriumhydrid (50-prozentig in Öl) erhalten. Ausbeute: 3.77 g (100 % der Theorie)
$C_{14}H_{19}ClN_2O_5$ (M= 330.77)
$R_f$-Wert: 0.35 (Kieselgel, Petrolether/Essigester 4:1)

Z40b) 4-(N-tert.Butoxycarbonyl-2-methylamino-ethoxy)-3-chlor-phenylamin

Das Produkt wurde analog zu Zwischenprodukt 29b ausgehend von 4.19 g (12.7 mmol) N-tert.Butoxycarbonyl-N-methyl-[2-(2-chlor-4-nitro-phenoxy)-ethyl]-amin durch Hydrierung mit 500 mg Raney-Nickel bei 3 bar erhalten.
Ausbeute: 3.68 g (94 % der Theorie)
$C_{14}H_{21}ClN_2O_3$ (M= 300.78)
Ber. Molpeak $(M+H)^+$: 301/303
Gef.: Molpeak $(M+H)^+$: 301/303
$R_f$-Wert: 0.60 (Kieselgel, Dichlormethan/Methanol 19:1)

Zwischenprodukt 41:

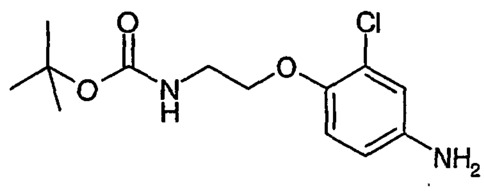

Z41a) N-tert.Butoxycarbonyl-[2-(2-chlor-4-nitro-phenoxy)-ethyl]-amin

Das Produkt wurde analog zu Zwischenprodukt 29a ausgehend von 2.00 g (11.4 mmol) 3-Chlor-4-fluor-nitrobenzol, 1.85 mL (12.0 mmol) N-tert.Butoxycarbonyl-aminoethanol und 820 mg (17.1 mmol) Natriumhydrid (50-prozentig in Öl) erhalten. Ausbeute: 2.25 g (62 % der Theorie)
$C_{13}H_{17}ClN_2O_5$ (M= 316.74)
Ber. Molpeak $(M-H)^-$: 315/317
Gef.: Molpeak $(M-H)^-$: 315/317
$R_f$-Wert: 0.45 (Kieselgel, Petrolether/Essigester 4:1)

Z41b) 4-(N-tert.Butoxycarbonyl-2-amino-ethoxy)-3-chlor-phenylamin

Das Produkt wurde analog zu Zwischenprodukt 29b ausgehend von 2.25 g (7.10 mmol) N-tert.Butoxycarbonyl-[2-(2-chlor-4-nitro-phenoxy)-ethyl]-amin durch Hydrierung mit 500 mg Raney-Nickel bei 3 bar erhalten.

Ausbeute: 1.95 g (81 % der Theorie)
$C_{13}H_{19}ClN_2O_3$ (M= 286.76)
Ber. Molpeak $(M+H)^+$: 287/289
Gef.: Molpeak $(M+H)^+$: 287/289
$R_f$-Wert: 0.55 (Kieselgel, Dichlormethan/Methanol 19:1)

Zwischenprodukt 42:

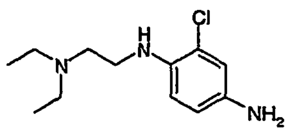

Z42a) *N'*-(2-Chlor-4-nitro-phenyl)-*N,N*-diethyl-ethan-1,2-diamin

Zu einer Lösung von 4.96 mL (34.160 mmol) *N*[1],*N*[1]-diethyl-ethan-1,2-diamin in 64 mL DMF wurde 9.440 g (34.120 mmol) Kaliumcarbonat zugegeben und das Gemisch 15 Min. bei RT gerührt. 6.120 g (68.240 mmol) 2-Chlor-1-fluor-4-nitro-benzol wurde zugegeben und 16 h bei RT gerührt. Das Reaktionsgemisch wrude auf Eiswasser gegossen, der Niederschlag abfiltriert und mittels Säulenchromatographie (Alox, neutral, Akt. II-III, Dichlormethan /MeOH 49:1) gereinigt.
Ausbeute: 9.20 g (99 % der Theorie)
$C_{12}H_{18}ClN_3O_2$ (M= 271.749)
Ber. Molpeak $(M+H)^+$: 272/274
Gef.: Molpeak $(M+H)^+$: 272/274
$R_f$-Wert: 0.72 (Alox, Dichlormethan/Methanol 49:1)

Z42b) 2-Chlor-N'-(2-diethylamino-ethyl)-benzol-1,4-diamin

Eine Suspension von 8.850 g (32.570 mmol) *N'*-(2-Chlor-4-nitro-phenyl)-*N,N*-diethyl-ethan-1,2-diamin und 4.00 g Raney-Nickel in 200 mL THF wurden 7 h bei 20 psi Wasserstoffdruck hydriert. Der Katalysator wurde abfiltriert, das Filtrat i. vac. eingeengt und der Rückstand mittels Säulenchromatographie (Alox, neutral, Akt. II-III, Gradient Dichlormethan /MeOH 100:0 → 24:1) gereinigt.
Ausbeute: 6.150 g (78 % der Theorie)
$C_{12}H_{20}ClN_3$ (M= 241.766)
Ber. Molpeak $(M+H)^+$: 242/244
Gef.: Molpeak $(M+H)^+$: 242/244
$R_f$-Wert: 0.62 (Alox, Dichlormethan/Methanol 49:1)

Zwischenprodukt 43:

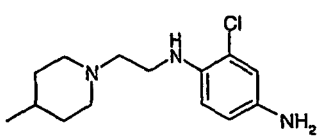

Z43a) [2-(4-Methyl-piperidin-1-yl)-ethyl]-carbaminsäure-tert-butylester

Eine Lösung von 10.00 g (43.280 mmol) (2-Brom-ethyl)-carbaminsäure-tert-butylester und 11.60 mL (96.000 mmol) 4-Methyl-piperidin in 100 mL Dichlormethan wurde 16 h bei RT gerührt. Das Rohprodukt wurde über Alox (neutral, Akt. II-III, Dichlormethan / MeOH 49:1) filtriert und das Filtrat i. vac. eingeengt.

Ausbeute: 6.150 g (78 % der Theorie)
$C_{13}H_{26}N_2O_2$ (M= 242.364)
Ber. Molpeak $(M+H)^+$: 243
Gef.: Molpeak $(M+H)^+$: 243
$R_f$-Wert: 0.65 (Alox, Dichlormethan/Methanol 19:1)

Z43b) 2-(4-Methyl-piperidin-1-yl)-ethylamin-bis-trifluoracetat

Zu einer Lösung von 8.500 g (35.070 mmol) [2-(4-Methyl-piperidin-1-yl)-ethyl]-carbaminsäure-tert-butylester in 100 mL Dichlormethan wurde bei RT 11.56 mL (150 mmol) TFA zugegeben und das Gemisch 16 h bei RT gerührt. Das Reaktionsgemisch wurde i. vac. eingeengt und der Rückstand mit Ether verrührt. Der Niederschlag wurde abfiltriert, mit Ether gewaschen und im HV getrocknet. Das Produkt wurde als Bistrifluoracetat-Salz erhalten.
Ausbeute: 12.10 g (93 % der Theorie)
$C_8H_{18}N_2$ * 2 $C_2HF_3O_2$ (M= 370.295)
Ber. Molpeak $(M+H)^+$: 143
Gef.: Molpeak $(M+H)^+$: 143

Z43c) (2-Chlor-4-nitro-phenyl)-[2-(4-methyl-piperidin-1-yl)-ethyl]-amin

Hergestellt analog Zwischenprodukt Z42a ausgehend von 12.02 g (32.450 mol) 2-(4-Methyl-piperidin-1-yl)-ethylamin-bis-trifluoracetat, 5.810 g (32.450 mmol) 2-Chlor-1-fluor-4-nitro-benzol und 17.94 g (129.64 mmol) Kaliumcarbonat.
Ausbeute: 8.85 g (92% der Theorie)
$C_{14}H_{20}ClN_3O_2$ (M= 297.787)
Ber. Molpeak $(M+H)^+$: 298/300
Gef.: Molpeak $(M+H)^+$: 298/300

Z43d) 2-Chlor-*N*'-[2-(4-methyl-piperidin-1-yl)-ethyl]-benzene-1,4-diamin

Hergestellt analog Zwischenprodukt Z42b ausgehend von 8.715 g (29.270 mmol) (2-Chlor-4-nitro-phenyl)-[2-(4-methyl-piperidin-1-yl)-ethyl]-amin.
Ausbeute: 7.00 g (89 % der Theorie)
$C_{14}H_{22}ClN_3$ (M= 267.805)
Ber. Molpeak $(M+H)^+$: 268/280
Gef.: Molpeak $(M+H)^+$: 268/280 (Cl)
$R_f$-Wert: 0.60 (Alox, Dichlormethan/Methanol 49:1)

Zwischeprodukt 44:

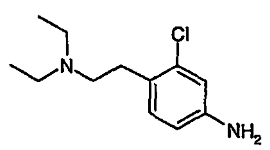

Z44a) (2-Chlor-4-nitro-phenyl)-acetyl-chlorid

Eine Suspension von 8.100 g (37.571 mmol) (2-Chlor-4-nitro-phenyl)-essigsäure in 40 mL Thionylchlorid wurde 2 h unter Rückfluss erhitzt, auf RT gekühlt und i. vac. eingeengt. Das Rohprodukt wurde ohne weitere Reinigung in den nachfolgenden Reaktionsschritt eingesetzt. Ausbeute: 8.80 g (quant. Ausbeute) $C_8H_5Cl_2NO_3$ (M= 234.040)

Z44b) 2-(2-Chlor-4-nitro-phenyl)-*N,N*-diethyl-acetamid

Zu einer Lösung von 5.67 mL (54.000 mmol) Diethylamin in 50 mL EtOAc wurde bei 0 °C eine Lösung von 3.20 g (13.673 mmol) (2-Chlor-4-nitro-phenyl)-acetyl-chlorid in 50 mL EtOAc langsam zugetropft, anschliessend das Kühlbad entfernt und das Gemisch 2 h bei RT gerührt. Das Reaktionsgemisch wurde mit EtOAc verdünnt, die org. Phase

mit Wasser und ges. wässr. NaCl-Lösung gewaschen, über Natriumsulfat getrocknet und i. vac. eingeengt.
Ausbeute: 3.70 g (quant. Ausbeute)
$C_{12}H_{15}ClN_2O_3$ (M= 270.718)
Ber. Molpeak $(M+H)^+$: 271/273
Gef.: Molpeak $(M+H)^+$: 271/273 (Cl)
$R_f$-Wert: 0.45 (Kieselgel, Petrolether /EtOAc 1:1)

Z44c) [2-(2-Chlor-4-nitro-phenyl)-ethyl]-diethyl-amin

Zu einer Lösung von 3.702 g (13.673 mmol) 2-(2-Chlor-4-nitro-phenyl)-*N,N*-diethylacetamid in 130 mL THF wurde bei RT 65 mL (65.000 mmol) Boran (1 M in THF) zugegeben und das Gemisch 4 h gerührt. Das Reaktionsgemisch wurde i. vac. eingeengt, der Rückstand mit 15 mL MeOH und 15 mL halbkonz. wässr. HCl versetzt und 15 Min. auf 100 ˚C erhitzt. Wasser wurde zugegeben, mit wässr. Natriumcarbonat-Lösung basisch gestellt und die wässr. Phase mit EtOAc extrahiert. Die vereinigten org. Phasen wurden mit Wasser und ges. wässr. NaCl-Lösung gewaschen, über Natriumsulfat getrocknet und i. vac. eingeengt. Das Rohprodukt wurde mittels Säulenchromatographie (Alox, neutral, Akt. II-III, Gradient Petrolether /EtOAc 8:1 → 4:1) gereinigt.
Ausbeute: 2.10 g (60% der Theorie)
$C_{12}H_{17}ClN_2O_2$ (M= 256.734)
$R_f$-Wert: 0.63 (Alox, Petrolether / EtOAc 3:1)

Z44d) 3-Chloro-4-(2-diethylamino-ethyl)-phenylamin

Eine Suspension von 2.00 g (7.790 mmol) [2-(2-Chlor-4-nitro-phenyl)-ethyl]-diethylamin und 0.80 g Raney-Nickel in THF wurde 2.5 h bei RT und 25 psi Wasserstoffdruck hydriert. Der Katalysator wurde abfiltriert und das Filtrat i. vac. eingeengt.
Ausbeute: 3.70 g (quant. Ausbeute)
$C_{12}H_{19}ClN_2$ (M= 226.752)
Ber. Molpeak $(M+H)^+$: 227/229
Gef.: Molpeak $(M+H)^+$: 227/229 (Cl)

Zwischenprodukt 45:

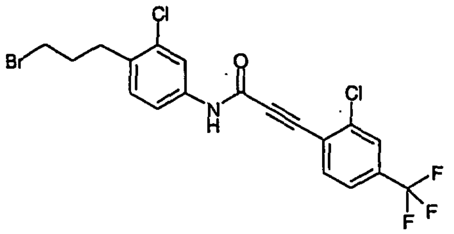

Z45a) 3-(2-Chlor-4-nitro-phenyl)-prop-2-yn-1-ol

Zu 5.013 g (21.200 mmol) 4-Brom-3-chlornitrobenzol, 8.72 mL (63.000 mmol) Triethylamin, 1.265 g (1.800 mmol) Bis(triphenylphosphin)-palladium(II)dichlorid und 0.343 g (1.800 mmol) Kupfer(I)iodid in 50 mL Acetonitril wurde unter Argon 1.059 mL (18.000 mmol) Propargylalkohol zugegeben und das Gemisch 4 h unter Rückfluss erhitzt. Das Reaktionsgemisch wurde über Celite filtriert und das Filtrat i. vac. eingeengt. Der Rückstand wurde in EtOAc gelöst, die org. Phase mit Wasser gewaschen, über Natriumsulfat getrocknet und i. vac. eingeengt. Das Rohprodukt wurde mittels Säulenchromatographie (Kieselgel, Petrolether /EtOAc 3:1) und durch Verreiben mit Petrolether gereinigt.
Ausbeute: 2.550 g (67% der Theorie)
$C_9H_6ClNO_3$ (M= 211.606)
Ber. Molpeak $(M+H)^+$: 211/213
Gef.: Molpeak $(M+H)^+$: 211/213 (Cl)
$R_f$-Wert: 0.38 (Kieselgel, Dichlormethan / MeOH 50:1)

Z45b) 3-(2-Chlor-4-nitro-phenyl)-propan-1-ol

Eine Suspension von 1.25 g (6.808 mmol) 3-(2-Chlor-4-nitro-phenyl)-prop-2-yn-1-ol und 1.00 g Raney-Nickel in 50 mL THF wurde 12 h bei RT und 25 psi Wasserstoffdruck hydriert. Der Katalysator wurde abfiltriert und das Filtrat i. vac. eingeengt.
Ausbeute: 1.26 g (quant. Ausbeute)
$C_9H_{12}ClNO$ (M= 185.655)
Ber. Molpeak $(M+H)^+$: 186/188
Gef.: Molpeak $(M+H)^+$: 186/188 (Cl)
$R_f$-Wert: 0.33 (Kieselgel, Dichlormethan / MeOH 19:1)

Z45c) (E)-*N*-[3-Chlor-4-(3-hydroxy-propyl)-phenyl]-3-(2-chlor-4-trifluormethyl-phenyl)-acrylamid

Hergestellt analog Zwischenprodukt Z32a ausgehend von (E)-3-(2-Chlor-4-trifluormethyl-phenyl)-acrylsäure (Z37b) und 3-(2-Chlor-4-nitro-phenyl)-propan-1-ol. Das Rohprodukt wurde mittels Säulenchromatographie (Kieselgel, Petrolether /EtOAc 1:1) gereinigt.
Ausbeute: 1.800 g (63% der Theorie)
$C_{19}H_{16}Cl_2F_3NO_2$ (M= 418.246)
Ber. Molpeak $(M+H)^+$: 418/420/422
Gef.: Molpeak $(M+H)^+$: 418/420/422 ($Cl_2$)

Z45d) (E)-*N*-[4-(3-Brom-propyl)-3-chlor-phenyl]-3-(2-chlor-4-trifluormethyl-phenyl)-acrylamid

Zu einer Suspension von 0.836 g (2.000 mmol) (E)-*N*-[3-Chlor-4-(3-hydroxy-propyl)-phenyl]-3-(2-chlor-4-trifluormethyl-phenyl)-acrylamid und 0.730 g (2.200 mmol) Tetrabrommethan in 10 mL Dichlormethan wurde portionsweise 0.577 g (2.200 mmol) Triphenylphosphin zugegeben und das Gemisch 48 h bei RT gerührt. Das Reaktionsgemisch wurde mittels Säulenchromatographie (Kieselgel, Dichlormethan) gereinigt und der Rückstand mit Petrolether verrieben.
Ausbeute: 0.620 g (64% der Theorie)
$C_{19}H_{15}BrCl_2F_3NO$ (M= 481.143)
Ber. Molpeak $(M+H)^+$: 480/482/484/486
Gef.: Molpeak $(M+H)^+$: 480/482/484/486 ($BrCl_2$)
$R_f$-Wert: 0.81 (Kieselgel, Dichlormethan)

Zwischenprodukt 46:

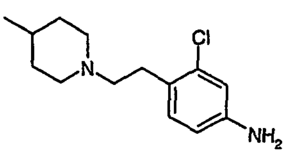

Z46a) 2-(2-Chlor-4-nitro-phenyl)-1-(4-methyl-piperidin-1-yl)-ethanon

Hergestellt analog Zwischenprodukt Z44b ausgehend von (2-Chlor-4-nitro-phenyl)-acetyl-chlorid (Z44a) und 4-Methylpiperidin.
Ausbeute: 1.050 g (77% der Theorie)
$C_{14}H_{17}ClN_2O_3$ (M= 296.756)
$R_f$-Wert: 0.51 (Kieselgel, Petrolether /EtOAc 1:1)

Z46b) 1-[2-(2-Chlor-4-nitro-phenyl)-ethyl]-4-methyl-piperidin

Hergestellt analog Zwischenprodukt Z44c ausgehend von -(2-Chlor-4-nitro-phenyl)-1-(4-methyl-piperidin-1-yl)-ethanon. Das Rohprodukt wurde mittels Säulenchromatographie (Alox, neutral, Akt. II-III, Gradient Petrolether /EtOAc 8:1 → 6:1) gereinigt.

Ausbeute: 0.820 g (57% der Theorie)
$C_{14}H_{19}ClN_2O_2$ (M= 282.773)
$R_f$-Wert: 0.73 (Kieselgel, Petrolether /EtOAc 2:1)

Z46c) 3-Chlor-4-[2-(4-methyl-piperidin-1-yl)-ethyl]-phenylamin

Hergestellt analog Zwischenprodukt Z44d ausgehend von 1-[2-(2-Chlor-4-nitro-phenyl)-ethyl]-4-methyl-piperidin.
Ausbeute: 0.820 g (57% der Theorie)
$C_{14}H_{21}ClN_2$ (M= 252.790)
Ber. Molpeak $(M+H)^+$: 253/255
Gef.: Molpeak $(M+H)^+$: 253/255 (Cl)

Allgemeine Arbeitsvorschrift I (TBTU-Kupplung):

Zu einer Lösung der Carbonsäure (1.0 eq.) in THF oder DMF wird nacheinander Triethylamin (1.5 eq.) oder Hünigbase (1.5eq.) und TBTU (1.0-1.5 eq.) gegeben. Die Mischung wird je nach Carbonsäure 10 Minuten -12 Stunden zwischen RT und 40°C gerührt bevor das Amin (1.0 eq.) zugegeben wird. Die Reaktion wird 30 Minuten - 24 Stunden zwischen RT und 40°C gerührt, bevor halbgesättigte $NaHCO_3$-Lösung zugegeben wird. Nach Extraktion der wässrigen Phase mit EtOAc wird die organische Phase über Magnesiumsulfat getrocknet. Das Lösungsmittel wird am Rotationsverdampfer entfernt; die weitere Reinigung erfolgt durch Säulenchromatographie oder Kristallisation. Die Reaktion kann auch im Syntheseautomaten Chemspeed durchgeführt werden.
Gemäß der allgemeinen Arbeitsvorschrift I wurden folgende Verbindungen hergestellt:

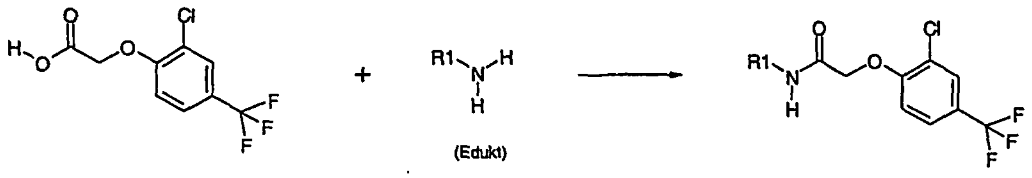


wobei in der nachstehenden Tabelle die Produkte über die Teilformel $R^1$-NH- und die zugehörigen Carbonsäureedukte durch Verweis auf die entsprechende Beispielnummer des Zwischenprodukts definiert sind.

| Beispiel | $R^1NH$- | Edukt | Summenformel | Massenspektrum | $R_f$-Wert | Ausbeute (%) |
|---|---|---|---|---|---|---|
| 1 | 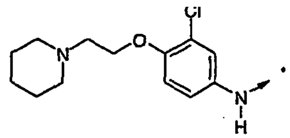 | Z10 | $C_{22}H_{23}Cl_2F_3N_2O_3$ | 491/493 $[M+H]^+$ | 0.45 (A) | 44 |
| 2 | 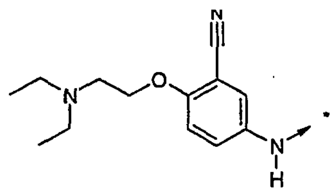 | Z11b | $C_{22}H_{23}ClF_3N_3O_3$ | 470/472 $[M+H]^+$ | 0.36 (A) | 64 |
| 3 | 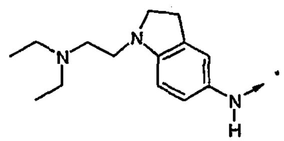 | Z12b | $C_{23}H_{27}ClF_3N_3O_2$ | 470/472 $[M+H]^+$ | 0.22 (A) | 44 |
| 4 | 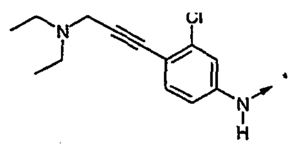 | Z13b | $C_{22}H_{21}Cl_2F_3N_2O_2$ | 473/475/4 77 $[M+H]^+$ | 0.42 (A) | 21 |
| 5 | 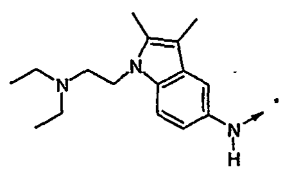 | Z14b | $C_{25}H_{29}ClF_3N_3O_2$ | 496/498 $[M+H]^+$ | 0.30 (A) | 48 |
| 6 | 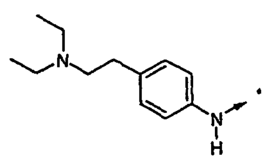 | Lit. | $C_{21}H_{24}ClF_3N_2O_2$ | 429/431 $[M+H]^+$ | 0.33 (A) | 36 |

(fortgesetzt)

| Beispiel | $R^1NH$- | Edukt | Summenformel | Massenspektrum | $R_f$-Wert | Ausbeute (%) |
|---|---|---|---|---|---|---|
| 7 | 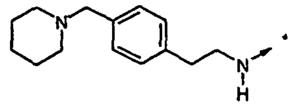 | Z24b | $C_{23}H_{26}ClF_3N_2O_2$ | 455/457 $[M+H]^+$ | 0.46 (A) | 50 |
| 8 | 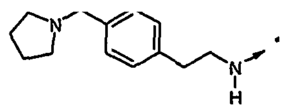 | Z23f | $C_{22}H_{24}ClF_3N_2O_2$ | 441/443 $[M+H]^+$ | 0.37 (A) | 46 |
| 9 | 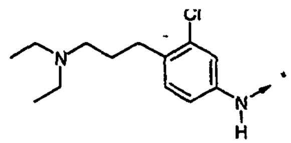 | Z15 | $C_{22}H_{25}Cl_2F_3N_2O_2$ | 477/479/4 81 $[M+H]^+$ | 0.22 (A) | 31 |
| 10 | 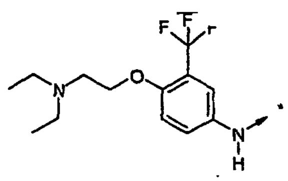 | Z16b | $C_{22}H_{23}ClF_6N_2O_3$ | 513/515 $[M+H]^+$ | 0.27 (A) | 39 |
| 11 | 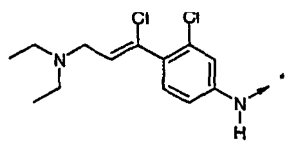 | Z17 | $C_{22}H_{22}Cl_3F_3N_2O_2$ | 509/11/13/ 15 $[M+H]^+$ | 0.48 (A) | 1 |
| 12 | 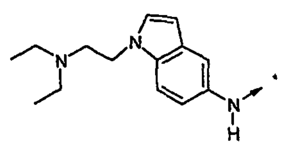 | Z18b | $C_{23}H_{25}ClF_3N_3O_2$ | 468/470 $[M+H]^+$ | 0.63 (A) | 1 |

(fortgesetzt)

| Beispiel | $R^1NH$- | Edukt | Summenformel | Massenspektrum | $R_f$-Wert | Ausbeute (%) |
|---|---|---|---|---|---|---|
| 13 | 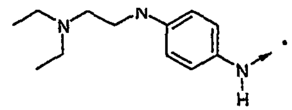 | Z19b | $C_{21}H_{25}ClF_3N_3O_2$ | 444/446 $[M+H]^+$ | 0.35 (A) | 35 |
| 14 | 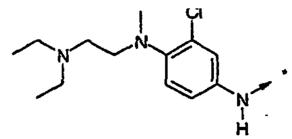 | Z20b | $C_{22}H_{26}Cl_2F_3N_3O_2$ | 492/494/4 96 $[M+H]^+$ | 0.46 (A) | 49 |
| 15 | 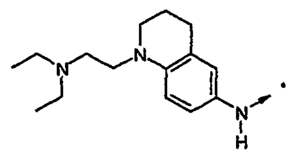 | Z21b | $C_{24}H_{29}ClF_3N_3O_2$ | 484/486 $[M+H]^+$ | 0.86 (B) | 42 |

Lit.: Literatur-bekannt
Fließmittel: A) Dichlormethan / MeOH / konz. wässriger Ammoniak = 90:10:1
B) EtOAc / MeOH / konz. wässriger Ammoniak = 90:10:1

Allgemeine Arbeitsvorschrift II:

Zu einer Lösung aus 1.0 eq. 3-Chlor-4-(2-diethylamino-ethoxy)-phenylamin und 4.5-6.0 eq. Triethylamin in THF wird bei 5°C eine Lösung aus 1.0 eq. Säurechlorid in THF langsam zugetropft. Das Reaktionsgemisch wird 3 Stunden bei 25-30°C gerührt, abfiltriert und mit THF gewaschen. Das Filtrat wird i. vac. eingeengt und der Rückstand mittels Säulenchromatographie gereinigt. Das Zwischenprodukt wird in Acetonitril gelöst, mit etherischer HCl angesäuert und mit Ether ausgefällt. Die weitere Reinigung erfolgt durch Umkristallisation.

Gemäß der allgemeinen Arbeitsvorschrift II wurden folgende Verbindungen hergestellt:

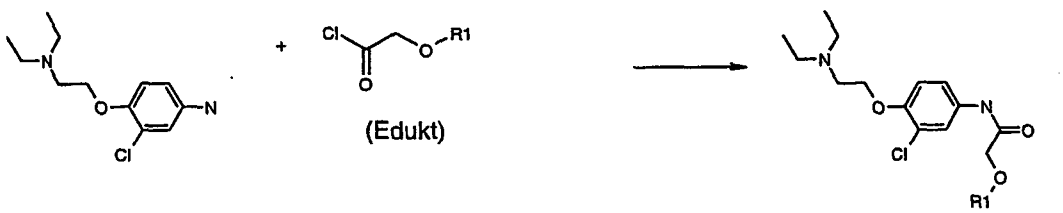


wobei in der nachstehenden Tabelle die Produkte über den Rest $R^1$- definiert sind. Die zugehörigen Aminedukte sind kommerziell erhältlich und/oder literaturbekannt.

| Beispiel | $R^1$ | Summenformel | Schmelzpunkt | Ausbeute (%) |
|---|---|---|---|---|
| 16 | 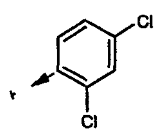 | $C_{20}H_{23}Cl_3N_2O_3$ x HCl | 186-188°C | 63 |
| 17 | 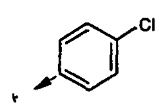 | $C_{20}H_{24}Cl_2N_2O_3$ x HCl | 171-172°C | 62 |
| 18 | 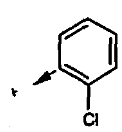 | $C_{20}H_{24}Cl_2N_2O_3$ x HCl | 183-185°C | 63 |

Beispiel 19:

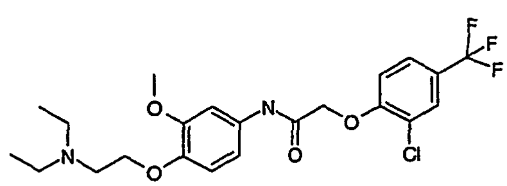

19) 2-(2-Chlor-4-trifluormethyl-phenoxy)-*N*-[4-(2-diethylamino-ethoxy)-3-methoxyphenyl]-acetamid

Zu einer Lösung von 185 mg (0.73 mmol) (2-Chlor-4-trifluormethyl-phenoxy)-essigsäure (siehe Zwischenprodukt Z2b) in 5 mL Tetrahydrofuran wurde 171 mg (0.82 mmol) CDI gegeben und die Reaktionsmischung 30 Minuten bei 50°C gerührt. Dann wurden 0.1 mL (0.73 mmol) Triethylamin und 200 mg (0.73 mmol) 4-(2-Diethylamino-ethoxy)-3-methoxy-phenylamin (siehe Zwischenprodukt Z6b) zugegeben und die Lösung 16 Stunden bei RT gerührt. Die Reaktionslösung wurde auf Wasser gegeben und 45 Minuten bei RT gerührt. Nach Filtration wurde der Rückstand im Umlufttrockenschrank getrocknet.
Ausbeute: 170 mg (49 % der Theorie)
$C_{22}H_{26}ClF_3N_2O_4$ (M= 474.912)
Ber. Molpeak $(M+H)^+$: 475/477
gef.: Molpeak $(M+H)^+$: 475/477 (Cl)
$R_f$-Wert: 0.30 (Kieselgel, Dichlormethan / MeOH / konz. wässriger Ammoniak 90:10:1).

Beispiel 20:

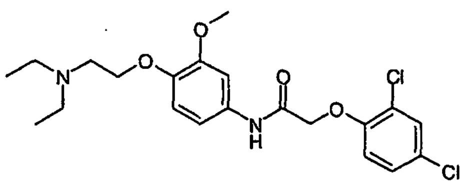

20) 2-(2,4-Dichlor-phenoxy)-N-[4-(2-diethylamino-ethoxy)-3-methoxy-phenyl]-acetamid

Zu einer Lösung von 66 mg (0.278 mmol) 4-(2-Diethylamino-ethoxy)-3-methoxy-phenylamin (Zwischenprodukt Z6b) und 96 µL (0.56 mmol) Ethyl-diisopropylamin in 1.5 mL abs. Dichlormethan wurde eine Lösung von 70 mg (0.290 mmol) (2,4-Dichlor-phenoxy)-acetylchlorid in 0.5 mL Dichlormethan zugegeben und das Gemisch 15 Stunden bei RT gerührt. Das Reaktionsgemisch wurde i. vac. eingeengt und der Rückstand mittels Säulenchromatographie (Kieselgel, Dichlormethan / MeOH 9:1) gereinigt.
Ausbeute: 77 mg (61 % der Theorie)
$C_{21}H_{26}Cl_2N_2O_4$ (M= 441.358)
Ber. Molpeak $(M+H)^+$: 441/443/445
gef.: Molpeak $(M+H)^+$: 4.41/443/445
$R_f$-Wert: 0.32 (Kieselgel, Dichlormethan / MeOH 9:1)

Analog zu Beispiel 20 wurden folgende Verbindungen hergestellt:

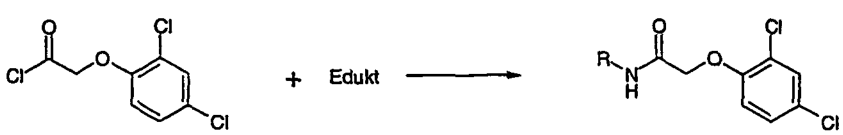


wobei in der nachstehenden Tabelle die Produkte über den Rest R und die zugehörigen Edukte durch Verweis auf die entsprechende Beispielnummer des Zwischenprodukts definiert oder als aus der Literatur (Lit.) bekannt angegeben sind.

| Beispiel | R | Edukt | Summenformel | Massenspektrum | $R_f$-Wert | Ausbeute (%) |
|---|---|---|---|---|---|---|
| 21 | 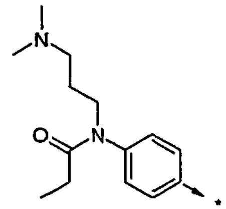 | Lit. | $C_{22}H_{27}Cl_2N_3O_3$ | 452/454/457 $[M+H]^+$ | 0.12 (A) | 65 % |
| 22 | 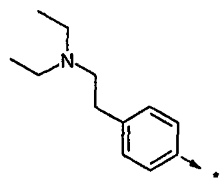 | Lit. | $C_{20}H_{24}Cl_2N_2O_2$ | 395/397/399 $[M+H]^+$ | 0.38 (A) | 46% |
| 23 | 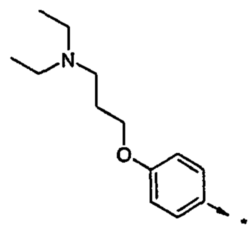 | Lit. | $C_{21}H_{26}Cl_2N_2O_3$ | 425/427/429 $[M+H]^+$ | 0.31 (A) | 69 % |
| 24 | 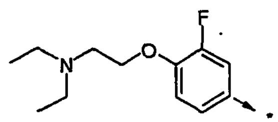 | Z8b | $C_{20}H_{23}Cl_2FN_2O_3$ | 429/431/433 $[M+H]^+$ | 0.34 (A) | 66% |
| 25 | 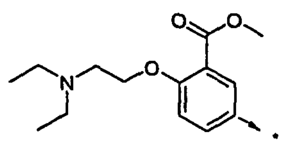 | Z7b | $C_{22}H_{26}Cl_2N_2O_5$ | 469/471/473 $[M+H]^+$ | 0.30 (A) | 40 % |

(fortgesetzt)

| Beispiel | R | Edukt | Summenformel | Massenspektrum | $R_f$-Wert | Ausbeute (%) |
|---|---|---|---|---|---|---|
| 26 | 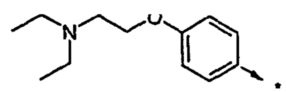 | Z5b | $C_{20}H_{24}Cl_2N_2O_3$ | 411/413/415 $[M+H]^+$ | 0.33 (A) | 89 % |
| 27 | 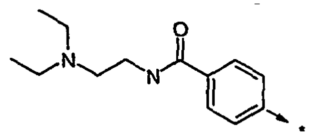 | Lit. | $C_{21}H_{25}Cl_2N_3O_3$ | 438/440/442 $[M+H]^+$ | 0.28 (A) | 52 % |
| 28 | 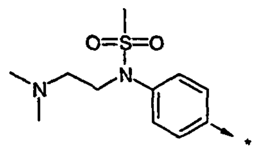 | Z23c | $C_{19}H_{23}Cl_2N_3O_4S$ | 460/462 $[M+H]^+$ | 0.40 (A) | 36 % |
| 29 | 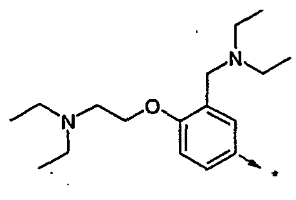 | Z9c | $C_{25}H_{39}Cl_2N_3O_3$ | 496/498/500 $[M+H]^+$ | 0.21 (A) | 84 % |
| 30 | 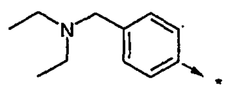 | Lit. | $C_{19}H_{22}Cl_2N_2O_2$ | 381/383 $[M+H]^+$ | 0.48 (A) | 35 % |

(fortgesetzt)

| Beispiel | R | Edukt | Summenformel | Massenspektrum | $R_f$-Wert | Ausbeute (%) |
|---|---|---|---|---|---|---|
| 31 | 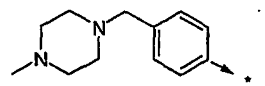 | Lit. | $C_{20}H_{23}Cl_2N_3O_2$ | 408/410/412 $[M+H]^+$ | 0.35 (A) | 40 % |
| $R_f$-Wert: A= (Kieselgel, Dichlormethan / MeOH 9:1)<br>B= (Kieselgel, EtOAc) | | | | | | |

Beispiel 32:

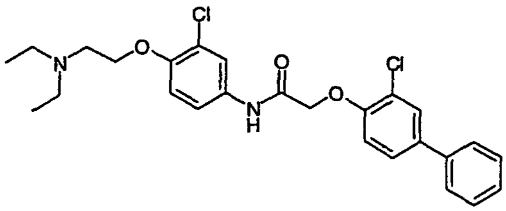

32) 2-(3-Chlor-biphenyl-4-yloxy)-N-[3-chlor-4-(2-diethylamino-ethoxy)-phenyl]-acetamid

Zu einer Lösung von 70 mg (0.159 mmol) 2-Brom-N-[3-chlor-4-(2-diethylamino-ethoxy)-phenyl]-acetamid-hydrobromid (Zwischenprodukt Z1c) und 64 mg (0.314 mmol) 3-Chlor-biphenyl-4-ol in 1 mL abs. DMF wurde 65 mg (0.47 mmol) Kaliumcarbonat zugegeben und das Gemisch 1 Stunde bei 40 ˚C und 15 Stunden bei RT gerührt. Das Reaktionsgemisch wurde mit Dichlormethan verdünnt, die org. Phase mit ges. wässriger Natriumbicarbonat-Lösung und Wasser gewaschen und über Magnesiumsulfat getrocknet. Säulenchromatographie (Kieselgel, Dichlormethan / MeOH 9:1) ergab das Produkt.

Ausbeute: 51 mg (67 % der Theorie)

$C_{26}H_{28}Cl_2N_2O_3$ (M= 487.431)

Ber. Molpeak $(M+H)^+$: 487/489/491

gef.: Molpeak $(M+H)^+$: 487/489/491 ($Cl_2$)

$R_f$-Wert: 0.43 (Kieselgel, Dichlormethan / MeOH 9:1)

Analog zu Beispiel 32 wurden folgende Verbindungen hergestellt:

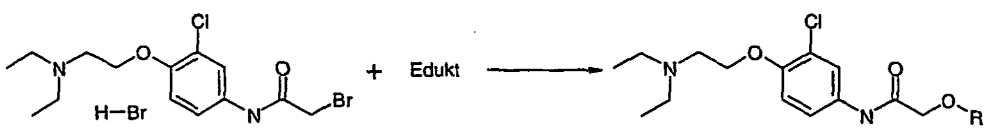


wobei in der nachstehenden Tabelle die Produkte über den Rest R definiert und die zugehörigen Edukte kommerziell erhältlich sind.

| Beispiel | R | Summenformel | Massenspektrum | $R_f$-Wert | Ausbeute (%) |
|---|---|---|---|---|---|
| 33 | 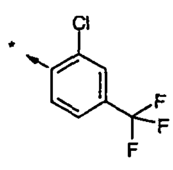 | $C_{21}H_{23}Cl_2F_3N_2O_3$ | 479/481/483 $[M+H]^+$ | 0.34 (A) | 67 % |
| 34 | 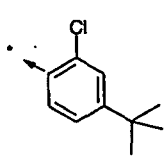 | $C_{24}H_{32}Cl_2N_2O_3$ | 467/469/471 $[M+H]^+$ | 0.31 (A) | 63 % |

(fortgesetzt)

| Beispiel | R | Summenformel | Massenspektrum | $R_f$-Wert | Ausbeute (%) |
|---|---|---|---|---|---|
| 35 | 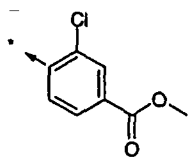 | $C_{22}H_{26}Cl_2N_2O_5$ | 469/471/473 $[M+H]^+$ | 0.30 (A) | 80 % |
| 36 | 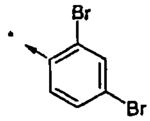 | $C_{20}H_{23}Br_2ClN_2O_3$ | 533/535/537 $[M+H]^+$ | 0.31 (A) | 82 % |
| 37 | 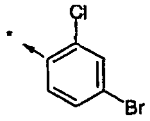 | $C_{20}H_{23}BrCl_2N_2O_3$ | 489/491/495/4 95 $[M+H]^+$ | 0.25 (A) | 74 % |
| 38 | 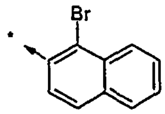 | $C_{24}H_{26}BrClN_2O_3$ | 505/507/509 $[M+H]^+$ | 0.36 (A) | 80 % |
| 39 | 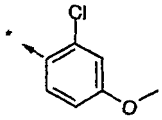 | $C_{21}H_{26}Cl_2N_2O_4$ | 441/443/445 $[M+H]^+$ | 0.38 (A) | 60 % |
| 40 | 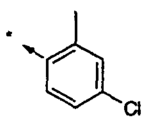 | $C_{21}H_{26}Cl_2N_2O_3$ | 425/427/429 $[M+H]^+$ | 0.31 (A) | 85 % |
| 41 | 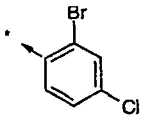 | $C_{20}H_{23}BrCl_2N_2O_3$ | 489/491/493/4 95 $[M+H]^+$ | 0.32 (A) | 57 % |
| 42 | 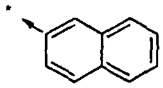 | $C_{24}H_{27}ClN_2O_3$ | 427/429 $[M+H]^+$ | 0.31 (A) | 78 % |
| 43 | 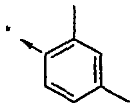 | $C_{22}H_{29}ClN_2O_3$ | 405/407 $[M+H]^+$ | 0.30 (A) | 73 % |
| 44 | 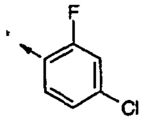 | $C_{20}H_{23}Cl_2FN_2O_3$ | 429/431/433 $[M+H]^+$ | 0.26 (A) | 74 % |

(fortgesetzt)

| Beispiel | R | Summenformel | Massenspektrum | $R_f$-Wert | Ausbeute (%) |
|---|---|---|---|---|---|
| 45 | 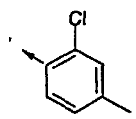 | $C_{21}H_{26}Cl_2N_2O_3$ | 425/427/429 $[M+H]^+$ | 0.19 (A) | 54 % |
| 46 | 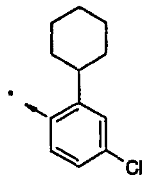 | $C_{26}H_{34}Cl_2N_2O_3$ | 493/495/497 $[M+H]^+$ | 0.24 (A) | 62 % |
| 47 | 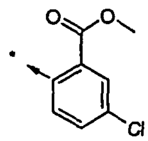 | $C_{22}H_{26}Cl_2N_2O_5$ | 469/471/473$[ M+H]^+$ | 0.25 (A) | 68 % |
| 48 | 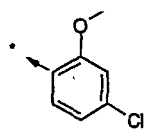 | $C_{21}H_{26}Cl_2N_2O_4$ | 441/443/445 $[M+H]^+$ | 0.31 (A) | 80 % |
| 49 | 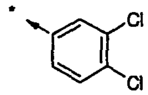 | $C_{20}H_{23}Cl_3N_2O_3$ | 445/447/449/4 51$[M+H]^+$ | 0.26(A) | 66 % |
| 50 | 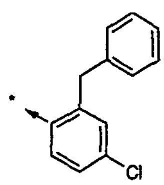 | $C_{27}H_{30}Cl_2N_2O_3$ | 501/503/505$[ M+H]^+$ | 0.36 (A) | 93 % |
| 51 | 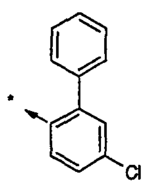 | $C_{26}H_{28}Cl_2N_2O_3$ | 487/489/491$[ M+H]^+$ | 0.36 (A) | 83 % |
| 52 | 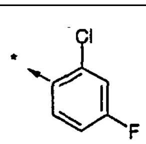 | $C_{20}H_{23}Cl_2FN_2O_3$ | 429/431/433$[ M+H]^+$ | 0.36 (A) | 64 % |
| 53 | 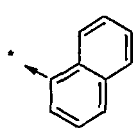 | $C_{24}H_{27}ClN_2O_3$ | 427/429 $[M+H]^+$ | 0.32 (A) | 84 % |

(fortgesetzt)

| Beispiel | R | Summenformel | Massenspektrum | $R_f$-Wert | Ausbeute (%) |
|---|---|---|---|---|---|
| 54 | 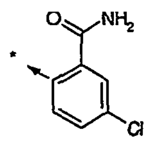 | $C_{21}H_{25}Cl_2N_3O_4$ | 454/456/458[ M+H]$^+$ | 0.08 (A) | 72 % |
| 55 | 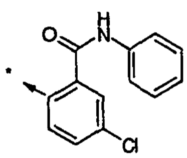 | $C_{27}H_{29}Cl_2N_3O_4$ | 530/532/534[ M+H]$^+$ | 0.23 (A) | 48 % |
| $R_f$-Wert: A= (Kieselgel, Dichlormethan / MeOH 9:1) | | | | | |

Allgemeine Arbeitsvorschrift III (Phenolalkylierungl):

Zu einer Lösung des Alkylbromides (siehe Zwischenprodukt Z1c) (1.0 eq.) in DMF wird nacheinander Phenol (2.0 eq.) und Kaliumcarbonat (3.0-5.0 eq.) gegeben. Die Mischung wird 48-72 Stunden bei RT unter Stickstoffatmosphäre gerührt, bevor auf Wasser gegeben wird. Nach Extraktion der wässrigen Phase mit EtOAc wird die organische Phase über Magnesiumsulfat getrocknet. Das Lösungsmittel wird am Rotationsverdampfer entfernt; die weitere Reinigung erfolgt durch Säulenchromatographie oder Kristallisation.

Gemäß der allgemeinen Arbeitsvorschrift III wurden folgende Verbindungen hergestellt:

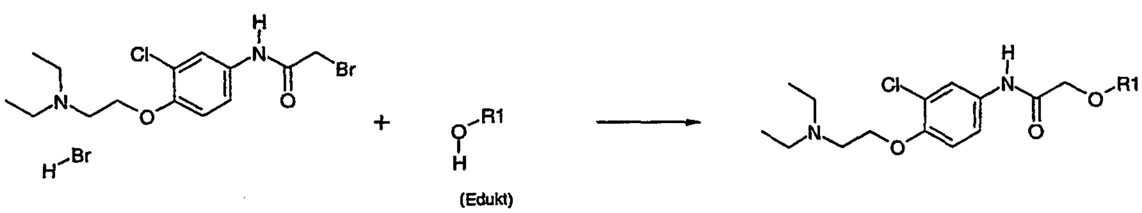


wobei in der nachstehenden Tabelle die Produkte über den Rest R1 definiert und die zugehörigen Edukte kommerziell erhältlich sind.

| Beispiel | R1 | Summenformel | Massenspektrum | $R_f$-Wert | Ausbeute (%) |
|---|---|---|---|---|---|
| 56 | 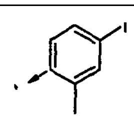 | $C_{21}H_{26}ClIN_2O_3$ | 517/519 [M+H]$^+$ | 0.32 (A) | 47 |
| 57 | 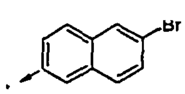 | $C_{24}H_{26}BrClN_2O_3$ | 505/507/509 [M+H]$^+$ | 0.42 (A) | 20 |
| 58 | 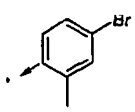 | $C_{21}H_{27}BrClN_2O_3$ | 469/471/473 [M+H]$^+$ | 0.33 (A) | 49 |

(fortgesetzt)

| Beispiel | R1 | Summenformel | Massenspektrum | $R_f$-Wert | Ausbeute (%) |
|---|---|---|---|---|---|
| 59 | 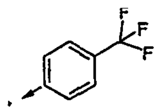 | $C_{21}H_{24}ClF_3N_2O_3$ | 445/447 $[M+H]^+$ | 0.32 (A) | 56 |
| 60 | 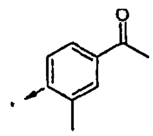 | $C_{23}H_{29}ClN_2O_4$ | 433/435 $[M+H]^+$ | 0.37 (A) | 23 |
| 61 | 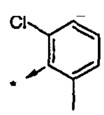 | $C_{21}H_{26}Cl_2N_2O_3$ | 425/427/429 $[M+H]^+$ | 0.42 (A) | 24 |
| 62 | 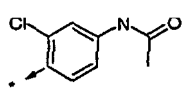 | $C_{22}H_{27}C_2N_3O_4$ | 468/470/472 $[M+H]^+$ | 0.26 (A) | 21 |
| 63 | 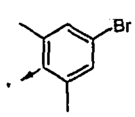 | $C_{22}H_{28}BrClN_2O_3$ | 483/485/487 $[M+H]^+$ | 0.32 (A) | 48 |
| 64 | 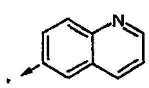 | $C_{23}H_{26}ClN_3O_3$ | 428/430 $[M+H]^+$ | 0.23 (A) | 12 |
| Fließmittel: A) Dichlormethan / MeOH / Ammoniak = 90:10:1 | | | | | |

<u>Allgemeine Arbeitsvorschrift IV (Phenolalkylierung II):</u>

Zu einer Lösung des Anilines (siehe Zwischenprodukt Z1b) (1.0 eq.) in DMF wird bei-10°C Bromacetylbromid (1.0 eq.) in Dioxan zugetropft. Anschließend wird auf RT erwärmt und nacheinander Phenol (1.0 eq.) in DMF und Kalium-*tert*-butylat (2.0 eq.) in *tert*-Butanol zugegeben. Die Mischung wird 4 Stunden auf 80°C erhitzt. DMF wird im Vakuum abgezogen und der Rückstand in EtOAc gelöst. Die Essigesterlösung wird 1x mit 10 %-iger $K_2CO_3$-Lösung, dann 2x mit Wasser gewaschen. Der EtOAc wird im Vakuum entfernt. Die weitere Reinigung erfolgt durch Säulenchromatographie. Gemäß der allgemeinen Arbeitsvorschrift IV wurden folgende Verbindungen hergestellt:

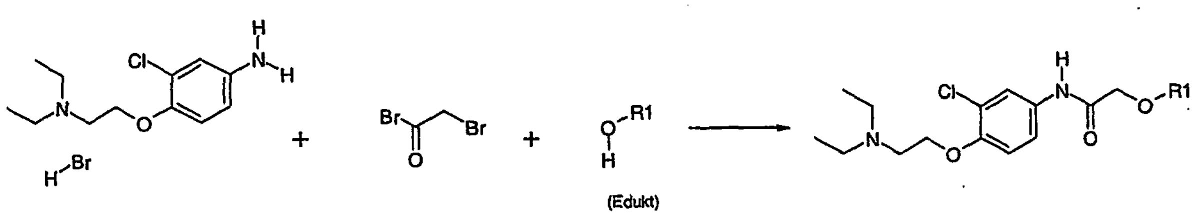


wobei in der nachstehenden Tabelle die Produkte über den Rest R1 definiert und die zugehörigen Edukte kommerziell erhältlich sind.

| Beispiel | R1 | Summenformel | Massenspektrum | $R_f$-Wert | Ausbeute (%) |
|---|---|---|---|---|---|
| 65 | 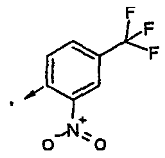 | $C_{21}H_{23}ClF_3N_3O_5$ | 490/492 $[M+H]^+$ | 0.24 (A) | 6 |
| 66 | 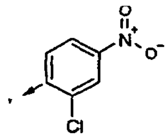 | $C_{20}H_{23}Cl_2N_3O_5$ | 456/458/460 $[M+H]^+$ | 0.28 (A) | 7 |
| 67 | 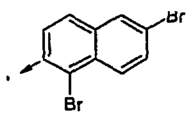 | $C_{24}H_{25}Br_2ClN_2O_5$ | 583/85/87/89 $[M+H]^+$ | 0.50 (A) | 7 |
| 68 | 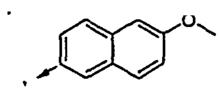 | $C_{25}H_{29}ClN_2O_4$ | 455/457 $[M+H]^+$ | 0.24 (A) | 13 |
| 69 | 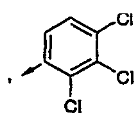 | $C_{20}H_{22}Cl_4N_2O_3$ | 479/81/83/85 /87 $[M+H]^+$ | 0.28 (A) | 10 |
| 70 | 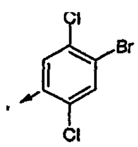 | $C_{20}H_{22}BrCl_3N_2O_3$ | 521/23/25/27 $[M+H]^+$ | 0.28 (A) | 10 |
| 71 | 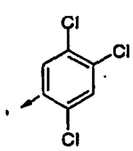 | $C_{20}H_{22}Cl_4N_2O_3$ | 477/79/81/83 $[M+H]^+$ | 0.23 (A) | 8 |
| 72 | 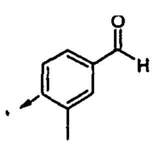 | $C_{22}H_{27}ClN_2O_4$ | 419/421 $[M+H]^+$ | 0.25 (A) | 5 |
| 73 | 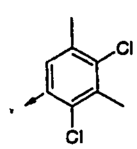 | $C_{22}H_{27}Cl_3N_2O_3$ | 473/75/77/79 $[M+H]^+$ | 0.31 (A) | 8 |
| 74 | 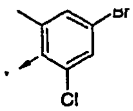 | $C_{21}H_{25}BrCl_2N_2O_3$ | 503/05/07/09 $[M+H]^+$ | 0.28 (A) | 8 |
| Fließmittel: A) Dichlormethan / MeOH / Ammoniak = 90:10:1 | | | | | |

Beispiel 75:

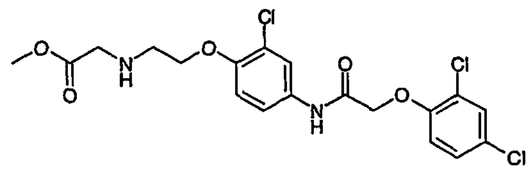

75) (2-{2-Chlor-4-[2-(2,4-dichlor-phenoxy)-acetylamino]-phenoxy}-ethylamino)-essigsäure-methylester

Zu einer Suspension von 45 mg (0.36 mmol) Amino-essigsäure-methylesterhydrochlorid in 2 mL Dichlormethan / THF (1:1) wurde 75 µL (0.54 mmol) Triethylamin und 70 mg (0.18 mmol) N-[3-Chlor-4-(2-oxo-ethoxy)-phenyl]-2-(2,4-dichlor-phenoxy)-acetamid zugegeben. 114 mg (0.54 mmol) Natriumtriacetoxyborhydrid wurde zugegeben und das Gemisch 3 Stunden bei RT gerührt. 100 mL 2 N wässrige Natriumcarbonat-Lösung wurde zugegeben und die wässriger Phase mit Chloroform erschöpfend extrahiert. Die vereinigten org. Extrakte wurden über Magnesiumsulfat getrocknet, i. vac. eingeengt und mittels Säulenchromatographie (Kieselgel, EtOAc / MeOH 9:1) gereinigt.
Ausbeute: 71 mg (78 % der Theorie)
$C_{19}H_{19}Cl_3N_2O_5$ (M= 461.733)
Ber. Molpeak $(M+H)^+$: 461/463/465/467
gef.: Molpeak $(M+H)^+$: 461/463/465/467 ($Cl_3$)
$R_f$-Wert: 0.32 (Kieselgel, EtOAc)
Analog zu Beispiel 75 wurden folgende Verbindungen hergestellt:

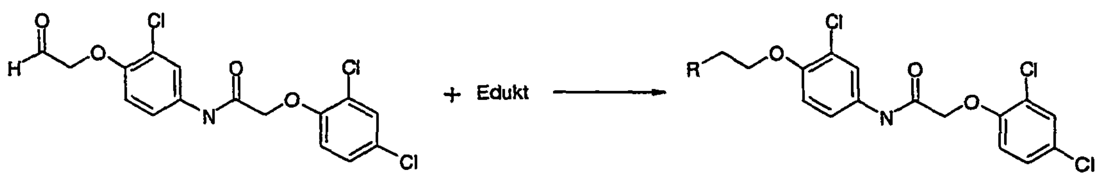


wobei in der nachstehenden Tabelle die Produkte über den Rest R definiert und die zugehörigen Edukte kommerziell erhältlich sind.

| Beispiel | R | Summenformel | Massenspektrum | $R_f$-Wert | Ausbeute (%) |
|---|---|---|---|---|---|
| 76 | 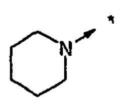 | $C_{21}H_{23}Cl_3N_2O_3$ | 457/459/461/463 $[M+H]^+$ | 0.30 (A) | 66 % |
| 77 | 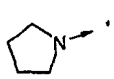 | $C_{20}H_{21}Cl_3N_2O_3$ | 443/445/447/449 $[M+H]^+$ | 0.28 (A) | 70 % |
| 78 | 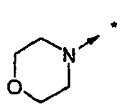 | $C_{20}H_{21}Cl_3N_2O_4$ | 459/461/463/465 $[M+H]^+$ | 0.18 (B) | 72 % |

(fortgesetzt)

| Beispiel | R | Summenformel | Massenspektrum | $R_f$-Wert | Ausbeute (%) |
|---|---|---|---|---|---|
| 79 | 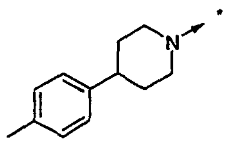 | $C_{28}H_{29}Cl_3N_2O_3$ | 547/549/551/553 $[M+H]^+$ | 0.19 (B) | 52 % |
| 80 | 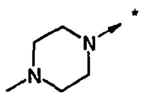 | $C_{21}H_{24}Cl_3N_3O_3$ | 472/474/476/478 $[M+H]^+$ | 0.31 (A) | 66 % |
| 81 | 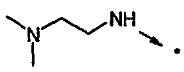 | $C_{20}H_{24}Cl_3N_3O_3$ | 460/462/464/466 $[M+H]^+$ | 0.19 (A) | 42 % |
| 82 | 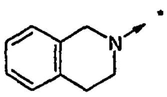 | $C_{25}H_{23}Cl_3N_2O_3$ | 505/507/509/511 $[M+H]^+$ | 0.61 (B) | 78 % |
| 83 | 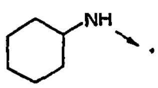 | $C_{22}H_{25}Cl_3N_2O_3$ | 471/473/475/477 $[M+H]^+$ | 0.41 (B) | 64 % |
| 84 | 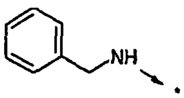 | $C_{23}H_{21}Cl_3N_2O_3$ | 479/481/483/485 $[M+H]^+$ | 0.16 (B) | 69 % |
| 85 | 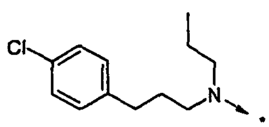 | $C_{28}H_{30}Cl_3N_2O_3$ | 583/585/587/589 $[M+H]^+$ | 0.51 (B) | 56 % |
| 86 | 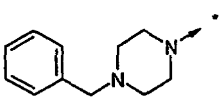 | $C_{27}H_{28}Cl_3N_3O_3$ | 548/550/552/554 $[M+H]^+$ | 0.10 (B) | 82 % |
| 87 | 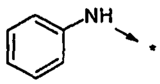 | $C_{22}H_{19}Cl_3N_2O_3$ | 465/467/469/471 $[M+H]^+$ | 0.51 (C) | 58 % |

$R_f$-Wert: A= (Kieselgel, Dichlormethan / MeOH 9:1)
B= (Kieselgel, EtOAc)
C= (Kieselgel, EtOAc / Hexan 1:1)

Allgemeine Arbeitsvorschrift V (Reduktive-Aminierung):

Zu einer Lösung des Aldehydes (siehe Zwischenprodukt Z3d) (1.0 eq.) und Amin (2.0 eq.) in THF wird konz. Salzsäure gegeben (2.0 eq.) oder mit Eisessig ein pH Wert zwischen 4-6 eingestellt. Die Mischung wird 10 Minuten bei RT gerührt und dann wird Natriumcyanborhydrid (2.0 eq.) in THF oder Natriumtriacetoxyborhydrid (2.0 eq.) zugegeben. Die Reaktionsmischung wird je nach Amin bei RT bis 60°C 30 Minuten - 24 Stunden gerührt, bevor mit ges. wässriger Natriumbicarbonat-Lösung versetzt wird. Nach Extraktion der wässrigen Phase mit Ether wird die organische Phase über Magnesiumsulfat getrocknet. Das Lösungsmittel wird am Rotationsverdampfer entfernt; die weitere Reinigung erfolgt durch Säulenchromatographie oder Kristallisation.
Gemäß der allgemeinen Arbeitsvorschrift V wurden folgende Verbindungen hergestellt:

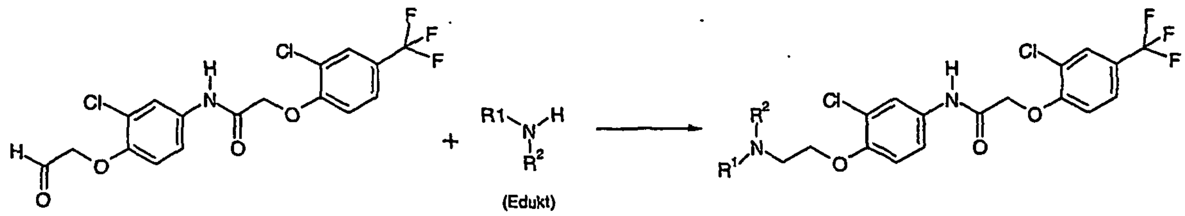


wobei in der nachstehenden Tabelle die Produkte über den Rest $R^1R^2N$- definiert und die zugehörigen Edukte kommerziell erhältlich oder aus der Literatur bekannt sind.

| Beispiel | $R^1R^2N$- | Summenformel | Massenspektrum | $R_f$-Wert | Ausbeute (%) |
|---|---|---|---|---|---|
| 88 | 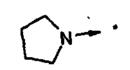 | $C_{21}H_{21}Cl_2F_3N_2O_3$ | 477/479/481 $[M+H]^+$ | 0.13 (A) | 27 |
| 89 | 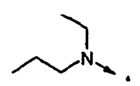 | $C_{22}H_{25}Cl_2F_3N_2O_3$ | 493/495/497 $[M+H]^+$ | 0.26 (B) | 8 |
| 90 | 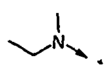 | $C_{20}H_{21}Cl_2F_3N_2O_3$ | 465/67/69 $[M+H]^+$ | 0.25 (B) | 4 |
| 91 | 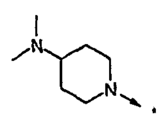 | $C_{24}H_{28}Cl_2F_3N_3O_3$ | 534/536 $[M+H]^+$ | 0.10 (B) | 30 |
| 92 | 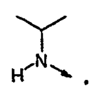 | $C_{20}H_{21}Cl_2F_3N_2O_3$ | 465/467/469 $[M+H]^+$ | 0.28 (B) | 9 |
| 93 | 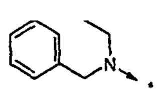 | $C_{26}H_{25}Cl_2F_3N_2O_3$ | 541/543/545 $[M+H]^+$ | 0.80 (B) | 10 |
| 94 | 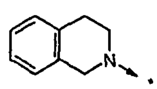 | $C_{26}H_{23}Cl_2F_3N_2O_3$ | 539/541/543 $[M+H]^+$ | 0.35 (B) | 19 |
| 95 | 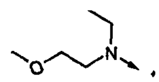 | $C_{22}H_{25}Cl_2F_3N_2O_4$ | 509/511/513 $[M+H]^+$ | 0.37 (B) | 7 |
| 96 | 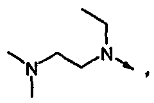 | $C_{23}H_{28}Cl_2F_3N_3O_3$ | 522/524/526 $[M+H]^+$ | 0.18 (B) | 8 |
| 97 | 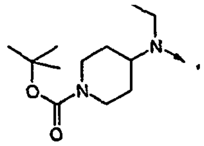 | $C_{29}H_{36}Cl_2F_3N_3O_5$ | 634/636/638 $[M+H]^+$ | 0.32 (B) | 6 |

(fortgesetzt)

| Beispiel | $R^1R^2N$- | Summenformel | Massenspektrum | $R_f$-Wert | Ausbeute (%) |
|---|---|---|---|---|---|
| 98 | 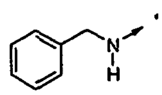 | $C_{24}H_{21}Cl_2F_3N_2O_3$ | 513/515/517 $[M+H]^+$ | 0.47 (C) | 27 |
| 99 | 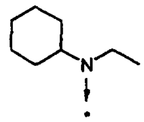 | $C_{25}H_{29}Cl_2F_3N_2O_3$ | 533/535/537 $[M+H]^+$ | 0.37 (C) | 1 |
| 100 | 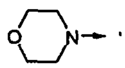 | $C_{21}H_{21}Cl_2F_3N_2O_4$ | 493/495 $[M+H]^+$ | 0.33 (B) | 13 |
| 101 | 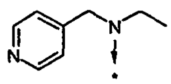 | $C_{25}H_{24}Cl_2F_3N_3O_3$ | 542/544/546 $[M+H]^+$ | 0.35 (B) | 10 |
| 102 | 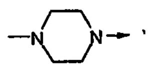 | $C_{22}H_{24}Cl_2F_3N_3O_3$ | 506/508/510 $[M+H]^+$ | 0.15 (B) | 1 |
| 103 | 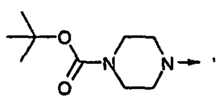 | $C_{26}H_{30}Cl_2F_3N_3O_5$ | 592/594/596 $[M+H]^+$ | 0.55 (B) | 21 |
| 104 | 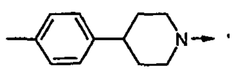 | $C_{29}H_{29}Cl_2F_3N_2O_3$ | 581/583/585 $[M+H]^+$ | 0.55 (B) | 18 |
| 105 | 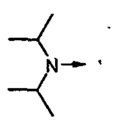 | $C_{23}H_{27}Cl_2F_3N_2O_3$ | 507/509/511 $[M+H]^+$ | 0.65 (B) | 6 |
| Fließmittel: A) Dichlormethan / MeOH / konz. wässriger Ammoniak = 95:5:0.5<br>B) Dichlormethan / MeOH / konz. wässriger Ammoniak = 90:10:1<br>C) Dichlormethan / MeOH = 9:1 | | | | | |

Beispiel 106:

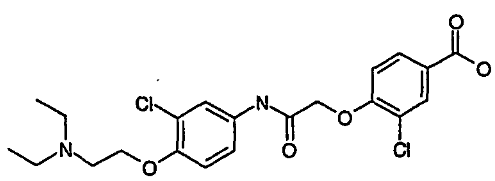

106) 3-Chlor-4-{[3-chlor-4-(2-diethylamino-ethoxy)-phenylcarbamoyl]-methoxy}-benzoesäure

Eine Lösung aus 1.8 g (3.835 mmol) 3-Chlor-4-{[3-chlor-4-(2-diethylamino-ethoxy)-phenylcarbamoyl]-methoxy}-benzoesäuremethylester (aus Beispiel 35) und 2 mL 2 M wässrige NaOH-Lösung in 20 mL MeOH wurde 1 Stunde

refluxiert. Die Reaktionslösung wurde i. vac. eingeengt, mit Wasser verdünnt und mit HCl schwach angesäuert. Nach 3 Tagen bei RT wurde die Lösung i. vac. eingeengt. Der Rückstand wurde mit kaltem EtOH verrieben und der Niederschlag abfiltriert.
Ausbeute: 230 mg (13 % der Theorie)
$C_{21}H_{24}Cl_2N_2O_5$ (M= 455.342)
Ber. Molpeak $(M+H)^+$: 454/456/458
gef.: Molpeak $(M+H)^+$: 454/456/458 ($Cl_2$)
$R_f$-Wert: 0.05 (Kieselgel, Dichlormethan / MeOH / konz. wässriger Ammoniak 90:10:1).

Allgemeine Arbeitsvorschrift VI:

Eine Lösung aus 1.0 eq. 3-Chlor-4-{[3-chlor-4-(2-diethylamino-ethoxy)-phenylcarbamoyl]-methoxy}-benzoesäure (aus Beispiel 106) und 1.07 eq. TBTU in DMF wird bei RT vorgelegt. Nach Zugabe von 1.07 eq. Triethylamin wird 10 Minuten gerührt. Anschließend wurden 7.0 eq. Amin zugegeben und 16 Stunden bei RT gerührt. Das Reaktionsgemisch wurde mit Wasser oder 5%iger Natriumcarbonat-Lösung versetzt. Der ausgefallene Feststoff wurde abfiltriert, mit Wasser gewaschen und i. vac. getrocknet.
Gemäß der allgemeinen Arbeitsvorschrift VI wurden folgende Verbindungen hergestellt:

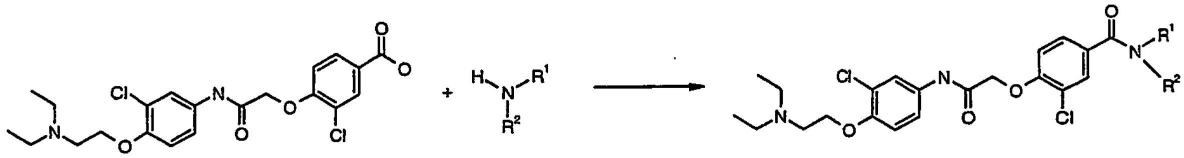

wobei in der nachstehenden Tabelle die Produkte über den Rest $R^1R^2N$- definiert und die zugehörigen Edukte kommerziell erhältlich oder aus der Literatur bekannt sind.

| Beispiel | $R^1R^2N$- | Summenformel | Massenspektrum | $R_f$-Wert | Ausbeute (%) |
|---|---|---|---|---|---|
| 107 | 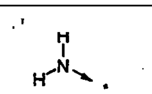 aus $(NH_4)_2CO_3$ | $C_{21}H_{25}Cl_2F_3N_3O_4$ | 454/456/458 $[M+H]^+$ | 0.37 (A) | 59 |
| 108 | 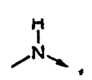 | $C_{22}H_{27}Cl_2F_3N_3O_4$ | 468/470/472 $[M+H]^+$ | 0.38 (A) | 57 |
| 109 | 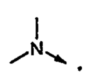 | $C_{23}H_{29}Cl_2F_3N_3O_4$ | 482/484/486 $[M+H]^+$ | 0.38 (A) | 55 |
| Fließmittel: A) Dichlormethan / MeOH / konz. wässriger Ammoniak = 90:10:1 | | | | | |

Allgemeine Arbeitsvorschrift VII (Suzuki-Kupplung):

Zu einer Lösung des Iodides (1.0 eq.; siehe Beispiel 56) in Toluol und 2M-Natriumcarbonat-Lösung (4.0 eq.) werden nacheinander Boronsäure (2.0 eq.) und Tetrakis-(triphenylphosphin)-palladium (0.1 eq.) gegeben und über Nacht bei 80°C gerührt. Die Reaktionslösung wird mit 10 % wässrige $Na_2CO_3$-Lösung versetzt und die wässrige Phase wird mit EtOAc extrahiert. Die organischen Phasen werden vereinigt und das Lösungsmittel wird im Vakuum entfernt. Die weitere Reinigung erfolgt durch Säulenchromatographie.

Gemäß der allgemeinen Arbeitsvorschrift VII wurden folgende Verbindungen hergestellt:

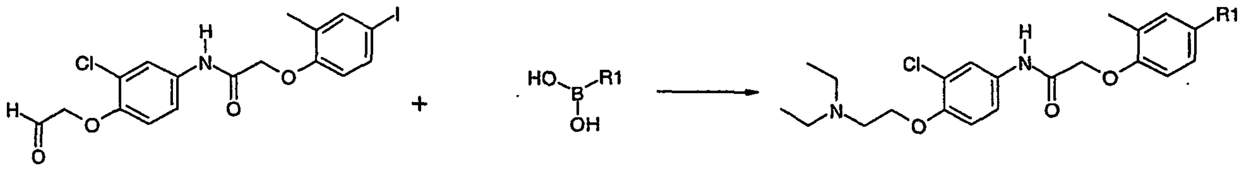

wobei in der nachstehenden Tabelle die Produkte über den Rest R1 definiert und die zugehörigen Edukte kommerziell erhältlich oder aus der Literatur bekannt sind.

| Beispiel | R1 | Summenformel | Massenspektrum | $R_f$-Wert | Ausbeute (%) |
|---|---|---|---|---|---|
| 110 | 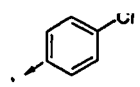 | $C_{27}H_{30}Cl_2N_2O_3$ | 501/503/505 $[M+H]^+$ | 0.30 (A) | 4 |
| 111 | 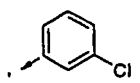 | $C_{27}H_{30}Cl_2N_2O_3$ | 501/503/505 $[M+H]^+$ | 0.30 (A) | 4 |
| 112 | 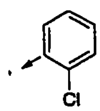 | $C_{27}H_{30}Cl_2N_2O_3$ | 501/503/505 $[M+H]^+$ | 0.30 (A) | 6 |
| 113 | 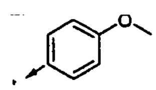 | $C_{28}H_{33}ClN_2O_4$ | 497/499/501 $[M+H]^+$ | 0.27 (A) | 8 |
| 114 | 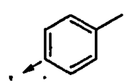 | $C_{28}H_{33}ClN_2O_4$ | 481/483 $[M+H]^+$ | 0.6 (B) | 21 |
| Fließmittel: A) Dichlormethan / MeOH / konz. wässriger Ammoniak = 90:10:1<br>B) EtOAc / MeOH / konz. wässriger Ammoniak = 90:10:1 | | | | | |

Beispiel 115:

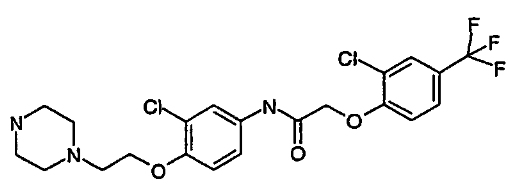

115) N-[3-Chlor-4-(2-piperazin-1-yl-ethoxy)-phenyl]-2-(2-chlor-4-trifluormethyl-phenoxy)-acetamid

0.200g(0.338mmol)4-(2-{2-Chlor-4-[2-(2-chlor-4-trifluormethyl-phenoxy)-acetylamino]-phenoxy}-ethyl)-piperazin-1-carbonsäure-tert-butylester (aus Beispiel 103) wurden in 5.0 mL Dichlormethan gelöst. Nach Zugabe von 0.5 mL (6.760 mmol) Trifluoressigsäure wurde 2 Stunden bei RT gerührt. Die Reaktionslösung wurde i. vac. eingeengt und der Rückstand mit ges. wässriger Natriumhydrogencarbonat-Lösung versetzt. Die wässrige Phase wurde mit EtOAc extrahiert. Die org. Phase wurde über Magnesiumsulfat getrocknet, filtriert und i. vac. eingeengt. Die weitere Reinigung erfolgte mittels Säulenchromatographie.

Ausbeute: 0.032 g (16 % der Theorie)
$C_{21}H_{22}Cl_2F_3N_3O_3$ * 2 $CH_2O_2$ (M= 584.381)
Ber. Molpeak $(M+H)^+$: 492/494/496 ($Cl_2$)
gef.: Molpeak $(M+H)^+$: 492/494/496 ($Cl_2$)
$R_f$-Wert: 0.22 (Kieselgel, Dichlormethan / MeOH / konz. wässriger Ammoniak 90:10:1).

Beispiel 116:

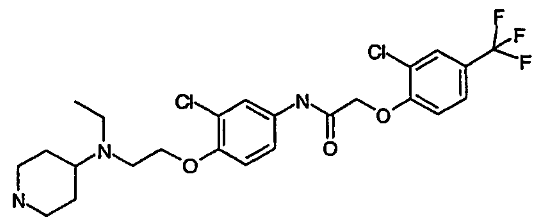

116) N-{3-Chlor-4-[2-(ethyl-piperidin-4-yl-amino)-ethoxy]-phenyl}-2-(2-chlor-4-trifluormethyl-phenoxy)-acetamid

0.180 g (0.284 mmol) 4-[(2-{2-Chlor-4-[2-(2-chlor-4-trifluormethyl-phenoxy)-acetylamino]-phenoxy}-ethyl)-ethyl-amino]-piperdin-1-carbonsäure-tert-butylester (aus Beispiel 97) wurden in 5.0 mL Dichlormethan gelöst. Nach Zugabe von 0.44 mL (5.680 mmol) Trifluoressigsäure wurde 2 Stunden bei RT gerührt. Die Reaktionslösung wurde i. vac. eingeengt und der Rückstand mit ges. wässriger Natriumhydrogencarbonat-Lösung versetzt. Die wässrige Phase wurde mit EtOAc extrahiert. Die org. Phase wurde über Magnesiumsulfat getrocknet, filtriert und i. vac. eingeengt. Die weitere Reinigung erfolgte mittels Säulenchromatographie.
Ausbeute: 0.011 g (6 % der Theorie)
$C_{24}H_{28}Cl_2F_3N_3O_3$ * 2 $CH_2O2$ (M= 626.462)
Ber. Molpeak $(M+H)^+$: 534/536/538 ($Cl_2$)
gef.: Molpeak $(M+H)^+$: 534/536/538 ($Cl_2$)
$R_f$-Wert: 0.25 (Kieselgel, Dichlormethan / MeOH / konz. wässriger Ammoniak 90:10:1).

Beispiel 117:

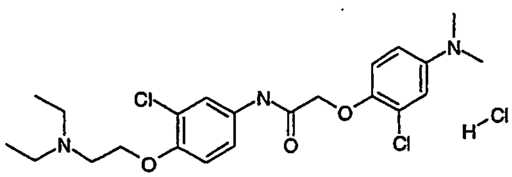

117) N-[3-Chlor-4-(2-diethylamino-ethoxy)-phenyl]-2-(2-chlor-4-dimethylamino-phenoxy)-acetamid

94.7 mg (0.200 mmol) 2-(4-Amino-2-chlor-phenoxy)-N-[3-chlor-4-(2-diethylamino-ethoxy)-phenyl]-acetamid (aus Beispiel 118), 0.149 mL (37%ig, 2.000 mmol) Formaldehyd-Lösung und 62.8 mg (1.000 mmol) Natriumcyanoborhydrid wurde bei RT in 5.0 mL Acetonitril vorgelegt. Unter Rühren wurde mit Eisessig pH 4-5 eingestellt. Nach 1 Stunde wurde das Reaktionsgemisch mit 12 %iger HCl angesäuert und 10 Minuten gerührt. Anschließend wurde mit 20 %iger Kaliumcarbonat-Lösung schwach alkalisch gestellt. Die wässrige Phase wurde mit EtOAc extrahiert. Die org. Phase wurde über Magnesiumsulfat getrocknet, filtriert und i. vac. eingeengt. Der Rückstand wurde mittels Säulenchromatographie gereinigt (Kieselgel; EtOAc/10 % konz. wässriger Ammoniak in MeOH 100:0 → 5:95). Der ölige Rückstand wurde mit etherischer HCl versetzt, i. vac. eingeengt und in 10 mL Isopropanol gelöst. Der ausgefallene Niederschlag wurde abfiltriert und i. vac. getrocknet.
Ausbeute: 0.035 g (36 % der Theorie)
$C_{22}H_{29}Cl_2N_3O_3$ * HCl (M= 490.862)

Ber. Molpeak $(M+H)^+$: 454/456/458 ($Cl_2$)
gef.: Molpeak $(M+H)^+$: 454/456/458 ($Cl_2$)
$R_f$-Wert: 0.40 (Kieselgel, Dichlormethan / MeOH/konz. wässriger Ammoniak 90:10:1)

Beispiel 118:

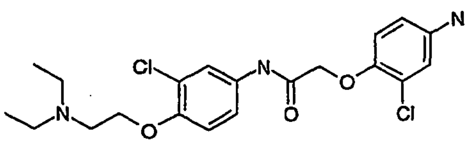

118) 2-(4-Amino-2-chlor-phenoxy)-N-[3-chlor-4-(2-diethylamino-ethoxy)-phenyl]-acetamid

0.310 g (0.679 mmol) N-[3-Chlor-4-(2-diethylamino-ethoxy)-phenyl]-2-(2-chlor-4-nitro-phenoxy)-acetamid (aus Beispiel 66) wurde in 10.0 mL EtOAc gelöst. Nach Zugabe von 0.030 g Pt/C (5%) wurde bei RT und 15 psi $H_2$-Atmosphäre 5 Stunden hydriert. Das Reaktionsgemisch wurde filtriert und das Filtrat i. vac. eingeengt. Der Rückstand wurde mit wenig EtOH gelöst. Der ausgefallene Niederschlag wurde abfiltriert und i. vac. getrocknet.
Ausbeute: 0.050 g (17 % der Theorie)
$C_{20}H_{25}Cl_2N_3O_3$ (M= 426.347)
Ber. Molpeak $(M+H)^+$: 426/428/430 ($Cl_2$)
gef.: Molpeak $(M+H)^+$: 426/428/430 ($Cl_2$)
$R_f$-Wert: 0.24 (Kieselgel, Dichlormethan / MeOH/konz. wässriger Ammoniak 90:10:1).

Beispiel 119:

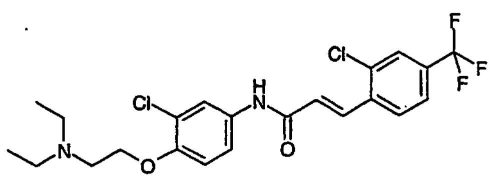

119) (E)-N-[3-Chlor-4-(2-diethylamino-ethoxy)-phenyl]-3-(2-chlor-4-trifluormethylphenyl)-acrylamid

Zu einer Lösung von 0.28 g (1.00 mmol) 3-Chlor-4-(2-diethylamino-ethoxy)-phenylamin-hydrochlorid (Zwischenprodukt Z1b), 0.25 g (1.00 mmol) (E)-3-(2-Chlor-4-trifluormethyl-phenyl)-acrylsäure (Z37b) und 0.34 g (1.05 mmol) TBTU in 10 mL abs.
THF wurde 0.29 mL (2.10 mmol) Triethylamin zugegeben und das Gemisch 1 Stunde bei RT gerührt. Das Reaktionsgemisch wurde i. vac. eingeengt und der Rückstand mit Dichlormethan und Wasser versetzt. Die org. Phase wurde abgetrennt, mit ges. wässriger Natriumbicarbonat-Lösung und Wasser gewaschen und i. vac. eingeengt.
Der Rückstand wurde mittels Säulenchromatographie (Kieselgel, Gradient Dichlormethan/10 % konz. wässriger Ammoniak in MeOH 100:0 → 5:95) gereinigt.
Ausbeute: 150 mg (32 % der Theorie)
$C_{22}H_{23}Cl_2F_3N_2O_2$ (M= 475.342)
Ber. Molpeak $(M+H)^+$: 475/477/479
gef.: Molpeak $(M+H)^+$: 475/477/479 ($Cl_2$)
$R_f$-Wert: 0.2 (Kieselgel, Dichlormethan / MeOH / konz. wässriger Ammoniak 95:5:1)

Beispiel 120:

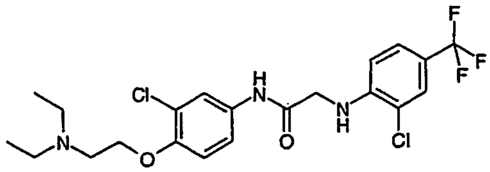

120) N-[3-Chlor-4-(2-diethylamino-ethoxy)-phenyl]-2-(2-chlor-4-trifluormethyl-phenylamino)-acetamid

Eine Lösung von 0.228 g (0.511 mmol) 2-Brom-N-[3-chlor-4-(2-diethylamino-ethoxy)-phenyl]-acetamid-hydrobromid (Zwischenprodukt Z1c) und 0.200 g (1.023 mmol) 2-Chlor-4-trifluormethyl-phenylamin in 5 mL DMF wurde 16 Stunden bei 90 ˚C und anschließend 24 Stunden bei 120 ˚C gerührt. Das Reaktionsgemisch wurde auf RT abgekühlt, mit Wasser verdünnt und mit EtOAc erschöpfend extrahiert. Die vereinigten org. Extrakte wurden über Magnesiumsulfat getrocknet und i. vac. eingeengt. Der Rückstand wurde in DMF gelöst und mittels HPLC-MS (Stable Bond C18; 3.5 µm; Wasser:Acetonitril:Ameisensäure 9:1:0.01 → 1:9:0.01 über 9 min) gereinigt. Ausbeute: 11 mg (5 % der Theorie)
$C_{21}H_{24}Cl_2F_3N_3O_2$ (M= 478.346)
Ber. Molpeak $(M+H)^+$: 478/480/482
gef.: Molpeak $(M+H)^+$: 478/480/482 ($Cl_2$)
$R_f$-Wert: 0.24 (Kieselgel, Dichlormethan / MeOH/konz. wässriger Ammoniak 90:10:1).

Beispiel 121:

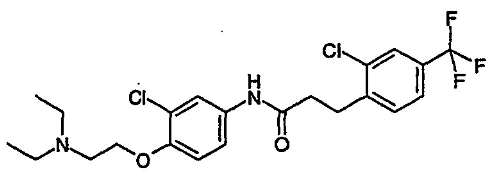

121a) 3-(2-Chlor-4-trifluormethyl-phenyl)-propionsäure

Zu einer Suspension von 0.500 g Raney-Nickel in abs. MeOH wurde bei RT 2.00 g (7.981 mmol) (E)-3-(2-Chlor-4-trifluormethyl-phenyl)-acrylsäure (Z37b) zugegeben und das Gemisch 4 Stunden bei 50 psi $H_2$-Atmosphäre hydriert. Der Katalysator wurde abfiltriert und das Filtrat i. vac. eingeengt.
Ausbeute: 1.90 g (94 % der Theorie)
$C_{10}H_8ClF_3O_2$ (M= 252.622)
Ber. Molpeak $(M-H)^-$: 251/253
gef.: Molpeak $(M+H)^+$: 251/253 (Cl)

121 b) N-[3-Chlor-4-(2-diethylamino-ethoxy)-phenyl]-3-(2-chlor-4-trifluormethyl-phenyl)-propionamid

Das Produkt wurde analog zu Beispiel 119 ausgehend von 0.400 g (1.433 mmol) 3-Chlor-4-(2-diethylamino-ethoxy)-phenylamin-hydrochlorid (Zwischenprodukt Z1 b) und 0.362 g (1.433 mmol) 3-(2-Chlor-4-trifluormethyl-phenyl)-propionsäure erhalten.
Ausbeute: 340 mg (50% der Theorie)
$C_{22}H_{25}Cl_2F_3N_2O_2$ (M= 477.358)
Ber. Molpeak $(M-H)^-$: 477/479/481
gef.: Molpeak $(M+H)^+$: 477/479/481 ($Cl_2$)
$R_f$-Wert: 0.30 (Kieselgel, Dichlormethan / MeOH / konz. wässriger Ammoniak 90:10:1)

Beispiel 122:

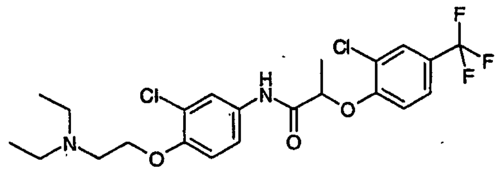

122a) 2-(2-Chlor-4-trifluormethyl-phenoxy)-propionsäure-ethylester

10.00 g (50.87 mmol) 2-Chlor-4-trifluormethyl-phenol, 7.11 mL (55.00 mmol) 2-Brompropionsäure-ethylester und 7.60 g (55 mmol) Kaliumcarbonat in 100 mL DMF wurde 16 Stunden bei 50 ˚C gerührt und anschließend filtriert. Das Filtrat wurde i. vac. eingeengt, mit Wasser versetzt und mit EtOAc erschöpfend extrahiert. Die vereinigten org. Extrakte wurden mit 10% wässriger Natriumcarbonat-Lösung und Wasser gewaschen, über Natriumsulfat getrocknet und i. vac. eingeengt.
Ausbeute: 14.10 g (93 % der Theorie)
$C_{12}H_{12}ClF_3O_3$ (M= 296.676)
Ber. Molpeak $(M+Na)^+$: 319/321
gef.: Molpeak $(M+Na)^+$: 319/321 (Cl)
$R_f$-Wert: 0.6 (Kieselgel, EtOAc/Petrolether 4:1)

122b) 2-(2-Chlor-4-trifluormethyl-phenoxy)-propionsäure

Zu einer Lösung von 14.00 g (0.047 mol) 2-(2-Chlor-4-trifluormethyl-phenoxy)-propionsäure-ethylester in 100 mL EtOH wurde 50 mL (0.100 mol) 2 M wässrige NaOH-Lösung zugegeben und das Gemisch 1 Stunde unter Rückfluss erhitzt. EtOH wurde i. vac. abgedampft, der Rückstand mit Eiswasser verdünnt und mit 2 M wässriger HCl angesäuert. Der gebildete Niederschlag wurde abfiltriert, mit Wasser gewaschen und bei 70 ˚C i. vac. getrocknet.
Ausbeute: 12.10 g (96 % der Theorie)
$C_{10}H_8ClF_3O_3$ (M= 268.622)
Ber. Molpeak $(M-H)^-$: 267/269
gef.: Molpeak $(M-H)^-$: 267/269 (Cl)

122c) N-[3-Chlor-4-(2-diethylamino-ethoxy)-phenyl]-2-(2-chlor-4-trifluormethyl-phenoxy)-propionamid-hydrochlorid

Zu einer Lösung von 0.364 g (1.500 mmol) 3-Chlor-4-(2-diethylamino-ethoxy)-phenylamin (Zwischenprodukt Z1b), 0.403 g (1.500 mmol) 2-(2-Chlor-4-trifluormethyl-phenoxy)-propionsäure und 0.562 g (1.750 mmol) TBTU in 10 mL abs. THF wurde 0.342 mL (2.000 mmol) Ethyl-diisopropylamin zugegeben und das Gemisch 1 Stunde bei RT gerührt. Das Reaktionsgemisch wurde i. vac. eingeengt und der Rückstand mit Dichlormethan und Wasser versetzt. Die org. Phase wurde abgetrennt, mit ges. wässriger Natriumbicarbonat-Lösung und Wasser gewaschen und i. vac. eingeengt. Der Rückstand wurde mittels Säulenchromatographie (Kieselgel, Gradient Dichlormethan / 10 % konz. wässriger Ammoniak in MeOH 100:0 → 5:95) gereinigt. Ausbeute: 450 mg (57 % der Theorie)
$C_{22}H_{25}Cl_2F_3N_2O_3$ * HCl (M= 529.818)
Ber. Molpeak $(M+H)^+$: 493/495/497
gef.: Molpeak $(M+H)^+$: 493/495/497 ($Cl_2$)
$R_f$-Wert: 0.30 (Kieselgel, Dichlormethan / MeOH / konz. wässriger Ammoniak 95:5:0.5).

Beispiel 123:

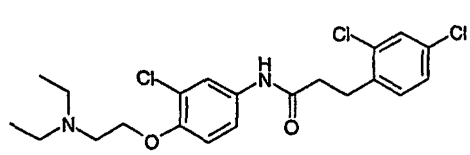

123) N-[3-Chlor-4-(2-diethylamino-ethoxy)-phenyl]-3-(2,4-dichlor-phenyl)-propionamid

Eine Lösung von 0.271 g (1.236 mmol) (3-(2,4-Dichlor-phenyl)-propionsäure in 3.00 mL Thionylchlorid wurde 2 Stunden bei RT gerührt, i. vac. eingeengt und in 10 mL Dichlormethan gelöst. Diese Lösung des Säurechlorids wurde langsam unter Eiskühlung zu einer Lösung von 0.300 (1.236 mmol) 3-Chlor-4-(2-diethylamino-ethoxy)-phenylamin (Zwischenprodukt Z1b) und 0.32 mL (1.854 mmol) Ethyl-diisopropylamin in 10 mL Dichlormethan zugetropft und das Gemisch 16 Stunden bei RT gerührt. Das Reaktionsgemisch wurde mit ges. wässriger Natriumbicarbonat-Lösung gewaschen, über Magnesiumsulfat getrocknet und i. vac. eingeengt. Der Rückstand wurde in EtOAc gelöst und mittels Säulenchromatographie (Kieslegel, EtOAc / MeOH / konz. wässriger Ammoniak 90:10:1) gereinigt.
Ausbeute: 60 mg (11 % der Theorie)
$C_{21}H_{25}Cl_3N_2O_2$ (M= 443.)
Ber. Molpeak $(M-Na)^-$: 441/443/445
gef.: Molpeak $(M-Na)^-$: 441/443/445 ($Cl_2$)
$R_f$-Wert: 0.27 (Kieselgel, Dichlormethan / MeOH / konz. wässriger Ammoniak 90:10:1)

Beispiel 124:

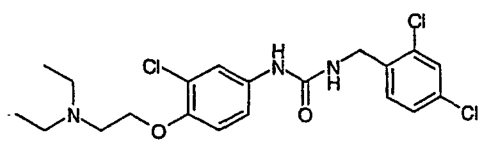

124) 1-[3-Chlor-4-(2-diethylamino-ethoxy)-phenyl]-3-(2,4-dichlor-benzyl)-harnstoff

Zu einer Lösung von 345 mg (1.236 mmol) 3-Chlor-4-(2-diethylamino-ethoxy)-phenylamin-hydrochlorid (Zwischenprodukt Z1b) und 0.56 mL (4.000 mmol) Triethylamin in 40 mL THF wurde 203 mg (1.236 mmol) CDT in 4 mL DMF zugegeben und das Gemisch 2 Stunden bei RT gerührt. 176 mg (1.236 mmol) 2,4-Dichlorbenzylamin wurde zugegeben, das Reaktionsgemisch 4 Stunden unter Rückfluss erhitzt und anschließend i. vac. eingeengt. Der Rückstand wurde mittels Säulenchromatographie (Kieselgel, Dichlormethan / MeOH / konz. wässriger Ammoniak 80:20:1) gereinigt und das Produkt mit Diisopropylether verrieben. Ausbeute: 300 mg (55 % der Theorie)
$C_{20}H_{24}Cl_3N_3O_2$ (M= 444.792)
Ber. Molpeak $(M+H)^+$: 444/446/448
gef.: Molpeak $(M+H)^+$: 444/446/448 ($Cl_3$)
$R_f$-Wert: 0.73 (Kieselgel, Dichlormethan / MeOH / konz. wässriger Ammoniak 80:20:1)

Beispiel 125:

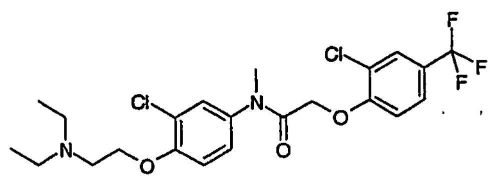

125a) [3-Chlor-4-(2-diethylamino-ethoxy)-phenyl]-carbaminsäure-tert-butylester

Zu einer Lösung von 0.500 g (2.06 mmol) 3-Chlor-4-(2-diethylamino-ethoxy)-phenylamin und 0.495 g (2.266 mmol) Boc-Anhydrid in 10 mL Dichlormethan wurde bei RT 0.31 mL (2.266 mmol) Triethylamin zugegeben und das Gemisch 48 Stunden gerührt. Das Reaktionsgemisch wurde mit Dichlormethan verdünnt und die org. Phase mit ges. wässriger Natriumbicarbonat-Lösung gewaschen. Die vereinigten org. Extrakte wurden über Magnesiumsulfat getrocknet und i. vac. eingeengt. Säulenchromatographie (Kieselgel, Dichlormethan / MeOH / konz. wässriger Ammoniak 90:

10:1) ergab das Produkt.
Ausbeute: 500 mg (71 % der Theorie)
$C_{17}H_{27}ClN_2O_3$ (M= 342.869)
Ber. Molpeak (M+H$^+$: 343/345
gef.: Molpeak (M+H)$^+$: 343/345 (Cl)

125b) [3-Chlor-4-(2-diethylamino-ethoxy)-phenyl]-methyl-amin

Unter Stickstoffatmosphäre wurde zu einer Suspension von 165 mg (4.374 mol) Lithiumaluminiumhydrid in 20 mL abs. THF eine Lösung von 500 mg (1.458 mmol) [3-Chlor-4-(2-diethylamino-ethoxy)-phenyl]-carbaminsäure-tert-butylester in 10 mL THF langsam zugetropft und das Gemisch 16 Stunden bei RT gerührt. 165 μL Wasser, 165 μL 15% wässrige NaOH-Lösung und weitere 495 μL Wasser wurde zugegeben und der gebildete Niederschlag abfiltriert. Das Filtrat wurde über Magnesiumsulfat getrocknet, i. vac. eingeengt und der Rückstand mittels Säulenchromatographie (Kieselgel, EtOAc / MeOH / konz. wässriger Ammoniak 90:10:1) gereinigt.
Ausbeute: 180 mg (48 % der Theorie)
$C_{13}H_{21}ClN_2O$ (M= 256.778)
Ber. Molpeak (M+H)$^+$: 257/259
gef.: Molpeak (M+H)$^+$: 257/259 (Cl)
$R_f$-Wert: 0.61 (Kieselgel, EtOAc / MeOH / konz. wässriger Ammoniak 90:10:1)

125c) N-[3-Chlor-4-(2-diethylamino-ethoxy)-phenyl]-2-(2-chlor-4-trifluormethyl-phenoxy)-N-methyl-acetamid

Zu einer Suspension von 231 mg (0.911 mmol) (2-Chlor-4-trifluormethyl-phenoxy)-essigsäure (Zwischenprodukt Z2b) in 5 mL abs. THF wurden 293 mg (0.911 mmol) TBTU und 123 mg (0.911 mmol) HOBT zugegeben und das Gemisch 10 Minuten bei RT gerührt. 180 mg (0.701 mmol) [3-Chlor-4-(2-diethylamino-ethoxy)-phenyl]-methylamin und 0.18 mL (1.051 mmol) Ethyl-diisopropylamin wurde zugegeben, das Gemisch 16 Stunden bei RT gerührt und i. vac. eingeengt. Der Rückstand wurde mittels Säulenchromatographie (Kieselgel, Dichlormethan / MeOH / konz. wässriger Ammoniak 85:15:1) gereinigt.
Ausbeute: 150 mg (43 % der Theorie)
$C_{22}H_{25}Cl_2F_3N_2O_3$ (M= 493.357)
Ber. Molpeak (M+H)$^+$: 493/495/497
gef.: Molpeak (M+H)$^+$: 493/495/497 ($Cl_2$)
$R_f$-Wert: 0.416 (Kieselgel, Dichlormethan / MeOH / konz. wässriger Ammoniak 90:10:1).

Beispiel 126:

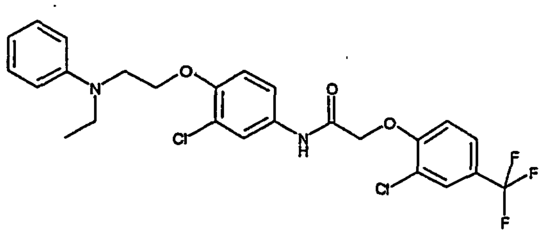

126) N-{3-Chlor-4-[2-(ethyl-phenyl-amino)-ethoxy]-phenyl}-2-(2-chlor-4-trifluoromethyl-phenoxy)-acetamid

Das Produkt wurde gemäß der allgemeinen Arbeitsvorschrift V ausgehend von 0.422 g (0.616 mmol) N-[3-Chlor-4-(2-oxo-ethoxy)-phenyl]-2-(2-chlor-4-trifluormethyl-phenoxy)-acetamid (Z3d) und 0.094 mL (0.739 mmol) N-Ethylanilin erhalten.
Ausbeute: 20 mg (6.2% der Theorie)
$C_{25}H_{23}Cl_2F_3N_2O_3$ (M= 527.375)
Ber. Molpeak (M-H)$^-$: 525/527/529
gef.: Molpeak (M-H)$^-$: 525/527/529 ($Cl_2$)
$R_f$-Wert: 0.94 (Kieselgel, EtOAc)

Beispiel 127:

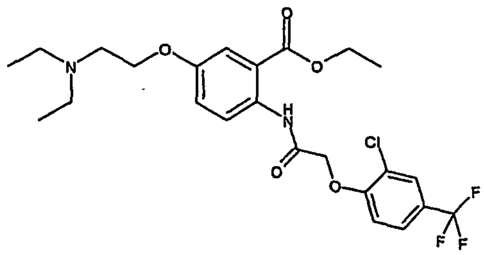

2-[2-(2-Chlor-4-trifluormethyl-phenoxy)-acetylamino]-5-(2-diethylamino-ethoxy)-benzoesäure-ethylester

Das Produkt wurde gemäß der allgemeinen Arbeitsvorschrift I ausgehend von 1.00 g (3.567 mmol) (2-Chlor-4-trifluormethyl-phenoxy)-essigsäure (Z2b) und 0.908 g (3.567 mmol) 2-Amino-5-(2-diethylamino-ethoxy)-benzoesäure-ethylester (Z25c) erhalten. Ausbeute: 1.700 g (92% der Theorie)
$C_{24}H_{28}ClF_3N_2O_5$ (M= 516.949)
Ber. Molpeak $(M-H)^-$: 515/517
gef.: Molpeak $(M-H)^-$: 515/517 (Cl)
$R_f$-Wert: 0.35 (Kieselgel, EtOAc / MeOH / konz. wässriger Ammoniak 90:10:1).

Beispiel 128:

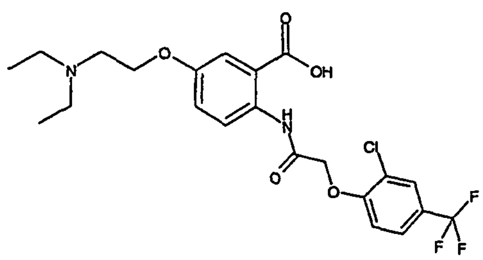

128) 2-[2-(2-Chlor-4-trifluormethyl-phenoxy)-acetylamino]-5-(2-diethylamino-ethoxy)-benzoesäure

Zu einer Lösung von 0.900 g (1.741 mmol) 2-[2-(2-Chlor-4-trifluormethyl-phenoxy)-acetylamino]-5-(2-diethylamino-ethoxy)-benzoesäure-ethylester (Beispiel 127) in 30 mL EtOH wurde 10 mL wässr. NaOH (1N) zugegeben und das Gemisch 2 h bei RT gerührt. Das Reaktionsgemisch wurde mit 10 mL wässr. HCl (1 M) angesäuert und 30 Min. mit Ultraschall behandelt. Der Niederschlag wurde abfiltriert, mit Wasser gewaschen und bei 50 ˚C i. vac. getrocknet.
Ausbeute: 0.640 g (75% der Theorie)
$C_{22}H_{24}ClF_3N_2O_5$ (M= 488.895)
Ber. Molpeak $(M-H)^-$: 487/489
gef.: Molpeak $(M-H)^-$: 487/489 (Cl)

Beispiel 129:

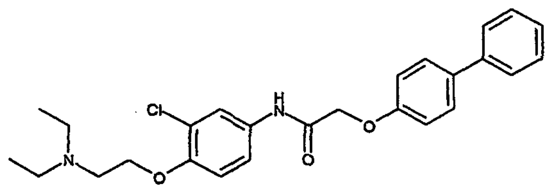

129) 2-(Biphenyl-4-yloxy)-N-[3-chlor-4-(2-diethylamino-ethoxy)-phenyl]-acetamid

Das Produkt wurde gemäß der allgemeinen Arbeitsvorschrift I aus (Biphenyl-4-yloxy)-essigsäure und 3-Chlor-4-(2-diethylamino-ethoxy)-phenylamin hergestellt.
Ausbeute: 0.560 g (58% der Theorie)
$C_{26}H_{29}ClN_2O_3$ (M= 452.986)
Ber. Molpeak $(M+H)^+$: 453/455
gef.: Molpeak $(M+H)^+$: 453/455 (Cl)
$R_f$-Wert: 0.76 (Kieselgel, Dichlormethan / MeOH / Cyclohexan / konz. wässriger Ammoniak 75:15:15:1.).

Beispiel 130:

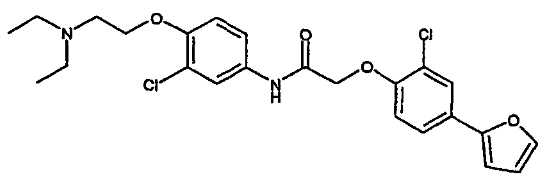

130a) (2-Chlor-4-furan-2-yl-phenoxy)-essigsäure-ethylester

Zu einer Lösung von 1.00 g (3.407 mmol) (4-Brom-2-chlor-phenoxy)-essigsäure-ethylester (Z26), 0.784 g (6.800 mmol) 2-Furanboronsäure und 0.196 g (0.170 mmol) Tetrakis-triphenylphosphin-palladium in 65 mL Dioxan wurde 3.5 mL wässr. Natriumcarbonat-Lösung (1 M) zugegeben und das Gemisch 4 h unter Rückfluss erhitzt. Das Reaktionsgemisch wurde auf RT gekühlt, i. vac. eingeengt, mit Wasser verdünnt und mit EtOAc erschöpfend extrahiert. Die vereinigten org. Phasen wurden mit ges. wässr. Natriumbicarbonat, Wasser und ges. wässr. NaCl gewaschen, über Natriumsulfat getrocknet und i. vac. eingeengt. Säulenchromatographie (Kieselgel, Petrolether / EtOAc 3:1) ergab das Produkt.
Ausbeute: 0.800 g (84% der Theorie)
$C_{14}H_{13}ClO_4$ (M= 280.710)
Ber. Molpeak $(M+H)^+$: 281/283
gef.: Molpeak $(M+H)^+$: 281/283 (Cl)
$R_f$-Wert: 0.56 (Kieselgel, Petrolether / EtOAc 3:1).

130b) (2-Chlor-4-furan-2-yl-phenoxy)-essigsäure

Zu einer Lösung von 0.280 g (2.672 mmol) (2-Chlor-4-furan-2-yl-phenoxy)-essigsäure-ethylester (130a) in 20 mL abs. EtOH wurde 0.40 g (10.00 mmol) NaOH in 5 mL Wasser zugegeben und das Gemisch 16 h bei RT gerührt. Das Rektionsgemisch wurde mit Wasser verdünnt und mit halbkonz. wässr. HCl auf pH 1 angesäuert. Der Niederschlag wurde abfiltriert, mit Wasser gewaschen und im HV getrocknet.
Ausbeute: 0.630 g (93% der Theorie)
$C_{12}H_9ClO_4$ (M= 252.656)
Ber. Molpeak $(M-H)^-$: 251/253
gef.: Molpeak $(M-H)^-$: 251/253 (Cl)

130c) N-[3-Chlor-4-(2-diethylamino-ethoxy)-phenyl]-2-(2-chlor-4-furan-2-yl-phenoxy)-acetamid

Das Produkt wurde gemäß der allgemeinen Arbeitsvorschrift I aus 2-Chlor-4-furan-2-yl-phenoxy)-essigsäure (130b) und 3-Chlor-4-(2-diethylamino-ethoxy)-phenylamin (Z1b) hergestellt.
Ausbeute: 0.380 g (79% der Theorie)
$C_{24}H_{26}Cl_2N_2O_4$ (M= 477.392)
Ber. Molpeak $(M+H)^+$: 477/479/481
gef.: Molpeak $(M+H)^+$: 477/479/481 ($Cl_2$)
$R_f$-Wert: 0.70 (Alox, Dichlormethan / MeOH 30:1).

Beispiel 131:

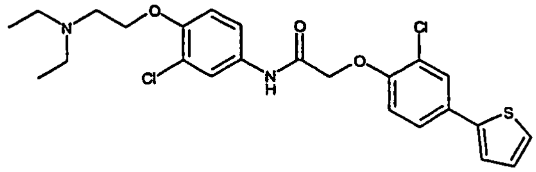

131a) (2-Chlor-4-thiophen-2-yl-phenoxy)-essigsäure-ethylester

Hergestellt analog Beispiel 130a aus (4-Brom-2-chlor-phenoxy)-essigsäure-ethylester (Z26) und Thiophen-2-boronsäure.
Ausbeute: 0.730 g (72% der Theorie)
$C_{14}H_{13}ClO_3S$ (M= 296.775)
Ber. Molpeak $(M+H)^+$: 297/299
gef.: Molpeak $(M+H)^+$: 297/299 (Cl)
$R_f$-Wert: 0.60 (Kieselgel, Petrolether / EtOAc 3:1).

131b) (2-Chlor-4-thiophen-2-yl-phenoxy)-essigsäure

Hergestellt analog Beispiel 130b aus (2-Chlor-4-thiophen-2-yl-phenoxy)-essigsäure-ethylester (131a).
Ausbeute: 0.600 g (95% der Theorie)
$C_{12}H_9ClO_3S$ (M= 268.721)
Ber. Molpeak $(M+H)^+$: 267/269
gef.: Molpeak $(M+H)^+$: 267/269 (Cl)

131c) N-[3-Chlor-4-(2-diethylamino-ethoxy)-phenyl]-2-(2-chlor-4-thiophen-2-yl-phenoxy)-acetamid

Das Produkt wurde gemäß der allgemeinen Arbeitsvorschrift I aus 2-Chlor-4-thiophen-2-yl-phenoxy)-essigsäure (131b) und 3-Chlor-4-(2-diethylamino-ethoxy)-phenylamin (Z1b) hergestellt.
Ausbeute: 0.410 g (83% der Theorie)
$C_{24}H_{26}Cl_2N_2O_3S$ (M= 493.456)
Ber. Molpeak $(M+H)^+$: 491/493/495
gef.: Molpeak $(M+H)^+$: 491/493/495 ($Cl_2$)
$R_f$-Wert: 0.64 (Alox, Dichlormethan / MeOH 30:1).

Beispiel 132:

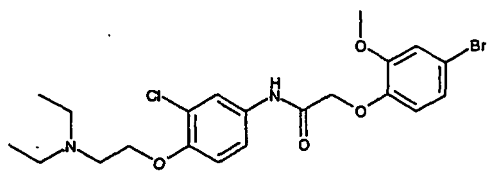

132a) 2-(4-Brom-2-methoxy-phenoxy)-essigsäure-ethylester

Hergestellt analog Zwischenprodukt Z2a aus Brom-essigsäure-ethylester und 2-Methoxy-4-bromphenol.
Ausbeute: 3.600 g (83% der Theorie)
$C_{11}H_{13}BrO_4$ (M= 289.128)
Ber. Molpeak $(M+H)^+$: 289/291
gef.: Molpeak $(M+H)^+$: 289/291 (Br).

132b) 2-(4-Brom-2-methoxy-phenoxy)-essigsäure

Hergestellt analog Beispiel 130b aus 2-(4-Brom-2-methoxy-phenoxy)-essigsäure-ethylester (132a).
Ausbeute: 3.250 g (quant. Ausbeute)
$C_9H_9BrO_4$ (M= 289.128)
Ber. Molpeak $(M+H)^+$: 261/263
gef.: Molpeak $(M+H)^+$: 261/263 (Br).

132c) 2-(4-Brom-2-methoxy-phenoxy)-N-[3-chloro-4-(2-diethylamino-ethoxy)-phenyl]-acetamid

Das Produkt wurde gemäß der allgemeinen Arbeitsvorschrift I aus 2-(4-Brom-2-methoxy-phenoxy)-essigsäure (132b) und 3-Chlor-4-(2-diethylamino-ethoxy)-phenylamin (Z1b) hergestellt.
Ausbeute: 0.050 g (20% der Theorie)
$C_{21}H_{26}BrClN_2O_4$ (M= 485.809)
Ber. Molpeak $(M+H)^+$: 485/487/489
gef.: Molpeak $(M+H)^+$: 485/487/489 (BrCl)
$R_f$-Wert: 0.55 (Kieselgel, Dichlormethan / MeOH 9:1).

Beispiel 133:

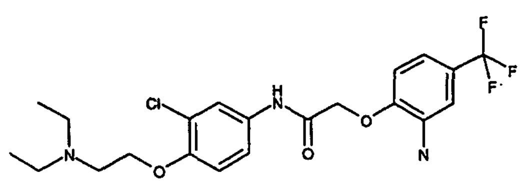

133) 2-(2-Amino-4-trifluormethyl-phenoxy)-N-[3-chlor-4-(2-diethylamino-ethoxy)-phenyl]-acetamid

Eine Suspension von 0.280 g (0.570 mmol) 2-(2-Amino-4-trifluormethyl-phenoxy)-N-[3-chlor-4-(2-diethylamino-ethoxy)-phenyl]-acetamid (Beispiel 65) und 50 mg Raney-Nickel in 10 mL MeOH wurde 5 h bei 50 psi hydriert. Der Katalysator wurde abfiltriert und das Filtrat i. vac. eingeengt. Säulenchromatographie (Alox, N, Akt. II-III, Gradient Dichlormethan / MeOH 49:1 $\rightarrow$ 19:1) ergab das Produkt.
Ausbeute: 0.180 g (69% der Theorie)
$C_{21}H_{25}ClF_3N_3O_3$ (M= 459.900)
Ber. Molpeak $(M+H)^+$: 460/462
gef.: Molpeak $(M+H)^+$: 460/462 (Cl)
$R_f$-Wert: 0.42 (Alox, Dichlormethan / MeOH 19:1).

Beispiel 134:

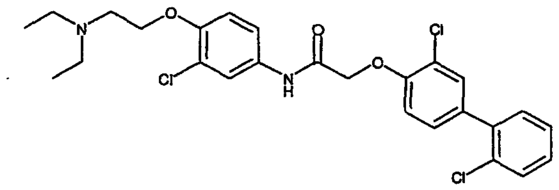

134a) (3,2'-Dichlor-biphenyl-4-yloxy)-essigsäure-ethylester

Hergestellt analog Beispiel 130a aus (4-Brom-2-chlor-phenoxy)-essigsäure-ethylester (Z26) und 2-Chlorphenylboronsäure.
Ausbeute: 1.070 g (97% der Theorie)
$C_{16}H_{14}Cl_2O_3$ (M= 325.194)
$R_f$-Wert: 0.58 (Kieselgel, Petrolether / EtOAc 3:1).

134b) (3,2'-Dichlor-biphenyl-4-yloxy)-essigsäure

Hergestellt analog Beispiel 130b aus (3,2'-dichlor-biphenyl-4-yloxy)-essigsäure-ethylester (134a).
Ausbeute: 0.900 g (92% der Theorie)
$C_{14}H_{10}Cl_2O_3$ (M= 297.140)
Ber. Molpeak $(M-H)^-$: 295/297/299
gef.: Molpeak $(M-H)^-$: 295/297/299 ($Cl_2$)

134c) N-[3-Chlor-4-(2-diethylamino-ethoxy)-phenyl]-2-(3,2'-dichlor-biphenyl-4-yloxy)-acetamid

Das Produkt wurde gemäß der allgemeinen Arbeitsvorschrift I aus (3,2'-dichlorbiphenyl-4-yloxy)-essigsäure (134b) und 3-Chlor-4-(2-diethylamino-ethoxy)-phenylamin (Z1b) hergestellt.
Ausbeute: 0.250 g (48% der Theorie)
$C_{26}H_{27}Cl_3N_2O_3$ (M= 521.876)
Ber. Molpeak $(M+H)^+$: 521/523/525/527
gef.: Molpeak $(M+H)^+$: 521/523/525/527 (Cl3)
$R_f$-Wert: 0.65 (Alox, Dichlormethan / MeOH 30:1).

Beispiel 135:

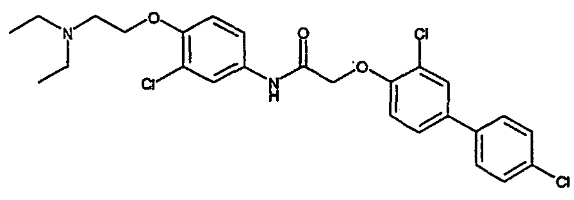

135a) (3,4'-Dichlor-biphenyl-4-yloxy)-essigsäure-ethylester

Hergestellt analog Beispiel 130a aus (4-Brom-2-chlor-phenoxy)-essigsäure-ethylester (Z26) und 4-Chlorphenylboronsäure.
Ausbeute: 0.430 g (68% der Theorie)
$C_{16}H_{14}Cl_2O_3$ (M= 325.194)
$R_f$-Wert: 0.62 (Kieselgel, Petrolether / EtOAc 3:1).

135b) (3,4'-Dichlor-biphenyl-4-yloxy)-essigsäure

Hergestellt analog Beispiel 130b aus (3,4'-dichlor-biphenyl-4-yloxy)-essigsäure-ethylester (135a).
Ausbeute: 0.299 g (76% der Theorie)
$C_{14}H_{10}Cl_2O_3$ (M= 297.140)
Ber. Molpeak $(M-H)^-$: 295/297/299
gef.: Molpeak $(M-H)^-$: 295/297/299 ($Cl_2$)

135c) N-[3-Chlor-4-(2-diethylamino-ethoxy)-phenyl]-2-(3,4'-dichlor-biphenyl-4-yloxy)-acetamid

Das Produkt wurde gemäß der allgemeinen Arbeitsvorschrift I aus (3,4'-dichlorbiphenyl-4-yloxy)-essigsäure (135b) und 3-Chlor-4-(2-diethylamino-ethoxy)-phenylamin (Z1b) hergestellt.
Ausbeute: 0.280 g (60% der Theorie)
$C_{26}H_{27}Cl_3N_2O_3$ (M= 521.876)
Ber. Molpeak $(M+H)^+$: 521/523/525/527
gef.: Molpeak $(M+H)^+$: 521/523/525/527 ($Cl_3$)
$R_f$-Wert: 0.48 (Alox, Dichlormethan / MeOH 50:1).

Beispiel 136:

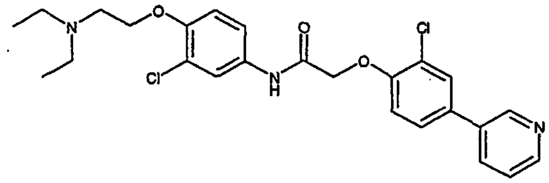

136a) (2-Chlor-4-pyridin-3-yl-phenoxy)-essigsäure-ethylester

Hergestellt analog Beispiel 130a aus (4-Brom-2-chlor-phenoxy)-essigsäure-ethylester (Z26) und Pyridin-3-boronsäure.
Ausbeute: 0.420 g (58 % der Theorie)
$C_{16}H_{14}ClNO_3$ (M= 291.737)
$R_f$-Wert: 0.45 (Kieselgel, Petrolether / EtOAc 1:3).

136b) (2-Chlor-4-pyridin-3-yl-phenoxy)-essigsäure

Zu einer Lösung von 0.400 g (1.371 mmol) (2-Chlor-4-pyridin-3-yl-phenoxy)-essigsäure-ethylester in 40 mL abs. EtOH wurde 250 mg (6.250 mmol) NaOH in 10 mL Wasser zugegeben und das Gemisch 1 h bei RT gerührt. Das Reaktionsgemisch wurde mit wässr. HCl (1 M) auf pH 7.5 angesäuert, der Niederschlag abfiltriert und mit Wasser gewaschen.
Ausbeute: 0.269 g (74 % der Theorie)
$C_{13}H_{10}ClNO_3$ (M= 263.683)
Ber. Molpeak $(M+Na)^+$: 264/266
gef.: Molpeak $(M+Na)^+$: 264/266 ($Cl_3$)

136c) N-[3-Chlor-4-(2-diethylamino-ethoxy)-phenyl]-2-(2-chlor-4-pyridin-3-yl-phenoxy)-acetamid

Das Produkt wurde gemäß der allgemeinen Arbeitsvorschrift I aus (2-Chlor-4-pyridin-3-yl-phenoxy)-essigsäure (136b) und 3-Chlor-4-(2-diethylamino-ethoxy)-phenylamin (Z1b) hergestellt.
Ausbeute: 0.328 g (75% der Theorie)
$C_{25}H_{27}Cl_2N_3O_3$ (M= 488.418)
Ber. Molpeak $(M+H)^+$: 488/490/492
gef.: Molpeak $(M+H)^+$: 488/490/492 ($Cl_2$)
$R_f$-Wert: 0.47 (Alox, Dichlormethan / MeOH 30:1).

Beispiel 137:

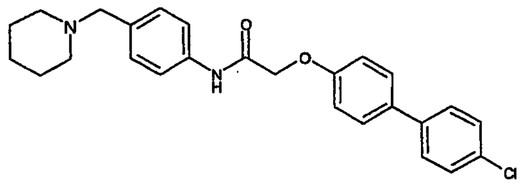

137a) (4'-Chlor-biphenyl-4-yloxy)-essigsäure

Zu einer Lösung von 0.750 g (2.000 mmol) (4'-Chlor-biphenyl-4-yloxy)-essigsäure-tert-butylester in 20 mL Dichlormethan wurde 1.5 mL TFA zugegeben und das Gemisch 16 h bei RT gerührt. Das Reaktionsgemisch wurde i. vac. eingeengt und mit 1 M wässr. NaOH und EtOAc verdünnt. Der Niederschlag wurde abfiltriert und bei 80 ˚C i. vac. getrocknet.
Ausbeute: 0.522 g (quant. Ausbeute)
$C_{14}H_{11}ClO_3$ (M= 262.695)
Ber. Molpeak $(M-H)^-$: 261/263
gef.: Molpeak $(M-H)^-$: 261/263 (Cl)

137b) 2-(4'-Chlor-biphenyl-4-yloxy)-N-(4-piperidin-1-ylmethyl-phenyl)-acetamid

Das Produkt wurde gemäß der allgemeinen Arbeitsvorschrift I aus (4'-Chlor-biphenyl-4-yloxy)-essigsäure (137a) und 4-Piperidin-ylmethyl-phenylamin hergestellt.
Ausbeute: 0.140 g (40% der Theorie)
$C_{26}H_{27}ClN_2O_2$ (M= 434.970)
Ber. Molpeak $(M+H)^+$: 435/437
gef.: Molpeak $(M+H)^+$: 435/437 (Cl)
$R_f$-Wert: 0.40 (Kieselgel, Dichlormethan / MeOH 9:1).

Beispiel 138:

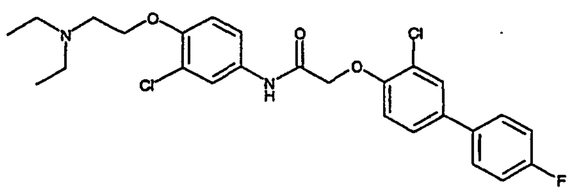

138a) (3-Chlor-4'-fluor-biphenyl-4-yloxy)-essigsäure-ethylester

Hergestellt analog Beispiel 130a aus (4-Brom-2-chlor-phenoxy)-essigsäure-ethylester (Z26) und 4-Fluor-phenylboronsäure.
Ausbeute: 0.850 g (81 % der Theorie)
$C_{16}H_{14}ClFO_3$ (M= 308.740)
Ber. Molpeak $(M+H)^+$: 309/311
gef.: Molpeak $(M+H)^+$: 309/311 (Cl)
$R_f$-Wert: 0.45 (Kieselgel, Petrolether / EtOAc 3:1).

138b) (3-Chlor-4'-fluor-biphenyl-4-yloxy)-essigsäure

Hergestellt analog Beispiel 130b aus (3-Chlor-4'-fluor-biphenyl-4-yloxy)-essigsäure-ethylester (138a).
Ausbeute: 0.520 g (67% der Theorie)
$C_{14}H_{10}ClFO_3$ (M= 280.685)
Ber. Molpeak $(M-H)^-$: 279/281
gef.: Molpeak $(M-H)^-$: 279/281 (Cl)

138c) N-[3-Chlor-4-(2-diethylamino-ethoxy)-phenyl]-2-(3-chlor-4'-fluor-biphenyl-4-yloxy)-acetamid

Das Produkt wurde gemäß der allgemeinen Arbeitsvorschrift I aus (3-Chlor-4'-fluor-biphenyl-4-yloxy)-essigsäure (138b) und 3-Chlor-4-(2-diethylamino-ethoxy)-phenylamin (Z1b) hergestellt.
Ausbeute: 0.440 g (87% der Theorie)
$C_{26}H_{27}Cl_2FN_2O_3$ (M= 505.421)
Ber. Molpeak $(M+H)^+$: 505/507/509
gef.: Molpeak $(M+H)^+$: 505/507/509 ($Cl_2$)
$R_f$-Wert: 0.46 (Alox, Dichlormethan / MeOH 50:1).

Beispiel 139:

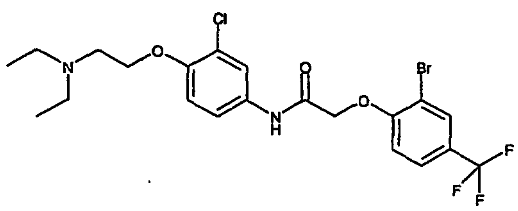

139a) (2-Brom-4-trifluormethyl-phenoxy)-essigsäure-ethylester

Eine Lösung von 2.650 g (11.000 mmol) 2-Brom-4-trifluormethyl-phenol und 1.47 mL 13.20 mmol) Bromessigsäure-ethylester in 20 mL Hünigbase wurde 5 h bei 120 °C gerührt. Das Reaktionsgemisch wurde mit EtOAc verdünnt und die org. Phase mit Wasser, ges, wässr. Natriumbicarbonat und ges. wässr. NaCl gewaschen, über Magnesiumsulfat getrocknet und i. vac. eingeengt. Säulechromatographie (Kieselgel, Gradient Dichlormethan / MeOH 100:0 → 19:1) ergab das Produkt. Ausbeute: 2.80 g (78% der Theorie)
$C_{11}H_{10}BrF_3O_3$ (M= 327.100)
Ber. Molpeak $(M+H)^+$: 327/329
gef.: Molpeak $(M+H)^+$: 327/329 (Br)
$R_f$-Wert: 0.85 (Kieselgel, Dichlormethan).

139b) (2-Brom-4-trifluormethyl-phenoxy)-essigsäure

Hergestellt analog Beispiel 130b aus (2-Brom-4-trifluormethyl-phenoxy)-essigsäure-ethylester (139a).
Ausbeute: 2.150 g (84% der Theorie)
$C_9H_6BrF_3O_3$ (M= 299.046)
Ber. Molpeak $(M-H)^-$: 297/299
gef.: Molpeak $(M-H)^-$: 297/299 (Br)
$R_f$-Wert: 0.35 (Kieselgel, Dichlormethan / MeOH 49:1).

139c) 2-(2-Brom-4-trifluormethyl-phenoxy)-N-[3-chlor-4-(2-diethylamino-ethoxy)-phenyl]-acetamid

Das Produkt wurde gemäß der allgemeinen Arbeitsvorschrift I aus (2-Brom-4-trifluormethyl-phenoxy)-essigsäure (139b) und 3-Chlor-4-(2-diethylamino-ethoxy)-phenylamin (Z1b) hergestellt.
Ausbeute: 0.450 g (86% der Theorie)
$C_{21}H_{23}BrClF_3N_2O_3$ (M= 523.781)

Ber. Molpeak $(M+H)^+$: 523/525/527
gef.: Molpeak $(M+H)^+$: 523/525/527 (BrCl)
$R_f$-Wert: 0.45 (Kieselgel, Dichlormethan / MeOH 9:1).

Beispiel 140:

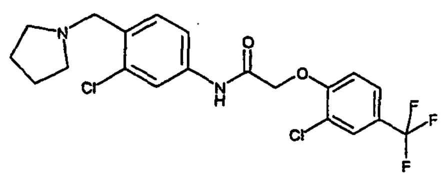

140a) 1-(2-Ghlor-4-nitro-benzyl)-pyrrolidin

Zu einer Lösung von 7.400 g (29.543 mmol) 1-Brommethyl-2-chlor-4-nitro-benzol und 5.00 mL (59.300 mmol) Pyrrolidin in 150 mL Acetonitril wurde 10.50 g (74.452 mmol) Kaliumcarbonat zugegeben und das Gemisch 16 h bei RT gerührt. Das Reaktionsgemisch wurde mit 200 mL Dichlormethan verdünnt, filtriert und das Filtrat i. vac. eingeengt. Der Rückstand wurde in EtOAc aufgenommen, die org. Phase mit Wasser gewaschen, über Magnesiumsulfat getrocknet und i. vac. eingeengt. Das Rohprodukt wurde ohne weitere Reinigung in den nachfolgenden Reaktionsschritt eingesetzt.
Ausbeute: 6.100 g (86% der Theorie)
$C_{11}H_{13}ClN_2O_2$ (M= 240.691)
Ber. Molpeak $(M+H)^+$: 241/243
gef.: Molpeak $(M+H)^+$: 241/243 (Cl)
$R_f$-Wert: 0.38 (Kieselgel, Petrolether / EtOAc 2:1).

140b) 3-Chlor-4-pyrrolidin-1-ylmethyl-phenylamin

Eine Suspension von 1.00 g (4.155 mmol) 1-(2-Chlor-4-nitro-benzyl)-pyrrolidin (140a) und 100 mg Raney-Nickel in 30 mL MeOH wurden bei RT und 10 psi hydriert. Der Katalysator wurde abfiltriert und das Filtrat i. vac. eingeengt.
Ausbeute: 0.840 g (96% der Theorie)
$C_{11}H_{15}ClN_2$ (M= 210.709)
Ber. Molpeak $(M+H)^+$: 211/213
gef.: Molpeak $(M+H)^+$: 211/213 (Cl)
$R_f$-Wert: 0.78 (Kieselgel, Dichlormethan / MeOH / Cyclohexan / konz. wässr. Ammoniak 75:15:15:1).

140c) N-(3-Chlor-4-pyrrolidin-1-ylmethyl-phenyl)-2-(2-chlor-4-trifluormethyl-phenoxy)-acetamid

Das Produkt wurde gemäß der allgemeinen Arbeitsvorschrift I aus (2-Chlor-4-trifluormethyl-phenoxy)-essigsäure (Z2b) und 3-Chlor-4-pyrrolidin-1-ylmethyl-phenylamin (140b) hergestellt.
Ausbeute: 0.360 g (51 % der Theorie)
$C_{20}H_{19}C_{12}F_3N_2O_2$ (M= 447.288)
Ber. Molpeak $(M+H)^+$: 447/449/451
gef.: Molpeak $(M+H)^+$: 447/449/451 ($Cl_2$)
$R_f$-Wert: 0.58 (Kieselgel, Dichlormethan / EtOAc / MeOH / konz. wässr. Ammoniak 350:75:75:10).

Beispiel 141:

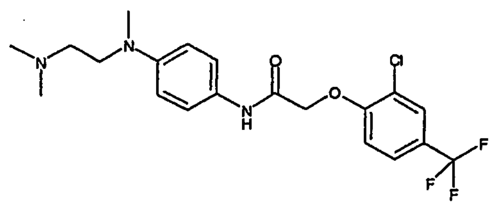

141a) N,N,N'-Trimethyl-N'-(4-nitro-phenyl)-ethan-1,2-diamin

3.600 g (25.514 mmol) 1-Fluor-4-nitrobenzol und 8.00 mL (62.948 mmol) N,N,N'-Trimethyl-ethylen-1,2-diamin wurden 1 h bei 100 ˚C gerührt und anschließend auf RT gekühlt. Das Reaktionsgemisch wurde mit Wasser verdünnt und mit Ether erschöpfend extrahiert. Die vereinigten org. Phasen wurden über Natriumsulfat getrocknet und i. vac. eingeengt.
Ausbeute: 5.771 g (quant. Ausbeute)
$C_{11}H_{17}N_3O_2$ (M= 223.277)
Ber. Molpeak $(M+H)^+$: 224
gef.: Molpeak $(M+H)^+$: 224
$R_f$-Wert: 0.32 (Kieselgel, EtOAc / EtOH / konz. wässr. Ammoniak 50:10:1).

141b) N-(2-Dimethylamino-ethyl)-N-methylbenzol-1,4-diamin

Eine Suspension von 5.770 g (25.842 mmol), N,N'-Trimethyl-N'-(4-nitro-phenyl)-ethan-1,2-diamin (141a) und 0.60 g Pd/C (10%) in EtOH wurde 4.5 h bei 50 psi hydriert. Der Katalysator wurde abfiltriert und das Filtrat i. vac. eingeengt. Das Rohprodukt wurde sofort weiter umgesetzt.
Ausbeute: 3.162 g (63% der Theorie)
$C_{11}H_{19}N_3$ (M= 1943.294)

141c) 2-(2-Chlor-4-trifluormethyl-phenoxy)-N-{4-[(2-dimethylamino-ethyl)-methyl-amino]-phenyl}-acetamid

Das Produkt wurde gemäß der allgemeinen Arbeitsvorschrift I aus (2-Chlor-4-trifluormethyl-phenoxy)-essigsäure (Z2b) und N-(2-Dimethylamino-ethyl)-N-methylbenzol-1,4-diamin (141b) hergestellt.
Ausbeute: 0.110 g (37% der Theorie)
$C_{20}H_{23}ClF_3N_3O_2$ (M= 429.873)
Ber. Molpeak $(M+H)^+$: 430/432
gef.: Molpeak $(M+H)^+$: 430/432 (Cl)
$R_f$-Wert: 0.37 (Kieselgel, Dichlormethan / MeOH / konz. wässr. Ammoniak 90:10:1).

Beispiel 142:

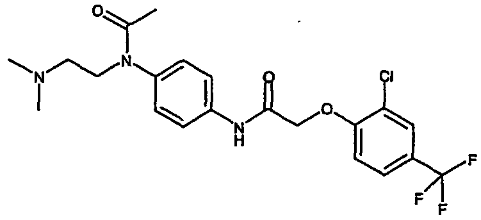

142a) N-(2-Dimethylamino-ethyl)-N-(4-nitro-phenyl)-acetamid

Eine Lösung von 50 g (0.239 mol) N,N-Dimethyl-N'-(4-nitro-phenyl)-ethan-1,2-diamin in 500 mL Essigsäureanhydrid wurde 3.5 h bei 130 ˚C gerührt, anschließend i. vac. eingeengt und mit ges. wässr. Natriumbicarbonat neutralisiert. Der Rückstand wurde mit EtOAc erschöpfend extrahiert, die vereinigten org. Phasen über Natriumsulfat getrocknet

und i. vac. eingeengt.
Ausbeute: 58.36 g (97% der Theorie)
$C_{12}H_{17}N_3O_3$ (M= 251.288)
Ber. Molpeak $(M+H)^+$: 252
gef.: Molpeak $(M+H)^+$: 252
$R_f$-Wert: 0.55 (Kieselgel, Dichlormethan / MeOH / konz. wässr. Ammoniak 90:10:1).

142b) N-(4-Amino-phenyl)-N-(2-dimethylamino-ethyl)-acetamid

Hergestellt analog zu Beispiel 141b ausgehend von -(2-Dimethylamino-ethyl)-N-(4-nitro-phenyl)-acetamid) (142a).
Ausbeute: 50.66 g (99% der Theorie)
$C_{12}H_{19}N_3O$ (M= 221.305)
Ber. Molpeak $(M+H)^+$: 222
gef.: Molpeak $(M+H)^+$: 222
$R_f$-Wert: 0.50 (Kieselgel, Dichlormethan / MeOH / konz. wässr. Ammoniak 90:10:1).

142c) N-{4-[Acetyl-(2-dimethylamino-ethyl)-amino]-phenyl}-2-(2-chlor-4-trifiuormethyl-phenoxy)-acetamid

Das Produkt wurde gemäß der allgemeinen Arbeitsvorschrift I aus (2-Chlor-4-trifluormethyl-phenoxy)-essigsäure (Z2b) und N-(4-Amino-phenyl)-N-(2-dimethylaminoethyl)-acetamid (142b) hergestellt.
Ausbeute: 0.200 g (64% der Theorie)
$C_{21}H_{23}ClF_3N_3O_3$ (M= 457.884)
Ber. Molpeak $(M+H)^+$: 458/460
gef.: Molpeak $(M+H)^+$: 458/460 (Cl)
$R_f$-Wert: 0.64 (Alox, Dichlormethan / MeOH 19:1).

Beispiel 143:

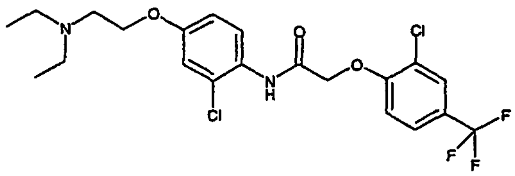

143) N-[2-Chlor-4-(2-diethylamino-ethoxy)-phenyl]-2-(2-chlor-4-trifluormethyl-phenoxy)-acetamid

Zu einer Lösung von 0.254 g (0.982 mmol) (2-Chlor-4-trifluormethyl-phenoxy)-essigsäure (Z2b) und 0.119 mL (1.080 mmol) N-Methylmorpholin in 20 mL abs. THF wurde bei -10 °C 0.141 mL (1.080 mmol) Chlorameisensäureisopropylester zugetropft und das Gemisch weitere 10 Min. gerührt. 0.250 g (1.031 mmol) 2-Chlor-4-(2-diethylamino-ethoxy)-phenylamin (Z27b) wurde zugegeben, das Gemisch 1 h bei RT gerührt und anschließend i. vac. eingeengt. Wasser wurde zugegeben und die wässrige Phase mit Dichlormethan erschöpfend extrahiert. Die vereinigten org. Phasen wurde über Natriumsulfat getrocknet, i. vac. eingeengt und der Rückstand mit Ether verrührt. Der Niederschlag wurde abfiltriert, mit Ether gewaschen und im HV getrocknet.
Ausbeute: 0.210 g (45% der Theorie)
$C_{21}H_{23}C1_2F_3N_2O_3$ (M= 479.330)
Ber. Molpeak $(M+H)^+$; 479/481/483
gef.: Molpeak $(M+H)^+$: 479/481/483 ($Cl_2$)
$R_f$-Wert: 0.67 (Alox, Dichlormethan / MeOH 39:1).

Beispiel 144:

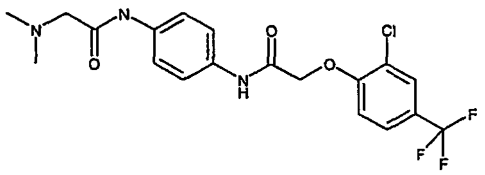

144) 2-(2-Chlor-4-trifluormethyl-phenoxy)-N-[4-(2-dimethylamino-acetylamino)-phenyl]-acetamid

Hergestellt analog Beispiel 143 ausgehend von (2-Chlor-4-trifluormiethyl-phenoxy)-essigsäure (Z2b) und N-(4-Amino-phenyl)-2-dimethylamino-acetamid.
Ausbeute: 0.270 g (64% der Theorie)
$C_{19}H_{19}ClF_3N_3O_3$ (M= 429.830)
Ber. Molpeak $(M+H)^+$: 430/432
gef.: Molpeak $(M+H)^+$: 430/432 (Cl)
$R_f$-Wert: 0.82 (Alox, Dichlormethan / MeOH 39:1).

Beispiel 145:

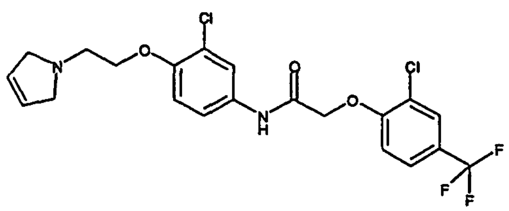

145) N-{3-Chlor-4-[2-(2,5-dihydro-pyrrol-1-yl)-ethoxy]-phenyl}-2-(2-chlor-4-trifluormethyl-phenoxy)-acetamid

Eine Lösung von 0.145 g (0.300 mmol) N-[4-(2-Brom-ethoxy)-3-chlor-phenyl]-2-(2-chlor-4-trifluormethyl-phenoxy)-acetamid (Z28b) und 30.6 μL (0.400 mmol) 2,5-Dihydro-1H-pyrrol in 2 mL Hünigbase wurde 3h bei 80 °C gerührt und anschließend i. vac. eingeengt. Säulenchromatographie (Alox, neutral, Akt. II-III, Gradient Dichlormethan / MeOH 20:0 → 19:1) ergab das Produkt.
Ausbeute: 85 mg (60% der Theorie)
$C_{21}H_{19}Cl_2F_3N_2O_3$ (M= 475.298)
Ber. Molpeak $(M+H)^+$: 475/477/479
gef.: Molpeak $(M+H)^+$: 475/477/479($Cl_2$)
$R_f$-Wert: 0.35 (Alox, Dichlormethan / MeOH 19:1).

Beispiel 146:

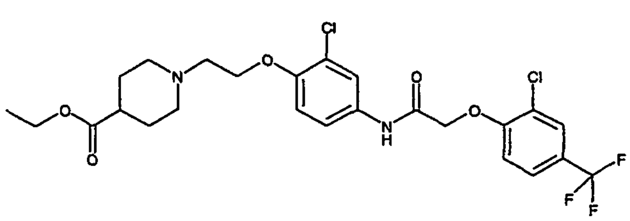

146) 1-(2-{2-Chlor-4-[2-(2-chlor-4-trifluormethyl-phenoxy)-acetylamino]-phenoxy}-ethyl)-piperidin-4-carbonsäure-ethylester

Hergestellt analog zu Beispiel 145 ausgehend von N-[4-(2-Brom-ethoxy)-3-chlorphenyl]-2-(2-chlor-4-trifluormethyl-phenoxy)-acetamid (Z28b) und Piperidin-4-carbonsäure-ethylester.
Ausbeute: 190 mg (82% der Theorie)
$C_{25}H_{27}Cl_2F_3N_2O_5$ (M= 563.406)
Ber. Molpeak $(M+H)^+$: 563/565/567
gef.: Molpeak $(M+H)^+$: 563/565/567 ($Cl_2$)
$R_f$-Wert: 0.52 (Kieselgel, Dichlormethan / MeOH 9:1).

Beispiel 147:

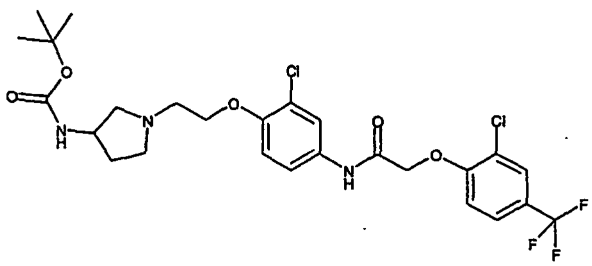

147) [1-(2-{2-Chlor-4-[2-(2-chlor-4-trifluormethyl-phenoxy)-acetylamino]-phenoxy}-ethyl)-pyrrolidin-3-yl]-carbaminsäure-tert-butylester

Hergestellt analog zu Beispiel 145 ausgehend von N-[4-(2-Brom-ethoxy)-3-chlorphenyl]-2-(2-chlor-4-trifluormethyl-phenoxy)-acetamid (Z28b) und Pyrrolidin-3-yl-carbaminsäure-tert-butylester.
Ausbeute: 230 mg (95% der Theorie)
$C_{26}H_{30}Cl_2F_3N_3O_5$ (M= 592.447)
Ber. Molpeak $(M+H)^+$: 592/594/596
gef.: Molpeak $(M+H)^+$: 592/594/596 ($Cl_2$)
$R_f$-Wert: 0.55 (Kieselgel, Dichlormethan / MeOH 19:1).

Beispiel 148:

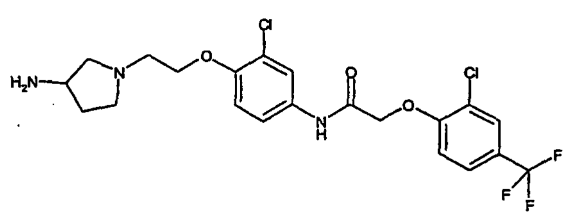

148) N-{4-[2-(3-Amino-pyrrolidin-1-yl)-ethoxy]-3-chlor-phenyl}-2-(2-chlor-4-trifluormethyl-phenoxy)-acetamid

Zu einer Lösung von 0.230 g (0.340 mmol) [1-(2-{2-Chlor-4-[2-(2-chlor-4-trifluormethyl-phenoxy)-acetylamino]-phenoxy}-ethyl)-pyrrolidin-3-yl]-carbaminsäure-tert-butylester (Beispiel 147) in 2 mL Dichlormethan wurde bei RT 0.80 mL (10.380 mmol) TFA zugegeben und das Gemisch 16 h gerührt. Das Reaktionsgemisch wurde i. vac. eingeengt und der Rückstand mit Ether verrieben. Der Niederschlag wurde abfiltriert, mit Ether gewaschen und im HV getrocknet. Das Produkt wurde als Bis-trifluoracetat-Salz erhalten.
Ausbeute: 230 mg (94% der Theorie)

$C_{21}H_{22}Cl_2F_3N_3O_3$ * 2 $C_2HF_3O_2$ (M= 720.378)
Ber. Molpeak $(M+H)^+$: 492/494/496
gef.: Molpeak $(M+H)^+$: 492/494/496 ($Cl_2$)
$R_f$-Wert: 0.45 (Alox, Dichlormethan / MeOH 19:1).

Beispiel 149:

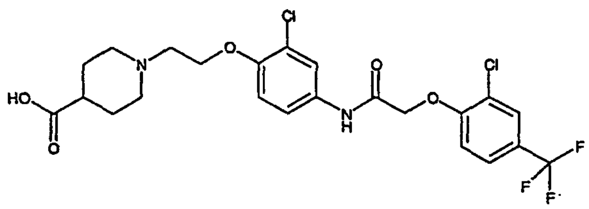

149) 1-(2-{2-Chlor-4-[2-(2-chlor-4-trifluormethyl-phenoxy)-acetylamino]-phenoxy}-ethyl)-piperidin-4-carbonsäure

Zu einer Lösung von 150 mg (0.270 mmol) 1-(2-{2-Chlor-4-[2-(2-chlor-4-trifluormethyl-phenoxy)-acetylamino]-phenoxy}-ethyl)-piperidin-4-carbonsäure-ethylester (Beispiel 146) in 4 mL EtOH wurde bei RT 2 mL wässr. NaOH (1 M) zugegeben und das Gemisch 16 h gerührt. Das Reaktionsgemisch wurde mit 2 mL wässr. HCl (1 M) neutralisiert, i. vac. eingeengt und das Rohprodukt mittels Säulenchromatographie (Kieselgel, Dichlormethan / MeOH 4:1) gereinigt.
Ausbeute: 100 mg (69% der Theorie)
$C_{23}H_{23}Cl_2FsN_2O_5$ (M= 535.351)
Ber. Molpeak $(M+H)^+$: 535/537/539
gef.: Molpeak $(M+H)^+$: 535/537/539 ($Cl_2$)
$R_f$-Wert: 0.35 (Kieselgel, Dichlormethan / MeOH 4:1).

Beispiel 150:

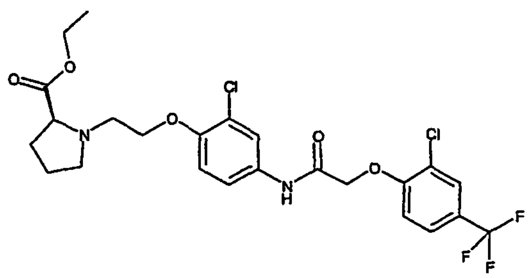

150) (S)-1-(2-{2-Chlor-4-[2-(2-chlor-4-trifluormethyl-phenoxy)-acetylamino]-phenoxy}-ethyl)-pyrrolidin-2-carbonsäure-ethylester

Hergestellt analog zu Beispiel 145 ausgehend von N-[4-(2-Brom-ethoxy)-3-chlorphenyl]-2-(2-chlor-4-trifluormethyl-phenoxy)-acetamid (Z28b) und (S)-Pyrrolidin-2-carbonsäure-ethylester.
Ausbeute: 200 mg (89% der Theorie)
$C_{24}H_{25}Cl_2F_3N_2O_5$ (M= 549.378)
Ber. Molpeak $(M+H)^+$: 549/551/553
gef.: Molpeak $(M+H)^+$: 549/551/553 ($Cl_2$)
$R_f$-Wert: 0.53 (Alox, Dichlormethan / MeOH 49:1).

Beispiel 151:

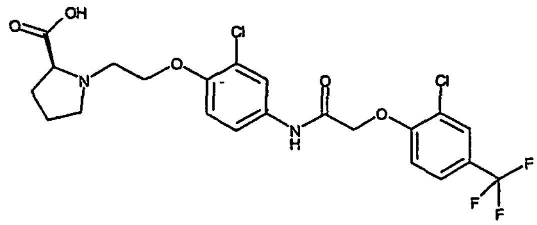

151) (S)-1-(2-{2-Chlor-4-[2-(2-chlor-4-trifluormethyl-phenoxy)-acetylamino]-phenoxy}-ethyl)-pyrrolidin-2-carbonsäure

Hergestellt analog Beispiel 149 ausgehend von (S)-1-(2-{2-Chlor-4-[2-(2-chlor-4-trifluormethyl-phenoxy)-acetylamino]-phenoxy}-ethyl)-pyrrolidin-2-carbonsäure-ethylester (Beispiel 150).
Ausbeute: 114 mg (30% der Theorie)
$C_{22}H_{21}Cl_2F_3N_2O_5$ (M= 521.324)
Ber. Molpeak $(M+H)^+$: 521/523/525
gef.: Molpeak $(M+H)^+$: 521/523/525 ($Cl_2$).

Beispiel 152:

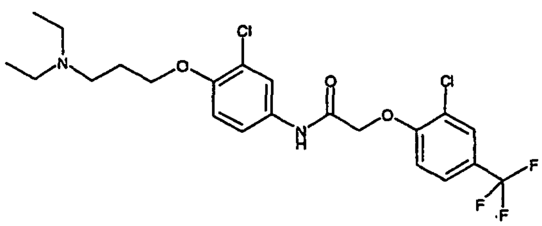

152) N-[3-Chlor-4-(3-diethylamino-propoxy)-phenyl]-2-(2-chlor-4-trifluormethyl-phenoxy)-acetamid

Hergestellt analog Beispiel 145 ausgehend von N-[4-(2-Brom-ethoxy)-3-chlor-phenyl]-2-(2-chlor-4-trifluormethyl-phenoxy)-acetamid (Z28b) und 3-Chlor-4-(3-diethylamino-propoxy)-phenylamin (Z29b).
Ausbeute: 330 mg (68% der Theorie)
$C_{22}H_{25}Cl_2F_3N_2O_3$ (M= 493.357)
Ber. Molpeak $(M+H)^+$: 493/495/497
gef.: Molpeak $(M+H)^+$: 493/495/497 ($Cl_2$).
$R_f$-Wert: 0.30 (Kieselgel, Dichlormethan / MeOH / konz. wässr. Ammoniak 90:10:0.1).

Beispiel 153:

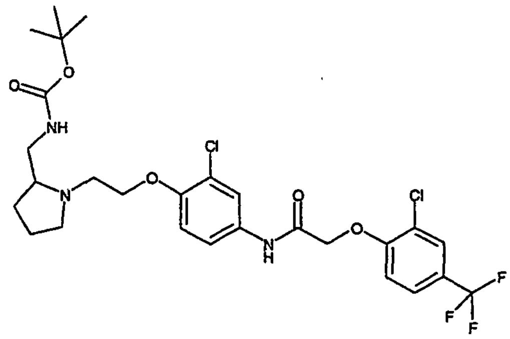

153) [1-(2-{2-Chlor-4-[2-(2-chlor-4-trifluormethyl-phenoxy)-acetylamino]-phenoxy}-ethyl)-pyrrolidin-2-ylmethyl]-carbaminsäure-tert-butyl-ester

Hergestellt analog Beispiel 143 ausgehend von N-[4-(2-Brom-ethoxy)-3-chlor-phenyl]-2-(2-chlor-4-trifluormethyl-phenoxy)-acetamid (Z28b) und Pyrrolidin-2-ylmethyl-carbaminsäure-tert-butylester.
Ausbeute: 280 mg (quant. Ausbeute)
$C_{27}H_{32}Cl_2F_3N_3O_5$ (M= 606.474)
Ber. Molpeak $(M-H)^-$: 604/606/608
gef.: Molpeak $(M-H)^-$: 604/606/608 ($Cl_2$).
$R_f$-Wert: 0.85 (Alox, Dichlormethan / MeOH 19:1).

Beispiel 154:

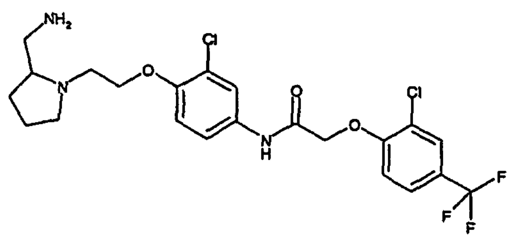

154) N-{4-[2-(2-Aminomethyl-pyrrolidin-1-yl)-ethoxy]-3-chlor-phenyl}-2-(2-chlor-4-trifluormethyl-phenoxy)-acetamid

Hergestellt analog Beispiel 148 ausgehend von [1-(2-{2-Chlor-4-[2-(2-chlor-4-trifluormethyl-phenoxy)-acetylamino]-phenoxy}-ethyl)-pyrrolidin-2-ylmethyl]-carbaminsäure-tert-butyl-ester (Beispiel 153).
Ausbeute: 280 mg (quant. Ausbeute)
$C_{22}H_{24}Cl_2F_3N_3O3 * C_2HF_3O_2$ (M= 734.405)
Ber. Molpeak $(M-H)^-$: 504/506/508
gef.: Molpeak (M-H)-: 504/506/508 ($Cl_2$).
$R_f$-Wert: 0.14 (Alox, Dichlormethan / MeOH 19:1).

Beispiel 155:

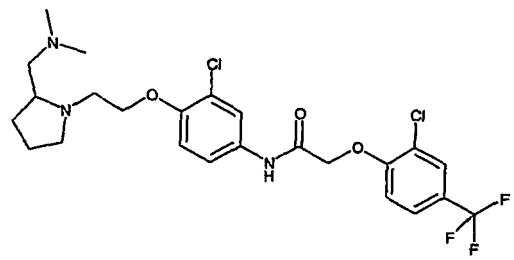

155)N-{3-Chlor-4-[2-(2-dimethylaminomethyl-pyrrolidin-1-yl)-ethoxy]-phenyl}-2-(2-chlor-4-trifluormethyl-phenoxy)-acetamid

Zu einer Lösung von 250 mg (0.340 mmol) N-{4-[2-(2-Aminomethyl-pyrrolidin-1-yl)-ethoxy]-3-chlor-phenyl}-2-(2-chlor-4-trifluormethyl-phenoxy)-acetamid (Beispiel 154) in 7 mL MeOH wurde 0.46 mL (6.080 mmol) Formaldehyd (37% in Wasser) zugegeben und das Gemisch 1 h bei RT gerührt. 103 mg (2.720 mmol) Natriumborhydrid wurde portionenweise zugegeben und weitere 16 h bei RT gerührt. Das Reaktionsgemisch wurde i. vac. eingeengt und der Rückstand in Dichlormethan aufgenommen. Die org. Phase wurde mit ges. wässr. Natriumbicarbonat gewaschen, über Natriumsulfat getrocknet und i. vac. eingeengt. Säulenchromatographie (Kieselgel, Dichlormethan / MeOH 49:1) ergab das Produkt.
Ausbeute: 170 mg (94% der Theorie)
$C_{24}H_{28}Cl_2F_3N_3O_3$ (M= 534.410)
Ber. Molpeak $(M+H)^+$: 534/536/538
gef.: Molpeak $(M+H)^+$: 534/536/538 ($Cl_2$).
$R_f$-Wert: 0.58 (Alox, Dichlormethan / MeOH 19:1).

Beispiel 156:

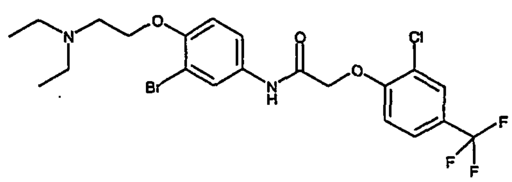

156) N-[3-Brom-4-(2-diethylamino-ethoxy)-phenyl]-2-(2-chlor-4-trifluormethyl-phenoxy)-acetamid

Hergestellt analog Beispiel 143 ausgehend von (2-Chlor-4-trifluormethyl-phenoxy)-essigsäure (Z2b) und 3-Brom-4-(2-diethylamino-ethoxy)-phenylamin (Z30b).
Ausbeute: 0.310 g (60% der Theorie)
$C_{21}H_{23}BrClF_3N_2O_3$ (M= 523.781)
Ber. Molpeak $(M+H)^+$: 523/525/527
gef.: Molpeak $(M+H)^+$: 523/525/527 (BrCl).
$R_f$-Wert: 0.64 (Alox, Dichlormethan / MeOH 39:1).

Beispiel 157:

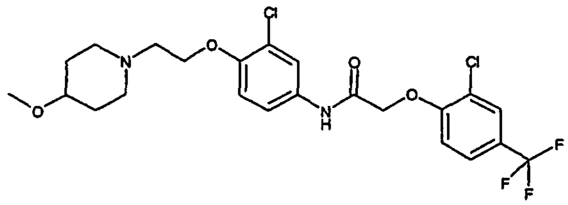

157) N-{3-Chlor-4-[2-(4-methoxy-piperidin-1-yl)-ethoxy]-phenyl}-2-(2-chlor-4-trifluormethyl-phenoxy)-acetamid

Hergestellt analog Beispiel 145 ausgehend von N-[4-(2-Brom-ethoxy)-3-chlor-phenyl]-2-(2-chlor-4-trifluormethyl-phenoxy)-acetamid (Z28b) und 4-Methoxy-piperidin. Ausbeute: 0.200 g (94% der Theorie)
$C_{23}H_{25}Cl_2F_3N_2O_4$ (M= 521.368)
Ber. Molpeak $(M+H)^+$: 521/523/525
gef.: Molpeak $(M+H)^+$: 521/523/525 ($Cl_2$).
$R_f$-Wert: 0.75 (Alox, Dichlormethan / MeOH 49:1).

Beispiel 158:

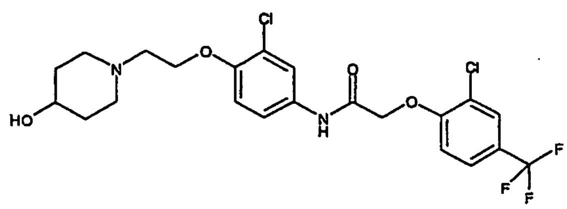

158) N-{3-Chlor-4-[2-(4-hydroxy-piperidin-1-yl)-ethoxy]-phenyl}-2-(2-chlor-4-trifluormethyl-phenoxy)-acetamid

Hergestellt analog Beispiel 145 ausgehend von N-[4-(2-Brom-ethoxy)-3-chlor-phenyl]-2-(2-chlor-4-trifluormethyl-phenoxy)-acetamid (Z28b) und 4-Hydroxy-piperidin. Ausbeute: 0.190 g (91% der Theorie)
$C_{22}H_{23}Cl_2F_3N_2O_4$ (M= 507.341)
Ber. Molpeak $(M+H)^+$: 507/509/511
gef.: Molpeak $(M+H)^+$: 507/509/511 ($Cl_2$).
$R_f$-Wert: 0.55 (Alox, Dichlormethan / MeOH 19:1).

Beispiel 159:

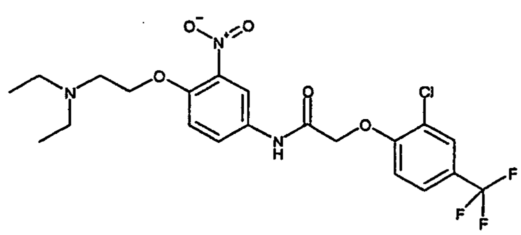

159) 2-(2-Chlor-4-trifluormethyl-phenoxy)-N-[4-(2-diethylamino-ethoxy)-3-nitro-phenyl]-acetamid

Hergestellt analog zu Beispiel 143 ausgehend von (2-Chlor-4-trifluormethyl-phenoxy)-essigsäure (Z2b) und 4-(2-Diethylamino-ethoxy)-3-nitro-phenylamin (Z31a).
Ausbeute: 0.410 g (45% der Theorie)
$C_{21}H_{23}ClF_3N_3O_5$ (M= 489.883)

Ber. Molpeak $(M+H)^+$: 490/492
gef.: Molpeak $(M+H)^+$: 490/492 (Cl).
$R_f$-Wert: 0.46 (Kieselgel, Dichlormethan / MeOH 9:1).

Beispiel 160:

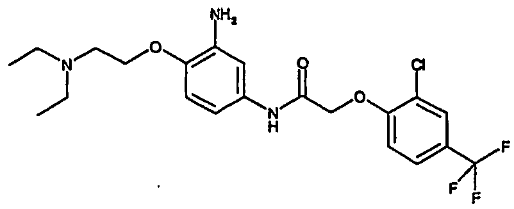

160) N-[3-Amino-4-(2-diethylamino-ethoxy)-phenyl]-2-(2-chlor-4-trifluormethyl-phenoxy)-acetamid

Eine Suspension von 330 mg (0.674 mmol) 2-(2-Chlor-4-trifluormethyl-phenoxy)-N-[4-(2-diethylamino-ethoxy)-3-nitro-phenyl]-acetamid (Beispiel 160) und 200 mg Raney-Nickel wurde bei RT und 3 bar hydriert. Der Katalysator wurde abfiltriert und das Filtrat i. vac. eingeengt.
Ausbeute: 0.310 g (quant. Ausbeute)
$C_{21}H_{25}ClF_3N_3O_3$ (M= 459.900)
Ber. Molpeak $(M+H)^+$: 460/462
gef.: Molpeak $(M+H)^+$: 460/462 (Cl).
$R_f$-Wert: 0.45 (Kieselgel, Dichlormethan / MeOH 9:1).

Beispiel 161:

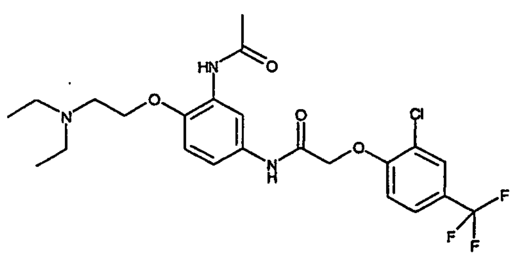

161) N-[3-Acetylamino-4-(2-diethylamino-ethoxy)-phenyl]-2-(2-chlor-4-trifluormethyl-phenoxy)-acetamid

Eine Lösung von 100 mg (0.217 mmol) N-[3-Amino-4-(2-diethylamino-ethoxy)-phenyl]-2-(2-chlor-4-trifluormethyl-phenoxy)-acetamid (Beispiel 160) in 10 mL Essigsäure-anhydrid wurde 3 h bei 100 °C gerührt und anschließend i. vac. eingeengt. Der Rückstand wurde mit ges. wässr. Natriumbicarbonat versetzt und mit EtOAc erschöpfend extrahiert. Die vereinigten org. Phasen wurde über Natriumsulfat getrocknet, i. vac. eingeengt und der Rückstand mit Ether verrührt. Der Niederschlag wurde abfiltriert, mit Ether gewaschen und im HV getrocknet.
Ausbeute: 0.108 g (quant. Ausbeute)
$C_{23}H_{27}ClF_3N_3O_4$ (M= 501.938)
Ber. Molpeak $(M+H)^+$: 502/504
gef.: Molpeak $(M+H)^+$: 502/504 (Cl).
$R_f$-Wert: 0.42 (Kieselgel, Dichlormethan / MeOH 9:1).

Beispiel 162:

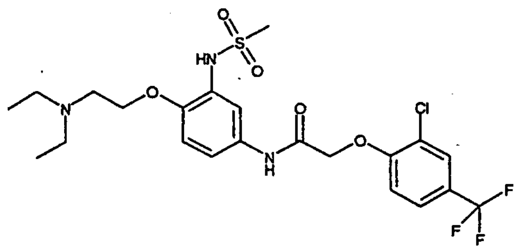

162) 2-(2-Chlor-4-trifluormethyl-phenoxy)-N-[4-(2-diethylamino-ethoxy)-3-methansulfon-ylamino-phenyl]-acetamid

Eine Lösung von 100 mg (0.217 mmol) N-[3-Amino-4-(2-diethylamino-ethoxy)-phenyl]-2-(2-chlor-4-trifluormethyl-phenoxy)-acetamid (Beispiel 160) in 5 mL Pyridin wurde bei 0 ˚C 18 μL (0.239 mmol) Methansulfonylchlorid zugegeben, das Gemisch langsam auf RT erwärmt und bei RT 3 h gerührt. Das Reaktionsgemisch wurde auf Eiswasser gegossen und die wässr. Phase mit EtOAc erschöpfend extrahiert. Die vereinigten org. Phasen wurden über Natriumsulfat getrocknet und i. vac. eingeengt. Der Rückstand wurde lyophilisiert.
Ausbeute: 0.080 g (87% der Theorie)
$C_{22}H_{27}ClF_3N_3O_5S$ (M= 537.990)
Ber. Molpeak $(M+H)^+$: 538/540
gef.: Molpeak $(M+H)^+$: 538/540 (Cl).
$R_f$-Wert: 0.40 (Kieselgel, Dichlormethan / MeOH 9:1).

Beispiel 163:

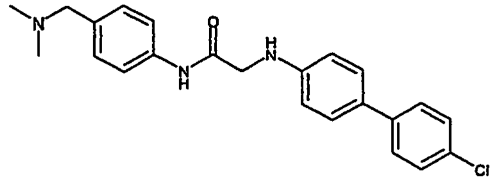

163a) 2-(4-Brom-phenylamino)-N-(4-dimethylaminomethyl-phenyl)-acetamid

Das Produkt wurde gemäß der allgemeinen Arbeitsvorschrift I aus (4-Bromo-phenylamino)-essigsäure und 4-Dimethylaminomethyl-phenylamin hergestellt. Ausbeute: 1.340 g (28% der Theorie)
$C_{17}H_{20}BrN_3O$ (M= 362.272)
Ber. Molpeak $(M+H)^+$: 362/364
gef.: Molpeak $(M+H)^+$: 362/364 (Cl).
$R_f$-Wert: 0.68 (Alox, Dichlormethan / MeOH 19:1).

163b) 2-(4'-Chlor-biphenyl-4-ylamino)-N-(4-dimethylaminomethyl-phenyl)-acetamid

Hergestellt analog zu Beispiel 130a ausgehend von 2-(4-Brom-phenylamino)-N-(4-dimethylaminomethyl-phenyl)-acetamid (Beispiel 163a) und 4-Chlor-phenylboronsäure.
Ausbeute: 0.160 g (41 % der Theorie)
$C_{23}H_{24}ClN_3O$ (M= 393.920)
Ber. Molpeak $(M+H)^+$: 394/396
gef.: Molpeak $(M+H)^+$: 394/396 (Cl).
$R_f$-Wert: 0.48 (Kieselgel, Dichlormethan / MeOH / konz. wässr. Ammoniak 90:10:1).

Beispiel 164:

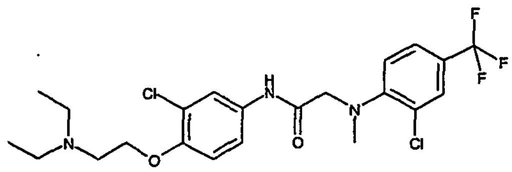

164a) (2-Chlor-4-trifluormethyl-phenyl)-methyl-amin

Zu einer Lösung von 2.100 g (10.738 mmol) 2-Chlor-4-trifluormethoxy-phenylamin in 10 mL DMF wurde unter Stickstoff-Atmosphäre 7.04 mL (59.059 mmol) N,N-Dimethylformamid-dimethylacetal zugegeben und das Gemisch 5 h bei 60 ˚C und 16 h bei RT gerührt. 1.421 g (37.583 mmol) Natriumborhydrid wurde zugegeben und das Gemisch weiter 3 h bei 60 ˚C gerührt. Ges. wässr. Natriumbicarbonat wurde zugegeben und das Gemisch mit EtOAc extrahiert. Die org. Phase wurde über Natriumsulfat getrocknet und i. vac. bei 30 ˚C eingeengt. Säulenchromatographie (Kieselgel, Petrolether) ergab das Produkt.
Ausbeute: 1.100 g (49% der Theorie)
$C_8H_7ClF_3N$ (M= 209.600)
Ber. Molpeak $(M+H)^+$: 210/212
gef.: Molpeak $(M+H)^+$: 210/212 (Cl).
$R_f$-Wert: 0.75 (Kieselgel, Petrolether / EtOAc 9:1).

164b) N-[3-Chlor-4-(2-diethylamino-ethoxy)-phenyl]-2-[(2-chlor-4-trifluormethyl-phenyl)-methyl-amino]-acetamid

Eine Lösung von 0.200 g (0.450 mmol) 2-Brom-*N*-[3-chlor-4-(2-diethylamino-ethoxy)-phenyl]-acetamid-hydrobromid und 0.141 g (0.675 mmol) (2-Chlor-4-trifluormethylphenyl)-methyl-amin (Beispiel 164a) in 2 mL DMF wurde 2 h in der Mikrowelle auf 100 ˚C erhitzt. Das Reaktionsgemisch wurde i. vac. eingeengt, mit 3 mL DMF verdünnt und mittels HPLC-MS (Stable Bond C18; 3.5 μm; Wasser:Acetonitril:Ameisensäure 9:1:0.01 → 1:9:0.01 über 9 min) gereinigt. Das Produkt wurde als Formiat-Salz erhalten.
Ausbeute: 36 mg (15% der Theorie)
$C_{22}H_{26}Cl_2F_3N_3O_2 * CH_2O_2$ (M= 538.399)
Ber. Molpeak $(M+H)^+$: 492/494/496
gef.: Molpeak $(M+H)^+$: 492/494/496 ($Cl_2$).
$R_f$-Wert: 0.69 (Kieselgel, EtOAc / MeOH / konz. wässr. Ammoniak 90:10:1).

Beispiel 165:

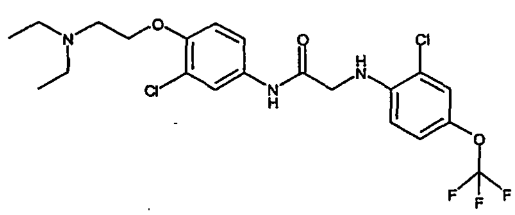

165a) (2-Chlor-4-trifluormethoxy-phenyl-amino)-essigsäure-ethylester

Zu einer Lösung von 0.95 g (4.266 mmol) (2-Chlor-4-trifluormethyl-phenyl)-methylamin in 10 mL Hünigbase wurde bei RT 0.566 mL (5.000 mmol) Bromessigsäure-ethylester zugegeben und das Gemisch 4 h unter Rückfluss erhitzt. Wasser wurde zugegeben und die wässr. Phase mit EtOAc erschöpfend extrahiert. Die vereinigten org. Phasen wurden mit Wasser, ges. wässr. Natriumbicarbonat und ges. wässr. NaCl gewaschen, über Natriumsulfat getrocknet und i. vac. eingeengt. Das Rohprodukt wurde ohne weitere Reinigung in den nachfolgenden Reaktionsschritt eingesetzt.
Ausbeute: 1.200 g (57% der Theorie)
$C_{11}H_{11}ClF_3NO_3$ (M= 297.663)
$R_f$-Wert: 0.68 (Kieselgel, Petrolether / EtOAc 3:1).

165b) (2-Chlor-4-trifluormethoxy-phenyl-amino)-essigsäure

Hergestellt analog Zwischenprodukt Z2b ausgehend von [(2-Chlor-4-trifluormethylphenyl)-methyl-amino]-essigsäure-ethylester (Beispiel 165a).
Ausbeute: 0.630 g (97% der Theorie)
$C_9H_7ClF_3NO_3$ (M= 209.600)
Ber. Molpeak $(M+H)^+$: 210/212
gef.: Molpeak $(M+H)^+$: 210/212 (Cl).
$R_f$-Wert: 0.75 (Kieselgel, Petrolether / EtOAc 9:1).

165c) N-[3-Chlor-4-(2-diethylamino-ethoxy)-phenyl]-2-(2-chlor-4-trifluormethoxy-phenylamino)-acetamid

Das Produkt wurde gemäß der allgemeinen Arbeitsvorschrift I aus (2-Chlor-4-trifluormethoxy-phenyl-amino)-essigsäure (Beispiel 165b) und 3-Chlor-4-(2-diethylamino-ethoxy)-phenylamin (Z1b) hergestellt.
Ausbeute: 0.220 g (44% der Theorie)
$C_{21}H_{24}C_{12}F_3N_3O_3$ (M= 494.345)
Ber. Molpeak $(M-H)^-$: 492/494/496
gef.: Molpeak $(M-H)^-$: 492/494/496 (Cl).
$R_f$-Wert: 0.48 (Alox, Dichlormethan / MeOH 30:1).

Beispiel 166:

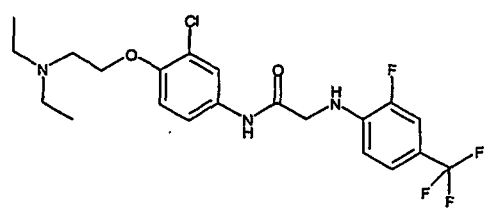

166a) (2-Fluor-4-trifluormethyl-phenylamino)-essigsäure-ethylester

Hergestellt analog Beispiel 165a ausgehend von 2-Fluor-4-trifluormethyl-phenylamin und Bromessigsäureethylester. Das Rohprodukt wurde mittels Säulenchromatographie (Kieselgel, Gradient Dichlormethan / MeOH 20:0 → 19:1) gereinigt.
Ausbeute: 0.980 g (82% der Theorie)
$C_{11}H_{11}F_4NO_2$ (M= 265.209)
Ber. Molpeak $(M+H)^+$: 266
gef.: Molpeak $(M+H)^+$: 266
$R_f$-Wert: 0.72 (Kieselgel, Petrolether / EtOAc 3:1).

166b) (2-Fluor-4-trifluormethyl-phenylamino)-essigsäure

Hergestellt analog Zwischenprodukt Z2b ausgehend von (2-Fluor-4-trifluormethyl-phenylamino)-essigsäureethylester (Beispiel 166a). Das Rohprodukt wurde mittels Säulenchromatographie (Kieselgel, Gradient Dichlormethan / MeOH 20:0 → 19:1) gereinigt.
Ausbeute: 0.670 g (79% der Theorie)
$C_9H_7F_4NO_2$ (M= 237.155)
Ber. Molpeak $(M-H)^-$: 236
gef.: Molpeak $(M-H)^-$: 236.

166c) N-[3-Chlor-4-(2-diethylamino-ethoxy)-phenyl]-2-(2-fluor-4-trifluormethyl-phenylamino)-acetamid

Das Produkt wurde gemäß der allgemeinen Arbeitsvorschrift I ausgehend von (2-Fluor-4-trifluormethyl-phenylamino)-essigsäure (Beispiel 166b) und 3-Chlor-4-(2-diethylamino-ethoxy)-phenylamin (Z1b) erhalten.

Ausbeute: 0.150 g (32% der Theorie)
$C_{21}H_{24}ClF_4N_3O_2$ (M= 461.891)
Ber. Molpeak $(M+H)^+$: 462/464
gef.: Molpeak $(M+H)^+$: 462/464 (Cl)

Beispiel 167:

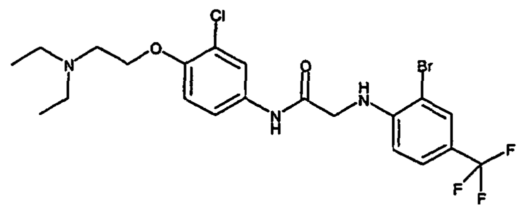

167a) (2-Brom-4-trifluormethyl-phenylamino)-essigsäure-ethylester

Hergestellt analog Beispiel 166a ausgehend von 2-Brom-4-trifluormethyl-phenylamin und Bromessigsäure-ethylester.
Ausbeute: 1.200 g (36% der Theorie)
$C_{11}H_{11}BrF_3NO_2$ (M= 326.115)
$R_f$-Wert: 0.72 (Kieselgel, Petrolether / EtOAc 3:1).

167b) (2-Brom-4-trifluormethyl-phenylamino)-essigsäure

Hergestellt analog Zwischenprodukt Z2b ausgehend von (2-Brom-4-trifluormethyl-phenylamino)-essigsäure-ethylester (Beispiel 167a).
Ausbeute: 0.438 g (quant. Ausbeute)
$C_9H_7BrF_3NO_2$ (M= 298.061)
Ber. Molpeak $(M-H)^-$: 296/298
gef.: Molpeak $(M-H)^-$: 296/298.

167c) 2-(2-Brom-4-trifluormethyl-phenylamino)-N-[3-chlor-4-(2-diethylamino-ethoxy)-phenyl]-acetamid

Das Produkt wurde gemäß der allgemeinen Arbeitsvorschrift I ausgehend von (2-Brom-4-trifluormethyl-phenylamino)-essigsäure (Beispiel 167b) und 3-Chlor-4-(2-diethylamino-ethoxy)-phenylamin (Z1b) erhalten.
Ausbeute: 0.300 g (84% der Theorie)
$C_{21}H_{24}BrClF_3N_3O_2$ (M= 522.797)
Ber. Molpeak $(M+H)^+$: 522/524/526
gef.: Molpeak $(M+H)^+$: 522/524/526 (BrCl)
$R_f$-Wert: 0.45 (Kieselgel, Dichlormethan / MeOH 9:1).

Beispiel 168:

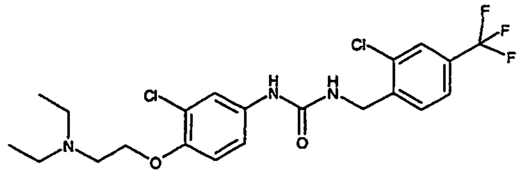

168a) 2-Chlor-4-trifluormethyl-benzylamin

Eine Suspension von 1.000 g (4.865 mmol) 2-Chlor-4-trifluormethyl-benzonitril und 100 mg Raney-Nickel in konz. methanolischem Ammoniak wurde 20 h bei RT und 3 bar hydriert. Der Katalysator wurde abfiltriert und das Filtrat i. vac. eingeengt.
Ausbeute: 0.870 g (85% der Theorie)
$C_8H_7ClF_3N$ (M= 209.600)
Ber. Molpeak $(M+H)^+$: 210/212
gef.: Molpeak $(M+H)^+$: 210/212 (Cl)
$R_f$-Wert: 0.48 (Kieselgel, Petrolether / EtOAc 2:1).

168b) 1-[3-Chlor-4-(2-diethylamino-ethoxy)-phenyl]-3-(2-chlor-4-trifluormethyl-benzyl)-harnstoff

Zu einer Lösung von 0.870 g (4.151 mmol) 2-Chlor-4-trifluormethyl-benzylamin (Beispiel 168a) in 50 mL abs. THF wurde bei 0 ˚C 0.770 g (4.457 mmol) CDT zugegeben und das Gemisch 1 h bei RT gerührt. 1.080 g (4.449 mmol) 3-Chlor-4-(2-diethylamino-ethoxy)-phenylamin (Z1b) wurde zugegeben und das Gemisch 5 h unter Rückfluss erhitzt. Das Reaktionsgemisch wurde i. vac. eingeengt und der Rückstand in Dichlormethan aufgenommen. Die org. Phase wurde mit 15% wässr. Kaliumcarbonat gewaschen, über Natriumsulfat getrocknet und i. vac. eingeengt. Das Rohprodukt wurde mit Diisopropylether verrieben, der Niederschlag abfiltriert, mit Diisopropylether gewaschen und im HV getrocknet.
Ausbeute: 0.570 g (29% der Theorie)
$C_{21}H_{24}Cl_2F_3N_3O_2$ (M= 478.346)
Ber. Molpeak $(M+H)^+$: 478/480/482
gef.: Molpeak $(M+H)^+$: 478/480/482 ($Cl_2$)
$R_f$-Wert: 0.36 (Kieselgel, Dichlormethan / MeOH / Cyclohexan / konz. wässr. Ammoniak 70:15:15:2).

Beispiel 169:

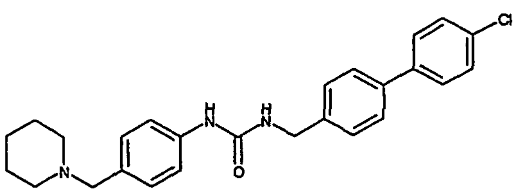

169) 1-(4'-Chlor-biphenyl-4-ylmethyl)-3-(4-piperidin-1-ylmethyl-phenyl)-harnstoff

Hergestellt analog Beispiel 168b ausgehend von C-(4'-Chlor-biphenyl-4-yl)-methylamin und 4-Piperidin-1-ylmethyl-phenylamin. Das Rohprodukt wurde mittels Säulenchromatographie (Alox, neutral, Akt. II-III, Dichlormethan / MeOH 98:2) gereinigt.
Ausbeute: 0.390 g (90% der Theorie)
$C_{26}H_{28}ClN_3O$ (M= 433.986)
Ber. Molpeak $(M+H)^+$: 434/436
gef.: Molpeak $(M+H)^+$: 434/436 (Cl)
$R_f$-Wert: 0.42 (Alox, Dichlormethan / MeOH 39:1).

Beispiel 170:

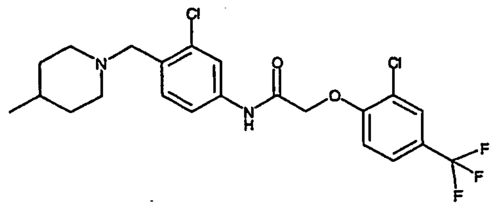

170) N-[3-Chlor-4-(4-methyl-piperidin-1-ylmethyl)-phenyl]-2-(2-chlor-4-trifluormethyl-phenoxy)-acetamid

Zu einer Lösung von 0.206 g (0.500 mmol) N-(3-Chlor-4-chlormethyl-phenyl)-2-(2-chlor-4-trifluormethyl-phenoxy)-acetamid (Z32b) in 5 mL abs. THF wurde 0.24 mL (2.000 mmol) 4-Methylpiperidin zugegeben und das Gemisch 2 h unter Rückfluss erhitzt. Das Reaktionsgemisch wurde auf Eiswasser gegossen, der Niederschlag abfiltriert, mit Wasser gewaschen und im HV getrocknet.
Ausbeute: 0.214 g (90% der Theorie)
$C_{22}H_{23}Cl_2F_3N_2O_2$ (M= 475.342)
Ber. Molpeak $(M+H)^+$: 475/477/479
gef.: Molpeak $(M+H)^+$: 475/477/479 ($Cl_2$)
$R_f$-Wert: 0.66 (Alox, Petrolether / EtOAc 3:1).

Beispiel 171:

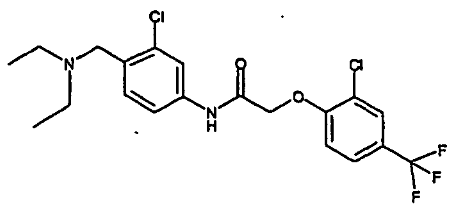

171) N-(3-Chlor-4-diethylaminomethyl-phenyl)-2-(2-chlor-4-trifluormethyl-phenoxy)-acetamid

Hergestellt analog Beispiel 170 ausgehend von N-(3-Chlor-4-chlormethyl-phenyl)-2-(2-chlor-4-trifluormethyl-phenoxy)-acetamid (Z32b) und Diethylamin. Das Rohprodukt wurde aus Petrolether umkristallisiert.
Ausbeute: 0.154 g (69% der Theorie)
$C_{20}H_{21}Cl_2F_3N_2O_2$ (M= 449.304)
Ber. Molpeak $(M+H)^+$: 449/451/453
gef.: Molpeak $(M+H)^+$: 449/451/453 ($Cl_2$)
$R_f$-Wert: 0.53 (Alox, Petrolether / EtOAc 3:1).

Beispiel 172:

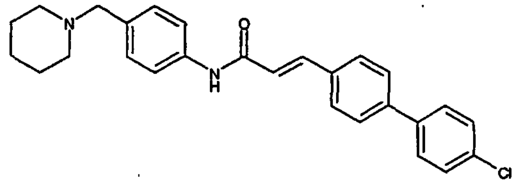

172) (E)-3-(4'-Chlor-biphenyl-4-yl)-N-(4-piperidin-1-ylmethyl-phenyl)-acrylamid

Eine Lösung von 0.544 g (2.103 mmol) (E)-3-(4'-Chloro-biphenyl-4-yl)-acrylsäure, 0.440 g (2.313 mmol) 4-Piperidin-1-ylmethyl-phenylamin, 0.736 g (2.313 mmol) TBTU, 0.313 g (2.313 mmol) HOBt und 1.025 mL (7.361 mmol) Triethylamin in 10 mL DMF wurde 3 h bei RT gerührt und dann auf Eiswasser und wenig EtOAc gegossen. Der Niederschlag wurde abfiltriert, mit Wasser gewaschen und i. vac. bei 80 °C getrocknet. Ausbeute: 0.154 g (69% der Theorie)
$C_{27}H_{27}ClN_2O$ (M= 430.982)
Ber. Molpeak $(M+H)^+$: 431/433
gef.: Molpeak $(M+H)^+$: 431/433 (Cl)
$R_f$-Wert: 0.31 (Kieselgel, Dichlormethan / MeOH 9:1).

Beispiel 173:

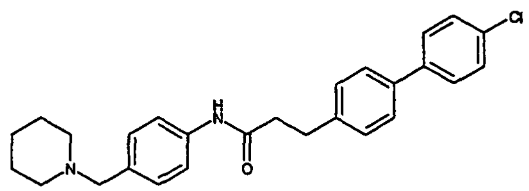

173) 3-(4'-Chlor-biphenyl-4-yl)-N-(4-piperidin-1-ylmethyl-phenyl)-propionamid

Eine Suspension von 0.200 g (0.464 mmol) (E)-3-(4'-Chlor-biphenyl-4-yl)-N-(4-piperidin-1-ylmethyl-phenyl)-acrylamid (Beispiel 172) und 100 mg Raney-Nickel in 50 mL EtOAc wurde bei RT und 50 psi hydriert. Der Katalysator wurde abfiltriert, das Filtrat i. vac. eingeengt und der Rückstand mit Ether verrieben. Der Niederschlag wurde abfiltriert, mit Ether gewaschen und bei 50 °C i. vac. getrocknet.
Ausbeute: 55 mg (27% der Theorie)
$C_{27}H_{29}ClN_2O$ (M= 432.998)
Ber. Molpeak $(M+H)^+$: 433/435
gef.: Molpeak $(M+H)^+$: 433/435 (Cl)
$R_f$-Wert: 0.23 (Kieselgel, Dichlormethan / MeOH 9:1).

Beispiel 174:

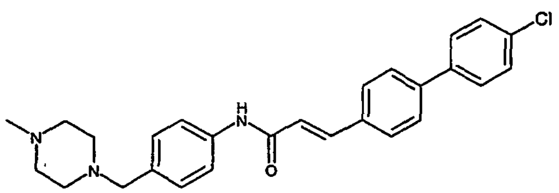

174) (E)-3-(4'-Chlor-biphenyl-4-yl)-N-[4-(4-methyl-piperazin-1-ylmethyl)-phenyl]-acrylamid

Hergestellt analog Beispiel 172 ausgehend von (E)-3-(4'-Chloro-biphenyl-4-yl)-acrylsäure und 4-(4-Methyl-piperazin-1-ylmethyl)-phenylamin. Das Rohprodukt wurde mittels Säulenchromatographie (Kieselgel, Dichlormethan / MeOH / konz. wässr. Ammoniak 90:10:0.1) gereinigt. Ausbeute: 0.410 g (61% der Theorie)
$C_{27}H_{28}ClN_3O$ (M= 445.997)
Ber. Molpeak $(M+H)^+$: 446/448
gef.: Molpeak $(M+H)^+$: 446/448 (Cl)
$R_f$-Wert: 0.40 (Kieselgel, Dichlormethan / MeOH 9:1).

Beispiel 175:

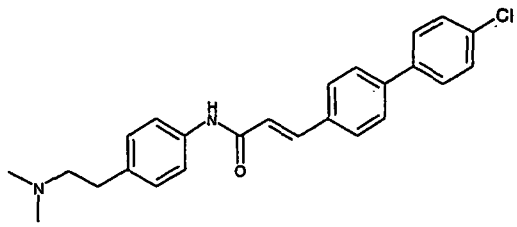

175) (E)-3-(4'-Chlor-biphenyl-4-yl)-N-[4-(2-dimethylamino-ethyl)-phenyl]-acrylamid

Hergestellt analog Beispiel 172 ausgehend von (E)-3-(4'-Chloro-biphenyl-4-yl)-acrylsäure und 4-(2-Dimethylamino-ethyl)-phenylamin.
Ausbeute: 0.350 g (58% der Theorie)
$C_{25}H_{25}ClN_2O$ (M= 404.944)
Ber. Molpeak $(M+H)^+$: 405/407
gef.: Molpeak $(M+H)^+$: 405/407 (Cl)
$R_f$-Wert: 0.40 (Kieselgel, Dichlormethan / MeOH / konz. wässr. Ammoniak 90:10:0.1).

Beispiel 176:

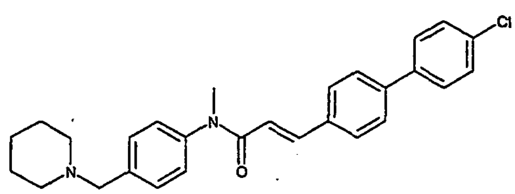

176) (E)-3-(4'-Chlor-biphenyl-4-yl)-N-methyl-N-(4-piperidin-1-ylmethyl-phenyl)-acrylamid

Hergestellt analog Beispiel 172 ausgehend von (E)-3-(4'-Chloro-biphenyl-4-yl)-acrylsäure und Methyl-(4-piperidin-1-ylmethyl-phenyl)-amin.
Ausbeute: 0.200 g (45% der Theorie)
$C_{28}H_{29}ClN_2O$ (M= 445.009)
Ber. Molpeak $(M+H)^+$: 445/447
gef.: Molpeak $(M+H)^+$: 445/447 (Cl)
$R_f$-Wert: 0.60 (Kieselgel, Dichlormethan / MeOH / konz. wässr. Ammoniak 90:10:0.1).

Beispiel 177:

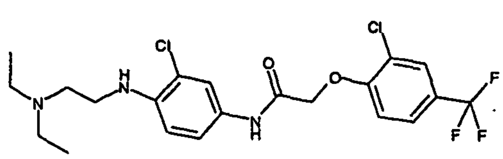

177) N-[3-Chlor-4-(2-diethylamino-ethylamino)-phenyl]-2-(2-chlor-4-trifluormethyl-phenoxy)-acetamid

Das Produkt wurde gemäß der allgemeinen Arbeitsvorschrift I ausgehend von 0.270 g (1.060 mmol) (2-Chloro-4-trifluormethyl-phenoxy)-essigsäure (Z2b) und 0.260 g (1.080 mmol) 2-Chlor-*N'*-(2-diethylamino-ethyl)-benzol-1,4-diamin (Z42b) hergestellt.
Ausbeute: 0.340 g (67% der Theorie)
$C_{21}H_{24}Cl_2N_3O_2$ (M= 478.346)
Ber. Molpeak $(M+H)^+$: 478/480/482
gef.: Molpeak $(M+H)^+$: 478/480/482 ($Cl_2$)
$R_f$-Wert: 0.45 (Alox, Dichlormethan / MeOH 45:1).

Beispiel 178:

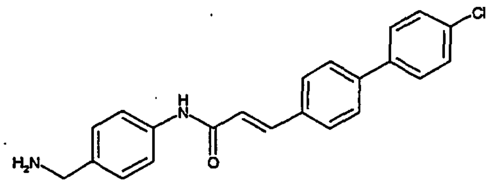

178a) {4-[(E)-3-(4'-Chlor-biphenyl-4-yl)-acryloylamino]-benzyl}-carbaminsäure-tert-butylester

Hergestellt analog Beispiel 172 ausgehend von (E)-3-(4'-Chloro-biphenyl-4-yl)-acrylsäure und (4-Amino-benzyl)-carbaminsäure-tert-butylester. Das Rohprodukt wurde mit MeOH verrührt, der Niederschlag abfiltriert und im HV getrocknet. Ausbeute: 1.000 g (72% der Theorie)
$C_{27}H_{27}ClN_2O_3$ (M= 462.981)
Ber. Molpeak $(M+H)^+$: 463/465
gef.: Molpeak $(M+H)^+$: 463/465 (Cl)
$R_f$-Wert: 0.70 (Kieselgel, Dichlormethan / MeOH 9:1).

178b) (E)-N-(4-Aminomethyl-phenyl)-3-(4'-chlor-biphenyl-4-yl)-acrylamid

Zu einer Suspension von 0.950 g (2.050 mmol) {4-[(E)-3-(4'-Chlor-biphenyl-4-yl)-acryloylamino]-benzyl}-carbaminsäure-tert-butylester in 50 mL abs. Dichlormethan wurde bei RT 5 mL TFA zugegeben und das Gemisch 2 h bei RT gerührt. Das Reaktionsgemisch wurde i. vac. eingeengt, mit Toluol versetzt und erneut i. vac. eingeengt. Der Rückstand wurde mit Ether verrieben, der Niederschlag abfiltriert und i. vac. bei 80 °C getrocknet. Das Produkt wurde als Trifluoracetat-Salz erhalten.
Ausbeute: 0.930 g (95% der Theorie)
$C_{22}H_{19}ClN_2O * C_2HF_3O_2$ (M= 476.887)
Ber. Molpeak $(M+H)^+$: 363/365
gef.: Molpeak $(M+H)^+$: 363/365 (Cl)
$R_f$-Wert: 0.40 (Kieselgel, Dichlormethan / MeOH / konz. wässr. Ammoniak 90:10:0.1).

Beispiel 179:

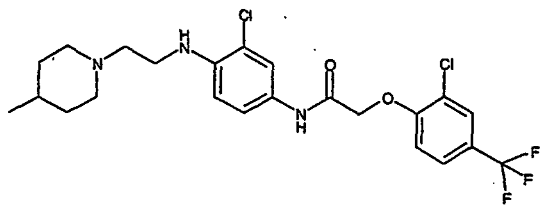

179) N-{3-Chlor-4-[2-(4-methyl-piperidin-1-yl)-ethylamino]-phenyl}-2-(2-chlor-4-trifluormethyl-phenoxy)-acetamid

Das Produkt wurde gemäß der allgemeinen Arbeitsvorschrift I ausgehend von 0.270 g (1.060 mmol) (2-Chloro-4-trifluormethyl-phenoxy)-essigsäure (Z2b) und 0.290 g (1.080 mmol) 2-Chlor-*N'*-[2-(4-methyl-piperidin-1-yl)-ethyl]-benzene-1,4-diamin (Z43d) hergestellt.
Ausbeute: 0.360 g (67% der Theorie)
$C_{23}H_{26}Cl_2F_3N_3O2$ (M= 503.384)
Ber. Molpeak $(M+H)^+$: 504/506/508
gef.: Molpeak $(M+H)^+$: 504/506/508 ($Cl_2$)
$R_f$-Wert: 0.44 (Alox, Dichlormethan / MeOH 49:1).

Beispiel 180:

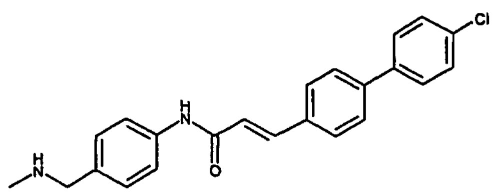

180a) {4-[(E)-3-(4'-Chlor-biphenyl-4-yl)-acryloylamino]-benzyl}-methyl-carbaminsäure-tert-butylester

Hergestellt analog Beispiel 172 ausgehend von (E)-3-(4'-Chloro-biphenyl-4-yl)-acrylsäure und (4-Amino-benzyl)-methyl-carbaminsäure-tert-butylester (Z33b). Das Rohprodukt wurde mit MeOH verrührt, der Niederschlag abfiltriert und im HV getrocknet.
Ausbeute: 0.620 g (43% der Theorie)
$C_{28}H_{29}ClN_2O_3$ (M= 477.008)
Ber. Molpeak $(M+H)^+$: 477/479
gef.: Molpeak $(M+H)^+$: 477/479 (Cl)
$R_f$-Wert: 0.80 (Kieselgel, Dichlormethan / MeOH 9:1).

180b) (E)-3-(4'-Chlor-biphenyl-4-yl)-N-(4-methylaminomethyl-phenyl)-acrylamid

Hergestellt analog Beispiel 178 ausgehend von {4-[(E)-3-(4'-Chlor-biphenyl-4-yl)-acryloylamino]-benzyl}-methyl-carbaminsäure-tert-butylester (Beispiel 179). Das Produkt wurde als Trifluoracetat-Salz erhalten.
Ausbeute: 0.540 g (87% der Theorie)
$C_{23}H_{21}ClN_2O * C_2HF_3O_2$ (M= 490.914)
Ber. Molpeak $(M+H)^+$: 377/379
gef.: Molpeak $(M+H)^+$: 377/379 (Cl)
$R_f$-Wert: 0.40 (Kieselgel, Dichlormethan / MeOH / konz. wässr. Ammoniak 90:10:0.1).

Beispiel 181:

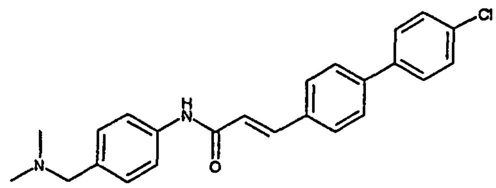

181) (E)-3-(4'-Chlor-biphenyl-4-yl)-N-(4-dimethylaminomethyl-phenyl)-acrylamid

Zu einer Lösung von 0.100 g (0.280 mmol) (E)-3-(4'-Chlor-biphenyl-4-yl)-N-(4-methylaminomethyl-phenyl)-acrylamid (Beispiel 180) in 1 mL Ameisensäure wurde bei 0 ˚C 1.00 mL Formaldehyd (37% in Wasser) zugegeben und das Gemisch 1 h bei RT und 2 h bei 90 ˚C gerührt. Das Reaktionsgemisch wurde auf Eiswasser gegossen und die wässr. Phase mit EtOAc erschöpfend extrahiert. Die vereinigten org. Phasen wurden über Natriumsulfat getrocknet, i. vac. eingeengt und der Rückstand mit Ether verrührt. Der Niederschlag wurde abfiltriert, mit Ether gewaschen und i. vac. bei 100 ˚C getrocknet.
Ausbeute: 81 mg (74% der Theorie)
$C_{24}H_{23}ClN_2O$ (M= 390.917)
Ber. Molpeak $(M+H)^+$: 391/393
gef.: Molpeak $(M+H)^+$: 391/393 (Cl)
$R_f$-Wert: 0.50 (Kieselgel, Dichlormethan / MeOH / konz. wässr. Ammoniak 90:10:0.1).

Beispiel 182:

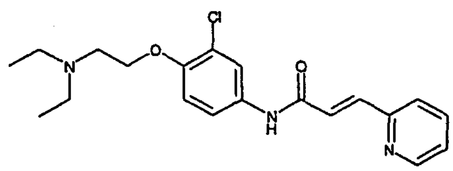

182) (E)-N-[3-Chlor-4-(2-diethylamino-ethoxy)-phenyl]-3-pyridin-2-yl-acrylamid

Hergestellt analog Beispiel 172 ausgehend von (E)-3-Pyridin-2-yl-acrylsäure und 3-Chlor-4-(2-diethylamino-ethoxy)-phenylamin (Z2b) bei RT (72 h) hergestellt. Das Rohprodukt wurde mittels Säulenchromatographie (Kieselgel, Dichlormethan / MeOH / konz. wässr. Ammoniak 90:10:0.1) und Umkristallisation aus Petrolether gereinigt. Ausbeute: 0.930 g (50% der Theorie)
$C_{20}H_{24}ClN_3O_2$ (M= 373.886)
Ber. Molpeak $(M+H)^+$: 374/376
gef.: Molpeak $(M+H)^+$: 374/376 (Cl)
$R_f$-Wert: 0.40 (Kieselgel, Dichlormethan / MeOH / konz. wässr. Ammoniak 90:10:0.1).

Beispiel 183:

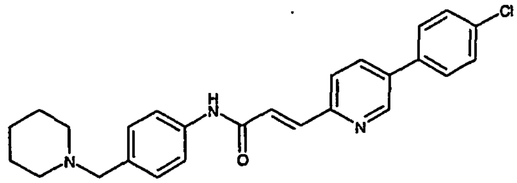

183) (E)-3-[5-(4-Chlor-phenyl)-pyridin-2-yl]-N-(4-piperidin-1-ylmethyl-phenyl)-acrylamid

Hergestellt analog Beispiel 143 ausgehend von (E)-3-[5-(4-Chlor-phenyl)-pyridin-2-yl]-acrylsäure (Z34b) und 4-Piperidin-1-ylmethyl-phenylamin. Das Rohprodukt wurde mittels Säulenchromatographie (Kieselgel, Dichlormethan / EtOH / konz. wässr. Ammoniak 50:10:0.1) gereinigt.
Ausbeute: 0.450 g (52% der Theorie)
$C_{26}H_{26}ClN_3O$ (M= 431.970)
Ber. Molpeak $(M+H)^+$: 432/434
gef.: Molpeak $(M+H)^+$: 432/434 (Cl)
$R_f$-Wert: 0.50 (Kieselgel, Dichlormethan / MeOH / konz. wässr. Ammoniak 90:10:0.1).

Beispiel 184:

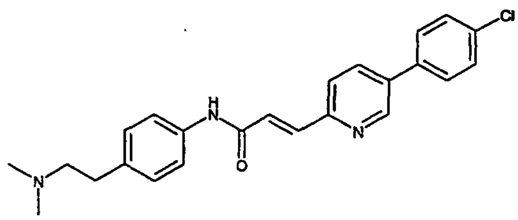

184) (E)-3-[5-(4-Chlor-phenyl)-pyridin-2-yl]-N-[4-(2-dimethylamino-ethyl)-phenyl]-acrylamid

Hergestellt analog Beispiel 143 ausgehend von (E)-3-[5-(4-Chlor-phenyl)-pyridin-2-yl]-acrylsäure (Z34b) und 4-(2-Dimethylamino-ethyl)-phenylamin. Das Rohprodukt wurde mittels Säulenchromatographie (Kieselgel, Dichlormethan / EtOH / konz. wässr. Ammoniak 50:10:0.1) gereinigt.
Ausbeute: 0.140 g (45% der Theorie)
$C_{24}H_{24}ClN_3O$ (M=405.931)
Ber. Molpeak $(M+H)^+$: 406/408
gef.: Molpeak $(M+H)^+$: 406/408 (Cl)
$R_f$-Wert: 0.60 (Kieselgel, Dichlormethan / MeOH / konz. wässr. Ammoniak 90:10:0.1).

Beispiel 185:

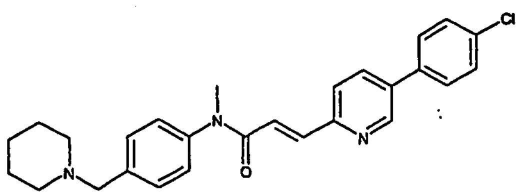

185) (E)-3-[5-(4-Chlor-phenyl)-pyridin-2-yl]-N-methyl-N-(4-piperidin-1-ylmethyl-phenyl)-acrylamid

Hergestellt analog Beispiel 143 ausgehend von (E)-3-[5-(4-Chlor-phenyl)-pyridin-2-yl]-acrylsäure (Z34b) und Methyl-(4-piperidin-1-ylmethyl-phenyl)-amin. Das Rohprodukt wurde mittels Säulenchromatographie (Kieselgel, Dichlormethan / EtOH / konz. wässr.
Ammoniak 50:10:0.1) gereinigt.
Ausbeute: 0.300 g (67% der Theorie)
$C_{27}H_{28}ClN_3O$ (M= 445.997)
Ber. Molpeak $(M+H)^+$: 446/448
gef.: Molpeak $(M+H)^+$: 446/448 (Cl)
$R_f$-Wert: 0.70 (Kieselgel, Dichlormethan / MeOH / konz. wässr. Ammoniak 90:10:0.1).

Beispiel 186:

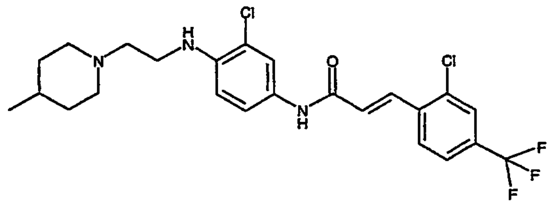

186) (E)-N-{3-Chlor-4-[2-(4-methyl-piperidin-1-yl)-ethylamino]-phenyl}-3-(2-chlor-4-trifluormethyl-phenyl)-acrylamid

Das Produkt wurde gemäß der allgemeinen Arbeitsvorschrift I ausgehend von 0.250 g (1.000 mmol) (E)-3-(2-Chlor-4-trifluormethyl-phenyl)-acrylsäure (Z37b) und 0.270 g (1.000 mmol) 2-Chlor-*N*'-[2-(4-methyl-piperidin-1-yl)-ethyl]-benzene-1,4-diamin (Z43d) hergestellt und mittels Säulenchromatographie (Alox, neutral, Akt. II-III Gradient Dichlormethan / MeOH 100:0 → 49:1) gereinigt.
Ausbeute: 0.220 g (44% der Theorie)
$C_{24}H_{26}Cl_2F_3N_3O$ (M= 500.396)
Ber. Molpeak $(M+H)^+$: 500/502/504
gef.: Molpeak $(M+H)^+$: 500/502/504 ($Cl_2$)
$R_f$-Wert: 0.70 (Alox, Dichlormethan / MeOH 49:1).

Beispiel 187:

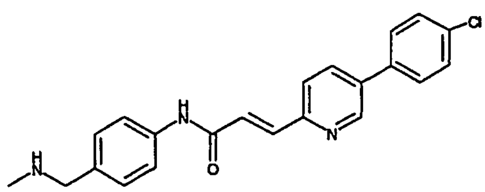

187a) (4-{(E)-3-[5-(4-Chlor-phenyl)-pyridin-2-yl]-acryloylamino}-benzyl)-methyl-carbaminsäure-tert-butylester

Hergestellt analog Beispiel 182 ausgehend von (E)-3-[5-(4-Chlor-phenyl)-pyridin-2-yl]-acrylsäure (Z34b) und (4-Amino-benzyl)-carbaminsäure-tert-butylester. Das Rohprodukt wurde mit MeOH verrührt, der Niederschlag abfiltriert und im HV getrocknet.
Ausbeute: 0.620 g (86% der Theorie)
$C_{27}H_{28}ClN_3O_3$ (M= 477.996)
Ber. Molpeak $(M+H)^+$: 478/480
gef.: Molpeak $(M+H)^+$: 478/480 (Cl)
$R_f$-Wert: 0.60 (Kieselgel, Dichlormethan / MeOH 9:1).

187b) (E)-3-[5-(4-Chlor-phenyl)-pyridin-2-yl]-N-(4-methylaminomethyl-phenyl)-acrylamid

Hergestellt analog Beispiel 178b ausgehend von (4-{(E)-3-[5-(4-Chlor-phenyl)-pyridin-2-yl]-acryloylamino}-benzyl)-methyl-carbaminsäure-tert-butylester. Das Produkt wurde als Trifluoracetat-Salz erhalten.
Ausbeute: 0.500 g (81 % der Theorie)
$C_{22}H_{20}ClN_3O * C_2HF_3O_2$ (M= 491.901)
Ber. Molpeak $(M+H)^+$: 378/380
gef.: Molpeak $(M+H)^+$: 278/380 (Cl)
$R_f$-Wert: 0.30 (Kieselgel, Dichlormethan / MeOH / konz. wässr. Ammoniak 90:10:0.1).

Beispiel 188:

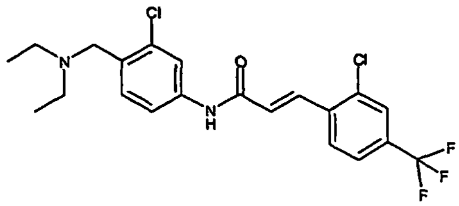

188) (E)-*N*-(3-Chlor-4-diethylaminomethyl-phenyl)-3-(2-chlor-4-trifluormethyl-phenyl)-acrylamid

Hergestellt analog Beispiel 143 ausgehend von (E)-3-(2-Chlor-4-trifluormethyl-phenyl)-acrylsäure (Z37b) und 3-Chlor-4-diethylaminomethyl-phenylamin (Z35b). Das Rohprodukt wurde mittels Säulenchromatographie (Alox, neutral, Akt. II-III, Petrolether / EtOAc 3:1) gereinigt.
Ausbeute: 0.192 g (43% der Theorie)
$C_{21}H_{21}Cl_2F_3N_2O$ (M= 445.316)
Ber. Molpeak $(M+H)^+$: 445/447/449
gef.: Molpeak $(M+H)^+$: 445/447/449 ($Cl_2$)
$R_f$-Wert: 0.53 (Alox, Petrolether / EtOAc 3:1).

Beispiel 189:

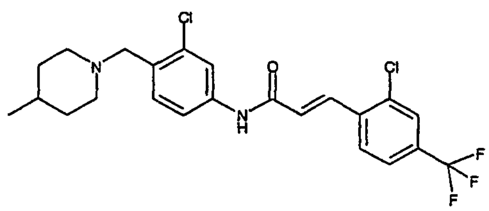

189) (E)-N-[3-Chlor-4-(4-methyl-piperidin-1-ylmethyl)-phenyl]-3-(2-chlor-4-trifluormethyl-phenyl)-acrylamid

Hergestellt analog Beispiel 143 ausgehend von (E)-3-(2-Chlor-4-trifluormethyl-phenyl)-acrylsäure (Z37b) und 3-Chlor-4-(4-methyl-piperidin-1-ylmethyl)-phenylamin (Z36b). Das Rohprodukt wurde mittels Säulenchromatographie (Alox, neutral, Akt. II-III, Petrolether / EtOAc 3:1) gereinigt.
Ausbeute: 0.176 g (37% der Theorie)
$C_{23}H_{23}Cl_2F_3N_2O$ (M= 471.354)
Ber. Molpeak $(M+H)^+$: 471/473/475
gef.: Molpeak $(M+H)^+$: 471/473/475 ($Cl_2$)
$R_f$-Wert: 0.47 (Alox, Dichlormethan / MeOH 50:1).

Beispiel 190:

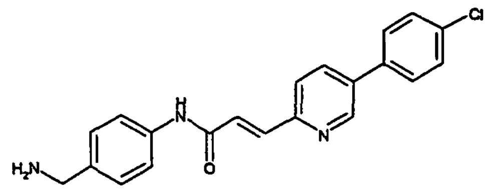

190a) (4-{(E)-3-[5-(4-Chlor-phenyl)-pyridin-2-yl]-acryloylamino}-benzyl)-carbaminsäure-tert-butylester

Hergestellt analog Beispiel 182 ausgehend von (E)-3-[5-(4-Chlor-phenyl)-pyridin-2-yl]-acrylsäure (Z34b) und (4-Amino-benzyl)-methyl-carbaminsäure-tert-butylester (Z33b). Das Rohprodukt wurde mit MeOH verrührt, der Niederschlag abfiltriert und im HV getrocknet.
Ausbeute: 0.610 g (88% der Theorie)
$C_{26}H_{26}ClN_3O_3$ (M= 463.968)
Ber. Molpeak $(M+H)^+$: 464/466
gef.: Molpeak $(M+H)^+$: 464/466 (Cl)
$R_f$-Wert: 0.60 (Kieselgel, Dichlormethan / MeOH 9:1).

190b) (E)-N-(4-Aminomethyl-phenyl)-3-[5-(4-chlor-phenyl)-pyridin-2-yl]-acrylamid

Hergestellt analog Beispiel 178 ausgehend von (4-{(E)-3-[5-(4-Chlor-phenyl)-pyridin-2-yl]-acryloylamino}-benzyl)-carbaminsäure-tert-butylester (Beispiel 186). Das Produkt wurde als Trifluoracetat-Salz erhalten.
Ausbeute: 0.600 g (99% der Theorie)
$C_{21}H_{18}ClN_3O * C_2HF_3O_3$ (M= 477.874)
Ber. Molpeak $(M-H)^-$: 362/364
gef.: Molpeak $(M-H)^-$: 362/364 (Cl)
$R_f$-Wert: 0.40 (Kieselgel, Dichlormethan / MeOH / konz. wässr. Ammoniak 90:10:0.1).

Beispiel 191:

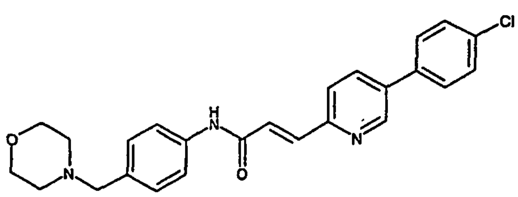

191) (E)-3-[5-(4-Chlor-phenyl)-pyridin-2-yl]-N-(4-morpholin-4-ylmethyl-phenyl)-acrylamid

Zu einer Lösung von 0.130 g (0.310 mmol) (E)-*N*-(4-Chlormethyl-phenyl)-3-[5-(4-chlorphenyl)-pyridin-2-yl]-acrylamid(Z38b) in 20 mL THF wurde 80 μL (0.930 mmol) Morpholin zugegeben und das Gemisch 5 h bei 50 °C und 5 h bei 75 °C gerührt. Weitere 200 μL Morpholin wurden zugegeben und das Gemisch 5 h bei 70 °C gerührt. Das Reaktionsgemisch wurde i. vac. eingeengt und der Rückstand mittels Säulenchromatographie (Kieselgel, Dichlormethan / MeOH / konz. wässr. Ammoniak 90:10:0.1) gereinigt. Das Produkt wurde mit Ether verrührt, der Niederschlag abfiltriert und i. vac. bei 70 °C getrocknet.
Ausbeute: 80 mg (60% der Theorie)
$C_{251}H_{24}ClN_3O_2$ (M= 433.942)
Ber. Molpeak $(M+H)^+$: 434/436
gef.: Molpeak $(M+H)^{+-}$: 434/436 (Cl)
$R_f$-Wert: 0.50 (Kieselgel, Dichlormethan / MeOH 9:1).

Beispiel 192:

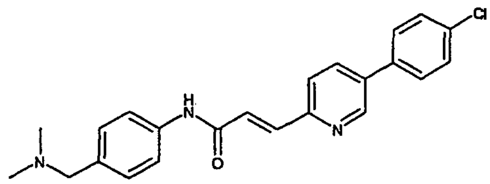

192) (E)-3-[5-(4-Chlor-phenyl)-pyridin-2-yl]-N-(4-dimethylaminomethyl-phenyl)-acrylamid

Hergestellt analog Beispiel 181 ausgehend von (E)-N-(4-Aminomethyl-phenyl)-3-[5-(4-chlor-phenyl)-pyridin-2-yl]-acrylamid (Beispiel 190).
Ausbeute: 0.120 g (56% der Theorie)
$C_{23}H_{22}ClN_3O$ (M= 391.904)
Ber. Molpeak $(M+H)^+$: 392/394
gef.: Molpeak $(M+H)^+$: 392/394 (Cl)
$R_f$-Wert: 0.30 (Kieselgel, Dichlormethan / MeOH / konz. wässr. Ammoniak 90:10:0.1).

Beispiel 193:

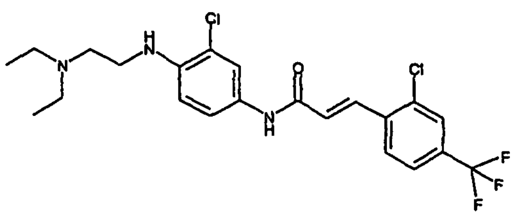

193) (E)-N-[3-Chlor-4-(2-diethylamino-ethylamino)-phenyl]-3-(2-chlor-4-trifluormethylphenyl)-acrylamid

Hergestellt in DMF analog Beispiel 143 ausgehend von (E)-3-(2-Chlor-4-trifluormethylphenyl)-acrylsäure (Z37b) und 2-Chlor-*N*'-(2-diethylamino-ethyl)-benzol-1,4-diamin (Z42b). Das Rohprodukt wurde mittels Säulenchromatographie (Alox, neutral, Akt. II-III, Dichlormethan / MeOH 49:1) und durch Verreiben in Ether/Petrolether gereinigt.
Ausbeute: 0.215 g (45% der Theorie)
$C_{22}H_{24}Cl_2F_3N_3O$ (M= 474.357)
Ber. Molpeak $(M+H)^+$: 474/476/478
gef.: Molpeak $(M+H)^+$: 474/476/478 ($Cl_2$)
$R_f$-Wert: 0.58 (Alox, Dichlormethan / MeOH 49:1).

Beispiel 194:

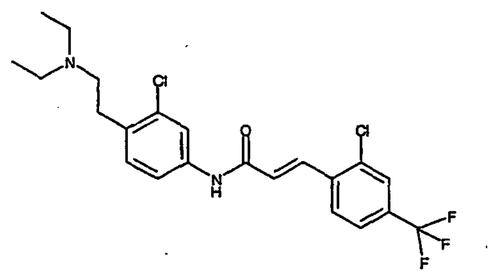

194) (E)-N-[3-Chlor-4-(2-diethylamino-ethyl)-phenyl]-3-(2-chlor-4-trifluormethyl-phenyl)-acrylamid

Hergestellt in DMF analog Beispiel 143 ausgehend von (E)-3-(2-Chlor-4-trifluormethylphenyl)-acrylsäure (Z37b) und 3-Chloro-4-(2-diethylamino-ethyl)-phenylamin (Z44d). Das Reaktionsgemisch wurde auf Eiswasser gegossen, der Niederschlag abfiltriert, mit Wasser gewaschen und im HV getrocknet. Der Rückstand wurde mit Ether verrieben, der Niederschlag abfiltriert, mit Ether gewaschen und im HV getrocknet.
Ausbeute: 0.278 g (60% der Theorie)
$C_{22}H_{23}Cl_2F_3N_2O$ (M= 459.343)
Ber. Molpeak $(M+H)^+$: 459/461/463
gef.: Molpeak $(M+H)^+$: 459/461/463 ($Cl_2$)
gef.: Molpeak $(M+H)^+$: 459/461/463 ($Cl_2$)
$R_f$-Wert: 0.32 (Alox, Petrolether / EtOAc 1:1).

Beispiel 195:

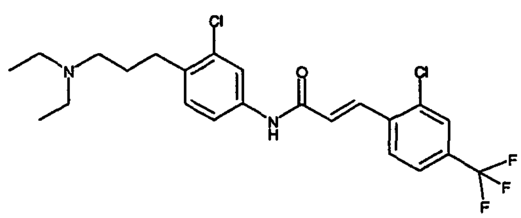

195) (E)-N-[3-Chlor-4-(3-diethylamino-propyl)-phenyl]-3-(2-chlor-4-trifluormethylphenyl)-acrylamid

Eine Lösung von 0.290 g (0.603 mmol) (E)-*N*-[4-(3-Brom-propyl)-3-chlor-phenyl]-3-(2-chlor-4-trifluormethylphenyl)-acrylamid (Z45d) in 2 mL Diethylamin wurde im geschlossenen Reaktionsgefäß in der Mikrowelle 5 Min. auf 100 °C erhitzt. Das Reaktionsgemisch wurde mit EtOAc verdünnt, die org. Phase mit Wasser gewaschen, über Natriumsulfat getrocknet und i. vac. eingeengt. Der Rückstand wurde mit Ether verrieben, der Niederschlag abfiltriert, mit Ether gewaschen und im HV getrocknet. Ausbeute: 0.180 g (63% der Theorie)
$C_{23}H_{25}Cl_2F_3N_2O$ (M= 473.370)
Ber. Molpeak $(M+H)^+$: 473/475/477
gef.: Molpeak $(M+H)^+$: 473/475/477 ($Cl_2$)
$R_f$-Wert: 0.33 (Alox, Dichlormethan / MeOH 30:1).

Beispiel 196:

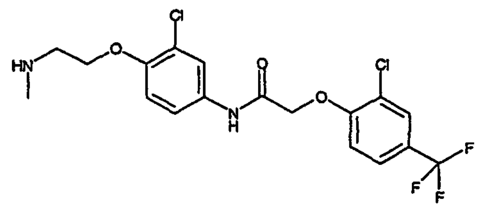

196a) N-[4-(N-tert.Butoxycarbonyl-2-methylamino-ethoxy)-3-chlor-phenylamin]-2-(2-chlor-4-trifluormethyl-phenoxy)-acetamid

Das Produkt wurde analog zu Beispiel 143 aus 260 mg (1.00 mmol) (2-Chlor-4-trifluormethyl-phenoxy)-essigsäure (Z2b), 0.120 mL(1.10 mmol) N-Methylmorpholin, 0.140 mL (1.10 mmol) Chlorameisensäure-isobutylester und 330 mg (1.10 mmol) 4-(N-tert.Butoxycarbonyl-2-methylamino-ethoxy)-3-chlor-phenylamin (Z40b) hergestellt. Ausbeute: 0.54 g (100% der Theorie)
$C_{23}H_{25}Cl_2F_3N_2O_5$ (M= 537.36)
Ber. Molpeak $(M-H)^-$: 535/537/539
gef.: Molpeak $(M-H)^-$: 535/537/539 ($Cl_2$)
$R_f$-Wert: 0.75 (Kieselgel, Dichlormethan/Methanol 19:1)

196b) N-[4-(2-Methylamino-ethoxy)-3-chlor-phenylamin]-2-(2-chlor-4-trifluormethyl-phenoxy)-acetamid

Das Produkt wurde analog zu Beispiel 115 aus 560 mg (1.04 mmol) N-[4-(N-tert.Butoxycarbonyl-2-methylamino-ethoxy)-3-chlor-phenylamin]-2-(2-chlor-4-trifluormethyl-phenoxy)-acetamid erhalten.
Ausbeute: 0.43 g (94% der Theorie)
$C_{18}H_{17}Cl_2F_3N_2O_3$ (M= 437.24)
Ber. Molpeak $(M+H)^+$: 437/439/441
gef.: Molpeak $(M+H)^+$: 437/439/441
$R_f$-Wert: 0.35 (Kieselgel, Dichlormethan / MeOH 9:1)

Beispiel 197:

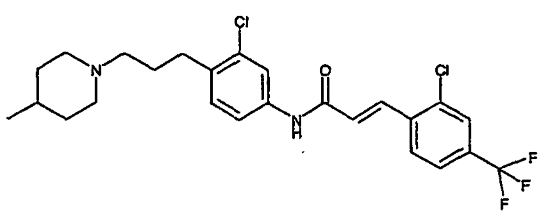

197) 2-(2-Chlor-4-trifluormethyl-phenoxy)-N-[4-(2-diethylamino-ethoxy)-3-methylphenyl]-acetamid

0.290 g (0.603 mmol) (E)-*N*-[4-(3-Brom-propyl)-3-chlor-phenyl]-3-(2-chlor-4-trifluormethyl-phenyl)-acrylamid (Z45d) und 0.270 g (2.614 mmol) 4-Methylpiperidin wurde im geschlossenen Reaktionsgefäß in der Mikrowelle 5 Min. auf 100 ˚C erhitzt. Das Reaktionsgemisch wurde mit EtOAc verdünnt, der gebildete Niederschlag abfiltriert, mit 1 N wässr. NaOH-Lösung verrührt, mit Wasser gewaschen und im HV getrocknet.
Ausbeute: 0.280 g (93% der Theorie)
$C_{25}H_{27}Cl_2F_3N_2O$ (M= 499.408)
Ber. Molpeak $(M+H)^+$: 499/501/503
gef.: Molpeak $(M+H)^+$: 499/501/503 ($Cl_2$)
$R_f$-Wert: 0.33 (Alox, Dichlormethan / MeOH 30:1).

Beispiel 198:

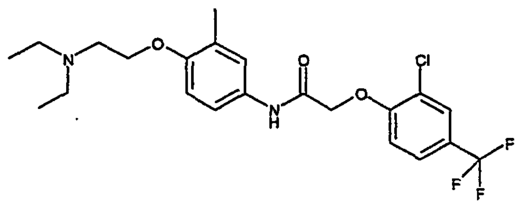

198) N-[4-(2-Diethylamino-ethoxy)-3-methyl-phenyl]-2-(2-chlor-4-trifluormethyl-phenoxy)-acetamid

Das Produkt wurde gemäß der allgemeinen Arbeitsvorschrift I aus 100 mg (0.40 mmol) (2-Chlor-4-trifluormethyl-phenoxy)-essigsäure (Z2b) und 100 mg (0.44 mmol) 4-(2-Diethylamino-ethoxy)-3-methyl-phenylamin (Z39b) hergestellt. Das Produkt wurde als Formiat-Salz erhalten.
Ausbeute: 51 mg (25% der Theorie)
$C_{22}H_{26}ClF_3N_2O_3$ (M= 458.91)
Ber. Molpeak $(M+H)^+$: 459/461
gef.: Molpeak $(M+H)^+$: 459/461
$R_f$-Wert: 0,4 (Kieselgel, Dichlormethan / MeOH 9:1)

Beispiel 199:

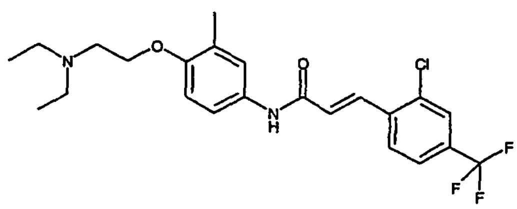

199) (E)-N-[4-(2-Diethylamino-ethoxy)-3-methyl-phenyl]-2-(2-chlor-4-trifluormethyl-phenoxy)-acrylamid

Das Produkt wurde analog zu Beispiel 119 aus 100 mg (0.40 mmol) (E)-3-(2-Chlor-4-trifluormethyl-phenyl)-acrylsäure (Z37b) und 100 mg (0.44 mmol) 4-(2-Diethylamino-ethoxy)-3-methyl-phenylamin (Z39b) hergestellt. Das Produkt wurde als Formiat-Salz erhalten.
Ausbeute: 44 mg (22% der Theorie)
$C_{23}H_{26}ClF_3N_2O_2$ (M= 454.92)
Ber. Molpeak $(M+H)^+$: 454/456
gef.: Molpeak $(M+H)^+$: 455/456
$R_f$-Wert: 0,2 (Kieselgel, Dichlormethan / MeOH 9:1)

Beispiel 200:

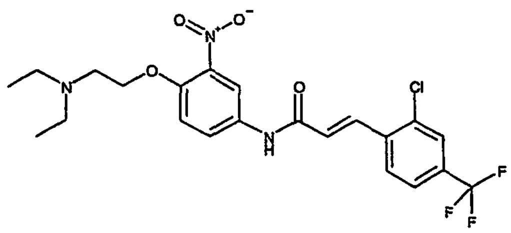

200) (E)-3-(2-Chlor-4-trifluormethyl-phenyl)-N-[4-(2-diethylamino-ethoxy)-3-nitrophenyl]-acrylamid

Das Produkt wurde analog zu Beispiel 119 aus 100 mg (0.40 mmol) (E)-3-(2-Chlor-4-trifluormethyl-phenyl)-acrylsäure (Z37b) und 110 mg (0.44 mmol) 4-(2-Diethylamino-ethoxy)-3-nitro-phenylamin (Z31 a) hergestellt. Das Produkt wurde als Formiat-Salz erhalten.
Ausbeute: 27 mg (13% der Theorie)
$C_{22}H_{23}ClF_3N_3O_4$ (M= 485.89)
Ber. Molpeak $(M+H)^+$: 485/487
gef.: Molpeak $(M+H)^+$: 486/488
$R_f$-Wert: 0,3 (Kieselgel, Dichlormethan / MeOH 9:1)

Beispiel 201:

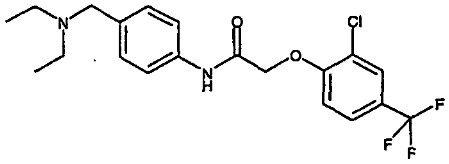

201) N-(4-Diethylaminomethyl-phenyl)-2-(2-chlor-4-trifluormethyl-phenoxy)-acetamid

Das Produkt wurde gemäß der allgemeinen Arbeitsvorschrift I aus 100 mg (0.40 mmol) (2-Chlor-4-trifluormethyl-phenoxy)-essigsäure (Z2b) und 80 mg (0.44 mmol) 4-Diethylaminomethyl-phenylamin (Herstellung siehe WO 01/27081) hergestellt. Das Produkt wurde als Formiat-Salz erhalten.
Ausbeute: 92 mg (50% der Theorie)
$C_{20}H_{22}ClF_3N_2O_2$ (M= 414.85)
Ber. Molpeak $(M+H)^+$: 414/416
gef.: Molpeak $(M+H)^+$: 415/417
$R_f$-Wert: 0,25 (Kieselgel, Dichlormethan / MeOH 9:1)

Beispiel 202:

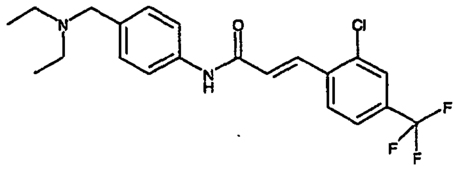

202) (E)-N-(4-Diethylaminomethyl-phenyl)-2-(2-chlor-4-trifluormethyl-phenoxy)-acrylamid

Das Produkt wurde analog zu Beispiel 119 aus 100 mg (0.40 mmol) (E)-3-(2-Chlor-4-trifluormethyl-phenyl)-acrylsäure (Z37b) und 80 mg (0.44 mmol) 4-Diethylaminomethylphenylamin (Herstellung siehe WO 01/27081) hergestellt. Das Produkt wurde als Formiat-Salz erhalten.
Ausbeute: 27 mg (15% der Theorie)
$C_{21}H_{22}ClF_3N_2O$ (M= 410.87)
Ber. Molpeak $(M+H)^+$: 410/412
gef.: Molpeak $(M+H)^+$: 411/413
$R_f$-Wert: 0,15 (Kieselgel, Dichlormethan / MeOH 9:1)

Beispiel 203:

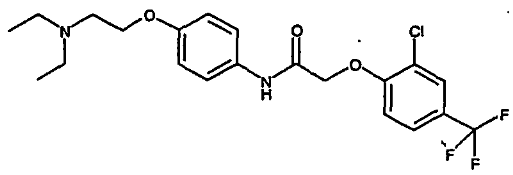

203) N-[4-(2-Diethylamino-ethoxy)-phenyl]-2-(2-chlor-4-trifluormethyl-phenoxy)-acetamid

Das Produkt wurde gemäß der allgemeinen Arbeitsvorschrift I aus 100 mg (0.40 mmol) (2-Chlor-4-trifluormethyl-phenoxy)-essigsäure (Z2b) und 90 mg (0.44 mmol) 4-(2-Diethylamino-ethoxy)-phenylamin (Z5b) hergestellt. Das Produkt wurde als Formiat-Salz erhalten.
Ausbeute: 45 mg (23% der Theorie)
$C_{21}H_{24}ClF_3N_2O_3$ (M= 444.88)
Ber. Molpeak $(M+H)^+$: 444/446
gef.: Molpeak $(M+H)^+$: 445/447
$R_f$-Wert: 0,3 (Kieselgel, Dichlormethan / MeOH 9:1)

Beispiel 204:

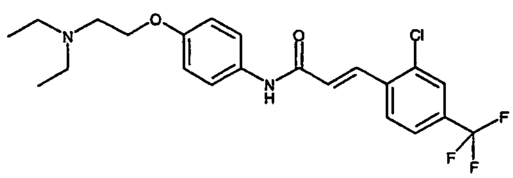

204) (E)-N-[4-(2-Diethylamino-ethoxy)-phenyl]-2-(2-chlor-4-trifluormethyl-phenoxy)-acrylamid

Das Produkt wurde analog zu Beispiel 119 aus 100 mg (0.40 mmol) (E)-3-(2-Chlor-4-trifluormethyl-phenyl)-acrylsäure (Z37b) und 90 mg (0.44 mmol) 4-(2-Diethylamino-ethoxy)-phenylamin (Z5b) hergestellt. Das Produkt wurde als Formiat-Salz erhalten.
Ausbeute: 35 mg (18% der Theorie)
$C_{22}H_{24}ClF_3N_2O_2$ (M= 440.89)
Ber. Molpeak $(M+H)^+$: 440/442
gef.: Molpeak $(M+H)^+$: 441/443
$R_f$-Wert: 0,2 (Kieselgel, Dichlormethan / MeOH 9:1)

Beispiel 205:

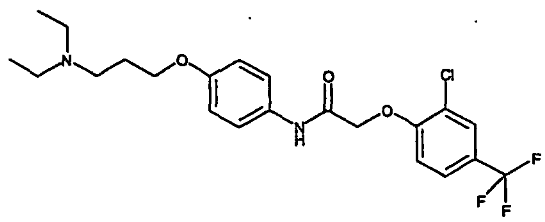

205) N-[4-(3-Diethylamino-propyloxy)-phenyl]-2-(2-chlor-4-trifluormethyl-phenoxy)-acetamid

Das Produkt wurde gemäß der allgemeinen Arbeitsvorschrift I aus 100 mg (0.40 mmol) (2-Chlor-4-trifluormethyl-phenoxy)-essigsäure (Z2b) und 100 mg (0.44 mmol) 4-(3-Diethylamino-propyloxy)-phenylamin (Herstellung siehe WO 99/52869) hergestellt. Das Produkt wurde als Formiat-Salz erhalten.
Ausbeute: 48 mg (23,8% der Theorie)
$C_{22}H_{26}ClF_3N_2O_3$ (M= 458.91)
Ber. Molpeak $(M+H)^+$: 458/460
gef.: Molpeak $(M+H)^+$: 459/61
$R_f$-Wert: 0,40 (Kieselgel, Dichlormethan / MeOH 9:1)

Beispiel 206:

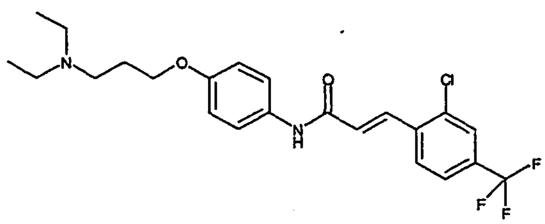

206) (E)-N-[4-(3-Diethylamino-propyloxy)-phenyl]-2-(2-chlor-4-trifluormethyl-phenoxy)-acrylamid

Das Produkt wurde analog zu Beispiel 119 aus 100 mg (0.40 mmol) (E)-3-(2-Chlor-4-trifluormethyl-phenyl)-acrylsäure (Z37b) und 100 mg (0.44 mmol) 4-(3-Diethylamino-propyloxy)-phenylamin (Herstellung siehe WO 99/52869) hergestellt. Das Produkt wurde als Formiat-Salz erhalten.
Ausbeute: 33 mg (16% der Theorie)
$C_{23}H_{26}ClF_3N_2O_2$ (M= 454.92)
Ber. Molpeak $(M+H)^+$: 454/456
gef.: Molpeak $(M+H)^+$: 455/7
$R_f$-Wert: 0,39 (Kieselgel, Dichlormethan / MeOH 9:1)

Beispiel 207:

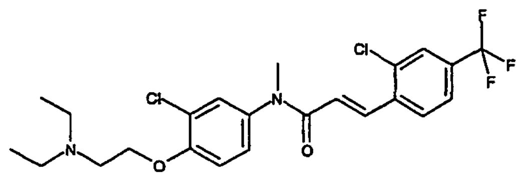

207) (E)-N-[3-Chlor-4-(2-diethylamino-ethoxy)-phenyl]-N-methyl-2-(2-chlor-4-trifluormethyl-phenoxy)-acrylamid

Das Produkt wurde analog zu Beispiel 119 aus 100 mg (0.40 mmol) (E)-3-(2-Chlor-4-trifluormethyl-phenyl)-acrylsäure (Z37b) und 110 mg (0.44 mmol) N-[3-Chlor-4-(2-diethylamino-ethoxy)-phenyl]-methylamin (Z125b) hergestellt. Das Produkt wurde als Formiat-Salz erhalten.
Ausbeute: 38 mg (18% der Theorie)
$C_{23}H_{25}Cl_2F_3N_2O_2$ (M= 489.36)
Ber. Molpeak $(M+H)^+$: 487/489/491
gef.: Molpeak $(M+H)^+$: 489/91/93
$R_f$-Wert: 0,48 (Kieselgel, Dichlormethan / MeOH 9:1)

Beispiel 208:

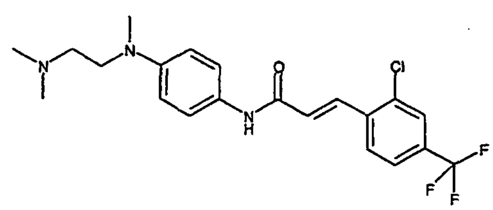

208) (E)-N-{4-[N-(2-Dimethylamino-ethyl)-N-methyl-amino]-phenyl}-2-(2-chlor-4-trifluormethyl-phenoxy)-acrylamid

Das Produkt wurde analog zu Beispiel 119 aus 100 mg (0.40 mmol) (E)-3-(2-Chlor-4-trifluormethyl-phenyl)-acrylsäure (Z37b) und 90 mg (0.44 mmol) N-(2-Dimethylaminoethyl)-N-methyl-benzol-1,4-diamin (Z141b) hergestellt. Das Produkt wurde als Formiat-Salz erhalten.
Ausbeute: 34 mg (18% der Theorie)
$C_{21}H_{23}ClF_3N_3O$ (M= 425.88)
Ber. Molpeak $(M+H)^+$: 425/427
gef.: Molpeak $(M+H)^+$: 426/8
$R_f$-Wert: 0,29 (Kieselgel, Dichlormethan / MeOH 9:1)

Beispiel 209:

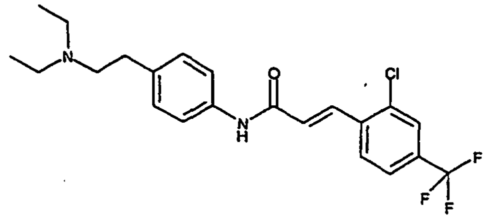

209) (E)-N-[4-(2-Diethylamino-ethyl)-phenyl]-2-(2-chlor-4-trifluormethyl-phenoxy)-acrylamid

Das Produkt wurde analog zu Beispiel 119 aus 100 mg (0.40 mmol) (E)-3-(2-Chlor-4-trifluormethyl-phenyl)-acrylsäure (Z37b) und 90 mg (0.44 mmol) 4-(2-Diethylaminoethyl)-phenylamin (Herstellung siehe WO 01/27081) hergestellt. Das Produkt wurde als Formiat-Salz erhalten.
Ausbeute: 27 mg (14% der Theorie)
$C_{22}H_{24}ClF_3N_2O$ (M= 424.89)
Ber. Molpeak $(M+H)^+$: 424/426
gef.: Molpeak $(M+H)^+$: 425/27
$R_f$-Wert: 0,30 (Kieselgel, Dichlormethan / MeOH 9:1)

Beispiel 210:

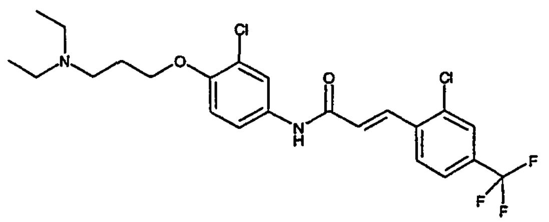

210) (E)-N-[3-Chlor-4-(3-diethylamino-propyloxy)-phenyl]-2-(2-chlor-4-trifluormethyl-phenoxy)-acrylamid

Das Produkt wurde analog zu Beispiel 119 aus 100 mg (0.40 mmol) (E)-3-(2-Chlor-4-trifluormethyl-phenyl)-acrylsäure (Z37b) und 110 mg (0.44 mmol) 3-Chlor-4-(3-diethylamino-propyloxy)-phenylamin (Z29b) hergestellt. Das Produkt wurde als Formiat-Salz erhalten.
Ausbeute: 38 mg (18% der Theorie)
$C_{23}H_{25}Cl_2F_3N_2O_2$ (M= 489.36)
Ber. Molpeak $(M+H)^+$: 489/491
gef.: Molpeak $(M+H)^+$: 489/91/93
$R_f$-Wert: 0.36 (Kieselgel, Dichlormethan / MeOH 9:1)

Beispiel 211:

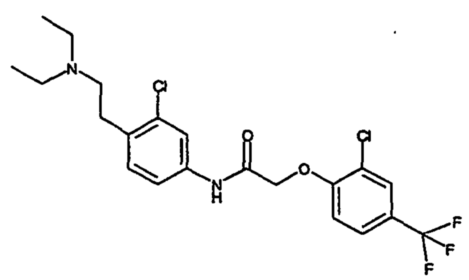

211) N-[3-Chlor-4-(2-diethylamino-ethyl)-phenyl]-2-(2-chlor-4-trifluormethyl-phenoxy)-acetamid

Hergestellt analog Beispiel 143 ausgehend von (2-Chloro-4-trifluormethyl-phenoxy)-essigsäure (Z2b) und 3-Chloro-4-(2-diethylamino-ethyl)-phenylamin (Z44d). Das Reaktionsgemisch wurde auf Eiswasser gegossen und die wässr. Phase mit EtOAc extrahiert. Die org. Phase wurde über Natriumsulfat getrocknet und i. vac. eingeengt. Der Rückstand wurde mittels Säulenchromatographie (Alox, neutral, Akt. II-III, Gradient Petrolether / EtOAc 5:2 → 1:1) gereinigt.

Ausbeute: 0.258 g (65% der Theorie)
$C_{21}H_{23}Cl_2F_3N_2O_2$ (M= 463.331)
Ber. Molpeak $(M+H)^+$: 463/465/467
gef.: Molpeak $(M+H)^+$: 463/465/467 ($Cl_2$)
$R_f$-Wert: 0.46 (Alox, Petrolether / EtOAc 1:1)

Beispiel 212:

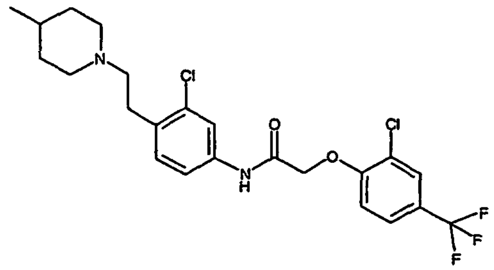

212) N-{3-Chlor-4-[2-(4-methyl-piperidin-1-yl)-ethyl]-phenyl}-2-(2-chlor-4-trifluormethyl-phenoxy)-acetamid

Hergestellt analog Beispiel 143 ausgehend von (2-Chloro-4-trifluormethyl-phenoxy)-essigsäure (Z2b) und 3-Chlor-4-[2-(4-methyl-piperidin-1-yl)-ethyl]-phenylamin (Z46c). Das Reaktionsgemisch wurde auf Eiswasser gegossen und die wässr. Phase mit EtOAc extrahiert. Die org. Phase wurde über Natriumsulfat getrocknet und i. vac. eingeengt. Der Rückstand wurde mittels Säulenchromatographie (Alox, neutral, Akt. II-III, Gradient Petrolether / EtOAc 3:1 → 1:1) gereinigt.
Ausbeute: 0.218 g (52% der Theorie)
$C_{23}H_{25}Cl_2F_3N_2O_2$ (M= 489.369)
Ber. Molpeak $(M+H)^+$: 489/491/493
gef.: Molpeak $(M+H)^+$: 489/491/493 ($Cl_2$)
$R_f$-Wert: 0.46 (Alox, Petrolether / EtOAc 1:1)

Beispiel 213:

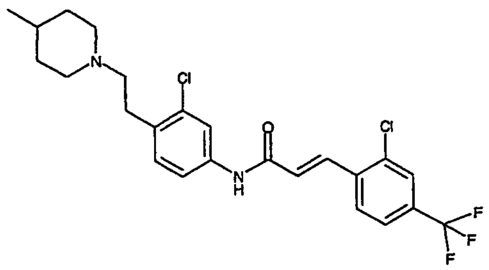

213) (E)-N-{3-Chlor-4-[2-(4-methyl-piperidin-1-yl)-ethyl]-phenyl}-3-(2-chlor-4-trifluormethyl-phenyl)-acrylamid

Hergestellt in DMF analog Beispiel 143 ausgehend von (E)-3-(2-Chlor-4-trifluormethylphenyl)-acrylsäure (Z37b) und 3-Chlor-4-[2-(4-methyl-piperidin-1-yl)-ethyl]-phenylamin (Z46c). Das Rohprodukt wurde mittels Säulenchromatographie (Alox, neutral, Akt. II-III, Gradient Petrolether / EtOAc 3:1 → 2:1) gereinigt und anschließend mit Petrolether verrieben.
Ausbeute: 0.240 g (51 % der Theorie)

$C_{24}H_{25}Cl_2F_3N_2O$ (M= 485.381)
Ber. Molpeak $(M+H)^+$: 485/487/489
gef.: Molpeak $(M+H)^+$: 485/487/489 ($Cl_2$)
$R_f$-Wert: 0.38 (Alox, Petrolether / EtOAc 1:1)

Beispiel 214:

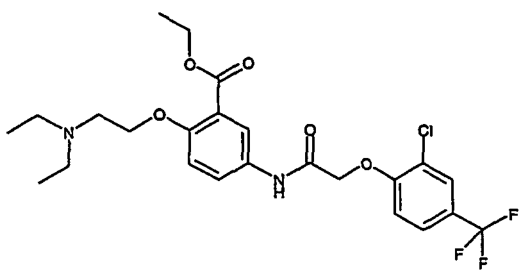

214) 5-[2-(2-Chlor-4-trifluormethyl-phenoxy)-acetylamino]-2-(2-diethylamino-ethoxy)-benzoesäure-ethylester

Hergestellt analog Beispiel 143 ausgehend von (2-Chlor-4-trifluormethyl-phenoxy)-essigsäure (Z2b) und 5-Amino-2-(2-diethylamino-ethoxy)-benzoesäure-ethylester. Das Reaktionsgemisch wurde i. vac. eingeengt und der Rückstand in Dichlormethan gelöst. Die org. Phase wurde mit Wasser gewaschen, über Natriumsulfat getrocknet und i. vac. eingeengt. Reinigung mittels Säulenchromatographie (Alox, neutral, Akt. II-III, Gradient Petrolether / EtOAc 70:30 → 50:50) ergab das Produkt.
Ausbeute: 1.220 g (59% der Theorie)
$C_{24}H_{28}ClF_3N_2O_5$ (M= 516.949)
Ber. Molpeak $(M+H)^+$: 517/519
gef.: Molpeak $(M+H)^+$: 517/519 (Cl)
$R_f$-Wert: 0.62 (Alox, Petrolether / EtOAc 2:1)

Beispiel 215:

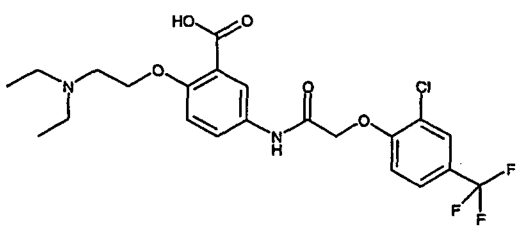

215) 5-[2-(2-Chlor-4-trifluormethyl-phenoxy)-acetylamino]-2-(2-diethylamino-ethoxy)-benzoesäure

Zu einer Lösung von 1.00 g (1.934 mmol) 5-[2-(2-Chlor-4-trifluormethyl-phenoxy)-acetylamino]-2-(2-diethylamino-ethoxy)-benzoesäure-ethylester in 30 mL EtOH wurde 4.00 mL 1 M wässr. NaOH-Lösung zugegeben und das Gemisch 3 h bei RT gerührt. 4.00 mL 1 M wässr. HCl wurde zugegeben und erneut 1 h gerührt. Das Reaktionsgemisch wurde i. vac. eingeengt, mit Wasser versetzt und der Niederschlag abfiltriert.
Ausbeute: 0.220 g (23% der Theorie)
$C_{22}H_{24}ClF_3N_2O_5$ (M= 488.895)
Ber. Molpeak $(M+H)^+$: 487/489
gef.: Molpeak $(M+H)^+$: 487/489 (Cl)
$R_f$-Wert: 0.36 (Kieselgel, Dichlormethan / MeOH 9:1)

Beispiel 216:

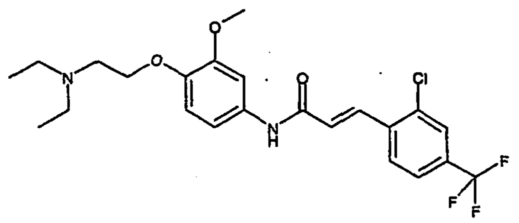

216) (E)-3-(2-Chlor-4-trifluormethyl-phenyl)-N-[4-(2-diethylamino-ethoxy)-3-methoxyphenyl]-acrylamid

Das Produkt wurde gemäß der allgemeinen Arbeitsvorschrift I ausgehend von 0.100g (0.400 mmol) (E)-3-(2-Chlor-4-trifluormethyl-phenyl)-acrylsäure (Z37b) und 0.121 g (0.440 mmol) 4-(2-Diethylamino-ethoxy)-3-methoxy-phenylamin hergestellt. Das Rohprodukt wurde mittels HPLC (Stable Bond C18; 3.5 $\mu$m; Wasser / Acetonitril / Ameisensäure 9:1:0.01 $\rightarrow$ 1:9:0.01 über 9 min) gereinigt und das Produkt als Formiat-Salz erhalten.
Ausbeute: 41 mg (20% der Theorie)
$C_{23}H_{26}ClF_3N_2O_3$ * $CH_2O_2$ (M= 516.949)
Ber. Molpeak $(M+H)^+$: 471/473
gef.: Molpeak $(M+H)^+$: 471/473 (Cl)
$R_f$-Wert: 0.35 (Kieselgel, Dichlormethan / MeOH 9:1)
In Analogie zu den vorstehend erwähnten Beispielen können folgende Verbindungen hergestellt werden:

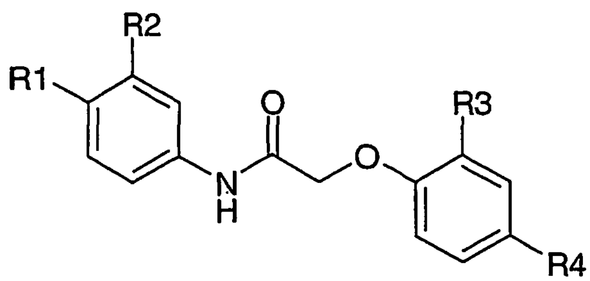

| Beispiel | R1 | R2 | R3 | R4 |
|---|---|---|---|---|
| 217 | 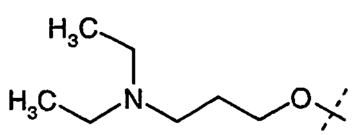 | -OMe | -Cl | $-CF_3$ |
| 218 | 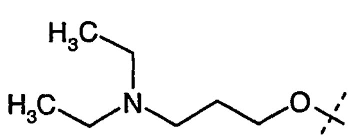 | -Br | -Cl | $-CF_3$ |
| 219 | 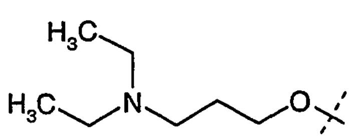 | -CN | -Cl | $-CF_3$ |

(fortgesetzt)

| Beispiel | R1 | R2 | R3 | R4 |
|---|---|---|---|---|
| 220 | 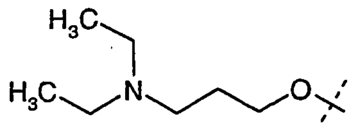 | -F | -Cl | $-CF_3$ |
| 221 | 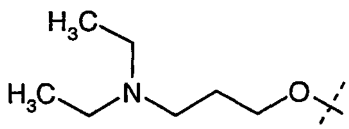 | $-CH_3$ | -Cl | $-CF_3$ |
| 222 | 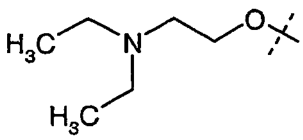 | -F | -Cl | $-CF_3$ |
| 223 | 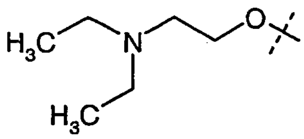 | -F | -Cl | $-CF_3$ |
| 224 | 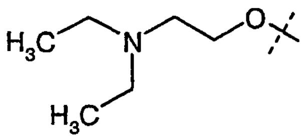 | -COOEt | -Cl | $-CF_3$ |
| 225 | 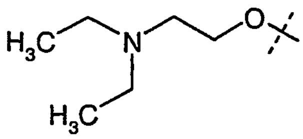 | -COOH | -Cl | $-CF_3$ |
| 226 | 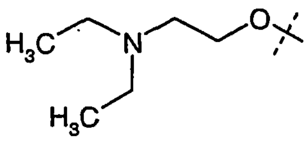 | $-CONH_2$ | -Cl | $-CF_3$ |
| 227 | 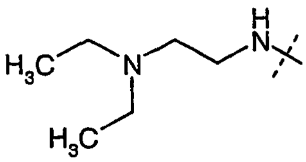 | -Cl | -Cl | $-CF_3$ |
| 228 | 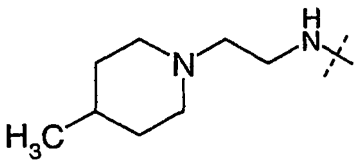 | -Cl | -Cl | $-CF_3$ |

(fortgesetzt)

| Beispiel | R1 | R2 | R3 | R4 |
|---|---|---|---|---|
| 229 | 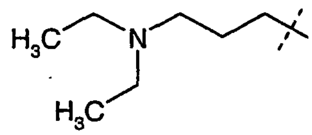 | -Cl | -Cl | $-CF_3$ |
| 230 | 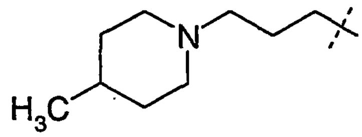 | -Cl | -Cl | $-CF_3$ |
| 231 | 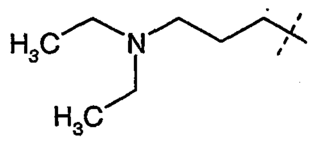 | -H | -Cl | $-CF_3$ |
| 232 | 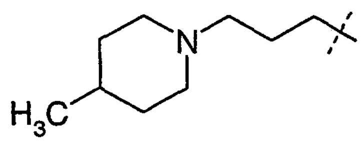 | -H | -Cl | $-CF_3$ |
| 233 | 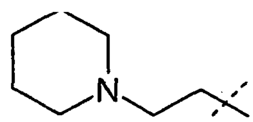 | -Cl | -Cl | $-CF_3$ |
| 234 | 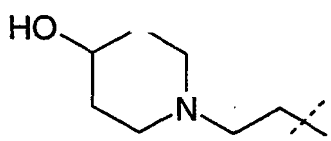 | -Cl | -Cl | $-CF_3$ |
| 235 | 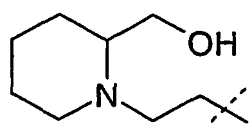 | -Cl | -Cl | $-CF_3$ |
| 236 | 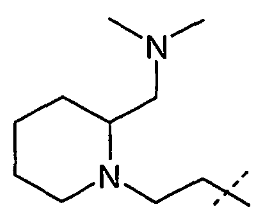 | -Cl | -Cl | $-CF_3$ |
| 237 | 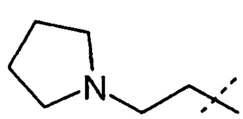 | -Cl | -Cl | $-CF_3$ |

(fortgesetzt)

| Beispiel | R1 | R2 | R3 | R4 |
|---|---|---|---|---|
| 238 | 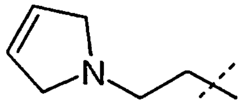 | -Cl | -Cl | $-CF_3$ |
| 240 | 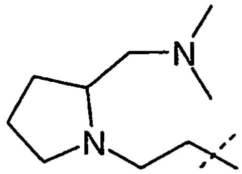 | -Cl | -Cl | $-CF_3$ |
| 241 | 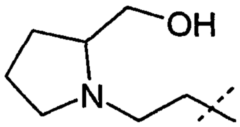 | -Cl | -Cl | $-CF_3$ |
| 242 | 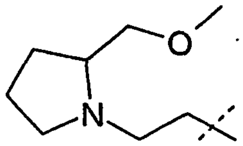 | -Cl | -Cl | $-CF_3$ |
| 243 | 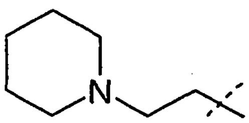 | -Br | -Cl | $-CF_3$ |
| 244 | 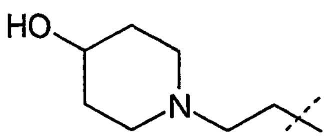 | -Br | -Cl | $-CF_3$ |
| 245 | 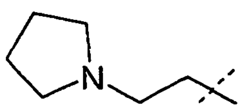 | -Br | -Cl | $-CF_3$ |
| 246 | 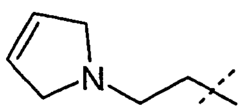 | -Br | -Cl | $-CF_3$ |
| 247 | 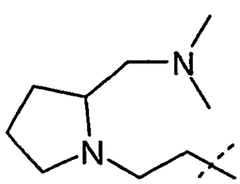 | -Br | -Cl | $-CF_3$ |

(fortgesetzt)

| Beispiel | R1 | R2 | R3 | R4 |
|---|---|---|---|---|
| 248 | 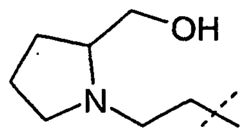 | -Br | -Cl | $-CF_3$ |
| 249 | 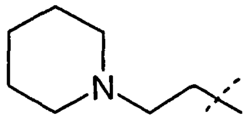 | -OMe | -Cl | $-CF_3$ |
| 250 | 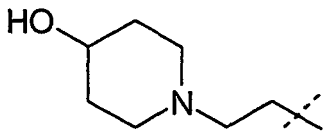 | -OMe | -Cl | $-CF_3$ |
| 251 | 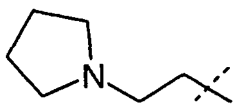 | -OMe | -Cl | $-CF_3$ |
| 252 | 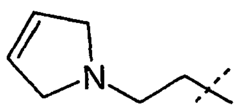 | -OMe | -Cl | $-CF_3$ |
| 253 | 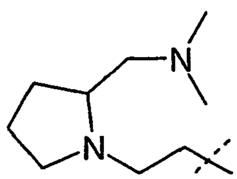 | -OMe | -Cl | $-CF_3$ |
| 254 | 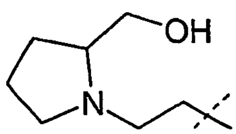 | -OMe | -Cl | $-CF_3$ |
| 255 | 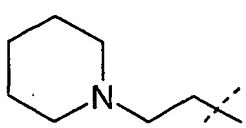 | -Cl | -Cl | -Br |
| 256 | 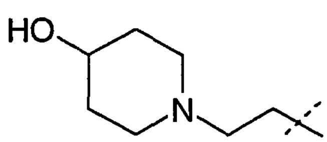 | -Cl | -Cl | -Br |
| 257 | 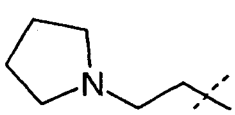 | -Cl | -Cl | -Br |

(fortgesetzt)

| Beispiel | R1 | R2 | R3 | R4 |
|---|---|---|---|---|
| 258 | 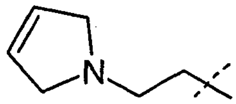 | -Cl | -Cl | -Br |
| 259 | 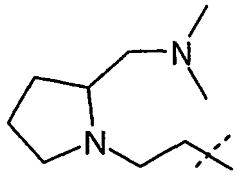 | -Cl | -Cl | -Br |
| 260 | 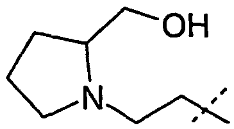 | -Cl | -Cl | -Br |
| 261 | 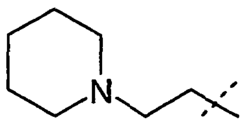 | -Cl | -Cl | -Ph |
| 262 | 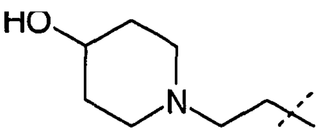 | -Cl | -Cl | -Ph |
| 263 | 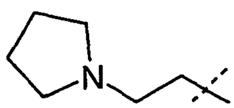 | -Cl | -Cl | -Ph |
| 364 | 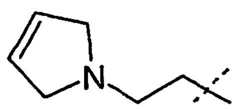 | -Cl | -Cl | -Ph |
| 265 | 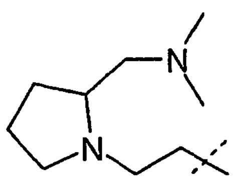 | -Cl | -Cl | -Ph |
| 266 | 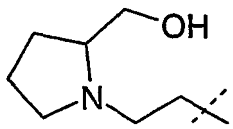 | -Cl | -Cl | -Ph |

(fortgesetzt)

| Beispiel | R1 | R2 | R3 | R4 |
|---|---|---|---|---|
| 267 | 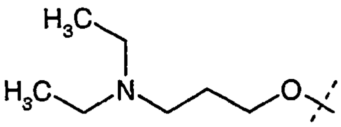 | -Cl | -Cl | -Ph |
| 268 | 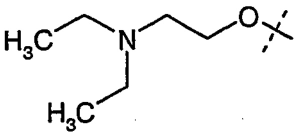 | -Br | -Cl | -Ph |
| 269 | 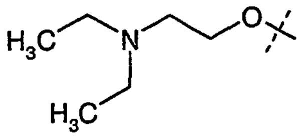 | -OMe | -Cl | -Ph |
| 270 | 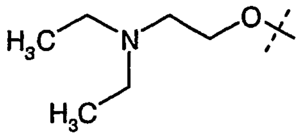 | -CN | -Cl | -Ph |

In Analogie zu den vorstehend erwähnten Beispielen können folgende Verbindungen hergestellt werden:

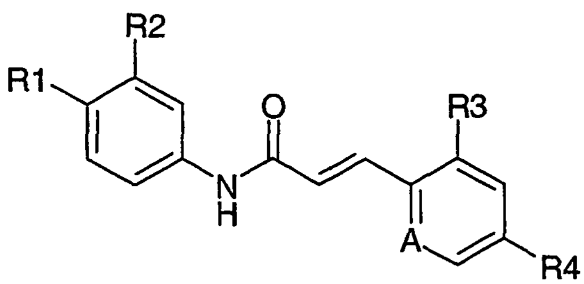

| Nummer | R1 | R2 | R3 | R4 | A |
|---|---|---|---|---|---|
| 271 | 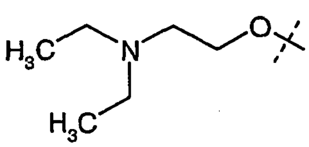 | -OMe | -Cl | $-CF_3$ | CH |
| 272 | 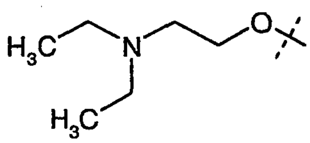 | -Br | -Cl | $-CF_3$ | CH |

(fortgesetzt)

| Nummer | R1 | R2 | R3 | R4 | A |
|---|---|---|---|---|---|
| 273 | 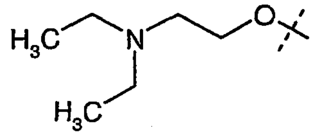 | -CN | -Cl | $-CF_3$ | CH |
| 274 | 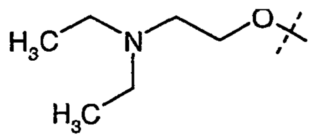 | -F | -Cl | $-CF_3$ | CH |
| 275 | 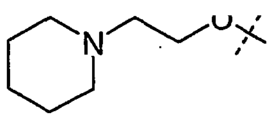 | -Cl | -Cl | $-CF_3$ | CH |
| 276 | 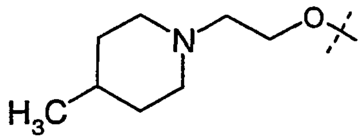 | -Cl | -Cl | $-CF_3$ | CH |
| 277 | 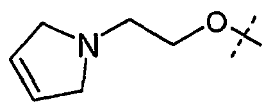 | -Cl | -Cl | $-CF_3$ | CH |
| 278 | 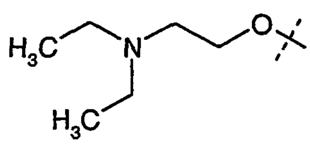 | $-NO_2$ | -Cl | $-CF_3$ | CH |
| 279 | 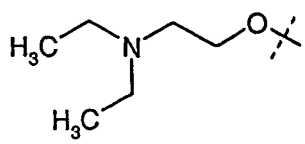 | -COOEt | -Cl | $-CF_3$ | CH |
| 280 | 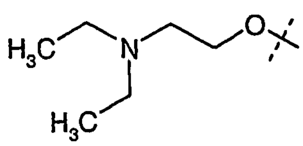 | -COOH | -Cl | $-CF_3$ | CH |
| 281 | 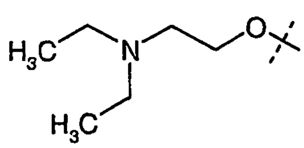 | $-CONH_2$ | -Cl | $-CF_3$ | CH |
| 282 | 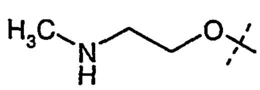 | -Cl | -Cl | $-CF_3$ | CH |
| 283 | 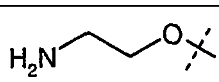 | -Cl | -Cl | $-CF_3$ | CH |

(fortgesetzt)

| Nummer | R1 | R2 | R3 | R4 | A |
|---|---|---|---|---|---|
| 284 | 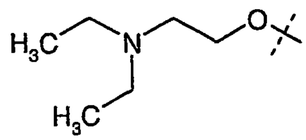 | -Cl | -Cl | -Ph | CH |
| 285 | 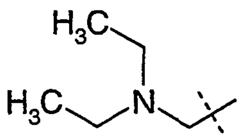 | -H | -H | 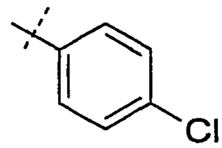 | CH |
| 286 | 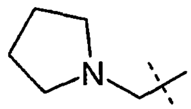 | -H | -H | 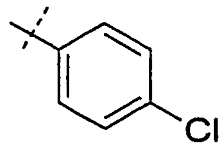 | CH |
| 287 | 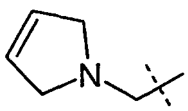 | -H | -H | 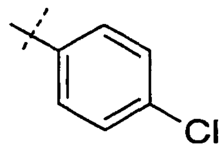 | CH |
| 288 | 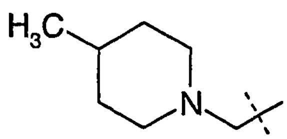 | -H | -H | 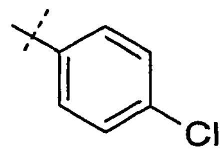 | CH |
| 289 | 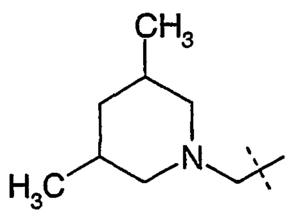 | -H | -H | 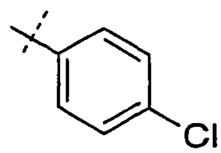 | CH |
| 290 | 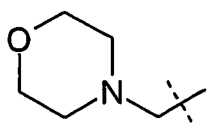 | -H | -H | 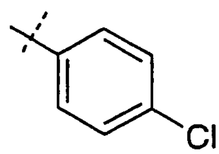 | CH |
| 291 | 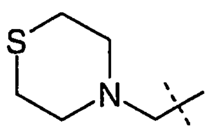 | -H | -H | 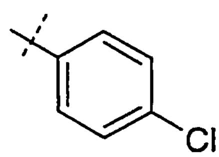 | CH |
| 292 | 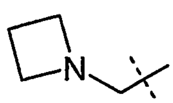 | -H | -H | 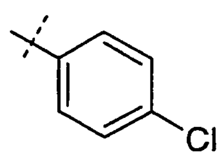 | CH |

(fortgesetzt)

| Nummer | R1 | R2 | R3 | R4 | A |
|---|---|---|---|---|---|
| 293 | 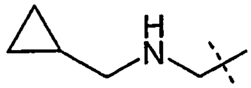 | -H | -H | 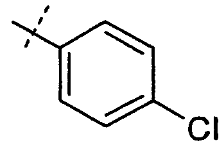 | CH |
| 294 | 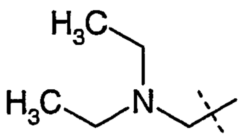 | -H | -H | 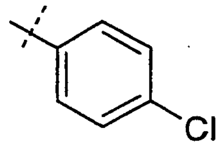 | N |
| 295 | 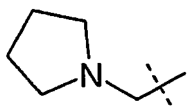 | -H | -H | 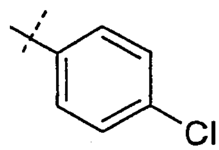 | N |
| 296 | 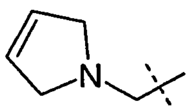 | -H | -H | 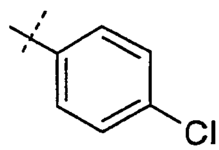 | N |
| 297 | 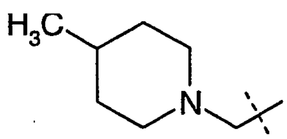 | -H | -H | 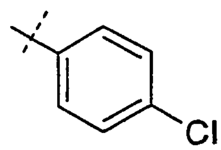 | N |
| 298 | 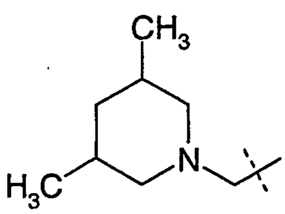 | -H | -H | 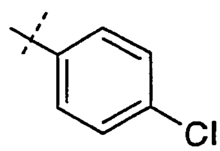 | N |
| 299 | 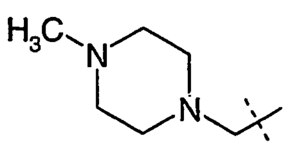 | -H | -H | 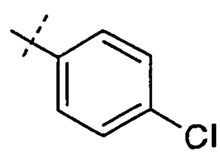 | N |
| 300 | 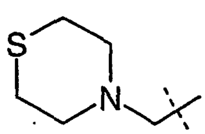 | -H | -H | 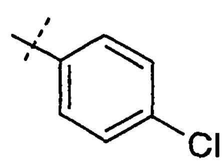 | N |

(fortgesetzt)

| Nummer | R1 | R2 | R3 | R4 | A |
|---|---|---|---|---|---|
| 301 | 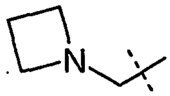 | -H | -H | 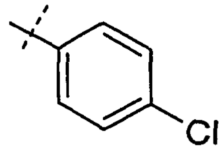 | N |
| 302 | 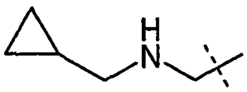 | -H | -H | 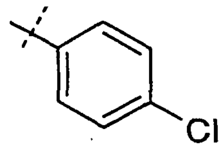 | N |
| 303 | 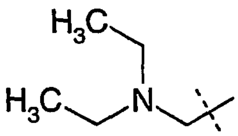 | -Cl | -H | 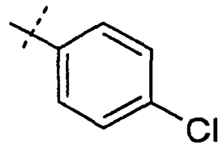 | CH |
| 304 | 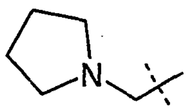 | -Cl | -H | 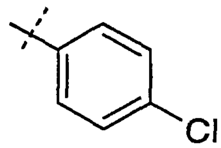 | CH |
| 305 | 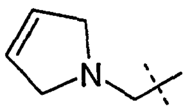 | -Cl | -H | 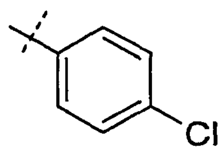 | CH |
| 306 | 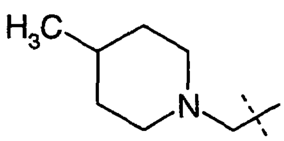 | -Cl | -H | 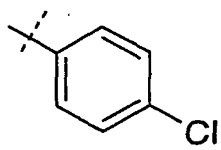 | CH |
| 307 | 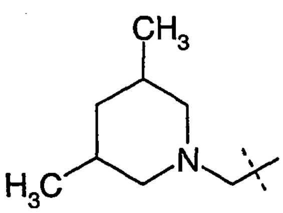 | -Cl | -H | 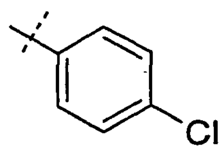 | CH |
| 308 | 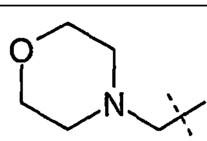 | -Cl | -H | 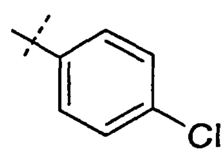 | CH |

(fortgesetzt)

| Nummer | R1 | R2 | R3 | R4 | A |
|---|---|---|---|---|---|
| 309 | 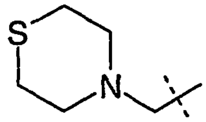 | -Cl | -H | 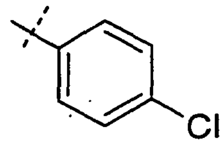 | CH |
| 310 | 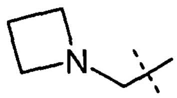 | -Cl | -H | 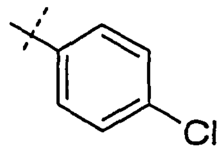 | CH |
| 311 | 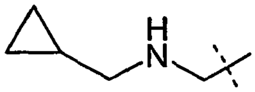 | -Cl | -H | 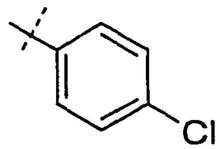 | CH |
| 312 | 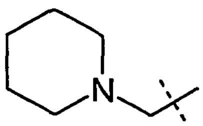 | -Cl | -H | 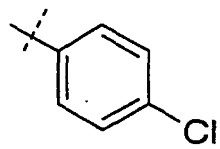 | CH |
| 313 | 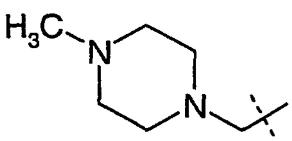 | -Cl | -H | 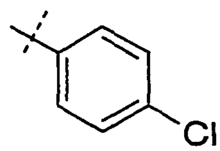 | CH |
| 314 | 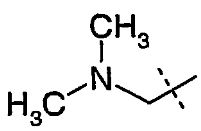 | -Cl | -H | 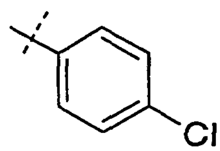 | CH |
| 315 | 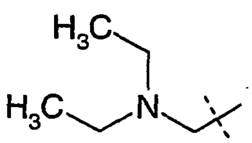 | -Cl | -H | 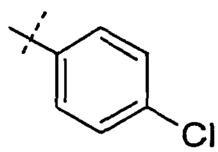 | N |
| 316 | 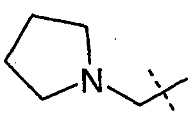 | -Cl | -H | 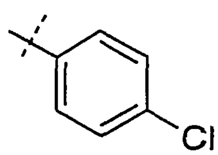 | N |
| 317 | 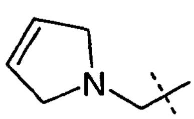 | -Cl | -H | 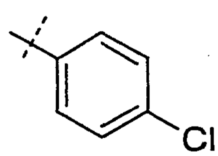 | N |

(fortgesetzt)

| Nummer | R1 | R2 | R3 | R4 | A |
|---|---|---|---|---|---|
| 318 | 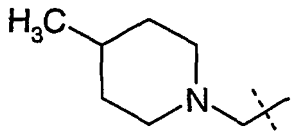 | -Cl | -H | 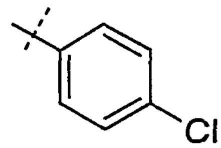 | N |
| 319 | 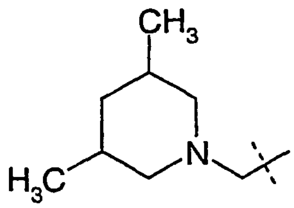 | -Cl | -H | 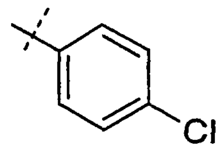 | N |
| 320 | 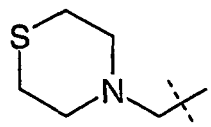 | -Cl | -H | 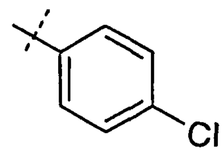 | N |
| 321 | 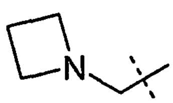 | -Cl | -H | 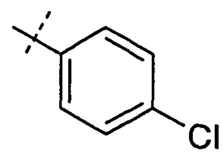 | N |
| 322 | 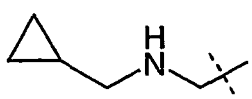 | -Cl | -H | 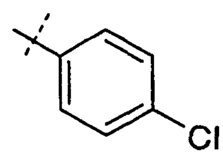 | N |
| 323 | 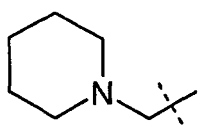 | -Cl | -H | 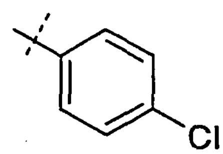 | N |
| 324 | 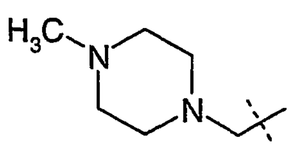 | -Cl | -H | 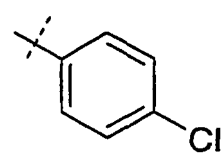 | N |
| 325 | 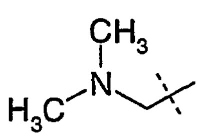 | -Cl | -H | 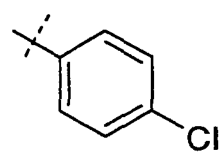 | N |
| 326 | 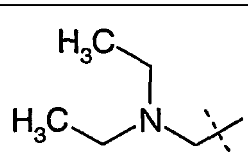 | -H | -H | 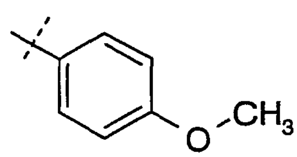 | CH |

(fortgesetzt)

| Nummer | R1 | R2 | R3 | R4 | A |
|---|---|---|---|---|---|
| 327 | 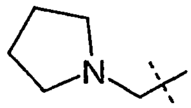 | -H | -H | 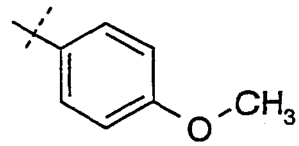 | CH |
| 328 | 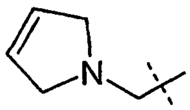 | -H | -H | 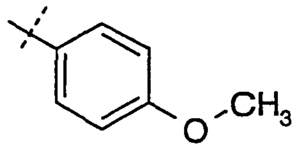 | CH |
| 329 | 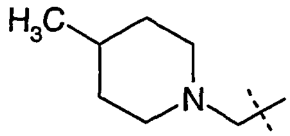 | -H | -H | 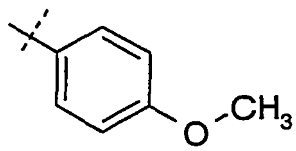 | CH |
| 330 | 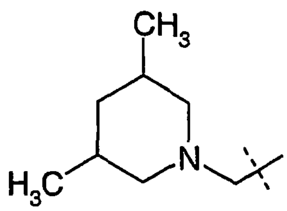 | -H | -H | 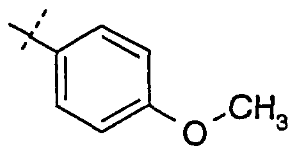 | CH |
| 331 | 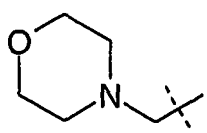 | -H | -H | 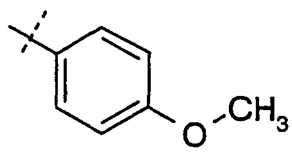 | CH |
| 332 | 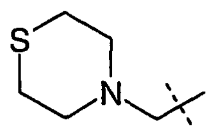 | -H | -H | 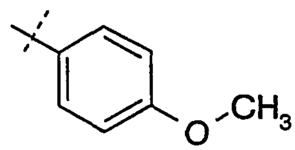 | CH |
| 333 | 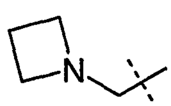 | -H | -H | 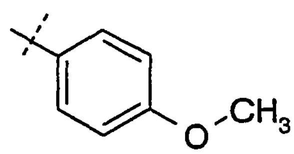 | CH |
| 334 | 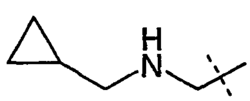 | -H | -H | 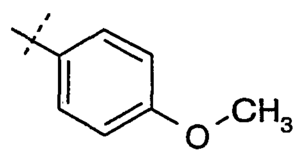 | CH |
| 335 | 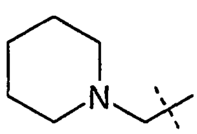 | -H | -H | 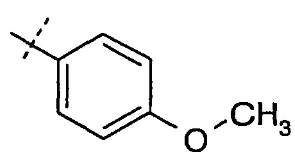 | CH |

(fortgesetzt)

| Nummer | R1 | R2 | R3 | R4 | A |
|---|---|---|---|---|---|
| 336 | 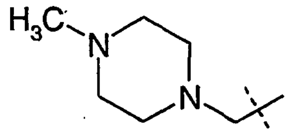 | -H | -H | 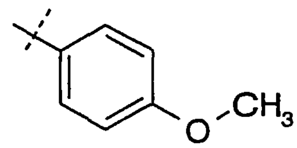 | CH |
| 337 | 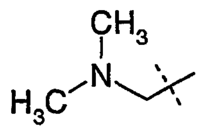 | -H | -H | 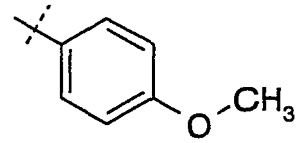 | CH |
| 338 | 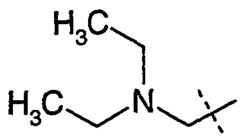 | -H | -H | 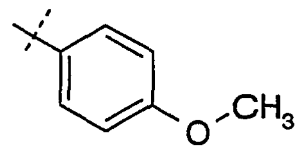 | N |
| 339 | 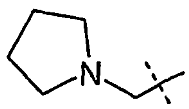 | -H | -H | 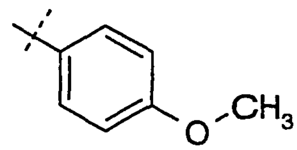 | N |
| 340 | 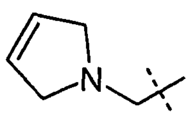 | -H | -H | 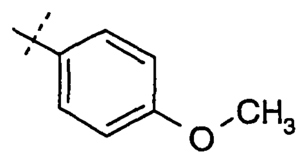 | N |
| 341 | 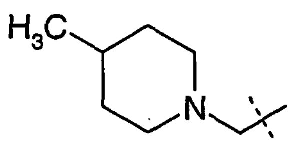 | -H | -H | 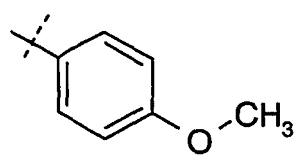 | N |
| 342 | 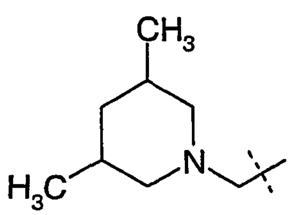 | -H | -H | 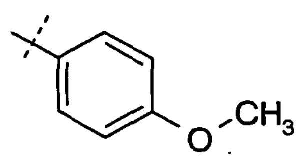 | N |
| 343 | 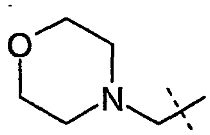 | -H | -H | 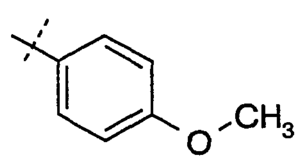 | N |

(fortgesetzt)

| Nummer | R1 | R2 | R3 | R4 | A |
|---|---|---|---|---|---|
| 344 | 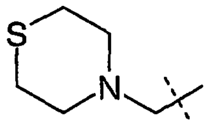 | -H | -H | 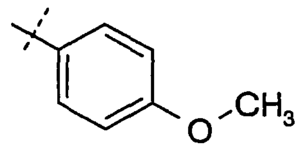 | N |
| 345 | 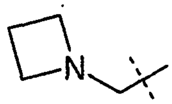 | -H | -H | 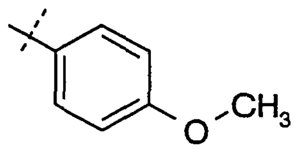 | N |
| 346 | 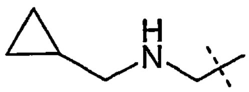 | -H | -H | 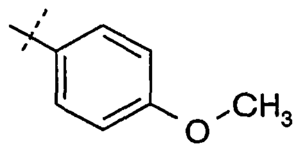 | N |
| 347 | 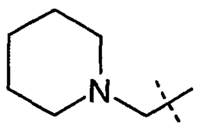 | -H | -H | 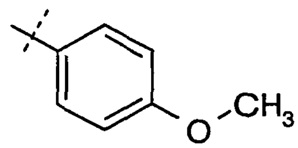 | N |
| 348 | 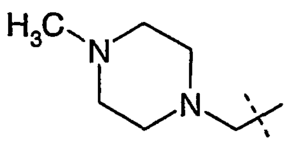 | -H | -H | 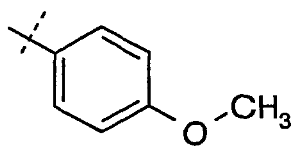 | N |
| 349 | 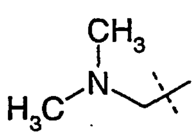 | -H | -H | 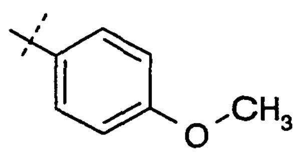 | N |
| 350 | 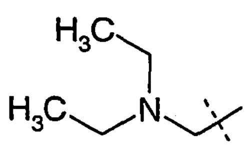 | -H | -F | 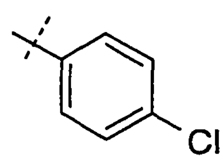 | CH |
| 351 | 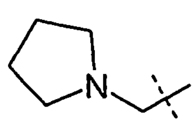 | -H | -F | 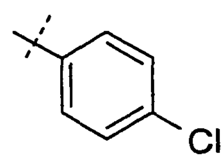 | CH |
| 352 | 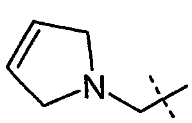 | -H | -F | 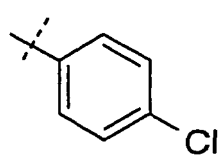 | CH |

(fortgesetzt)

| Nummer | R1 | R2 | R3 | R4 | A |
|---|---|---|---|---|---|
| 353 | 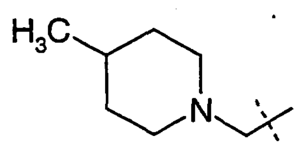 | -H | -F | 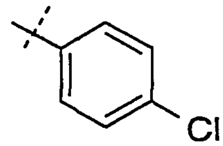 | CH |
| 354 | 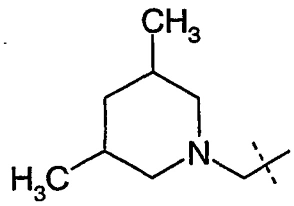 | -H | -F | 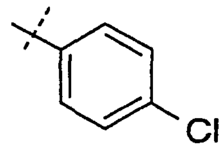 | CH |
| 355 | 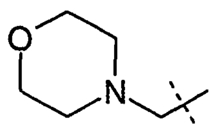 | -H | -F | 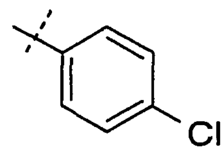 | CH |
| 356 | 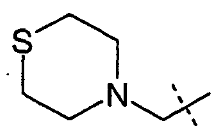 | -H | -F | 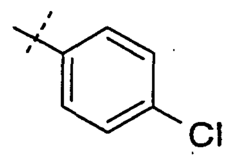 | CH |
| 357 | 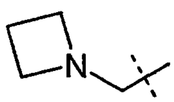 | -H | -F | 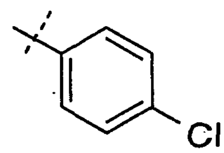 | CH |
| 358 | 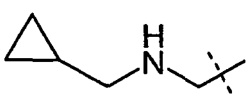 | -H | -F | 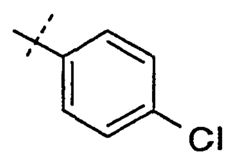 | CH |
| 359 | 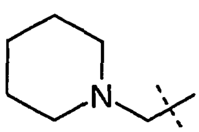 | -H | -F | 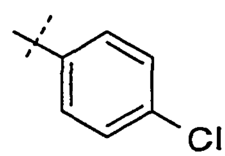 | CH |
| 360 | 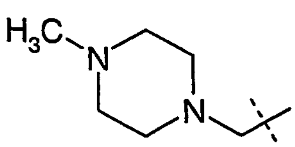 | -H | -F | 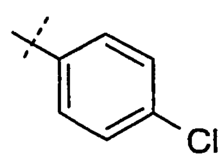 | CH |
| 361 | 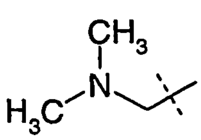 | -H | -F | 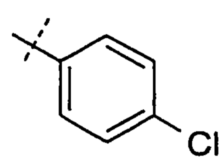 | CH |

(fortgesetzt)

| Nummer | R1 | R2 | R3 | R4 | A |
|---|---|---|---|---|---|
| 362 | 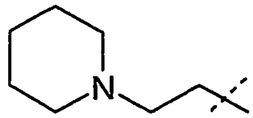 | -Cl | -Cl | $-CF_3$ | CH |
| 363 | 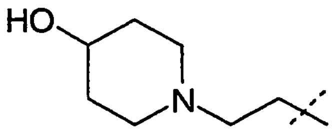 | -Cl | -Cl | $-CF_3$ | CH |
| 364 | 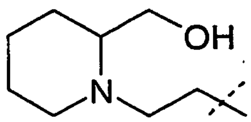 | -Cl | -Cl | $-CF_3$ | CH |
| 365 | 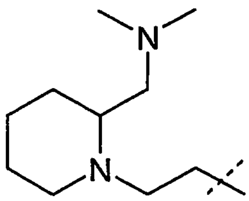 | -Cl | -Cl | $-CF_3$ | CH |
| 366 | 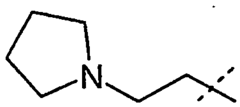 | -Cl | -Cl | $-CF_3$ | CH |
| 367 | 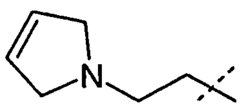 | -Cl | -Cl | $-CF_3$ | CH |
| 368 | 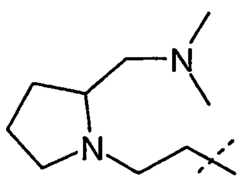 | -Cl | -Cl | $-CF_3$ | CH |
| 369 | 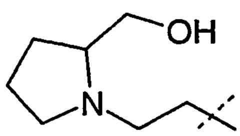 | -Cl | -Cl | $-CF_3$ | CH |
| 370 | 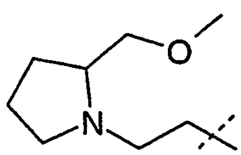 | -Cl | -Cl | $-CF_3$ | CH |
| 371 | 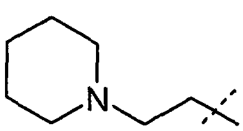 | -Br | -Cl | $-CF_3$ | CH |

(fortgesetzt)

| Nummer | R1 | R2 | R3 | R4 | A |
|---|---|---|---|---|---|
| 372 | 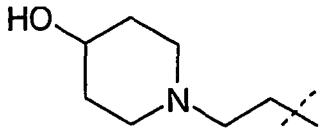 | -Br | -Cl | $-CF_3$ | CH |
| 373 | 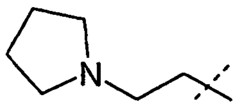 | -Br | -Cl | $-CF_3$ | CH |
| 374 | 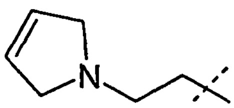 | -Br | -Cl | $-CF_3$ | CH |
| 375 | 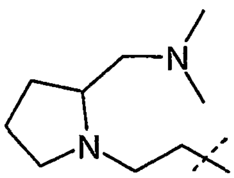 | -Br | -Cl | $-CF_3$ | CH |
| 376 | 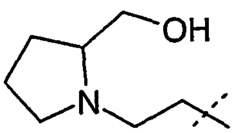 | -Br | -Cl | $-CF_3$ | CH |
| 377 | 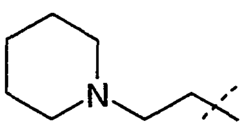 | -OMe | -Cl | $-CF_3$ | CH |
| 378 | 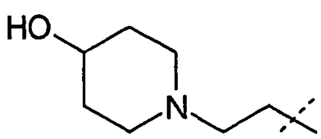 | -OMe | -Cl | $-CF_3$ | CH |
| 379 | 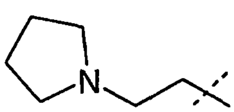 | -OMe | -Cl | $-CF_3$ | CH |
| 380 | 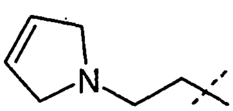 | -OMe | -Cl | $-CF_3$ | CH |
| 381 | 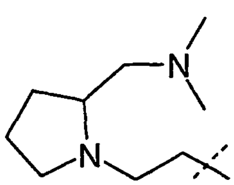 | -OMe | -Cl | $-CF_3$ | CH |

(fortgesetzt)

| Nummer | R1 | R2 | R3 | R4 | A |
|---|---|---|---|---|---|
| 382 | 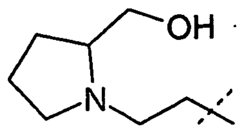 | -OMe | -Cl | $-CF_3$ | CH |
| 383 | 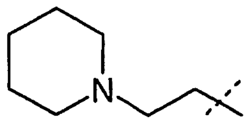 | -Cl | -Cl | -Ph | CH |
| 384 | 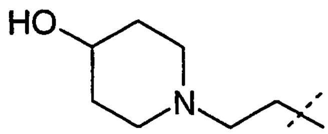 | -Cl | -Cl | -Ph | CH |
| 385 | 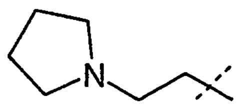 | -Cl | -Cl | -Ph | CH |
| 386 | 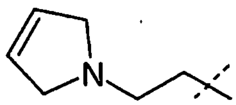 | -Cl | -Cl | -Ph | CH |
| 387 | 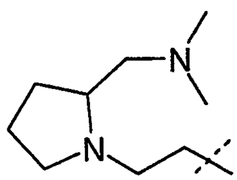 | -Cl | -Cl | -Ph | CH |
| 388 | 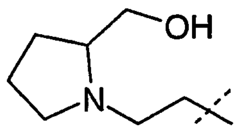 | -Cl | -Cl | -Ph | CH |
| 389 | 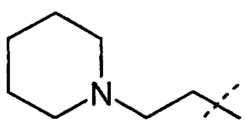 | -Cl | -H | -Ph | N |
| 390 | 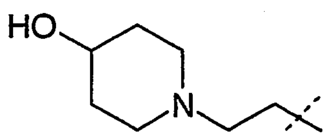 | -Cl | -H | -Ph | N |
| 391 | 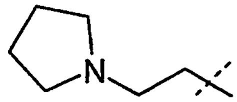 | -Cl | -H | -Ph | N |

(fortgesetzt)

| Nummer | R1 | R2 | R3 | R4 | A |
|---|---|---|---|---|---|
| 392 | 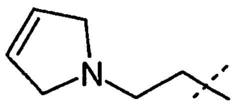 | -Cl | (H | -Ph | N |
| 393 | 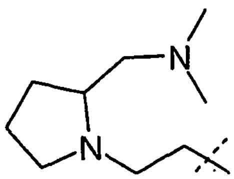 | -Cl | -H | -Ph | N |
| 394 | 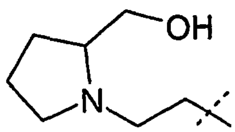 | -Cl | -H | -Ph | N |

Nachfolgend werden Testverfahren zur Bestimmung einer MCH-Rezeptor antagonistischen Aktivität beschrieben. Darüber hinaus können auch weitere dem Fachmann bekannte Testverfahren, beispielsweise über die Inhibition der MCH-Rezeptor vermittelten Hemmung der cAMP-Produktion, wie von Hoogduijn M et al. in "Melanin-concentrating hormone and its receptor are expressed and functional in human skin", Biochem. Biophys. Res Commun. 296 (2002) 698-701 sowie über die biosensorische Messung der Bindung von MCH an den MCH Rezeptor in Gegenwart antagonistischer Substanzen durch Plasmonresonanz, wie von Karlsson OP und Lofas S. in "Flow-Mediated On-Surface Reconstitution of G-Protein Coupled Receptors for Applications in Surface Plasmon Resonance Biosensors", Anal. Biochem. 300 (2002), 132-138 beschrieben, eingesetzt werden. Weitere Testmethoden auf MCH-Rezeptor antagonistische Aktivität sind in den einleitend genannten Literaturstellen und Patentdokumenten enthalten, deren Beschreibung der Testmethoden hiermit in diese Anmeldung aufgenommen wird.

**MCH-1 Rezeptorbindungstest**

| | |
|---|---|
| Methode: | MCH Bindung an hMCH-1 R transfizierten Zellen |
| Spezies: | Human |
| Testzelle: | hMCH-1 R stabil-transfiziert in CHO/Galpha16 Zellen |
| Resultate: | IC50 Werte |

Membranen aus mit humanem hMCH-1 R stabil-transfizierten CHO/Galpha16 Zellen werden mit Hilfe einer Spritze resuspendiert (Nadel 0.6 x 25 mm) und in Testpuffer (50 mM HEPES, 10 mM $MgCl_2$, 2 mM EGTA, pH 7.00; 0.1 % Rinderserum-Albumin (Protease-frei), 0.021 % Bacitracin, 1 μg/mL Aprotinin, 1 μg/mL Leupeptin and 1 μM Phosphoramidon) auf eine Konzentration von 5 bis 15 μg/mL verdünnt. 200 Mikroliter dieser Membranfraktion (enthält 1 bis 3 μg Protein) werden für 60 Minuten bei Raumtemperatur mit 100 pM $^{125}$I-tyrosyl melanine concentrating hormone ($^{125}$I-MCH kommerziell erhältlich von NEN) und steigende Konzentrationen der Testverbindung in einem Endvolumen von 250 Mikroliter inkubiert. Nach der Inkubation wird die Reaktion unter Benutzung eines Zellernters durch 0.5% PEI behandelte Glasfiberfilter (GF/B, Unifilter Packard) filtriert. Die membrangebundene auf dem Filter retenierte Radioaktivität wird anschliessend nach Zugabe von Szintillatorsubstanz (Packard Microscint 20) in einem Messgerät bestimmt (TopCount von Packard).
Die nichtspezifische Bindung ist definiert als gebundene Radioaktivität in Gegenwart von 1 Mikromolar MCH während der Inkubationsperiode.
Die Analyse der Konzentration-Bindungskurve erfolgt unter der Annahme einer Rezeptorbindungsstelle.

Standard:

Nichtmarkiertes MCH kompetiert mit markiertem $^{125}$I-MCH um die Rezeptorbindung mit einem IC50 Wert zwischen 0.06 bis 0.15 nM.
Der KD-Wert des Radioliganden beträgt 0.156 nM.

**MCH-1 Rezeptor-gekoppelter $Ca^{2+}$ Mobilisierungstest**

| | |
|---|---|
| Methode: | Calciummobilisierungstest mit humanem MCH (FLIPR$^{384}$) |
| Spezies: | Human |
| Testzellen: | Mit hMCH-R1 stabil-transfizierte CHO/ Galpha 16 Zellen |
| Resultate: : | 1. Messung: % Stimulation der Referenz (MCH $10^{-6}$M) |
| | 2. Messung: pKB Wert |

| | | |
|---|---|---|
| Reagentien: | HBSS (10x) | (GIBCO) |
| | HEPES Puffer (1 M) | (GIBCO) |
| | Pluronic F-127 | (Molecular Probes) |
| | Fluo-4 | (Molecular Probes) |
| | Probenecid | (Sigma) |
| | MCH | (Bachem) |
| | Rinderserum-Albumin (Protease frei) | (Serva) |
| | DMSO | (Serva) |
| | Ham's F12 | (BioWhittaker) |
| | FCS | (BioWhittaker) |
| | L-Glutamine | (GIBCO) |
| | Hygromycin B | (GIBCO) |
| | PENStrep | (BioWhittaker) |
| | Zeocin | (Invitrogen) |

Klonale CHO/Galpha16 hMCH-R1 Zellen werden in Ham's F12 Zellkulturmedium (mit L-Glutamine; BioWhittaker; Cat.Nr.: BE12-615F) kultiviert. Dieses enthält pro 500 mL 10% FCS, 1 % PENStrep, 5 mL L-Glutamine (200 mM Stocklösung), 3 mL Hygromycin B (50 mg/mL in PBS) and 1.25 mL Zeocin (100 µg/mL Stocklösung). Einen Tag vor dem Experiment werden die Zellen auf 384-Well-Mikrotiterplatte (schwarzwandig mit durchsichtigem Boden, Hersteller: Costar) in einer Dichte von 2500 Zellen pro Kavität ausplattiert und in dem obenbeschriebenen Medium über Nacht bei 37˚C, 5% $CO_2$ und 95% relativer Luftfeuchtigkeit kultiviert. Am Tag des Experiments werden die Zellen mit Zellkulturmedium, dem 2 mM Fluo-4 and 4.6 mM Probenicid zugesetzt ist, bei 37˚C für 45 Minuten inkubiert. Nach der Beladung mit Fluoreszenzfarbstoff werden die Zellen viermal mit Hanks Pufferlösung (1 x HBSS, 20 mM HEPES), welche mit 0.07% Probenicid versetzt ist, gewaschen. Die Testsubstanzen werden in Hanks Pufferlösung, versetzt mit 2.5% DMSO, verdünnt. Die Hintergrundsfluoreszenz nichtstimulierter Zellen wird in Gegenwart von Substanz in der 384-Well-Mikrotiterplatte fünf Minuten nach dem letzten Waschschritt im FLIPR$^{384}$-Gerät (Molecular Devices; Anregungswellenlänge: 488 nm; Emissionwellenlänge: bandpass 510 bis 570 nm) gemessen. Für die Zellstimulation wird MCH in Hanks Puffer mit 0.1% BSA verdünnt, 35 Minuten nach dem letzten Waschschritt zur 384-Well-Zellkulturplatte pipettiert und die MCH-stimulierte Fluoreszenz anschliessend im FLIPR$^{384}$ Gerät gemessen.

**Datenanalyse:**

1. Messung: Die zelluläre $Ca^{2+}$-Mobilisierung wird als Peak der relativen Fluoreszenz abzüglich Hintergrund gemessen und als Prozentanteil des Maximalsignals der Referenz (MCH $10^{-6}$M) ausgedrückt. Diese Messung dient der Identifizierung eines möglichen agonistischen Effektes einer Testsubstanz.
2. Messung: Die zelluläre $Ca^{2+}$-Mobilisierung wird als Peak der relativen Fluoreszenz abzüglich Hintergrund gemessen und als Prozentanteil des Maximalsignals der Referenz (MCH $10^{-6}$M, Signal wird auf 100% normiert)

ausgedrückt. Die EC50-Werte der MCH Dosis-Wirkungskurve mit und ohne Testsubstanz (definierte Konzentration) werden durch das GraphPad Prism 2.01 Kurvenprogramm graphisch ermittelt. MCH-Antagonisten bewirken in der erstellten Graphik eine Rechtsverschiebung der MCH-Stimulationskurve.

Die Inhibition wird pKB-Wert ausgedrückt:

$$pKB=\log(EC_{50(Testsubstanz+MCH)} / EC_{50(MCH)} -1) -\log c_{(Testsubstanz)}$$

Die erfindungsgemäßen Verbindungen, einschließlich deren Salze, zeigen in den genannten Tests eine MCH-Rezeptor antagonistische Wirkung. Unter Anwendung des zuvor beschriebenen MCH-1 Rezeptor-Bindungstests wird eine antagonistische Aktivität in einem Dosisbereich von etwa $10^{-10}$ bis $10^{-5}$ M, insbesondere von $10^{-9}$ bis $10^{-6}$ M, erhalten.

Folgende IC50 Werte wurden mit Hilfe des zuvor beschriebenen MCH-1 Rezeptor-Bindungstests bestimmt:

| Verbindung gemäß Beispiel-Nr. | Struktur | IC50-Wert |
|---|---|---|
| 12 | 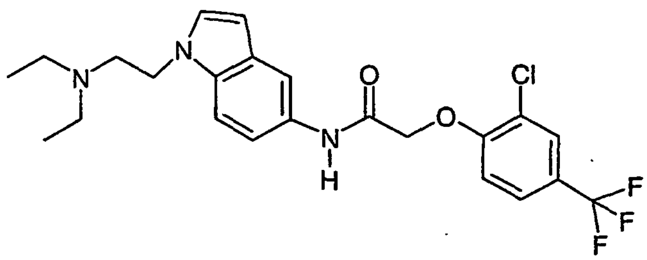 | 41 nM |
| 34 | 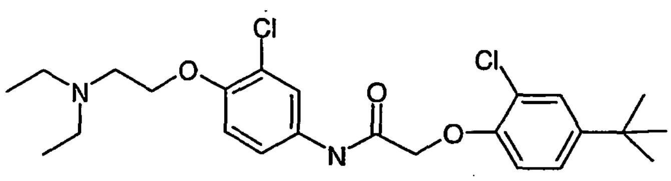 | 17 nM |

Nachfolgend werden Beispiele zu Darreichungsformen beschrieben, worin die Angabe "Wirkstoff" eine oder mehrere erfindungsgemäße Verbindungen, einschließlich deren Salze bedeutet. Im Falle einer der beschriebenen Kombinationen mit einem oder mehreren weiteren Wirksubstanzen umfasst der Begriff "Wirkstoff" auch die weiteren Wirksubstanzen.

Beispiel A

Kapseln zur Pulverinhalation mit 1 mg Wirkstoff

| Zusammensetzung: | |
|---|---|
| 1 Kapsel zur Pulverinhalation enthält: | |
| Wirkstoff | 1.0 mg |
| Milchzucker | 20.0 mg |
| Hartgelatinekapseln | 50.0 mg |
| | 71.0 mg |

Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

Beispiel B

Inhalationslösung für Respimat® mit 1 mg Wirkstoff

| Zusammensetzung: | |
|---|---|
| 1 Hub enthält: | |
| Wirkstoff | 1.0 mg |
| Benzalkoniumchlorid | 0.002 mg |
| Dinatriumedetat | 0.0075 mg |
| Wasser gereinigt ad | 15.0 μl |

Herstellungsverfahren:

Der Wirkstoff und Benzalkoniumchlorid werden in Wasser gelöst und in Respimat®-Kartuschen abgefüllt.

Beispiel C

Inhalationslösung für Vernebler mit 1 mg Wirkstoff

| Zusammensetzung: | |
|---|---|
| 1 Fläschchen enthält: | |
| Wirkstoff | 0.1 g |
| Natriumchlorid | 0.18 g |
| Benzalkoniumchlorid | 0.002 g |
| Wasser gereinigt ad | 20.0 ml |

Herstellungsverfahren:

Wirkstoff, Natriumchlorid und Benzalkoniumchlorid werden in Wasser gelöst.

Beispiel D

Treibgas-Dosieraerosol mit 1 mg Wirkstoff

| Zusammensetzung: | |
|---|---|
| 1 Hub enthält: | |
| Wirkstoff | 1.0 mg |
| Lecithin | 0.1 % |
| Treibgas ad | 50. μl |

Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

Beispiel E

Nasalspray mit 1 mg Wirkstoff

| Zusammensetzung: | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

Herstellungsverfahren:

Der Wirkstoff und die Hilfsstoffe werden in Wasser gelöst und in ein entsprechendes Behältnis abgefüllt.

Beispiel F

Injektionslösung mit 5 mg Wirksubstanz pro 5 ml

| Zusammensetzung: | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

Beispiel G

Iniektionslösung mit 100 mg Wirksubstanz pro 20 ml

| Zusammensetzung: | |
|---|---|
| Wirksubstanz | 100 mg |
| Monokaliumdihydrogenphosphat = $KH_2PO_4$ | 12 mg |
| Dinatriumhydrogenphosphat = $Na_2HPO_4 \cdot 2H_2O$ | 2 mg |
| Natriumchlorid | 180 mg |
| Human-Serum-Albumin | 50 mg |
| Polysorbat 80 | 20 mg |
| Wasser für Injektionszwecke ad | 20 ml |

Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf

Ansatzvolumen auffüllen; in Ampullen abfüllen.

Beispiel H

Lyophilisat mit 10 mg Wirksubstanz

| Zusammensetzung: | |
|---|---|
| Wirksubstanz | 10 mg |
| Mannit | 300 mg |
| Human-Serum-Albumin | 20 mg |

Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

| Lösungsmittel für Lyophilisat: | |
|---|---|
| Polysorbat 80 = Tween 80 | 20 mg |
| Mannit | 200 mg |
| Wasser für Injektionszwecke ad | 10 ml |

Herstellung:

Polysorbat 80 und Mannit in Wasser für Injektionszwecke (Wfl) auflösen; in Ampullen abfüllen.

Beispiel I

Tabletten mit 20 mg Wirksubstanz

| Zusammensetzung: | |
|---|---|
| Wirksubstanz | 20 mg |
| Lactose | 120 mg |
| Maisstärke | 40 mg |
| Magnesiumstearat | 2 mg |
| Povidon K 25 | 18 mg |

Herstellung:

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

Beispiel J

Kapseln mit 20 mg Wirksubstanz

| Zusammensetzung: | |
|---|---|
| Wirksubstanz | 20 mg |
| Maisstärke | 80 mg |
| Kieselsäure. hochdispers | 5 mg |

(fortgesetzt)

| | |
|---|---|
| Magnesiumstearat | 2.5 mg |

Herstellung:

Wirksubstanz, Maisstärke und Kieselsäure homogen mischen; mit Magnesiumstearat mischen; Mischung auf einer Kapselfüllmaschine in Hartgelatine-Kapseln Grösse 3 abfüllen.

Beispiel K

Zäpfchen mit 50 mg Wirksubstanz

| Zusammensetzung: | |
|---|---|
| Wirksubstanz | 50 mg |
| Hartfett (Adeps solidus) q.s. ad | 1700 mg |

Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgiessen.

Beispiel L

Injektionslösung mit 10 mg Wirksubstanz pro 1 ml

| Zusammensetzung: | |
|---|---|
| Wirksubstanz | 10 mg |
| Mannitol | 50 mg |
| Human-Serum-Albumin | 10 mg |
| Wasser für Injektionszwecke ad | 1 ml |

Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

**Patentansprüche**

**1.** Amid-Verbindungen der allgemeinen Formel I

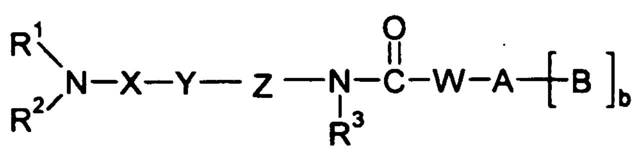

I

in der

$R^1$, $R^2$ unabhängig voneinander H, eine gegebenenfalls mit dem Rest $R^{11}$ substituierte $C_{1-8}$-Alkyl- oder $C_{3-7}$-Cy-

cloalkyl-Gruppe, wobei eine $-CH_2$-Gruppe in Position 3 oder 4 einer 5, 6 oder 7-gliedrigen Cycloalkylgruppe durch -O-, -S- oder $-NR^{13}$- ersetzt sein kann, oder ein gegebenenfalls mit dem Rest $R^{12}$ ein- oder mehrfach und/oder mit Nitro einfach substituierter Phenyl- oder Pyridinylrest, mit der Maßgabe, dass mindestens einer der Reste $R^1$, $R^2$ eine von H verschiedene Bedeutung aufweist, oder
$R^1$ und $R^2$ bilden eine $C_{2-8}$-Alkylen-Brücke, in der

- ein oder zwei $-CH_2$-Gruppen unabhängig voneinander durch
- CH=N- oder -CH=CH- ersetzt sein können und/oder
- ein oder zwei $-CH_2$-Gruppen unabhängig voneinander durch -O-,
- S-, -SO, $-(SO_2)$-, $-C=N-O-R^{18}$,-CO-, $-C(=CH_2)$- oder $-NR^{13}$-derart ersetzt sein können, dass Heteroatome nicht unmittelbar miteinander verbunden sind,

wobei in der zuvor definierten Alkylen-Brücke ein oder mehrere H-Atome durch $R^{14}$ ersetzt sein können, und wobei die zuvor definierte Alkylen-Brücke mit einer oder zwei gleichen oder verschiedenen carbo- oder heterocyclischen Gruppen Cy derart substituiert sein kann, dass die Bindung zwischen der Alkylenbrücke und der Gruppe Cy

- über eine Einfach- oder Doppelbindung,
- über ein gemeinsames C-Atom unter Ausbildung eines spirocyclischen Ringsystems,
- über zwei gemeinsame, benachbarte C- und/oder N-Atome unter Ausbildung eines kondensierten bicyclischen Ringsystems oder
- über drei oder mehrere C- und/oder N-Atome unter Ausbildung eines verbrückten Ringsystems erfolgt,

$R^3$ H, $C_{1-6}$-Alkyl, $C_{3-7}$-Cycloalkyl oder $C_{3-7}$-Cycloalkyl-$C_{1-4}$-Alkyl-,
X eine unverzweigte $C_{1-4}$-Alkylen-Brücke und für den Fall, dass die Gruppe Y über ein C-Atom mit X verbunden ist, auch $-CH_2-CH=CH-$, $-CH_2-C{\equiv}C-$, $C_{2-4}$-Alkylenoxy oder $C_{2-4}$-Alkylen-$NR^4$- bedeutet,
wobei die Brücke X mit $R^1$ unter Einschluss des mit $R^1$ und X verbundenen N-Atoms unter Ausbildung einer heterocyclischen Gruppe verbunden sein kann, wobei die Brücke X zusätzlich auch mit $R^2$ unter Einschluss des mit $R^2$ und X verbundenen N-Atoms unter Ausbildung einer heterocyclischen Gruppe verbunden sein kann, und
wobei ein C-Atom mit $R^{10}$ und/oder ein oder zwei C-Atome jeweils mit einem oder zwei gleichen oder verschiedenen Substituenten ausgewählt aus $C_{1-6}$-Alkyl-, $C_{2-6}$-Alkenyl-, $C_{2-6}$-Alkinyl-, $C_{3-7}$-Cycloalkyl, $C_{3-7}$-Cycloalkyl-$C_{1-3}$-alkyl-, $C_{4-7}$-Cycloalkenyl und $C_{4-7}$-Cycloalkenyl-$C_{1-3}$-alkyl- substituiert sein können, wobei zwei Alkyl- und/oder Alkenyl-Substituenten unter Ausbildung eines carbocyclischen Ringsystems miteinander verbunden sein können, mit der Maßgabe, dass die Gruppe X in der Bedeutung $C_{2-4}$-Alkylenoxy keinen Hydroxy-Substituenten aufweist; und
W $-CR^{6a}R^{6b}-O-$, $-CR^{7a}=CR^{7c}-$, $-CR^{6a}R^{6b}-NR^8-$, $-CR^{7a}R^{7b}-CR^{7c}R^{7d}-$ oder $-NR^8-CR^{6a}R^{6b}-$,
Z eine Einfachbindung, $C_{1-4}$-Alkylen, worin zwei benachbarte C-Atome mit einer zusätzlichen $C_{1-4}$-Alkylen-Brücke miteinander verbunden sein können,
wobei ein C-Atom der Alkylen-Brücke mit $R^{10}$ und/oder ein oder zwei C-Atome unabhängig voneinander mit einem oder zwei gleichen oder verschiedenen $C_{1-6}$-Alkyl-Resten substituiert sein können, wobei zwei Alkylreste unter Ausbildung eines carbocyclischen Rings miteinander verbunden sein können, und
Y ausgewählt ist aus der Gruppe der bivalenten cyclischen Gruppen

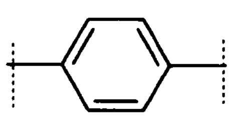 , 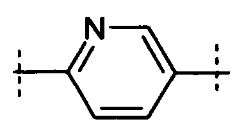 ,

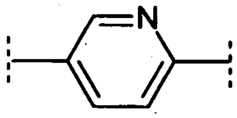 , 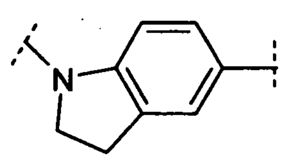 ,

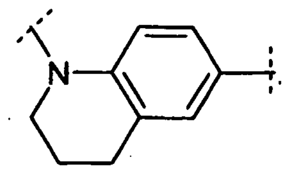 , 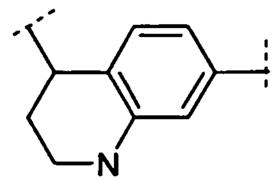 ,

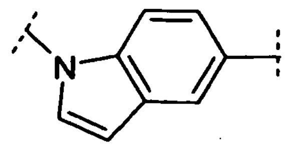 , 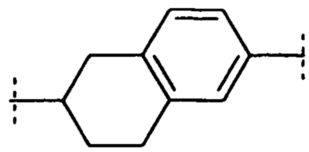 ,

wobei die zuvor genannten cyclischen Gruppen ein- oder mehrfach an ein oder mehreren C-Atomen mit $R^{20}$, im Falle einer Phenylgruppe auch zusätzlich einfach mit Nitro, und/oder eine oder mehrere NH-Gruppen mit $R^{21}$ substituiert sein können, und
A eine der für Cy angegebenen Bedeutungen,
B eine der für Cy angegebenen Bedeutungen,
b den Wert 0 oder 1,
Cy eine carbo- oder heterocyclische Gruppe ausgewählt aus einer der folgenden Bedeutungen

- eine gesättigte 3- bis 7-gliedrige carbocyclische Gruppe,
- eine ungesättigte 4- bis 7-gliedrige carbocyclische Gruppe,
- eine Phenyl-Gruppe,
- eine gesättigte 4- bis 7-gliedrige oder ungesättigte 5- bis 7-gliedrige heterocyclische Gruppe mit einem N-, O- oder S-Atom als Heteroatom,
- eine gesättigte oder ungesättigte 5- bis 7-gliedrige heterocyclische Gruppe mit zwei oder mehreren N-Atomen oder mit einem oder zwei N-Atomen und einem O- oder S-Atom als Heteroatome,
- eine aromatische heterocyclische 5- oder 6-gliedrige Gruppe mit einem oder mehreren gleichen oder verschiedenen Heteroatomen ausgewählt aus N, O und/oder S,

wobei die zuvor angeführten 4-, 5-, 6- oder 7-gliedrigen Gruppen über zwei gemeinsame, benachbarte C-Atome mit einen Phenyl- oder Pyridin-Ring kondensiert verbunden sein können, und
wobei in den zuvor genannten 5-, 6- oder 7-gliedrigen Gruppen eine oder zwei nicht benachbarte $-CH_2$-Gruppen unabhängig voneinander durch eine -CO-, $-C(=CH_2)-$, -(SO)- oder $-(SO_2)$-Gruppe ersetzt sein können, und
wobei die zuvor angeführten gesättigten 6- oder 7-gliedrigen Gruppen auch als verbrückte Ringsysteme mit einer Imino-, N-($C_{1-4}$-alkyl)-imino-, Methylen-, $C_{1-4}$-Alkyl-methylen- oder Di-($C_{1-4}$-alkyl)-methylen-Brücke vorliegen können, und
wobei die zuvor genannten cyclischen Gruppen ein- oder mehrfach an ein oder mehreren C-Atomen mit $R^{20}$, im Falle einer Phenylgruppe auch zusätzlich einfach mit Nitro, und/oder ein oder mehrere NH-Gruppen mit $R^{21}$ substituiert sein können,

$R^4$ H, $C_{1-6}$-Alkyl oder $C_{2-6}$-Alkenyl,
$R^{6a}$, $R^{6b}$ H, $C_{1-4}$-Alkyl oder $CF_3$,
$R^{7a}$, $R^{7b}$, $R^{7c}$, $R^{7d}$ H, F, Cl, $C_{1-4}$-Alkyl oder $CF_3$,
$R^8$ H, $C_{1-4}$-Alkyl, $C_{3-7}$-Cycloalkyl oder $C_{3-7}$-Cycloalkyl-$C_{1-3}$-alkyl-,
$R^{10}$ Hydroxy, ω-Hydroxy-$C_{1-3}$-alkyl, $C_{1-4}$-Alkoxy, ω-($C_{1-4}$-Alkoxy)-$C_{1-3}$-alkyl-, Carboxy, $C_{1-4}$-Alkoxycarbonyl, Amino, $C_{1-4}$-Alkyl-amino-, Di-($C_{1-4}$-alkyl)-amino-, Cyclo-$C_{3-6}$-alkylenimino-, Amino-$C_{1-3}$-alkyl-, $C_{1-4}$-Alkyl-amino-$C_{1-3}$-alkyl, Di-($C_{1-4}$-alkyl)-amino-$C_{1-3}$-alkyl, Cyclo-$C_{3-6}$-$_6$-alkylenimino-$C_{1-3}$-alkyl-, Amino-$C_{1-3}$-alkoxy-, $C_{1-4}$-Alkyl-amino-$C_{1-3}$-alkoxy-, Di-($C_{1-4}$-alkyl)-amino-$C_{1-3}$-alkoxy- oder Cyclo-$C_{3-6}$-alkylenimino-$C_{1-3}$-alkoxy-, Aminocarbonyl-, $C_{1-4}$-Alkyl-aminocarbonyl-, Di-($C_{1-4}$-alkyl)-aminocarbonyl- oder Cyclo-$C_{3-6}$-alkylenimino-carbonyl-,
$R^{11}$ $C_{2-6}$-Alkenyl, $C_{2-6}$-Alkinyl, $R^{15}$-O-, $R^{15}$-O-CO-, $R^{15}$-CO-O-, $R^{16}R^{17}N$-, $R^{18}R^{19}N$-CO- oder Cy-,
$R^{12}$ eine der für $R^{20}$ angegebenen Bedeutungen,

$R^{13}$ eine der für $R^{17}$ angegebenen Bedeutungen, ausgenommen Carboxy,

$R^{14}$ Halogen, $C_{1-6}$-Alkyl, $C_{2-6}$-Alkenyl, $C_{2-6}$-Alkinyl, $R^{15}$-O-, $R^{15}$-O-CO-, $R^{15}$-CO-, $R^{15}$-CO-O-, $R^{16}R^{17}$N-, $R^{18}R^{19}$N-CO-, $R^{15}$-O-$C_{1-3}$-alkyl , $R^{15}$-O-CO-$C_{1-3}$-alkyl, $R^{15}$-O-CO-NH-, $R^{15}$-$SO_2$-NH-, $R^{15}$-O-CO-NH-$C_{1-3}$-alkyl-, $R^{15}$-$SO_2$-NH-$C_{1-3}$-alkyl-, $R^{15}$-CO-$C_{1-3}$-alkyl-, $R^{15}$-CO-O-$C_{1-3}$-alkyl-, $R^{16}R^{17}$N-$C_{1-3}$-alkyl-, $R^{18}R^{19}$N-CO-$C_{1-3}$-alkyl- oder Cy-$C_{1-3}$-alkyl-,

$R^{15}$ H, $C_{1-4}$-Alkyl, $C_{3-7}$-Cycloalkyl, $C_{3-7}$-Cycloalkyl-$C_{1-3}$-alkyl-, Phenyl, Phenyl-$C_{1-3}$-alkyl-, Pyridinyl oder Pyridinyl-$C_{1-3}$-alkyl-,

$R^{16}$ H, $C_{1-6}$-Alkyl, $C_{3-7}$-Cycloalkyl, $C_{3-7}$-Cycloalkyl-$C_{1-3}$-alkyl-, $C_{4-7}$-Cycloalkenyl, $C_{4-7}$-Cycloalkenyl-$C_{1-3}$-alkyl-, ω-Hydroxy-$C_{2-3}$-alkyl-, ω-($C_{1-4}$-Alkoxy)-$C_{2-3}$-alkyl-, Amino-$C_{2-6}$-alkyl-, $C_{1-4}$-Alkyl-Amino-$C_{2-6}$-alkyl-, Di-($C_{1-4}$-alkyl)-Amino-$C_{2-6}$-alkyl- oder Cyclo-$C_{3-6}$-alkylenimino-$C_{2-6}$-alkyl-,

$R^{17}$ eine der für $R^{16}$ angegebenen Bedeutungen oder Phenyl, Phenyl-$C_{1-3}$-alkyl, Pyridinyl, Dioxolan-2-yl, -CHO, $C_{1-4}$-Alkylcarbonyl, Carboxy, Hydroxycarbonyl-$C_{1-3}$-alkyl-, $C_{1-4}$-Alkoxycarbonyl-, $C_{1-4}$-Alkoxycarbonyl-$C_{1-3}$-alkyl-,$C_{1-4}$-Alkylcarbonylamino-$C_{2-3}$-alkyl-,N-($C_{1-4}$-Alkylcarbonyl)-N-($C_{1-4}$-Alkyl)-amino-$C_{2-3}$-alkyl-,$C_{1-4}$-Alkylsulfonyl, $C_{1-4}$-Alkylsulfonylamino-$C_{2-3}$-alkyl oder N-($C_{1-4}$-Alkylsulfonyl)-N($C_{1-4}$-Alkyl)-amino-$C_{2-3}$-alkyl-,

$R^{18}$, $R^{19}$ unabhängig voneinander H oder $C_{1-6}$-Alkyl,

$R^{20}$ Halogen, Hydroxy, Cyano, $C_{1-6}$-Alkyl, $C_{2-6}$-Alkenyl, $C_{2-6}$-Alkinyl, $C_{3-7}$-Cycloalkyl, $C_{3-7}$-Cycloalkyl- $C_{1-3}$-alkyl-, Hydroxy-$C_{1-4}$-alkyl, $R^{22}$-$C_{1-3}$-alkyl oder eine der für $R^{22}$ angegebenen Bedeutungen,

$R^{21}$ $C_{1-4}$-Alkyl, ω-Hydroxy-$C_{2-3}$-alkyl, ω-$C_{1-4}$-Alkoxy-$C_{2-6}$-alkyl, ω-$C_{1-4}$-Alkyl-amino-$C_{2-6}$-alkyl-, ω-Di-($C_{1-4}$-alkyl)-amino-$C_{2-6}$-alkyl-, ω-Cyclo-$C_{3-6}$-alkylenimino-$C_{2-6}$-alkyl-, Phenyl-$C_{1-3}$-alkyl-, $C_{1-4}$-Alkyl-carbonyl, $C_{1-4}$-Alkoxy-carbonyl oder $C_{1-4}$-Alkylsulfonyl,

$R^{22}$ Phenyl-$C_{1-3}$-alkoxy, OHC-, HO-N=HC-, $C_{1-4}$-Alkoxy-N=HC-, $C_{1-4}$-Alkoxy, $C_{1-4}$-Alkylthio, Carboxy, $C_{1-4}$-Alkylcarbonyl, $C_{1-4}$-Alkoxycarbonyl, Aminocarbonyl, $C_{1-4}$-Alkylaminocarbonyl, Di-($C_{1-4}$-alkyl)-aminocarbonyl, Cyclo-$C_{3-6}$-alkyl-amino-carbonyl-, Cyclo-$C_{3-6}$-alkylenimino-carbonyl, Cyclo-$C_{3-6}$-alkylenimino-$C_{2-4}$-alkyl-aminocarbonyl, Phenyl-amino-carbonyl, $C_{1-4}$-Alkyl-sulfonyl, $C_{1-4}$-Alkyl-sulfinyl, $C_{1-4}$-Alkyl-sulfonylamino-, Amino-, $C_{1-4}$-alkylamino-, Di-($C_{1-4}$-alkyl)-amino-, $C_{1-4}$-Alkyl-carbonyl-amino-, Cyclo-$C_{3-6}$-alkylenimino-, Phenyl-$C_{1-3}$-alkylamino- oder N-($C_{1-4}$-Alkyl)-phenyl-$C_{1-3}$-alkylamino-, Acetylamino-, Propionylamino-, Phenylcarbonylamino, Phenylcarbonylmethylamino, Hydroxy-alkylaminocarbonyl, (4-Morpholinyl)carbonyl, (1-Pyrrolidinyl)-carbonyl, (1-Piperidinyl)carbonyl, (Hexahydro-1-azepinyl)carbonyl, (4-Methyl-1-piperazinyl)carbonyl, Methylendioxy, Aminocarbonylamino- oder Alkylaminocarbonylamino-,

wobei in den zuvor genannten Gruppen und Resten, insbesondere in insbesondere A, B, W, X, Y, Z, $R^1$ bis $R^4$, $R^{6a}$, $R^{6b}$, $R^{7a}$, $R^{7b}$, $R^{7c}$, $R^{7d}$, $R^8$, $R^{10}$ bis $R^{22}$, jeweils ein oder mehrere C-Atome zusätzlich ein- oder mehrfach mit F und/oder jeweils ein oder zwei C-Atome unabhängig voneinander zusätzlich einfach mit Cl oder Br und/oder jeweils ein oder mehrere Phenyl-Ringe unabhängig voneinander zusätzlich ein, zwei oder drei Substituenten ausgewählt aus der Gruppe F, Cl, Br, I, $C_{1-4}$-Alkyl, $C_{1-4}$-Alkoxy-, Difluormethyl-, Trifluormethyl-, Hydroxy-, Amino-, $C_{1-3}$-alkylamino-, Di-($C_{1-3}$-alkyl)-amino-, Acetylamino-, Aminocarbonyl-, Cyano, Difluormethoxy-, Trifluormethoxy-, Amino-$C_{1-3}$-alkyl-, $C_{1-3}$-Alkylamino-$C_{1-3}$-alkyl- und Di-($C_{1-3}$-Alkyl)-amino-$C_{1-3}$-alkyl- aufweisen und/oder einfach mit Nitro substituiert sein können, und

das H-Atom einer vorhandenen Carboxygruppe oder ein an ein N-Atom gebundenes H-Atom jeweils durch einen in-vivo abspaltbaren Rest ersetzt sein

kann, bedeuten,

deren Tautomere, deren Diastereomere, deren Enantiomere, deren Gemische und deren Salze,

mit folgenden Maßgaben (M2) und (M3)

(M2) für den Fall, wenn W -CH=CH- bedeutet und Y eine Phenylen-Gruppe und Z eine Einfachbindung ist, dann die Brücken X und Z an dem Phenylen-Ring der Gruppe Y in para-Stellung zueinander stehen und mindestens eine der folgenden Bedingungen erfüllt ist:

(a) die Gruppe Y in der Bedeutung Phenylen mindestens einfach substituiert ist,
(b) b den Wert 0 besitzt und der Rest A mindestens zweifach substituiert ist,
(c) b den Wert 1 besitzt;

(M3) dass folgende Einzelverbindungen nicht umfasst sind:

N-[4-(2-Diethylamino-ethoxy)-phenyl]-3-phenyl-propionamid,
N-[4-(2-Morpholin-4-ylethoxy)-phenyl]-3-phenyl-propionamid,
3-(4-Chlor-phenyl)-N-{2-[4-(2-diethylamino-ethoxy)-phenyl]-ethyl}-acrylamid,
N- {2-[3-(4- {2-[2-(4- Chlor- phenoxy)-acetylamino]-ethyl}-phenoxy)- 2- hydroxy- propylamino]-ethyl}-isobutyramid, Cyclopentancarbonsäure {2-[3-(4-{2-[2-(4-chlor-phenoxy)-acetylamino]-ethyl}-phenoxy)-2-hydroxy-propylamino]-ethyl}-amid,
2-(4- Chlor- phenoxy)-N-(2- {4-[2- hydroxy- 3-(2- phenylacetylamino- ethylamino)-propoxy]-phenyl}-ethyl)-acetamid.

**2.** Amid-Verbindungen gemäß Anspruch 1, **dadurch gekennzeichnet, dass**

$R^1$, $R^2$ unabhängig voneinander H, eine gegebenenfalls mit dem Rest $R^{11}$ substituierte $C_{1-8}$-Alkyl- oder $C_{3-7}$-Cycloalkyl-Gruppe, oder ein gegebenenfalls mit dem Rest $R^{12}$ ein- oder mehrfach und/oder mit Nitro einfach substituierter Phenylrest, mit der Maßgabe, dass mindestens einer der Reste $R^1$, $R^2$ eine von H verschiedene Bedeutung aufweist, oder
$R^1$ und $R^2$ bilden eine $C_{2-8}$-Alkylen-Brücke, in der

- ein oder zwei $-CH_2$-Gruppen unabhängig voneinander durch
- CH=N- oder -CH=CH- ersetzt sein können und/oder
- ein oder zwei $-CH_2$-Gruppen unabhängig voneinander durch -O-,
- S-, -CO-, $-C(=CH_2)$- oder $-NR^{13}$- derart ersetzt sein können, dass Heteroatome nicht unmittelbar miteinander verbunden sind,

wobei in der zuvor definierten Alkylen-Brücke ein oder mehrere H-Atome durch $R^{14}$ ersetzt sein können, und wobei die zuvor definierte Alkylen-Brücke mit einer oder zwei gleichen oder verschiedenen carbo- oder heterocyclischen Gruppen Cy derart substituiert sein kann, dass die Bindung zwischen der Alkylenbrücke und der Gruppe Cy

- über eine Einfach- oder Doppelbindung,
- über ein gemeinsames C-Atom unter Ausbildung eines spirocyclischen Ringsystems,
- über zwei gemeinsame, benachbarte C- und/oder N-Atome unter Ausbildung eines kondensierten bicyclischen Ringsystems oder
- über drei oder mehrere C- und/oder N-Atome unter Ausbildung eines verbrückten Ringsystems erfolgt,

X eine unverzweigte $C_{1-4}$-Alkylen-Brücke und
für den Fall, dass die Gruppe Y über ein C-Atom mit X verbunden ist, auch $-CH_2-CH=CH-$, $-CH_2-C\equiv C-$, $C_{2-4}$-Alkylenoxy oder $C_{2-4}$-Alkylen-$NR^4$- bedeutet,
wobei die Brücke X mit $R^1$ unter Einschluss des mit $R^1$ und X verbundenen N-Atoms unter Ausbildung einer heterocyclischen Gruppe verbunden sein kann, und
wobei ein C-Atom mit $R^{10}$ und/oder ein oder zwei C-Atome jeweils mit einem oder zwei gleichen oder verschiedenen $C_{1-6}$-Alkyl-Resten substituiert sein können, mit der Maßgabe, dass die Gruppe X in der Bedeutung $C_{2-4}$-Alkylenoxy keinen Hydroxy-Substituenten aufweist; und
Z eine Einfachbindung, $C_{1-4}$-Alkylen, worin zwei benachbarte C-Atome mit einer zusätzlichen $C_{1-4}$-Alkylen-Brücke miteinander verbunden sein können,
wobei ein C-Atom der Alkylen-Brücke mit $R^{10}$ und/oder ein oder zwei C-Atome unabhängig voneinander mit einem oder zwei gleichen oder verschiedenen $C_{1-6}$-Alkyl-Resten substituiert sein können,und
b den Wert 0 besitzt,
$R^{10}$ Hydroxy, ω-Hydroxy-$C_{1-3}$-alkyl, $C_{1-4}$-Alkoxy, ω-($C_{1-4}$-Alkoxy)-$C_{1-3}$-alkyl-, Amino, $C_{1-4}$-Alkyl-amino-, Di-($C_{1-4}$-alkyl)-amino-, Cyclo-$C_{3-6}$-alkylenimino-, Amino-$C_{1-3}$-alkyl-, $C_{1-4}$-Alkyl-amino-$C_{1-3}$-alkyl-, Di-($C_{1-4}$-alkyl)-amino-$C_{1-3}$-alkyl-, Cyclo-$C_{3-6}$-alkylenimino-$C_{1-3}$-alkyl-, Amino-$C_{1-3}$-alkoxy, $C_{1-4}$-Alkyl-amino-$C_{1-3}$-alkoxy, Di-($C_{1-4}$-alkyl)-amino-$C_{1-3}$-alkoxy oder Cyclo-$C_{3-6}$-alkylenimino-$C_{1-3}$-alkoxy,
$R^{14}$ Halogen, $C_{1-6}$-Alkyl, $R^{15}$-O-, $R^{15}$-O-CO-, $R^{15}$-CO-, $R^{15}$-CO-O-, $R^{16}R^{17}N$-, $R^{18}R^{19}N$-CO-, $R^{15}$-O-$C_{1-3}$-alkyl-, $R^{15}$-O-CO-$C_{1-3}$-alkyl-, $R^{15}$-CO-$C_{1-3}$-alkyl-, $R^{15}$-CO-O-$C_{1-3}$-alkyl-, $R^{16}R^{17}N$-$C_{1-3}$-alkyl-, $R^{18}R^{19}N$-CO-$C_{1-3}$-alkyl- oder Cy-$C_{1-3}$-alkyl-,
$R^{15}$ H, $C_{1-4}$-Alkyl, $C_{3-7}$-Cycloalkyl, $C_{3-7}$-Cycloalkyl-$C_{1-3}$-alkyl-, Phenyl oder Phenyl-$C_{1-3}$-alkyl-,
$R^{17}$ eine der für $R^{16}$ angegebenen Bedeutungen oder Phenyl, Phenyl-$C_{1-3}$-alkyl-, $C_{1-4}$-Alkylcarbonyl, Hydroxycarbonyl-$C_{1-3}$-alkyl-, $C_{1-4}$-Alkylcarbonylamino-$C_{2-3}$-alkyl-, N-($C_{1-4}$-Alkylcarbonyl)-N-($C_{1-4}$-Alkyl)-amino-$C_{2-3}$-alkyl-, $C_{1-4}$-Alkylsulfonyl, $C_{1-4}$-Alkylsulfonylamino-$C_{2-3}$-alkyl- oder N-($C_{1-4}$-Alkylsulfonyl)-N($C_{1-4}$-Alkyl)-amino-$C_{2-3}$-alkyl-,
$R^{20}$ Halogen, Hydroxy, Cyano, $C_{1-6}$-Alkyl, $C_{3-7}$-Cycloalkyl, $C_{3-7}$-Cycloalkyl- $C_{1-3}$-alkyl-, Hydroxy-$C_{1-4}$-alkyl-, $R^{22}$-$C_{1-3}$-alkyl oder eine der für $R^{22}$ angegebenen Bedeutungen,
$R^{21}$ $C_{1-4}$-Alkyl, ω-Hydroxy-$C_{2-3}$-alkyl, ω-$C_{1-4}$-Alkoxy-$C_{2-6}$-alkyl-, ω-$C_{1-4}$-Alkyl-amino-$C_{2-6}$-alkyl-, ω-Di-($C_{1-4}$-alkyl)-amino-$C_{2-6}$-alkyl-, ω-Cyclo-$C_{3-6}$-alkylenimino-$C_{2-6}$-alkyl-, Phenyl, Phenyl-$C_{1-3}$-alkyl-, $C_{1-4}$-Alkyl-carbonyl,

Carboxy, $C_{1-4}$-Alkoxy-carbonyl oder $C_{1-4}$-Alkylsulfonyl,
$R^{22}$ Phenyl, Phenyl-$C_{1-3}$-alkoxy, $C_{1-4}$-Alkoxy, $C_{1-4}$-Alkylthio, Carboxy, $C_{1-4}$-Alkylcarbonyl, $C_{1-4}$-Alkoxycarbonyl, Aminocarbonyl, $C_{1-4}$-Alkylaminocarbonyl, Di-($C_{1-4}$-alkyl)-aminocarbonyl, Cyclo-$C_{3-6}$-alkylenimino-carbonyl, $C_{1-4}$-Alkyl-sulfonyl, $C_{1-4}$-Alkyl-sulfinyl, $C_{1-4}$-Alkyl-sulfonylamino-, Amino-, $C_{1-4}$-alkylamino, Di-($C_{1-4}$-alkyl)-amino-, Cyclo-$C_{3-6}$-alkylenimino-, Phenyl-$C_{1-3}$-alkylamino- oder N-($C_{1-4}$-Alkyl)-phenyl-$C_{1-3}$-alkylamino-, Acetylamino-, Propionylamino-, Phenylcarbonylamino-, Phenylcarbonylmethylamino-, Hydroxy-alkylaminocarbonyl, (4-Morpholinyl)carbonyl, (1-Pyrrolidinyl)-carbonyl, (1-Piperidinyl)carbonyl, (Hexahydro-1-azepinyl)carbonyl, (4-Methyl-1-piperazinyl)carbonyl, Methylendioxy, Aminocarbonyl-amino- oder Alkylaminocarbonylamino-,
wobei die Gruppen und Reste $R^3$, $R^4$, $R^{6a}$, $R^{6b}$, $R^{7a}$, $R^{7b}$, $R^{7c}$, $R^{7d}$, $R^8$, $R^{11}$, $R^{12}$, $R^{16}$, $R^{18}$, $R^{19}$, W, Y, A, Cy die in Anspruch 1 angegebene Bedeutung besitzen.

**3.** Amid-Verbindungen gemäß Anspruch 1 oder 2, **dadurch gekennzeichnet, dass** $R^1$, $R^2$ unabhängig voneinander H, $C_{1-6}$-Alkyl, $C_{3-7}$-Cycloalkyl, $C_{3-7}$-Cycloalkyl-$C_{1-3}$-alkyl-, ω-Hydroxy-$C_{2-3}$-alkyl-, ω-($C_{1-4}$-Alkoxy)-$C_{2-3}$-alkyl-, $C_{1-4}$-Alkoxycarbonyl-$C_{1-4}$-alkyl-, Carboxyl-$C_{1-4}$-alkyl-, Amino-$C_{2-4}$-alkyl-, $C_{1-4}$-Alkyl-amino-$C_{2-4}$-alkyl-, Di-($C_{1-4}$-alkyl)-amino-$C_{2-4}$-alkyl-, Cyclo-$C_{3-6}$-alkylenimino-$C_{2-4}$-alkyl-, Pyrrolidinyl, N-($C_{1-4}$-alkyl)-pyrrolidinyl, Pyrrolidinyl-$C_{1-3}$-alkyl-, N-($C_{1-4}$-alkyl)-pyrrolidinyl-$C_{1-3}$-alkyl-, Piperidinyl, N-($C_{1-4}$-alkyl)-piperidinyl, Piperidinyl-$C_{1-3}$-alkyl, N-($C_{1-4}$-alkyl)-piperidinyl-$C_{1-3}$-alkyl-, Phenyl, Phenyl-$C_{1-3}$-alkyl-, Pyridyl oder Pyridyl-$C_{1-3}$-alkyl- bedeuten,
wobei in den zuvor angegebenen Gruppen und Resten ein oder mehrere C-Atome ein- oder mehrfach mit F und/oder ein oder zwei C-Atome unabhängig voneinander einfach mit Cl oder Br substituiert sein können und
wobei der Phenyl- oder Pyridylrest ein- oder mehrfach mit dem in Anspruch 1 definierten Rest $R^{12}$ und/oder einfach mit Nitro substituiert sein kann,
mit der Maßgabe, dass mindestens einer der Reste $R^1$, $R^2$ eine von H verschiedene Bedeutung aufweist.

**4.** Amid-Verbindungen gemäß einem oder mehreren der Ansprüche 1 bis 3, **dadurch gekennzeichnet, dass** $R^1$ und $R^2$ derart eine Alkylen-Brücke gemäß Anspruch 1 bilden, dass $R^1R^2N$- eine Gruppe ausgewählt aus Azetidin, Pyrrolidin, Piperidin, Azepan, 2,5-Dihydro-1H-pyrrol, 1,2,3,6-Tetrahydro-pyridin, 2,3,4,7-Tetrahydro-1H-azepin, 2,3,6,7-Tetrahydro-1H-azepin, Piperazin, worin die freie Imin-Funktion mit $R^{13}$ substituiert ist, Piperidin-4-on-oxim, Piperidin-4-on-O-$C_{1-4}$-alkyl-oxim, Morpholin und Thiomorpholin bildet,
wobei gemäß Anspruch 1 ein- oder mehrere H-Atome durch $R^{14}$ ersetzt sein können, und/ oder die in der in Anspruch 1 angegebenen Weise mit einer oder zwei gleichen oder verschiedenen carbo- oder heterocyclischen Gruppen Cy substituiert sein kann, und/oder
wobei $R^{13}$, $R^{14}$ und Cy die in Anspruch 1 oder 2 angegebene Bedeutung besitzen.

**5.** Amid-Verbindungen gemäß einem oder mehreren der vorherigen Ansprüche, **dadurch gekennzeichnet, dass** die Gruppe

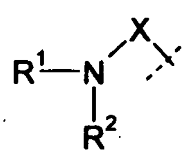

eine Bedeutung gemäß einer der folgenden Teilformeln besitzt

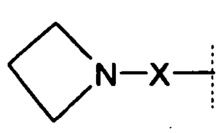 , 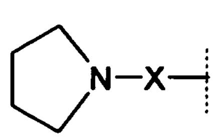 , 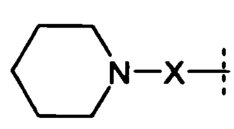 ,

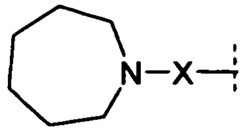 , 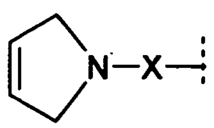 , 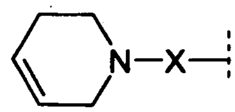 ,

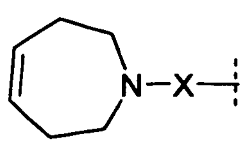 , 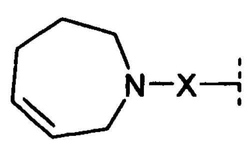 , 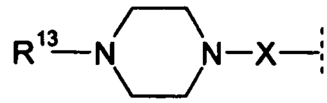 ,

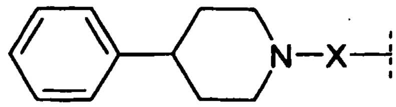 , 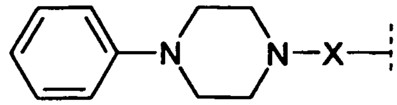 ,

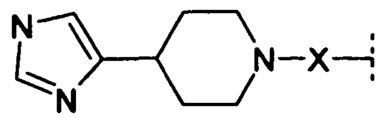 ,

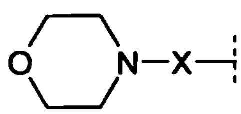 , 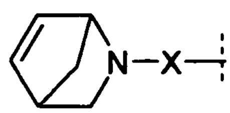 , 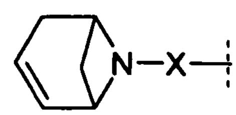 ,

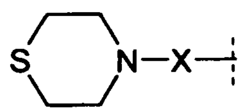 , 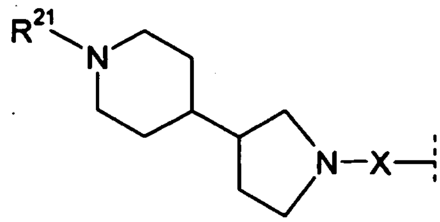 ,

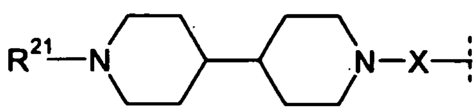 , 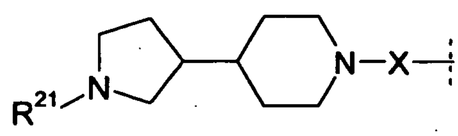 ,

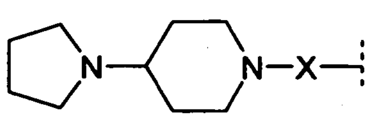 , 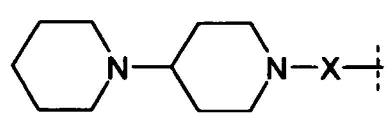 ,

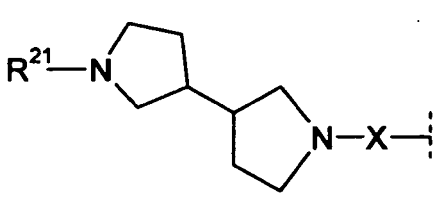 , 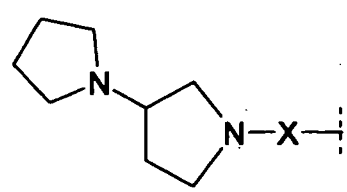 ,

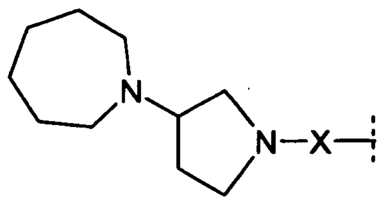 ,

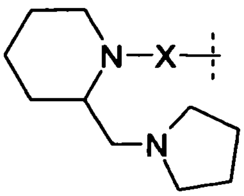 , 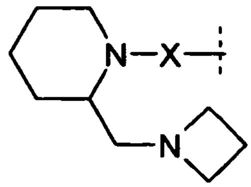 ,

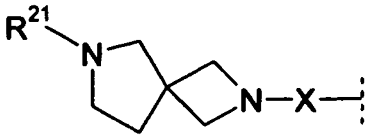 , ,

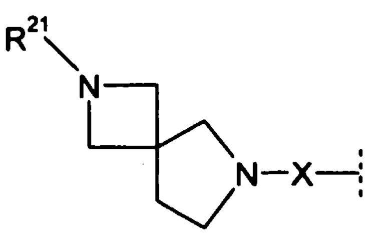 , 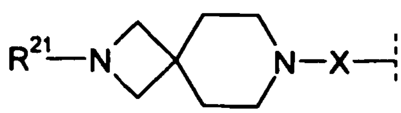 ,

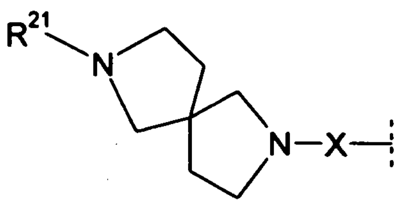 , 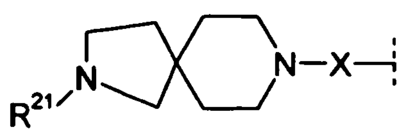 ,

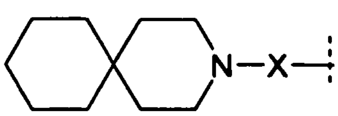 , 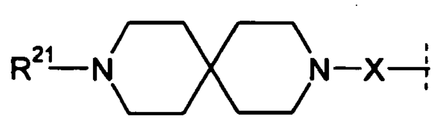 ,

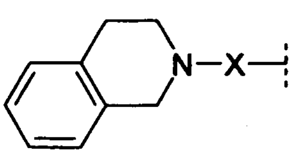 , 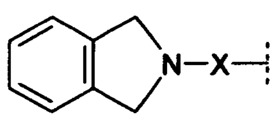 , 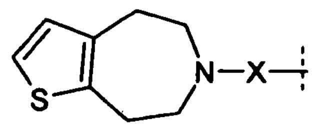 ,

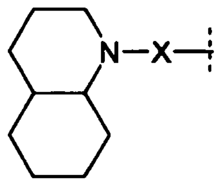 , 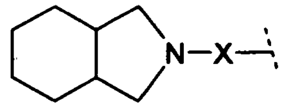 , 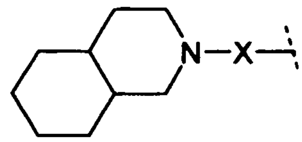 ,

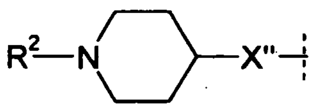 , 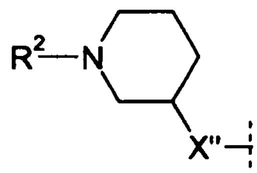 , 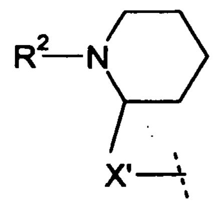 ,

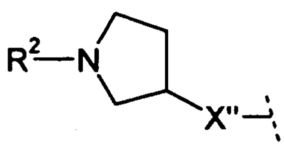 , 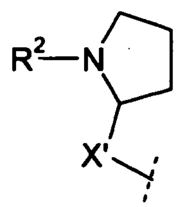 ,

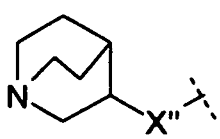 , 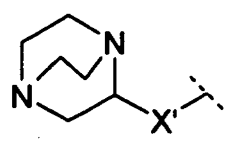 , 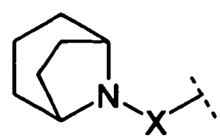 ,

worin ein- oder mehrere H-Atome des durch die Gruppe $R^1R^2N$- gebildeten Heterocyclus durch $R^{14}$ ersetzt sein können und der mit dem durch die Gruppe $R^1R^2N$- gebildeten Heterocyclus verbundene Ring ein- oder mehrfach an einem oder mehreren C-Atomen mit $R^{20}$, im Falle eines Phenyl-Rings auch zusätzlich einfach mit Nitro substituiert sein kann und

X', X" unabhängig voneinander eine Einfachbindung oder $C_{1-3}$-Alkylen und
für den Fall, dass die Gruppe Y über ein C-Atom mit X' bzw. X" verbunden ist, auch -$C_{1-3}$-Alkylen-O-, -$C_{1-3}$-Alkylen-NH- oder -$C_{1-3}$-Alkylen-N($C_{1-3}$-alkyl)-, und
wobei in den zuvor für X', X" genannten Bedeutungen jeweils ein C-Atom mit $R^{10}$ und/oder ein oder zwei C-Atome jeweils mit einem oder zwei gleichen oder verschiedenen Substituenten ausgewählt aus $C_{1-6}$-Alkyl-, $C_{2-6}$-Alkenyl-, $C_{2-6}$-Alkinyl-, $C_{3-7}$-Cycloalkyl, $C_{3-7}$-Cycloalkyl-$C_{1-3}$-alkyl-, $C_{4-7}$-Cycloalkenyl und $C_{4-7}$-Cycloalkenyl-$C_{1-3}$-alkyl- substituiert sein können, wobei zwei Alkyl- und/oder Alkenyl-Substituenten unter Ausbildung eines carbocyclischen Ringsystems miteinander verbunden sein können, und
wobei in X', X" unabhängig voneinander jeweils ein oder mehrere C-Atome ein- oder mehrfach mit F und/oder jeweils ein oder zwei C-Atome unabhängig voneinander einfach mit Cl oder Br substituiert sein können und

worin $R^2$, $R^{10}$, $R^{13}$, $R^{14}$, $R^{20}$, $R^{21}$ und X die in Anspruch 1 oder 2 angegebenen Bedeutungen besitzen.

6. Amid-Verbindungen nach einem oder mehreren der vorherigen Ansprüche, **dadurch gekennzeichnet, dass** X -$CH_2$-, -$CH_2$-$CH_2$- oder -$CH_2$-$CH_2$-$CH_2$- und
für den Fall, dass die Gruppe Y über ein C-Atom mit X verbunden ist, auch -$CH_2$-CH=CH-, -$CH_2$-C≡C-, -$CH_2$-$CH_2$-O-, -$CH_2$-$CH_2$-$CH_2$-O-,- $CH_2$-$CH_2$-$NR^4$- oder -$CH_2$-$CH_2$-$CH_2$-$NR^4$- bedeutet,

wobei die Brücke X mit $R^1$ unter Einschluss des mit $R^1$ und X verbundenen N-Atoms unter Ausbildung einer heterocyclischen Gruppe verbunden sein kann, und
wobei in X ein C-Atom mit einem Hydroxy-, ω-Hydroxy-$C_{1-3}$-alkyl-, ω-($C_{1-4}$-Alkoxy)-$C_{1-3}$-alkyl- und/oder $C_{1-4}$-Alkoxy-Rest, und/oder ein oder zwei C-Atome jeweils mit einem oder zwei gleichen oder verschiedenen Substituenten ausgewählt aus $C_{1-6}$-Alkyl-, $C_{3-7}$-Cycloalkyl, und $C_{3-7}$-Cycloalkyl-$C_{1-3}$-alkylsubstituiert sein können, wobei zwei Alkyl-Substituenten unter Ausbildung eines carbocyclischen Ringsystems miteinander verbunden sein können, und wobei jeweils ein oder mehrere C-Atome ein- oder mehrfach mit F und/oder jeweils ein oder zwei C-Atome unabhängig voneinander einfach mit Cl oder Br substituiert sein können und
mit der Maßgabe, dass die Gruppe X in der Bedeutung $C_{2-4}$-Alkylenoxy keinen Hydroxy-Substituenten aufweist;
worin $R^1$, $R^4$ und $R^{10}$ eine der in Anspruch 1 oder 2 angegebenen Bedeutungen besitzt.

**7.** Amid-Verbindungen nach einem oder mehreren der voherigen Ansprüche, **dadurch gekennzeichnet, dass** Z eine Einfachbindung, $-CH_2-$ oder $-CH_2-CH_2-$ ist, wobei ein oder zwei C-Atome unabhängig voneinander ein- oder zweifach mit F, $CH_3$ oder $CF_3$ und/oder einfach mit Cl substituiert sein können.

**8.** Amid-Verbindungen nach einem oder mehreren der voherigen Ansprüche, **dadurch gekennzeichnet, dass** W $-CH_2-O-$, $-CH_2-NR^8-$, $-CH_2-CH_2-$ oder -CH=CH- bedeutet,
worin jeweils ein oder zwei C-Atome unabhängig voneinander mit F, $CH_3$ oder $CF_3$ substituiert sein können,
worin $R^8$ eine der in Anspruch 1 oder 2 angegebenen Bedeutungen besitzt.

**9.** Amid-Verbindungen nach einem oder mehreren der vorherigen Ansprüche, **dadurch gekennzeichnet, dass** die Gruppe A Phenyl, Pyridyl oder Naphthyl bedeutet,
wobei die zuvor genannten cyclischen Gruppen ein- oder mehrfach an ein oder mehreren C-Atomen mit $R^{20}$, im Falle eines Phenylrings auch zusätzlich einfach mit Nitro, und/oder eine oder mehrere NH-Gruppen mit $R^{21}$ substituiert sein können, und
$R^{20}$ und $R^{21}$ die in Anspruch 1 oder 2 angegebenen Bedeutungen aufweisen.

**10.** Amid-Verbindungen nach einem oder mehreren der vorherigen Ansprüche, **dadurch gekennzeichnet, dass** b den Wert 0 besitzt.

**11.** Amid-Verbindungen nach einem oder mehreren der vorherigen Ansprüche, **dadurch gekennzeichnet, dass** b den Wert 1 besitzt und B eine Bedeutung ausgewählt aus der Gruppe bestehend aus Phenyl, Furanyl, Thienyl und Pyridyl besitzt,
wobei die zuvor genannten cyclischen Gruppen ein- oder mehrfach an ein oder mehreren C-Atomen mit $R^{20}$, im Falle eines Phenylrings auch zusätzlich einfach mit Nitro, substituiert sein können, und
$R^{20}$ die in Anspruch 1 oder 2 angegebene Bedeutung aufweist.

**12.** Amid-Verbindungen nach einem oder mehreren der vorherigen Ansprüche,
**dadurch gekennzeichnet, dass**

A eine Bedeutung gemäß Anspruch 9,
B eine Bedeutung gemäß Anspruch 11 und
b den Wert 0 oder 1 besitzt.

**13.** Amid-Verbindungen nach Anspruch 12, **dadurch gekennzeichnet, dass**

$R^1$, $R^2$ wie in Anspruch 3 oder 4 definiert sind,
X wie in Anspruch 6 definiert ist,
W wie in Anspruch 8 definiert ist und
Z wie in Anspruch 7 definiert ist.

**14.** Amid-Verbindungen nach einem oder mehreren der vorherigen Ansprüche,
**dadurch gekennzeichnet, dass**

$R^{20}$ F, Cl, Br, I, OH, Cyano, $C_{1-4}$-Alkyl, $C_{1-4}$-Alkoxy, Difluormethyl, Trifluormethyl, Difluormethoxy, Trifluormethoxy, Amino, $C_{1-3}$-Alkyl-amino-, Di-$C_{1-3}$-alkyl-amino-, Carboxy oder $C_{1-4}$-Alkoxy-carbonyl bedeutet, wobei mehrfach vorkommende Substituenten $R^{20}$ gleiche oder verschiedene Bedeutungen aufweisen können und im Falle eines Phenyl-Rings dieser zusätzlich auch einfach mit Nitro substituiert sein kann.

**15.** Amid-Verbindungen nach Anspruch 1 ausgewählt aus der Gruppe der Formeln

(1) N-[3-Chlor-4-(2-piperidin-1-yl-ethoxy)-phenyl]-2-(2-chlor-4-trifluormethyl-phenoxy)-acetamid
(2) 2-(2-Chloro-trifluormethyl-phenoxy)-N-[3-cyano-4-(2-diethylamino-ethoxy)-phenyl]-acetamid
(3) 2-(2-Chlor-4-trifluormethyl-phenoxy)-N-[1-(2-diethylamino-ethyl)-2,3-dihydro-1H-indol-5-yl]-acetamid
(4) N-[3-Chlor-4-(3-diethylamino-prop-1-ynyl)-phenyl]-2-(2-chlor-4-trifluormethyl-phenoxy)-acetamid
(5) 2-(2-Chlor-4-trifluormethyl-phenoxy)-N-[1-(2-diethylamino-ethyl)-2,3-dimethyl-1H-indol-5-yl]-acetamid
(6) 2-(2-Chlor-4-trifluormethyl-phenoxy)-N-[1-(2-diethylamino-ethyl)-1 H-indol-5-yl]-acetamid
(7) 2-(2-Chlor-4-trifluormethyl-phenoxy)-N-[4-(2-diethylamino-ethoxy)-3-methoxy-phenyl]-acetamid
(8) 2-(3-Chlor-biphenyl-4-yloxy)-N-[3-chlor-4-(2-diethylamino-ethoxy)-phenyl]-acetamid
(9) N-[3-Chlor-4-(2-diethylamino-ethoxy)-phenyl]-2-(2-chlor-4-trifluormethyl-phenoxy)-acetamid
(10) 2-(4-tert.-Butyl-2-chlor-phenoxy)-N-[3-chlor-4-(2-diethylamino-ethoxy)-phenyl]-acetamid
(11) 3-Chlor-4-{[3-chlor-4-(2-diethylamino-ethoxy)-phenylcarbamoyl]-methoxy}-benzoesäure-methylester
(12) N-[3-Chlor-4-(2-diethylamino-ethoxy)-phenyl]-2-(2,4-dibromo-phenoxy)-acetamid
(13) 2-(4-Brom-2-chlor-phenoxy)-N-[3-chlor-4-(2-diethylamino-ethoxy)-phenyl]-acetamid
(14) N-[3-Chlor-4-(2-diethylamino-ethoxy)-phenyl]-2-(4-iod-2-methyl-phenoxy)-acetamid
(15) (2-{2-Chlor-4-[2-(2,4-dichlor-phenoxy)-acetylamino]-phenoxy}-ethylamino)-essigsäure-methylester
(16) N-[3-Chlor-4-(2-pyrrolidin-1-yl-ethoxy)-phenyl]-2-(2-chlor-4-trifluormethyl-phenoxy)-acetamid
(17) N-{3-Chlor-4-[2-(ethyl-propyl-amino)-ethoxy]-phenyl}-2-(2-chlor-4-trifluormethyl-phenoxy)-acetamid
(18) N-{3-Chlor-4-[2-(ethyl-methyl-amino)-ethoxy]-phenyl}-2-(2-chlor-4-trifluormethyl-phenoxy)-acetamid
(19) N-[3-Chlor-4-(2-diethylamino-ethoxy)-phenyl]-2-(2-chlor-4-dimethylamino-phenoxy)-acetamid
(20) (E)-N-[3-Chlor-4-(2-diethylamino-ethoxy)-phenyl]-3-(2-chlor-4-trifluormethyl-phenyl)-acrylamid
(21) N-[3-Chlor-4-(2-diethylamino-ethoxy)-phenyl]-2-(2-chlor-4-trifluormethyl-phenylamino)-acetamid
(22) N-[3-Chlor-4-(2-diethylamino-ethoxy)-phenyl]-2-(2-chlor-4-furan-2-yl-phenoxy)-acetamid
(23) N-[3-Chlor-4-(2-diethylamino-ethoxy)-phenyl]-2-(2-chlor-4-thiophen-2-yl-phenoxy)-acetamid
(24) N-[3-Chlor-4-(2-diethylamino-ethoxy)-phenyl]-2-(2-chlor-4-pyridin-3-yl-phenoxy)-acetamid
(25) 2-(2-Brom-4-trifluormethyl-phenoxy)-N-[3-chlor-4-(2-diethylamino-ethoxy)-phenyl]-acetamid
(26) N-{3-Chlor-4-[2-(2,5-dihydro-pyrrol-1-yl)-ethoxy]-phenyl}-2-(2-chlor-4-trifluormethyl-phenoxy)-acetamid
(27) 1-(2-{2-Chlor-4-[2-(2-chlor-4-trifluormethyl-phenoxy)-acetylamino]-phenoxy}-ethyl)-piperidin-4-carbonsäure-ethylester
(28) N-[3-Chlor-4-(3-diethylamino-propoxy)-phenyl]-2-(2-chlor-4-trifluormethyl-phenoxy)-acetamid
(29) N-{4-[2-(2-Aminomethyl-pyrrolidin-1-yl)-ethoxy]-3-chlor-phenyl}-2-(2-chlor-4-trifluormethyl-phenoxy)-acetamid
(30) N-{3-Chlor-4-[2-(2-dimethylaminomethyl-pyrrolidin-1-yl)-ethoxy]-phenyl}-2-(2-chlor-4-trifluormethyl-phenoxy)-acetamid
(31) N-[3-Brom-4-(2-diethylamino-ethoxy)-phenyl]-2-(2-chlor-4-trifluormethyl-phenoxy)-acetamid
(32) N-{3-Chlor-4-[2-(4-methoxy-piperidin-1-yl)-ethoxy]-phenyl}-2-(2-chlor-4-trifluormethyl-phenoxy)-acetamid
(33) N-{3-Chlor-4-[2-(4-hydroxy-piperidin-1-yl)-ethoxy]-phenyl}-2-(2-chlor-4-trifluormethyl-phenoxy)-acetamid
(34) 2-(2-Chlor-4-trifluormethyl-phenoxy)-N-[4-(2-diethylamino-ethoxy)-3-nitro-phenyl]-acetamid
(35) N-[3-Chlor-4-(2-diethylamino-ethoxy)-phenyl]-2-(2-chlor-4-trifluormethoxy-phenylamino)-acetamid
(36) N-[3-Chlor-4-(2-diethylamino-ethoxy)-phenyl]-2-(2-fluor-4-trifluormethyl-phenylamino)-acetamid
(37) 2-(2-Brom-4-trifluormethyl-phenylamino)-N-[3-chlor-4-(2-diethylamino-ethoxy)-phenyl]-acetamid
(38) (E)-3-(4'-Chlor-biphenyl-4-yl)-N-(4-piperidin-1-ylmethyl-phenyl)-acrylamid
(39) N-[3-Chlor-4-(2-diethylamino-ethylamino)-phenyl]-2-(2-chlor-4-trifluormethyl-phenoxy)-acetamid
(40) N-{3-Chlor-4-[2-(4-methyl-piperidin-1-yl)-ethylamino]-phenyl}-2-(2-chlor-4-trifluormethyl-phenoxy)-acetamid
(41) (E)-3-(4'-Chlor-biphenyl-4-yl)-N-(4-dimethylaminomethyl-phenyl)-acrylamid
(42) (E)-3-[5-(4-Chlor-phenyl)-pyridin-2-yl]-N-(4-piperidin-1-ylmethyl-phenyl)-acrylamid
(43) (E)-N-{3-Chlor-4-[2-(4-methyl-piperidin-1-yl)-ethylamino]-phenyl}-3-(2-chlor-4-trifluormethyl-phenyl)-acrylamid
(44) (E)-N-[3-Chlor-4-(4-methyl-piperidin-1-ylmethyl)-phenyl]-3-(2-chlor-4-trifluormethyl-phenyl)-acrylamid
(45) 2-(2-Chlor-4-trifluormethyl-phenoxy)-N-[4-(2-diethylamino-ethoxy)-3-methyl-phenyl]-acetamid
(46) (E)-3-(2-Chlor-4-trifluormethyl-phenyl)-N-[4-(2-diethylamino-ethoxy)-3-methyl-phenyl]-acrylamid
(47) (E)-3-(2-Chlor-4-trifluormethyl-phenyl)-N-[4-(2-diethylamino-ethoxy)-3-methoxy-phenyl]-acrylamid
(48) (E)-N-[3-Chlor-4-(2-diethylamino-ethyl)-phenyl]-3-(2-chlor-4-trifluormethyl-phenyl)-acrylamid
(49) N-[3-Chlor-4-(2-diethylamino-ethyl)-phenyl]-2-(2-chlor-4-trifluormethyl-phenoxy)-acetamid
(50) N-{3-Chlor-4-[2-(4-methyl-piperidin-1-yl)-ethyl]-phenyl}-2-(2-chlor-4-trifluormethyl-phenoxy)-acetamid

einschließlich deren Salze.

**16.** Physiologisch verträgliche Salze der Amid-Verbindungen nach einem oder mehreren der Ansprüche 1 bis 15.

**17.** Zusammensetzung, enthaltend mindestens eine Amid-Verbindung nach einem oder mehreren der Ansprüche 1 bis 15 und/ oder ein Salz gemäß Anspruch 16 neben gegebenenfalls einem oder mehreren physiologisch verträglichen Hilfsstoffen.

**18.** Arzneimittel, enthaltend mindestens eine Amid-Verbindung nach einem oder mehreren der Ansprüche 1 bis 15 und/ oder ein Salz gemäß Anspruch 16 neben gegebenenfalls einem oder mehreren inerten Trägerstoffen und/oder Verdünnungsmitteln.

**19.** Nicht-therapeutische Verwendung mindestens einer Amid-Verbindung nach einem oder mehreren der Ansprüche 1 bis 15 und/ oder eines Salzes gemäß Anspruch 16, einschließlich der durch die Maßgaben (M2) und (M3) gemäß Anspruch 1 ausgeschlossenen Verbindungen, zur Beeinflussung des Essverhaltens eines Säugetiers durch Reduzierung des Hungers, Zügeln des Appetits, Kontrollieren des Essverhaltens und/oder Hervorrufen eines Sättigungsgefühls.

**20.** Nicht-therapeutische Verwendung mindestens einer Amid-Verbindung nach einem oder mehreren der Ansprüche 1 bis 15 und/ oder eines Salzes gemäß Anspruch 16, einschließlich der durch die Maßgaben (M2) und (M3) gemäß Anspruch 1 ausgeschlossenen Verbindungen, zur Reduzierung des Körpergewichts und/ oder zum Verhindern einer Zunahme des Körpergewichts eines Säugetiers.

**21.** Verwendung mindestens einer Amid-Verbindung nach einem oder mehreren der Ansprüche 1 bis 15 und/ oder eines Salzes gemäß Anspruch 16, einschließlich der durch die Maßgaben (M2) und (M3) gemäß Anspruch 1 ausgeschlossenen Verbindungen, zur Herstellung eines Arzneimittels mit MCH-Rezeptor antagonistischer Aktivität.

**22.** Verwendung mindestens einer Amid-Verbindung nach einem oder mehreren der Ansprüche 1 bis 15 und/ oder eines Salzes gemäß Anspruch 16, einschließlich der durch die Maßgaben (M2) und (M3) gemäß Anspruch 1 ausgeschlossenen Verbindungen, zur Herstellung eines Arzneimittels, welches zur Prophylaxe und/oder Behandlung von Erscheinungen und/oder Krankheiten, die durch MCH verursacht werden oder mit MCH in einem anderen kausalen Zusammenhang stehen, geeignet ist.

**23.** Verwendung mindestens einer Amid-Verbindung nach einem oder mehreren der Ansprüche 1 bis 15 und/ oder eines Salzes gemäß Anspruch 16, einschließlich der durch die Maßgaben (M2) und (M3) gemäß Anspruch 1 ausgeschlossenen Verbindungen, zur Herstellung eines Arzneimittels, welches zur Prophylaxe und/oder Behandlung von metabolischen Störungen und/oder Essstörungen, insbesondere von Obesitas, Bulimie, Bulimie nervosa, Cachexia, Anorexie, Anorexie nervosa und Hyperphagia, geeignet ist.

**24.** Verwendung mindestens einer Amid-Verbindung nach einem oder mehreren der Ansprüche 1 bis 15 und/ oder eines Salzes gemäß Anspruch 16, einschließlich der durch die Maßgaben (M2) und (M3) gemäß Anspruch 1 ausgeschlossenen Verbindungen, zur Herstellung eines Arzneimittels, welches zur Prophylaxe und/oder Behandlung von mit Obesitas einhergehenden Krankheiten und/oder Störungen, insbesondere von Diabetes, besonders Typ II Diabetes, diabetischen Komplikationen, einschließlich diabetischer Retinopathie, diabetischer Neuropathie, diabetischer Nephropathie, Insulin-Resistenz, pathologischer Glukosetoleranz, Herzinsuffizienz, Herzkreislauferkrankungen, insbesondere Arteriosklerose und Bluthochdruck, Arthritis und Gonitis geeignet ist.

**25.** Verwendung mindestens einer Amid-Verbindung nach einem oder mehreren der Ansprüche 1 bis 15 und/ oder eines Salzes gemäß Anspruch 16, einschließlich der durch die Maßgaben (M2) und (M3) gemäß Anspruch 1 ausgeschlossenen Verbindungen, zur Herstellung eines Arzneimittels, welches zur Prophylaxe und/oder Behandlung von Hyperlipidämie, Fettakkumulation, emotionalen Störungen, Affektivitätsstörungen, Depressionen und Angstzuständen, geeignet ist.

**26.** Arzneimittel, enthaltend
einen ersten Wirkstoff, der aus den Amid-Verbindungen nach einem oder mehreren der Ansprüche 1 bis 15 und/ oder eines Salzes gemäß Anspruch 16, einschließlich der durch die Maßgaben (M2) und (M3) gemäß Anspruch 1 ausgeschlossenen Verbindungen, ausgewählt ist sowie
einen zweiten Wirkstoff, der aus der Gruppe ausgewählt ist bestehend aus Wirkstoffen zur Behandlung von Diabetes,

Wirkstoffen zur Behandlung diabetischer Komplikationen, Wirkstoffen zur Behandlung von Obesitas, vorzugsweise anderen als MCH-Antagonisten, Wirkstoffen zur Behandlung von Bluthochdruck, Wirkstoffen zur Behandlung von Hyperlipidemia, einschließlich Arteriosklerose, Wirkstoffen zur Behandlung von Arthritis, Wirkstoffen zur Behandlung von Angstzuständen und Wirkstoffen zur Behandlung von Depressionen,
neben gegebenenfalls einem oder mehreren inerten Trägerstoffen und/oder Verdünnungsmitteln.

**Claims**

**1.** Amide compounds of general formula I

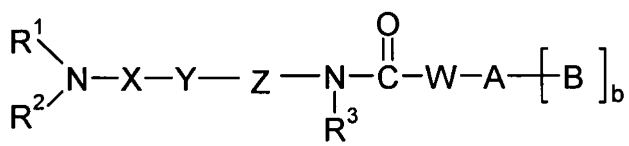

I

wherein

$R^1$, $R^2$ independently of one another denote H, a $C_{1-8}$-alkyl or $C_{3-7}$-cycloalkyl group optionally substituted by the group $R^{11}$, wherein a $-CH_2-$ group in position 3 or 4 of a 5-, 6- or 7-membered cycloalkyl group may be replaced by -O-, -S- or $-NR^{13}-$, or a phenyl or pyridinyl group optionally mono- or polysubstituted by the group $R^{12}$ and/or monosubstituted by nitro, with the proviso that at least one of the groups $R^1$, $R^2$ has a meaning other than H, or
$R^1$ and $R^2$ form a $C_{2-8}$-alkylene bridge wherein

- one or two $-CH_2-$ groups may be replaced independently of one another by -CH=N- or -CH=CH- and/or
- one or two $-CH_2-$ groups may be replaced independently of one another by -O-, -S-, -SO-, $-(SO_2)-$, $-C=N-O-R^{18}-$ -CO-,
- $C(=CH_2)-$ or $-NR^{13}-$ in such a way that heteroatoms are not directly connected to one another,

wherein in the above-defined alkylene bridge one or more H atoms may be replaced by $R^{14}$, and
wherein the above-defined alkylene bridge may be substituted by one or two identical or different carbo- or heterocyclic groups Cy in such a way that the bond between the alkylene bridge and the group Cy is formed

- via a single or double bond,
- via a common C atom forming a spirocyclic ring system,
- via two common, adjacent C and/or N atoms forming a fused bicyclic ring system or
- via three or more C and/or N atoms forming a bridged ring system,

$R^3$ denotes H, $C_{1-6}$-alkyl, $C_{3-7}$-cycloalkyl or $C_{3-7}$-cycloalkyl-$C_{1-4}$-alkyl ,
X denotes an unbranched $C_{1-4}$-alkylene bridge and
if the group Y is connected to X via a C atom, it may also denote $-CH_2-CH=CH-$, $-CH_2-C=C-$, $C_{2-4}$-alkylenoxy or $C_{2-4}$-alkylene-$NR^4$- ,
wherein the bridge X may be attached to $R^1$ including the N atom attached to $R^1$ and X forming a heterocyclic group, wherein the bridge X may additionally also be attached to $R^2$, including the N-atom attached to $R^2$ and X , forming a heterocyclic group, and
a C atom may be substituted by $R^{10}$ and/or one or two C atoms may each be substituted with one or two identical or different substituents selected from among $C_{1-6}$-alkyl, $C_{2-6}$-alkenyl, $C_{2-6}$-alkynyl, $C_{3-7}$-cycloalkyl, $C_{3-7}$-cycloalkyl-$C_{1-3}$-alkyl, $C_{4-7}$-cycloalkenyl and $C_{4-7}$-cycloalkenyl-$C_{1-3}$-alkyl, wherein two alkyl and/or alkenyl substituents may be joined together, forming a carbocyclic ring system, with the proviso that the group X denoting $C_{2-4}$-alkylenoxy does not contain a hydroxy substituent; and
W denotes $-CR^{6a}R^{6b}-O-$, $-CR^{7a}=CR^{7c}-$, $-CR^{6a}R^{6b}-NR^8-$, $-OR^{7a}p^{7b}-CR^{7c}R^{7d}-$ or $-NR^8-CR^{6a}R^{6b}-$,
Z denotes a single bond, $C_{1-4}$-alkylene, wherein two adjacent C atoms may be joined together with an additional $C_{1-4}$-alkylene bridge,
wherein a C atom of the alkylene bridge may be substituted with $R^{10}$ and/or one or two C atoms independently of one another may be substituted with one or two identical or different $C_{1-6}$-alkyl groups, wherein two alkyl

groups may be joined together, forming a carbocyclic ring, and
Y is selected from among the bivalent cyclic groups

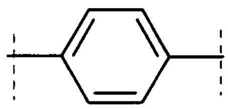 , 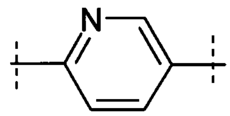 ,

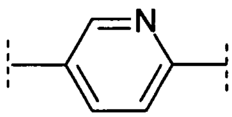 , 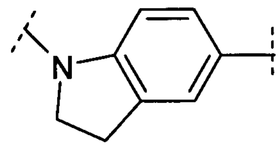 ,

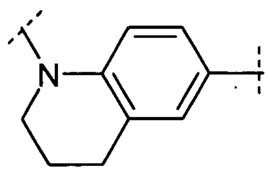 , 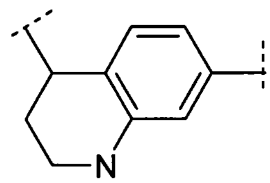 ,

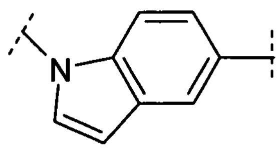 , 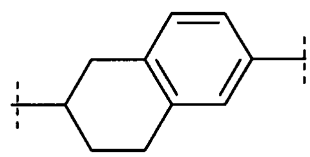 ,

wherein the above-mentioned cyclic groups may be mono- or polysubstituted by $R^{20}$ at one or more C atoms, and in the case of a phenyl group may also additionally be monosubstituted by nitro, and/or one or more NH groups may be substituted by $R^{21}$, and
A denotes one of the meanings given for Cy,
B denotes one of the meanings given for Cy,
b denotes the value 0 or 1,
Cy denotes a carbo- or heterocyclic group selected from one of the following meanings

- a saturated 3- to 7-membered carbocyclic group,
- an unsaturated 4- to 7-membered carbocyclic group,
- a phenyl group,
- a saturated 4- to 7-membered or unsaturated 5- to 7-membered heterocyclic group with an N, O or S atom as heteroatom,
- a saturated or unsaturated 5- to 7-membered heterocyclic group with two or more N atoms or with one or two N atoms and an O or S atom as heteroatoms,
- an aromatic heterocyclic 5- or 6-membered group with one or more identical or different heteroatoms selected from N, O and/or S,

wherein the above-mentioned 4-, 5-, 6- or 7-membered groups may be attached via two common, adjacent C atoms fused to a phenyl or pyridine ring, and
in the above-mentioned 5-, 6- or 7-membered groups one or two non-adjacent $-CH_2-$ groups may be replaced independently of one another by a -CO-, $-C(=CH_2)-$, -(SO)- or $-(SO_2)-$ group, and
the above-mentioned saturated 6- or 7-membered groups may also be present as bridged ring systems with an imino, N-($C_{1-4}$-alkyl)-imino, methylene, $C_{1-4}$-alkyl-methylene or di-($C_{1-4}$-alkyl)-methylene bridge, and

the above-mentioned cyclic groups may be mono- or polysubstituted at one or more C atoms with $R^{20}$, in the case of a phenyl group they may also additionally be monosubstituted with nitro, and/or one or more NH groups may be substituted with $R^{21}$,

$R^4$ denotes H, $C_{1-6}$-alkyl or $C_{2-6}$-alkenyl,

$R^{6a}$, $R^{6b}$ denotes H, $C_{1-4}$-alkyl or $CF_3$,

$R^{7a}$, $R^{7b}$, $R^{7c}$, $R^{7d}$ denotes H, F, Cl, $C_{1-4}$-alkyl or $CF_3$,

$R^8$ denotes H, $C_{1-4}$-alkyl, $C_{3-7}$-cycloalkyl or $C_{3-7}$-cycloalkyl-$C_{1-3}$-alkyl,

$R^{10}$ denotes hydroxy, ω-hydroxy-$C_{1-3}$-alkyl, $C_{1-4}$-alkoxy, ω-($C_{1-4}$-alkoxy)-$C_{1-3}$-alkyl, carboxy, $C_{1-4}$-alkoxycarbonyl, amino, $C_{1-4}$-alkyl-amino, di-($C_{1-4}$-alkyl)-amino, cyclo-$C_{3-6}$-alkyleneimino, amino-$C_{1-3}$-alkyl, $C_{1-4}$-alkyl-amino-$C_{1-3}$-alkyl, di-($C_{1-4}$-alkyl)-amino-$C_{1-3}$-alkyl, cyclo-$C_{3-6}$-alkyleneimino-$C_{1-3}$-alkyl, amino-$C_{1-3}$-alkoxy, $C_{1-4}$-alkyl-amino-$C_{1-3}$-alkoxy, di-($C_{1-4}$-alkyl)-amino-$C_{1-3}$-alkoxy or cyclo-$C_{3-6}$-alkyleneimino-$C_{1-3}$-alkoxy, aminocarbonyl, $C_{1-4}$-alkyl-aminocarbonyl, di-($C_{1-4}$-alkyl)-aminocarbonyl or cyclo-$C_{3-6}$-alkyleneimino-carbonyl,

$R^{11}$ denotes $C_{2-6}$-alkenyl, $C_{2-6}$-alkynyl, $R^{15}$-O, $R^{15}$-O-CO, $R^{15}$-CO-O, $R^{16}R^{17}N$, $R^{18}R^{19}N$-CO or Cy,

$R^{12}$ has one of the meanings given for $R^{20}$,

$R^{13}$ has one of the meanings given for $R^{17}$, with the exception of carboxy,

$R^{14}$ denotes halogen, $C_{1-6}$-alkyl, $C_{2-6}$-alkenyl, $C_{2-6}$-alkynyl, $R^{15}$-O, $R^{15}$-O-CO, $R^{15}$-CO, $R^{15}$-CO-O, $R^{16}R^{17}N$, $R^{18}R^{19}N$-CO, $R^{15}$-O-$C_{1-3}$-alkyl, $R^{15}$-O-CO-$C_{1-3}$-alkyl, $R^{15}$-O-CO-NH, $R^{15}$-$SO_2$-NH, $R^{15}$-O-CO-NH-$C_{1-3}$-alkyl, $R^{15}$-$SO_2$-NH-$C_{1-3}$-alkyl, $R^{15}$-CO-$C_{1-3}$-alkyl, $R^{15}$-O-O-$C_{1-3}$-alkyl, $R^{16}R^{17}N$-$C_{1-3}$-alkyl, $R^{18}R^{19}N$-CO-$C_{1-3}$-alkyl or Cy-$C_{1-3}$-alkyl,

$R^{15}$ denotes H, $C_{1-4}$-alkyl, $C_{3-7}$-cycloalkyl, $C_{3-7}$-cycloalkyl-$C_{1-3}$-alkyl, phenyl, phenyl-$C_{1-3}$-alkyl, pyridinyl or pyridinyl-$C_{1-3}$-alkyl,

$R^{16}$ denotes H, $C_{1-6}$-alkyl, $C_{3-7}$-cycloalkyl, $C_{3-7}$-cycloalkyl-$C_{1-3}$-alkyl, $C_{4-7}$-cycloalkenyl, $C_{4-7}$-cydoalkenyl-$C_{1-3}$-alkyl, ω-hydroxy-$C_{2-3}$-alkyl, ω-($C_{1-4}$-alkoxy)-$C_{2-3}$-alkyl, amino-$C_{2-6}$-alkyl, $C_{1-4}$-alkyl-amino-$C_{2-6}$-alkyl, di-($C_{1-4}$-alkyl)-amino-$C_{2-6}$-alkyl or cyclo-$C_{3-6}$-alkyleneimino-$C_{2-6}$-alkyl,

$R^{17}$ has one of the meanings given for $R^{16}$ or denotes phenyl, phenyl-$C_{1-3}$-alkyl, pyridinyl, dioxolan-2-yl, -CHO, $C_{1-4}$-alkylcarbonyl, carboxy, hydroxycarbonyl-$C_{1-3}$-alkyl, $C_{1-4}$-alkoxycarbonyl, $C_{1-4}$-alkoxycarbonyl-$C_{1-3}$-alkyl, $C_{1-4}$-alkylcarbonylamino-$C_{2-3}$-alkyl, N-($C_{1-4}$-alkylcarbonyl)-N-($C_{1-4}$-alkyl)-amino-$C_{2-3}$-alkyl, $C_{1-4}$-alkylsulphonyl, $C_{1-4}$-alkylsulphonylamino-$C_{2-3}$-alkyl or N-($C_{1-4}$-alkylsulphonyl)-N($C_{1-4}$-alkyl)-aminO-$C_{2-3}$-alkyl,

$R^{18}$, $R^{19}$ independently of one another denote H or $C_{1-6}$-alkyl,

$R^{20}$ denotes halogen, hydroxy, cyano, $C_{1-6}$-alkyl, $C_{2-6}$-alkenyl, $C_{2-6}$-alkynyl, $C_{3-7}$-cycloalkyl, $C_{3-7}$-cycloalkyl-$C_{1-3}$-alkyl, hydroxy-$C_{1-4}$-alkyl, $R^{22}$-$C_{1-3}$-alkyl or one of the meanings given for $R^{22}$,

$R^{21}$ denotes $C_1$-$_4$-alkyl, ω-hydroxy-$C_{2-3}$-alkyl, ω-$C_{1-4}$-alkoxy-$C_{2-6}$-alkyl, ω-$C_{1-4}$-alkyl-aminO-$C_{2-6}$-alkyl, ω-di-($C_{1-4}$-alkyl)-amino-$C_{2-6}$-alkyl, ω-cyclo-$C_{3-6}$-alkyleneimino-$C_{2-6}$-alkyl, phenyl-$C_{1-3}$-alkyl, $C_{1-4}$-alkylcarbonyl, $C_{1-4}$-alkoxy-carbonyl or $C_{1-4}$-alkylsulphonyl,

$R^{22}$ denotes phenyl-$C_{1-3}$-alkoxy, OHC, HO-N=HC, $C_{1-4}$-alkoxy-N=HC, $C_{1-4}$-alkoxy, $C_{1-4}$-alkylthio, carboxy, $C_{1-4}$-alkylcarbonyl, $C_{1-4}$-alkoxycarbonyl, aminocarbonyl, $C_{1-4}$-alkylaminocarbonyl, di-($C_{1-4}$-alkyl)-aminocarbonyl, cyclo-$C_{3-6}$-alkyl-amino-carbonyl, cyclo-$C_{3-6}$-alkyleneimino-carbonyl, cyclo-$C_{3-6}$-alkyleneimino-$C_{2-4}$-alkyl-aminocarbonyl, phenyl-amino-carbonyl, $C_{1-4}$-alkyl-sulphonyl, $C_{1-4}$-alkyl-sulphinyl, $C_{1-4}$-alkyl-sulphonylamino, amino, $C_{1-4}$-alkylamino, di-($C_{1-4}$-alkyl)-amino, $C_{1-4}$-alkyl-carbonyl-amino, cyclo-$C_{3-6}$-alkyleneimino, phenyl-$C_{1-3}$-alkylamino or N-($C_{1-4}$-alkyl)-phenyl-$C_{1-3}$-alkylamino, acetylamino, propionylamino, phenylcarbonylamino, phenylcarbonylmethylamino, hydroxy- alkylaminocarbonyl, (4- morpholinyl) carbonyl, (1- pyrrolidinyl)-carbonyl, (1- piperidinyl) carbonyl, (hexahydro- 1- azepinyl) carbonyl, (4- methyl- 1- piperazinyl) carbonyl, methylenedioxy, aminocarbonyl- amino or alkylaminocarbonylamino,

wherein in the above-mentioned groups and residues, especially in A, B, W, X, Y, Z, $R^1$ to $R^4$, $R^{6a}$, $R^{6b}$, $R^{7a}$, $R^{7b}$, $R^{7c}$, $R^{7d}$, $R^8$, $R^{10}$ to $R^{22}$, in particular, one or more C atoms in each case may additionally be mono- or polysubstituted by F and/or one or two C atoms in each case may additionally be monosubstituted by Cl or Br independently of one another and/or one or more phenyl rings in each case may additionally, independently of one another, have one, two or three substituents selected from among F, Cl, Br, I, $C_{1-4}$-alkyl, $C_{1-4}$-alkoxy, difluoromethyl, trifluoromethyl, hydroxy, amino, $C_{1-3}$-alkylamino, di-($C_{1-3}$-alkyl)-amino, acetylamino, aminocarbonyl, cyano, difluoromethoxy, trifluoromethoxy, amino-$C_{1-3}$-alkyl, $C_{1-3}$-alkylamino-$C_{1-3}$-alkyl and di-($C_{1-3}$-alkyl)-amino-$C_{1-3}$-alkyl and/or may be monosubstituted by nitro, and

the H atom of any carboxy group present or an H atom bonded to an N atom may each be replaced by a group which can be cleaved *in vivo*,

the tautomers, the diastereomers, the enantiomers, the mixtures thereof and the salts thereof,

with the following provisos (M2) and (M3)

(M2) in the event that W denotes -CH=CH- and Y denotes a phenylene group and Z is a single bond, then the bridges X and Z at the phenylene ring of the group Y are in the para position to one another and at least one of the

following conditions is met:

(a) the group Y meaning phenylene is at least monosubstituted,
(b) b has the value 0 and the group A is at least disubstituted,
(c) b has the value 1;

(M3) the following individual compounds are not included:

N-[4-(2-diethylamino-ethoxy)-phenyl]-3-phenyl-propionamide,
N-[4-(2-morpholin-4-ylethoxy)-phenyl]-3-phenyl-propionamide,
3-(4-chloro-phenyl)-N-{2-[4-(2-diethylamino-ethoxy)-phenyl]-ethyl}-acrylamide,
N-{2-[3-(4-{2-[2-(4-chloro-phenoxy)-acetylamino]-ethyl}-phenoxy)-2-hydroxy-propylamino]-ethyl}-isobutyramide,
cyclopentanecarboxylic acid {2-[3-(4-{2-[2-(4-chloro-phenoxy)-acetylamino]-ethyl}-phenoxy)-2-hydroxy-propylamino]-ethyl}-amide,
2-(4-chloro-phenoxy)-N-(2-{4-[2-hydroxy-3-(2-phenylacetylamino-ethylamino)-propoxy]-phenyl}-ethyl)-acetamide.

**2.** Amide compounds according to claim 1, **characterised in that**

$R^1$, $R^2$ independently of one another denote H, a $C_{1-8}$-alkyl or $C_{3-7}$-cycloalkyl group optionally substituted by the group $R^{11}$, or a phenyl group optionally mono- or polysubstituted by the group $R^{12}$ and/or monosubstituted by nitro, with the proviso that at least one of the groups $R^1$, $R^2$ has a meaning other than H, or
$R^1$ and $R^2$ form a $C_{2-8}$-alkylene bridge wherein

- one or two $-CH_2-$ groups independently of one another may be replaced by -CH=N- or -CH=CH- and/or
- one or two $-CH_2-$ groups independently of one another may be replaced by -O-, -S-, -CO-, $-C(=CH_2)-$ or $-NR^{13}-$ so that heteroatoms are not directly connected to one another,

wherein in the alkylene bridge defined above one or more H atoms may be replaced by $R^{14}$, and
wherein the alkylene bridge defined hereinbefore may be substituted with one or two identical or different carbo- or heterocyclic groups Cy so that the bond between the alkylene bridge and the group Cy is made

- via a single or double bond,
- via a common C atom forming a spirocyclic ring system,
- via two common adjacent C and/or N atoms forming a fused bicyclic ring system or
- via three or more C and/or N atoms forming a bridged ring system,

X denotes an unbranched $C_{1-4}$-alkylene bridge and
if the group Y is connected to X via a C atom, it may also denote $-CH_2-CH=CH-$, $-CH_2-C\equiv C-$, $C_{2-4}$-alkylenoxy or $C_{2-4}$-alkylene-$NR^4$-,
wherein the bridge X may be connected to $R^1$ including the N atom attached to $R^1$ and X forming a heterocyclic group, and
a C atom may be substituted by $R^{10}$ and/or one or two C atoms in each case may be substituted by one or two identical or different $C_{1-6}$-alkyl groups, with the proviso that the group X denoting $C_{2-4}$-alkylenoxy does not have a hydroxy substituent; and
Z denotes a single bond, $C_{1-4}$-alkylene, wherein two adjacent C atoms may be joined together by an additional $C_{1-4}$-alkylene bridge,
wherein a C atom of the alkylene bridge may be substituted by $R^{10}$ and/or one or two C atoms independently of one another may be substituted by one or two identical or different $C_{1-6}$-alkyl groups, and
b has the value 0,
$R^{10}$ denotes hydroxy, ω-hydroxy-$C_{1-3}$-alkyl, $C_{1-4}$-alkoxy, ω-($C_{1-4}$-alkoxy)-$C_{1-3}$-alkyl, amino, $C_{1-4}$-alkyl-amino, di-($C_{1-4}$-alkyl)-amino,cyclo-$C_{3-6}$-alkyleneimino,amino-$C_{1-3}$-alkyl,$C_{1-4}$-alkyl-amino-$C_{1-3}$-alkyl,di-($C_{1-4}$-alkyl)-amino-$C_{1-3}$-alkyl, cyclo-$C_{3-6}$-alkyleneimino-$C_{1-3}$-alkyl, amino-$C_{1-3}$-alkoxy, $C_{1-4}$-alkyl-amino-$C_{1-3}$-alkoxy, di-($C_{1-4}$-alkyl)-amino-$C_{1-3}$-alkoxy or cyclo-$C_{3-6}$-alkyleneimino-$C_{1-3}$-alkoxy,
$R^{14}$ denotes halogen, $C_{1-6}$-alkyl, $R^{15}$-O, $R^{15}$-O-CO, $R^{15}$-CO, $R^{15}$-CO-O, $R^{16}R^{17}N$, $R^{18}R^{19}N$-CO, $R^{15}$-O-$C_{1-3}$-alkyl-, $R^{15}$-O-CO-$C_{1-3}$-alkyl, $R^{15}$-CO-$C_{1-3}$-alkyl, $R^{15}$-CO-O-$C_{1-3}$-alkyl, $R^{16}R^{17}N$-$C_{1-3}$-alkyl, $R^{18}R^{19}N$-CO-$C_{1-3}$-alkyl or Cy-$C_{1-3}$-alkyl,

$R^{15}$ denotes H, $C_{1-4}$-alkyl, $C_{3-7}$-cycloalkyl, $C_{3-7}$-cycloalkyl-$C_{1-3}$-alkyl, phenyl or phenyl-$C_{1-3}$-alkyl,
$R^{17}$ has one of the meanings given for $R^{16}$ or denotes phenyl, phenyl-$C_{1-3}$-alkyl, $C_{1-4}$-alkylcarbonyl, hydroxycarbonyl-$C_{1-3}$-alkyl, $C_{1-4}$-alkylcarbonylaminO-$C_{2-3}$-alkyl, N-($C_{1-4}$-alkylcarbonyl)-N-($C_{1-4}$-alkyl)-amino-$C_{2-3}$-alkyl, $C_{1-4}$-alkylsulphonyl, $C_{1-4}$-alkylsulphonylaminO-$C_{2-3}$-alkyl or N-($C_{1-4}$-alkylsulphonyl)-N($C_{1-4}$-alkyl)-aminO-$C_{2-3}$-alkyl,
$R^{20}$ denotes halogen, hydroxy, cyano, $C_{1-6}$-alkyl, $C_{3-7}$-cycloalkyl, $C_{3-7}$-cycloalkyl-$C_{1-3}$-alkyl, hydroxy-$C_{1-4}$-alkyl, $R^{22}$-$C_{1-3}$-alkyl or one of the meanings given for $R^{22}$,
$R^{21}$ denotes $C_{1-4}$-alkyl, ω-hydroxy-$C_{2-3}$-alkyl, ω-$C_{1-4}$-alkoxy-$C_{2-6}$-alkyl, ω-$C_{1-4}$-alkyl-aminO-$C_{2-6}$-alkyl, ω-di-($C_{1-4}$-alkyl)-amino-$C_{2-6}$-alkyl, ω-cyclo-$C_{3-6}$-alkyleneimino-$C_{2-6}$-alkyl, phenyl, phenyl-$C_{1-3}$-alkyl, $C_{1-4}$-alkylcarbonyl, carboxy, $C_{1-4}$-alkoxy-carbonyl or $C_{1-4}$-alkylsulphonyl,
$R^{22}$ denotes phenyl, phenyl-$C_{1-3}$-alkoxy, $C_{1-4}$-alkoxy, $C_{1-4}$-alkylthio, carboxy, $C_{1-4}$-alkylcarbonyl, $C_{1-4}$-alkoxycarbonyl, aminocarbonyl, $C_{1-4}$-alkylaminocarbonyl, di-($C_{1-4}$-alkyl)-aminocarbonyl, cyclo-$C_{3-6}$-alkyleneiminocarbonyl, $C_{1-4}$-alkyl-sulphonyl, $C_{1-4}$-alkyl-sulphinyl, $C_{1-4}$-alkyl-sulphonylamino, amino, $C_{1-4}$-alkylamino, di-($C_{1-4}$-alkyl)-amino, cyclo-$C_{3-6}$-alkyleneimino, phenyl-$C_{1-3}$-alkylamino or N-($C_{1-4}$-alkyl)-phenyl-$C_{1-3}$-alkylamino, acetylamino, propionylamino, phenylcarbonylamino, phenylcarbonylmethylamino, hydroxyalkylaminocarbonyl, (4-morpholinyl)carbonyl, (1-pyrrolidinyl)carbonyl, (1-piperidinyl)carbonyl, (hexahydro-1-azepinyl)carbonyl, (4-methyl-1-piperazinyl)carbonyl, methylenedioxy, aminocarbonylamino or alkylaminocarbonylamino,
wherein the groups and residues $R^3$, $R^4$, $R^{6a}$, $R^{6b}$, $R^{7a}$, $R^{7b}$, $R^{7c}$, $R^{7d}$, $R^8$, $R^{11}$, $R^{12}$, $R^{16}$, $R^{18}$, $R^{19}$, W, Y, A, Cy have the meanings given in claim 1.

3. Amide compounds according to claim 1 or 2, **characterised in that** $R^1$, $R^2$ independently of one another denote H, $C_{1-6}$-alkyl, $C_{3-7}$-cydoalkyl, $C_{3-7}$-cycloalkyl-$C_{1-3}$-alkyl, ω-hydroxy-$C_{2-3}$-alkyl, ω-($C_{1-4}$-alkoxy)-$C_{2-3}$-alkyl, $C_{1-4}$-alkoxy-carbonyl-$C_{1-4}$-alkyl,carboxyl-$C_{1-4}$-alkyl,amino-$C_{2-4}$-alkyl,$C_{1-4}$-alkyl-amino-$C_{2-4}$-alkyl,di-($C_{1-4}$-alkyl)-amino-$C_{2-4}$-alkyl, cyclo-$C_{3-6}$-alkyleneimino-$C_{2-4}$-alkyl, pyrrolidinyl, N-($C_{1-4}$-alkyl)-pyrrolidinyl, pyrrolidinyl-$C_{1-3}$-alkyl, N-($C_{1-4}$-alkyl)-pyrrolidinyl-$C_{1-3}$-alkyl, piperidinyl, N-($C_{1-4}$-alkyl)-piperidinyl, piperidinyl-$C_{1-3}$-alkyl, N-($C_{1-4}$-alkyl)-piperidinyl-$C_{1-3}$-alkyl, phenyl, phenyl-$C_{1-3}$-alkyl, pyridyl or pyridyl-$C_{1-3}$-alkyl,
wherein in the above-mentioned groups and residues one or more C atoms may be mono- or polysubstituted by F and/or one or two C atoms may independently of one another be monosubstituted by Cl or Br, and
the phenyl or pyridyl group may be mono- or polysubstituted by the group $R^{12}$ as defined in claim 1 and/or may be monosubstituted by nitro,
with the proviso that at least one of the groups $R^1$, $R^2$ has a meaning other than H.

4. Amide compounds according to one or more of claims 1 to 3, **characterised in that** $R^1$ and $R^2$ form an alkylene bridge according to claim 1 such that $R^1R^2N$- denotes a group selected from azetidine, pyrrolidine, piperidine, azepan, 2,5-dihydro-1H-pyrrole, 1,2,3,6-tetrahydro-pyridine, 2,3,4,7-tetrahydro-1H-azepine, 2,3,6,7-tetrahydro-1H-azepine, piperazine, wherein the free imine function is substituted by $R^{13}$, piperidin-4-one-oxime, piperidin-4-one-O-$C_{1-4}$-alkyl-oxime, morpholine and thiomorpholine,
wherein according to claim 1 one or more H atoms may be replaced by $R^{14}$, and/ or Cy may be substituted in the manner indicated in claim 1 by one or two identical or different carbo- or heterocyclic groups, and/or
$R^{13}$, $R^{14}$ and Cy have the meanings given in claim 1 or 2.

5. Amide compounds according to one or more of the preceding claims,
**characterised in that**
the group

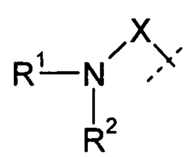

is defined according to one of the following partial formulae

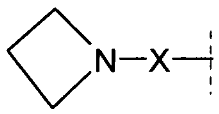 , 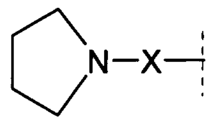 , 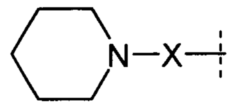 ,

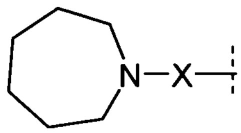 , 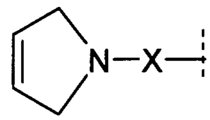 , 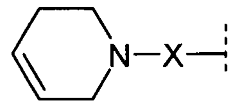 ,

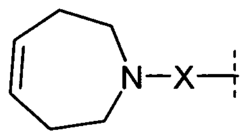 , 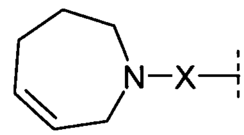 , 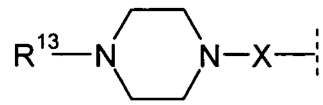 ,

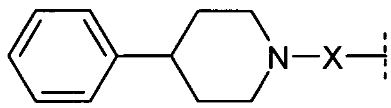 , 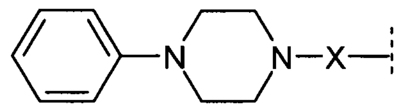 ,

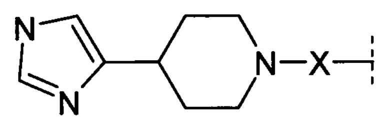 ,

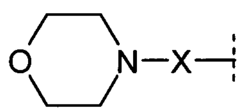 , 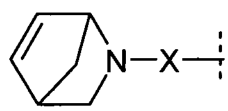 , 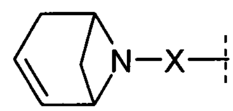 ,

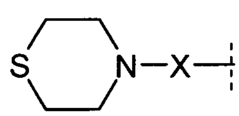 , 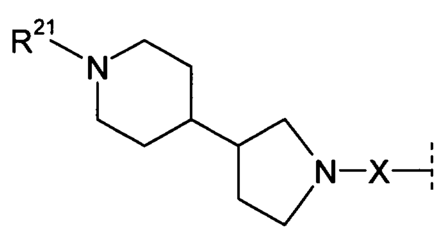 ,

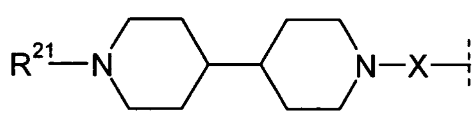 , 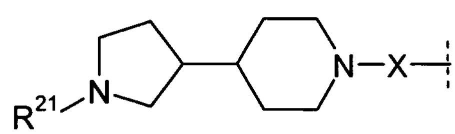 ,

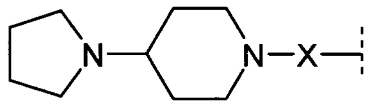 , 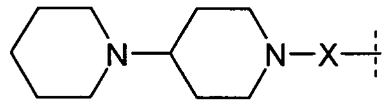 ,

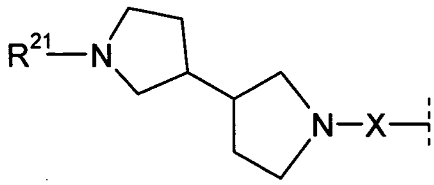 , 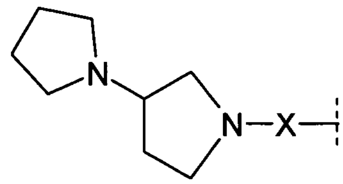 ,

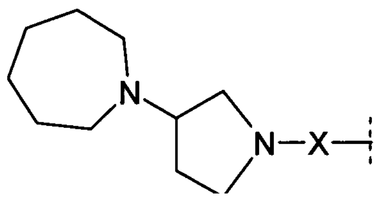 ,

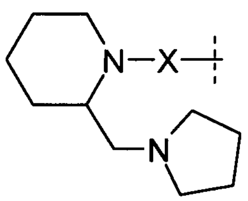 , 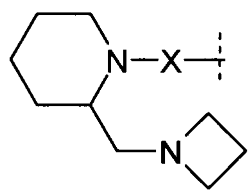 ,

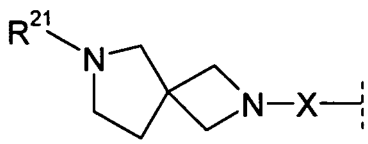 , ,

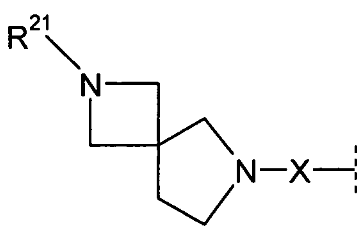 , 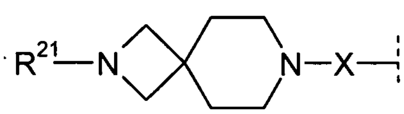 ,

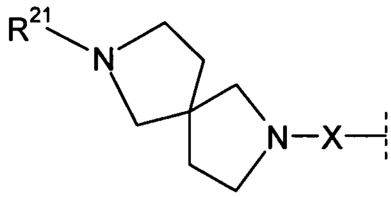 , 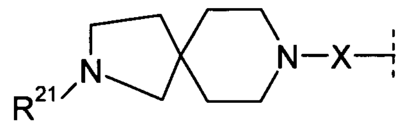 ,

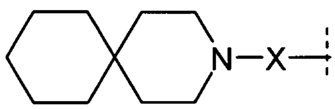 , 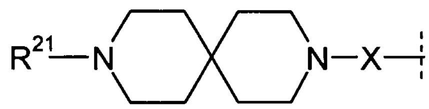 ,

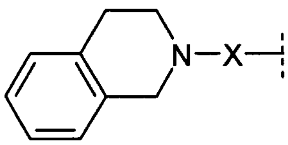 , 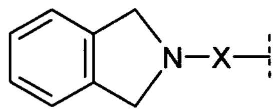 , 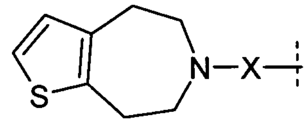 ,

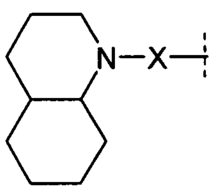 , 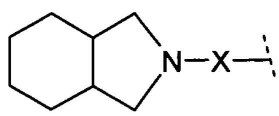 , 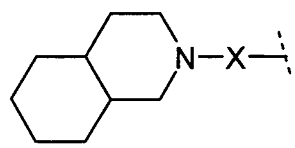 ,

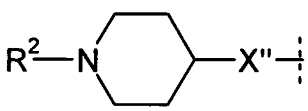 , 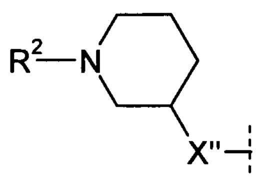 , 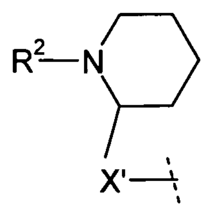 ,

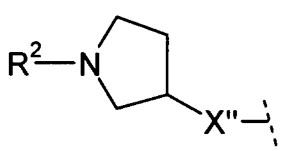 , 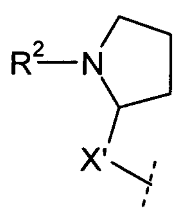 ,

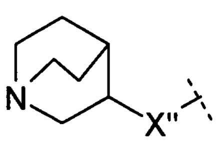 , 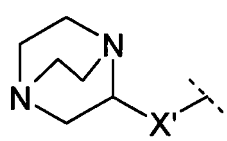 , 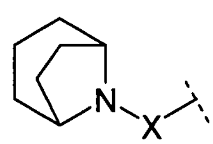 ,

wherein one or more H atoms of the heterocycle formed by the group $R^1R^2N$-may be replaced by $R^{14}$ and the ring

attached to the heterocycle formed by the group $R^1R^2N$- may be mono- or polysubstituted by $R^{20}$ at one or more C atoms, and in the case of a phenyl ring it may also additionally be monosubstituted by nitro and

X', X" independently of one another denote a single bond or $C_{1-3}$-alkylene and if the group Y is linked to X' or X" via a C atom, may also denote -$C_{1-3}$-alkylene-O-, -$C_{1-3}$-alkylene-NH- or -$C_{1-3}$-alkylene-N($C_{1-3}$-alkyl)-, and wherein in the definitions given hereinbefore for X', X" in each case a C atom may be substituted by $R^{10}$, and/or one or two C atoms may each be substituted by one or two identical or different substituents selected from $C_{1-6}$-alkyl, $C_{2-6}$-alkenyl, $C_{2-6}$-alkynyl, $C_{3-7}$-cycloalkyl, $C_{3-7}$-cycloalkyl-$C_{1-3}$-alkyl, $C_{4-7}$-cycloalkenyl and $C_{4-7}$-cycloalkenyl-$C_{1-3}$-alkyl, wherein two alkyl and/or alkenyl substituents may be joined together forming a carbocyclic ring system, and in X', X" independently of one another one or more C atoms may each be mono- or polysubstituted by F and/or one or two C atoms independently of one another may each be monosubstituted by Cl or Br and

wherein $R^2$, $R^{10}$, $R^{13}$, $R^{14}$, $R^{18}$, $R^{20}$, $R^{21}$ and X have the meanings given in claim 1 or 2.

6. Amide compounds according to one or more of the preceding claims, **characterised in that** X denotes -$CH_2$-, -$CH_2$-$CH_2$- or -$CH_2$-$CH_2$-$CH_2$-, and
if the group Y is linked to X via a C atom, it also denotes -$CH_2$-CH=CH-, -$CH_2$-C≡C-, -$CH_2$-$CH_2$-O-, -$CH_2$-$CH_2$-$CH_2$-O- or -$CH_2$-$CH_2$-$NR^4$- or -$CH_2$-$CH_2$-$CH_2$-$NR^4$-,
wherein the bridge X may be connected to $R^1$ including the N atom attached to $R^1$ and X, forming a heterocyclic group, and
in X a C atom may be substituted by a hydroxy, ω-hydroxy-$C_{1-3}$-alkyl, ω-($C_{1-4}$-alkoxy)-$C_{1-3}$-alkyl and/or $C_{1-4}$-alkoxy group, and/or one or two C atoms may each be substituted by one or two identical or different substituents selected from among $C_{1-6}$-alkyl, $C_{3-7}$-cycloalkyl and $C_{3-7}$-cycloalkyl-$C_{1-3}$-alkyl, wherein two alkyl substituents may be joined together, forming a carbocyclic ring system, and
one or more C atoms may each be mono- or polysubstituted by F and/or one or two C atoms may each independently of one another be monosubstituted by Cl or Br and
with the proviso that the group X denoting $C_{2-4}$-alkylenoxy does not have a hydroxy substituent;
wherein $R^1$, $R^4$ and $R^{10}$ have one of the meanings given in claim 1 or 2.

7. Amide compounds according to one or more of the preceding claims, **characterised in that** Z is a single bond, -$CH_2$- or -$CH_2$-$CH_2$-, wherein one or two C atoms independently of one another may be mono- or disubstituted by F, $CH_3$ or $CF_3$ and/or monosubstituted by Cl.

8. Amide compounds according to one or more of the preceding claims, **characterised in that** W denotes -$CH_2$-O-, -$CH_2$-$NR^8$-, -$CH_2$-$CH_2$- or -CH=CH-,
wherein in each case one or two C atoms may be substituted independently of one another by F, $CH_3$ or $CF_3$, wherein $R^8$ has one of the meanings given in claim 1 or 2.

9. Amide compounds according to one or more of the preceding claims, **characterised in that** the group A denotes phenyl, pyridyl or naphthyl,
wherein the above-mentioned cyclic groups may be mono- or polysubstituted by $R^{20}$ at one or more C atoms, and in the case of a phenyl group may also additionally be monosubstituted by nitro, and/or one or more NH groups may be substituted by $R^{21}$, and
$R^{20}$ and $R^{21}$ are defined as in claim 1 or 2.

10. Amide compounds according to one or more of the preceding claims, **characterised in that** b has the value 0.

11. Amide compounds according to one or more of the preceding claims, **characterised in that** b has the value 1 and B has a meaning selected from among phenyl, furanyl, thienyl and pyridyl,
wherein the above-mentioned cyclic groups may be mono- or polysubstituted by $R^{20}$ at one or more C atoms, and in the case of a phenyl group may also additionally be monosubstituted by nitro, and
$R^{20}$ has the meaning given in claim 1 or 2.

12. Amide compounds according to one or more of the preceding claims, **characterised in that**

A is defined according to claim 9,

B is defined according to claim 11 and
b has the value 0 or 1.

**13.** Amide compounds according to claim 12, **characterised in that**

$R^1$, $R^2$ are defined as in claim 3 or 4,
X is defined as in claim 6,
W is defined as in claim 8 and
Z is defined as in claim 7.

**14.** Amide compounds according to one or more of the preceding claims, **characterised in that**

$R^{20}$ denotes F, Cl, Br, I, OH, cyano, $C_{1-4}$-alkyl, $C_{1-4}$-alkoxy, difluoromethyl, trifluoromethyl, difluoromethoxy, trifluoromethoxy, amino, $C_{1-3}$-alkyl-amino, di-$C_{1-3}$-alkylamino, carboxy or $C_{1-4}$-alkoxy-carbonyl, wherein substituents $R^{20}$ occurring repeatedly may have the same or different meanings and in the case of a phenyl ring this may additionally also be monosubstituted by nitro.

**15.** Amide compounds according to claim 1 selected from the group of formulae

(1) N-[3-chloro-4-(2-piperidin-1-yl-ethoxy)-phenyl]-2-(2-chloro-4-trifluoromethyl-phenoxy)-acetamide
(2) 2-(2-chloro-4-trifluoromethyl-phenoxy)-N-[3-cyano-4-(2-diethylamino-ethoxy)-phenyl]-acetamide
(3) 2-(2-chloro-4-trifluoromethyl-phenoxy)-N-[1-(2-diethylaminoethyl)-2,3-dihydro-1H-indol-5-yl]-acetamide
(4) N-[3-chloro-4-(3-diethylamino-prop-1-ynyl)-phenyl]-2-(2-chloro-4-trifluoromethyl-phenoxy)-acetamide
(5) 2-(2-chloro-4-trifluoromethyl-phenoxy)-N-[1-(2-diethylaminoethyl)-2,3-dimethyl-1H-indol-5-yl]-acetamide
(6) 2-(2-chloro-4-trifluoromethyl-phenoxy)-N-[1-(2-diethylaminoethyl)-1H-indol-5-yl]-acetamide
(7) 2-(2-chloro-4-trifluoromethyl-phenoxy)-N-[4-(2-diethylaminoethoxy)-3-methoxy-phenyl]-acetamide
(8) 2-(3-chloro-biphenyl-4-yloxy)-N-[3-chloro-4-(2-diethylaminoethoxy)-phenyl]-acetamide
(9) N-[3-chloro-4-(2-diethylamino-ethoxy)-phenyl]-2-(2-chloro-4-trifluoromethyl-phenoxy)-acetamide
(10) 2-(4-tert.-butyl-2-chloro-phenoxy)-N-[3-chloro-4-(2-diethylamino-ethoxy)-phenyl]-acetamide
(11) methyl 3-chloro-4-{[3-chloro-4-(2-diethylamino-ethoxy)-phenylcarbamoyl]-methoxy}-benzoate
(12) N-[3-chloro-4-(2-diethylamino-ethoxy)-phenyl]-2-(2,4-dibromo-phenoxy)-acetamide
(13) 2-(4-bromo-2-chloro-phenoxy)-N-[3-chloro-4-(2-diethylamino-ethoxy)-phenyl]-acetamide
(14) N-[3-chloro-4-(2-diethylamino-ethoxy)-phenyl]-2-(4-iodo-2-methyl-phenoxy)-acetamide
(15) methyl (2-{2-chloro-4-[2-(2,4-dichloro-phenoxy)-acetylamino]-phenoxy}-ethylamino)-acetate
(16) N-[3-chloro-4-(2-pyrrolidin-1-yl-ethoxy)-phenyl]-2-(2-chloro-4-trifluoromethyl-phenoxy)-acetamide
(17) N-{3-chloro-4-[2-(ethyl-propyl-amino)-ethoxy]-phenyl}-2-(2-chloro-4-trifluoromethyl-phenoxy)-acetamide
(18) N-{3-chloro-4-[2-(ethyl-methyl-amino)-ethoxy]-phenyl}-2-(2-chloro-4-trifluoromethyl-phenoxy)-acetamide
(19) N-[3-chloro-4-(2-diethylamino-ethoxy)-phenyl]-2-(2-chloro-4-dimethylamino-phenoxy)-acetamide
(20) (E)-N-[3-chloro-4-(2-diethylamino-ethoxy)-phenyl]-3-(2-chloro-4-trifluoromethyl-phenyl)-acrylamide
(21) N-[3-chloro-4-(2-diethylamino-ethoxy)-phenyl]-2-(2-chloro-4-trifluoromethyl-phenylamino)-acetamide
(22) N-[3-chloro-4-(2-diethylamino-ethoxy)-phenyl]-2-(2-chloro-4-furan-2-yl-phenoxy)-acetamide
(23) N-[3-chloro-4-(2-diethylamino-ethoxy)-phenyl]-2-(2-chloro-4-thiophen-2-yl-phenoxy)-acetamide
(24) N-[3-chloro-4-(2-diethylamino-ethoxy)-phenyl]-2-(2-chloro-4-pyridin-3-yl-phenoxy)-acetamide
(25) 2-(2-bromo-4-trifluoromethyl-phenoxy)-N-[3-chloro-4-(2-diethylamino-ethoxy)-phenyl]-acetamide
(26) N-{3-chloro-4-[2-(2,5-dihydro-pyrrol-1-yl)-ethoxy]-phenyl}-2-(2-chloro-4-trifluoromethyl-phenoxy)-acetamide
(27) ethyl 1-(2-{2-chloro-4-[2-(2-chloro-4-trifluoromethyl-phenoxy)-acetylamino]-phenoxy}-ethyl)-piperidine-4-carboxylate
(28) N-[3-chloro-4-(3-diethylamino-propoxy)-phenyl]-2-(2-chloro-4-trifluoromethyl-phenoxy)-acetamide
(29) N-{4-[2-(2-aminomethyl-pyrrolidin-1-yl)-ethoxy]-3-chloro-phenyl}-2-(2-chloro-4-trifluoromethyl-phenoxy)-acetamide
(30) N-{3-chloro-4-[2-(2-dimethylaminomethyl-pyrrolidin-1-yl)-ethoxy]-phenyl}-2-(2-chloro-4-trifluoromethyl-phenoxy)-acetamide
(31) N-[3-bromo-4-(2-diethylamino-ethoxy)-phenyl]-2-(2-chloro-4-trifluoromethyl-phenoxy)-acetamide
(32) N-{3-chloro-4-[2-(4-methoxy-piperidin-1-yl)-ethoxy]-phenyl}-2-(2-chloro-4-trifluoromethyl-phenoxy)-acetamide
(33) N-{3-chloro-4-[2-(4-hydroxy-piperidin-1-yl)-ethoxy]-phenyl}-2-(2-chloro-4-trifluoromethyl-phenoxy)-acetamide

(34) 2-(2-chloro-4-trifluoromethyl-phenoxy)-N-[4-(2-diethylamino-ethoxy)-3-nitro-phenyl]-acetamide
(35) N-[3-chloro-4-(2-diethylamino-ethoxy)-phenyl]-2-(2-chloro-4-trifluoromethoxy-phenylamino)-acetamide
(36) N-[3-chloro-4-(2-diethylamino-ethoxy)-phenyl]-2-(2-fluoro-4-trifluoromethyl-phenylamino)-acetamide
(37) 2-(2-bromo-4-trifluoromethyl-phenylamino)-N-[3-chloro-4-(2-diethylamino-ethoxy)-phenyl]-acetamide
(38) (E)-3-(4'-chloro-biphenyl-4-yl)-N-(4-piperidin-1-ylmethyl-phenyl)-acrylamide
(39) N-[3-chloro-4-(2-diethylamino-ethylamino)-phenyl]-2-(2-chloro-4-trifluoromethyl-phenoxy)-acetamide
(40) N-{3-chloro-4-[2-(4-methyl-piperidin-1-yl)-ethylamino]-phenyl}-2-(2-chloro-4-trifluoromethyl-phenoxy)-acetamide
(41) (E)-3-(4'-chloro-biphenyl-4-yl)-N-(4-dimethylaminomethyl-phenyl)-acrylamide
(42) (E)-3-[5-(4-chloro-phenyl)-pyridin-2-yl]-N-(4-piperidin-1-ylmethyl-phenyl)-acrylamide
(43) (E)-N-{3-chloro-4-[2-(4-methyl-piperidin-1-yl)-ethylamino]-phenyl}-3-(2-chloro-4-trifluoromethyl-phenyl)-acrylamide
(44) (E)-N-[3-chloro-4-(4-methyl-piperidin-1-ylmethyl)-phenyl]-3-(2-chloro-4-trifluoromethyl-phenyl)-acrylamide
(45) 2-(2-chloro-4-trifluoromethyl-phenoxy)-N-[4-(2-diethylamino-ethoxy)-3-methyl-phenyl]-acetamide
(46) (E)-3-(2-chloro-4-trifluoromethyl-phenyl)-N-[4-(2-diethylamino-ethoxy)-3-methyl-phenyl]-acrylamide
(47) (E)-3-(2-chloro-4-trifluoromethyl-phenyl)-N-[4-(2-diethylamino-ethoxy)-3-methoxy-phenyl]-acrylamide
(48) (E)-N-[3-chloro-4-(2-diethylamino-ethyl)-phenyl]-3-(2-chloro-4-trifluoromethyl-phenyl)-acrylamide
(49) N-[3-chloro-4-(2-diethylamino-ethyl)-phenyl]-2-(2-chloro-4-trifluoromethyl-phenoxy)-acetamide
(50) N-{3-chloro-4-[2-(4-methyl-piperidin-1-yl)-ethyl]-phenyl}-2-(2-chloro-4-trifluoromethyl-phenoxy)-acetamide

including the salts thereof.

**16.** Physiologically acceptable salts of the amide compounds according to one or more of claims 1 to 15.

**17.** Composition, comprising at least one amide compound according to one or more of claims 1 to 15 and/ or a salt according to claim 16 optionally together with one or more physiologically acceptable excipients.

**18.** Medicament, comprising at least one amide compound according to one or more of claims 1 to 15 and/ or a salt according to claim 16 optionally together with one or more inert carriers and/or diluents.

**19.** Non-therapeutic use of at least one amide compound according to one or more of claims 1 to 15 and/ or a salt according to claim 16, including the compounds excluded by provisos (M2) and (M3) according to claim 1, for influencing the eating behaviour of a mammal by reducing hunger, reining in appetite, controlling eating behaviour and/or producing a feeling of satiation.

**20.** Non-therapeutic use of at least one amide compound according to one or more of claims 1 to 15 and/ or a salt according to claim 16, including the compounds excluded by provisos (M2) and (M3) according to claim 1, for reducing the body weight and/ or for preventing an increase in the body weight of a mammal.

**21.** Use of at least one amide compound according to one or more of claims 1 to 15 and/ or a salt according to claim 16, including the compounds excluded by provisos (M2) and (M3) according to claim 1, for preparing a medicament with an MCH receptor-antagonistic activity.

**22.** Use of at least one amide compound according to one or more of claims 1 to 15 and/ or a salt according to claim 16, including the compounds excluded by provisos (M2) and (M3) according to claim 1, for preparing a medicament which is suitable for the prevention and/or treatment of symptoms and/or diseases caused by MCH or causally connected with MCH in some other way.

**23.** Use of at least one amide compound according to one or more of claims 1 to 15 and/ or a salt according to claim 16, including the compounds excluded by provisos (M2) and (M3) according to claim 1, for preparing a medicament which is suitable for the prevention and/or treatment of metabolic disorders and/or eating disorders, particularly obesity, bulimia, bulimia nervosa, cachexia, anorexia, anorexia nervosa and hyperphagia.

**24.** Use of at least one amide compound according to one or more of claims 1 to 15 and/ or a salt according to claim 16, including the compounds excluded by provisos (M2) and (M3) according to claim 1, for preparing a medicament which is suitable for the prevention and/or treatment of illnesses and/or disorders which accompany obesity, particularly diabetes, especially type II diabetes, complications of diabetes including diabetic retinopathy, diabetic neuropathy, diabetic nephropathy, insulin resistance, pathological glucose tolerance, cardiac insufficiency, cardiovascular diseases, particularly arteriosclerosis and high blood pressure, arthritis and gonitis.

**25.** Use of at least one amide compound according to one or more of claims 1 to 15 and/ or a salt according to claim 16, including the compounds excluded by provisos (M2) and (M3) according to claim 1, for preparing a medicament which is suitable for the prevention and/or treatment of hyperlipidaemia, fat accumulation, emotional disorders, affective disorders, depression and anxiety.

**26.** Medicament, comprising
a first active substance selected from the amide compounds according to one or more of claims 1 to 15 and/ or a salt according to claim 16, including the compounds excluded by provisos (M2) and (M3) according to claim 1, and a second active substance selected from the group consisting of active substances for the treatment of diabetes, active substances for the treatment of diabetic complications, active substances for the treatment of obesity, preferably other than MCH antagonists, active substances for the treatment of high blood pressure, active substances for the treatment of hyperlipidaemia, including arteriosclerosis, active substances for the treatment of arthritis, active substances for the treatment of anxiety states and active substances for the treatment of depression,
optionally together with one or more inert carriers and/or diluents.

## Revendications

**1.** Composés amides de formule générale I

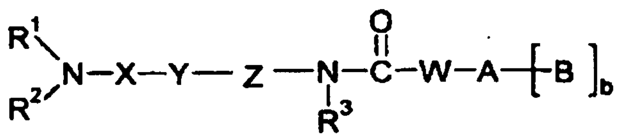

I

où
$R^1$, $R^2$ représentent indépendamment l'un de l'autre H, un groupe $C_{1-8}$-alkyle ou $C_{3-7}$-cycloalkyle éventuellement substitué avec le groupement $R^{11}$, où un groupe $-CH_2-$ en position 3 ou 4 d'un groupe cycloalkyle à 5, 7 ou 7 chaînons peut être remplacé par -O-, -S- ou $-NR^{13}-$, ou un groupement phényle ou pyridinyle éventuellement substitué une ou plusieurs fois avec le groupement $R^{12}$ et/ou une fois avec nitro, avec la condition qu'au moins l'un des groupements $R^1$, $R^2$ présente une signification différente de H, ou
$R^1$ et $R^2$ forment un pont $C_{2-8}$-alkylène où

- un ou deux groupes $-CH_2-$ peuvent être remplacés indépendamment l'un de l'autre par -CH=N- ou -CH=CH- et/ou
- un ou deux groupes $-CH_2-$ peuvent être remplacés indépendamment l'un de l'autre par -O-, -S-, -SO, $-(SO_2)-$, $-C=N-O-R^{18}$, -CO-, $-C(=CH_2)-$ ou
- $NR^{13}-$ de telle manière que les hétéroatomes ne sont pas liés entre eux directement,

où, dans le pont alkylène défini précédemment, un ou plusieurs atomes H peuvent être remplacés par $R^{14}$, et où le pont alkylène défini précédemment peut être substitué avec un ou deux groupes carbo- ou hétérocycliques Cy identiques ou différents de telle manière que la liaison entre le pont alkylène et le groupe Cy a lieu

- via une simple ou double liaison,
- via un atome C commun avec formation d'un système cyclique spirocyclique,
- via deux atomes C et/ou N voisins communs avec formation d'un système cyclique bicyclique condensé ou
- via trois ou plusieurs atomes C et/ou N avec formation d'un système cyclique ponté,

$R^3$ représente H, $C_{1-6}$-alkyle, $C_{3-7}$-cycloalkyle ou $C_{3-7}$cycloalkyl-$C_{1-4}$-alkyle,
X représente un pont $C_{1-4}$-alkylène non ramifié et
au cas où le groupe Y est lié à X via un atome C, représente aussi $-CH_2-CH=CH-$, $-CH_2-C\equiv C-$, $C_{2-4}$-alkylèneoxy ou $C_{2-4}$-alkylène-$NR^4$-,

où le pont X peut être lié à $R^1$ avec inclusion de l'atome N lié à $R^1$ et X avec formation d'un groupe hétérocyclique, où le pont X peut en outre être lié à $R^2$ avec inclusion de l'atome N lié à $R^2$ et X avec formation d'un groupe hétérocyclique, et

où un atome C peut être substitué avec $R^{10}$ et/ou un ou deux atomes C peuvent être substitués dans chaque cas avec un ou deux substituants identiques ou différents choisis parmi $C_{1-6}$-alkyle, $C_{2-6}$-alcényle, $C_{2-6}$-alcynyle, $C_{3-7}$-cycloalkyle, $C_{3-7}$-cycloalkyl-$C_{1-3}$-alkyle, $C_{4-7}$-cycloalcényle et $C_{4-7}$-cycloalcényl-$C_{1-3}$-alkyle, où deux substituants alkyle et/ou alcényle peuvent être liés l'un à l'autre en formant un système cyclique carbocyclique, avec la condition que le groupe X dans la signification $C_{2-4}$-alkylèneoxy ne présente aucun substituant hydroxy ; et

W représente $-CR^{6a}R^{6b}-O-$, $-CR^{7a}=CR^{7c}-$, $-CR^{6a}R^{6b}-NR^8-$, $-CR^{7a}R^{7b}-CR^{7c}R^{7d}-$ ou $-NR^8-CR^{6a}R^{6b}-$,

Z représente une simple liaison, $C_{1-4}$-alkylène, où deux atomes C voisins peuvent être liés l'un à l'autre avec un pont $C_{1-4}$-alkylène supplémentaire,

où un atome C du pont alkylène peut être substitué avec $R^{10}$ et/ou un ou deux atomes C peuvent être substitués indépendamment l'un de l'autre avec un ou deux groupements $C_{1-6}$-alkyle identiques ou différents, où deux groupements alkyle peuvent être liés l'un à l'autre en formant un cycle carbocyclique, et

Y est choisi dans le groupe des groupes cycliques divalents

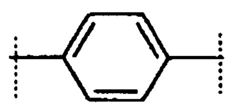 , 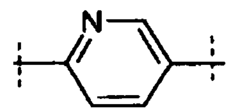 ,

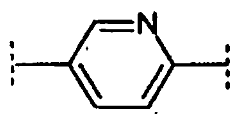 , 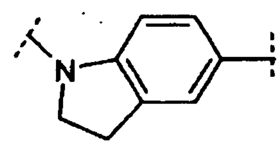 ,

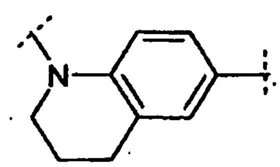 , 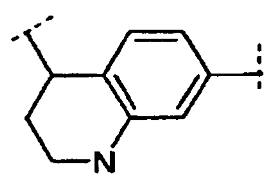 ,

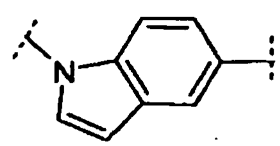 , 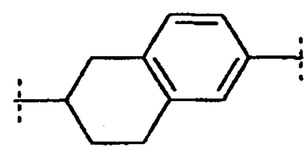 ,

où les groupes cycliques cités précédemment peuvent être substitués une ou plusieurs fois sur un ou plusieurs atomes C avec $R^{20}$, dans le cas d'un groupe phényle également en outre une fois avec nitro, et/ou un ou plusieurs groupes NH peuvent être substitués avec $R^{21}$, et

A a l'une des significations indiquées pour Cy,

B a l'une des significations indiquées pour Cy,

b a la valeur 0 ou 1,

Cy représente un groupe carbo- ou hétérocyclique choisi parmi l'une des significations suivantes

- un groupe carbocyclique à 3 à 7 chaînons saturé,
- un groupe carbocyclique à 4 à 7 chaînons insaturé,
- un groupe phényle,
- un groupe hétérocyclique à 4 à 7 chaînons saturé ou à 5 à 7 chaînons insaturé avec un atome N, O ou S

comme hétéroatome,

- un groupe hétérocyclique à 5 à 7 chaînons saturé ou insaturé avec deux ou plusieurs atomes N ou avec un deux atomes N et un atome O ou S comme hétéroatomes,
- un groupe hétérocyclique aromatique à 5 à 6 chaînons avec un ou plusieurs hétéroatomes identiques ou différents choisis parmi N, O et/ou S,

où les groupes à 4, 5, 6 ou 7 chaînons cités précédemment peuvent être liés par condensation via deux atomes C voisins communs avec un cycle phényle ou pyridine, et
où, dans les groupes à 5, 6 ou 7 chaînons cités précédemment, un ou deux groupes $-CH_2-$ non voisins peuvent être remplacés indépendamment l'un de l'autre par un groupe -CO-, $-C(=CH_2)-$, -(SO)- ou $-(SO_2)-$, et
où les groupes à 6 ou 7 chaînons saturés cités précédemment peuvent aussi être sous forme de systèmes cycliques pontés avec un pont imino, N-($C_{1-4}$-alkyl)-imino, méthylène, $C_{1-4}$-alkyl-méthylène ou di-($C_{1-4}$-alkyl)-méthylène, et
où les groupes cycliques cités précédemment peuvent être substitués une ou plusieurs fois sur un ou plusieurs atomes C avec $R^{20}$, dans le cas d'un groupe phényle également en outre une fois avec nitro, et/ou un ou plusieurs groupes NH peuvent être substitués avec $R^{21}$,
$R^4$ représente H, $C_{1-6}$-alkyle ou $C_{2-6}$-alcényle,
$R^{6a}$, $R^{6b}$ représentent H, $C_{1-4}$-alkyle ou $CF_3$,
$R^{7a}$, $R^{7b}$,
$R^{7c}$, $R^{7d}$ représentent H, F, Cl, $C_{1-4}$-alkyle ou $CF_3$,
$R^8$ représente H, $C_{1-4}$-alkyle, $C_{3-7}$-cycloalkyle ou $C_{3-7}$-cycloalkyl-$C_{1-3}$-alkyle,
$R^{10}$ représente hydroxy, ω-hydroxy-$C_{1-3}$-alkyle, $C_{1-4}$-alcoxy, ω-($C_{1-4}$-alcoxy)-$C_{1-3}$-alkyle, carboxy, $C_{1-4}$-alcoxycarbonyle, amino, $C_{1-4}$-alkyl-amino, di-($C_{1-4}$-alkyl)-amino, cyclo-$C_{3-6}$-alkylèneimino, amino-$C_{1-3}$-alkyle, $C_{1-4}$-alkyl-amino-$C_{1-3}$-alkyle, di-($C_{1-4}$-alkyl)-amino-$C_{1-3}$-alkyle, cyclo-$C_{3-6}$-alkylèneimino-$C_{1-3}$-alkyle, amino-$C_{1-3}$-alcoxy, $C_{1-4}$-alkyl-amino-$C_{1-3}$-alcoxy, di-($C_{1-4}$-alkyl)-amino-$C_{1-3}$-alcoxy ou cyclo-$C_{3-6}$-alkylèneimino-$C_{1-3}$-alcoxy, aminocarbonyle, $C_{1-4}$-alkyl-aminocarbonyle, di-($C_{1-4}$-alkyl)-aminocarbonyle ou cyclo-$C_{3-6}$-alkylèneimino-carbonyle,
$R^{11}$ représente $C_{2-6}$-alcényle, $C_{2-6}$-alcynyle, $R^{15}$-O-, $R^{15}$-O-CO-, $R^{15}$-CO-O-, $R^{16}R^{17}$N-, $R^{18}R^{19}$N-CO- ou Cy-,
$R^{12}$ présente l'une des significations indiquées pour $R^{20}$,
$R^{13}$ présente l'une des significations indiquées pour $R^{17}$, à l'exception de carboxy,
$R^{14}$ représente halogène, $C_{1-6}$-alkyle, $C_{2-6}$-alcényle, $C_{2-6}$-alcynyle, $R^{15}$-O-, $R^{15}$-O-CO-, $R^{15}$-CO-, $R^{15}$-CO-O-, $R^{16}R^{17}$N-, $R^{18}R^{19}$N-CO-, $R^{15}$-O-$C_{1-3}$-alkyle, $R^{15}$-O-CO-$C_{1-3}$-alkyle, $R^{15}$-O-CO-NH-, $R^{15}SO_2$-NH-, $R^{15}$-O-CO-NH-$C_{1-3}$-alkyle, $R^{15}$-$SO_2$-NH-$C_{1-3}$-alkyle, $R^{15}$-CO-$C_{1-3}$-alkyle, $R^{15}$-CO-O-$C_{1-3}$-alkyle, $R^{16}R^{17}$N-$C_{1-3}$-alkyle, $R^{18}R^{19}$N-CO-$C_{1-3}$-alkyle ou Cy-$C_{1-3}$-alkyle,
$R^{15}$ représente H, $C_{1-4}$-alkyle, $C_{3-7}$-cycloalkyle, $C_{3-7}$-cycloalkyl-$C_{1-3}$-alkyle, phényle, phényl-$C_{1-3}$-alkyle, pyridinyle ou pyridinyl-$C_{1-3}$-alkyle,
$R^{16}$ représente H, $C_{1-6}$-alkyle, $C_{3-7}$-cycloalkyle, $C_{3-7}$-cycloalkyl-$C_{1-3}$-alkyle, $C_{4-7}$-cycloalcényle, $C_{4-7}$-cycloalcényl-$C_{1-3}$-alkyle, ω-hydroxy-$C_{2-3}$-alkyle, ω-($C_{1-4}$-alcoxy)-$C_{2-3}$-alkyle, amino-$C_{2-6}$-alkyle, $C_{1-4}$-alkyl-amino-$C_{2-6}$-alkyle, di-($C_{1-4}$-alkyl)-amino-$C_{2-6}$-alkyle ou cyclo-$C_{3-6}$-alkylèneimino-$C_{2-6}$-alkyle, $R^{17}$ représente l'une des significations indiquées pour $R^{16}$ ou phényle, phényl-$C_{1-3}$-alkyle, pyridinyle, dioxolan-2-yle, -CHO, $C_{1-4}$-alkylcarbonyle, carboxy, hydroxycarbonyl-$C_{1-3}$-alkyle, $C_{1-4}$-alcoxycarbonyle, $C_{1-4}$-alcoxycarbonyl-$C_{1-3}$-alkyle, $C_{1-4}$-alkylcarbonylamino-$C_{2-3}$-alkyle, N-($C_{1-4}$-alkylcarbonyl)-N-($C_{1-4}$-alkyl)-amino-$C_{2-3}$-alkyle, $C_{1-4}$-alkylsulfonyle, $C_{1-4}$-alkylsulfonylamino-$C_{2-3}$-alkyle ou N-($C_{1-4}$-alkylsulfonyl)-N-($C_{1-4}$-alkyl)-amino-$C_{2-3}$-alkyle,
$R^{18}$, $R^{19}$ représentent indépendamment l'un de l'autre H ou $C_{1-6}$-alkyle,
$R^{20}$ représente halogène, hydroxy, cyano, $C_{1-6}$-alkyle, $C_{2-6}$-alcényle, $C_{2-6}$-alcynyle, $C_{3-7}$-cycloalkyle, $C_{3-7}$-cycloalkyl-$C_{1-3}$-alkyle, hydroxy-$C_{1-4}$-alkyle, $R^{22}$-$C_{1-3}$-alkyle ou l'une des significations indiquées pour $R^{22}$,
$R^{21}$ représente $C_{1-4}$-alkyle, ω-hydroxy-$C_{2-3}$-alkyle, ω-$C_{1-4}$-alcoxy-$C_{2-6}$-alkyle, ω-$C_{1-4}$-alkyl-amino-$C_{2-6}$-alkyle, ω-di-($C_{1-4}$-alkyl)-amino-$C_{2-6}$-alkyle, ω-cyclo-$C_{3-6}$-alkylèneimino-$C_{2-6}$-alkyle, phényl-$C_{1-3}$-alkyle, $C_{1-4}$-alkyl-carbonyle, $C_{1-4}$-alcoxycarbonyle ou $C_{1-4}$-alkylsulfonyle,
$R^{22}$ représente phényl-$C_{1-3}$-alcoxy, OHC-, HO-N=HC-, $C_{1-4}$-alcoxy-N=HC-, $C_{1-4}$-alcoxy, $C_{1-4}$-alkylthio, carboxy, $C_{1-4}$-alkylcarbonyle,$C_{1-4}$-alcoxycarbonyle,aminocarbonyle,$C_{1-4}$-alkylaminocarbonyle,di-($C_{1-4}$-alkyl)-aminocarbonyle, cyclo-$C_{3-6}$-alkyl-amino-carbonyle, cyclo-$C_{3-6}$-alkylèneimino-carbonyle, cyclo-$C_{3-6}$-alkylèneimino-$C_{2-4}$-alkyl-amino-carbonyle, phényl-amino-carbonyle, $C_{1-4}$-alkyl-sulfonyle, $C_{1-4}$-alkyl-sulfinyle, $C_{1-4}$-alkylsulfonylamino, amino, $C_{1-4}$-alkylamino, di-($C_{1-4}$-alkyl)-amino, $C_{1-4}$-alkyl-carbonyl-amino, cyclo-$C_{3-6}$-alkylèneimino, phényl-$C_{1-3}$-alkylamino ou N-($C_{1-4}$-alkyl)-phényl-$C_{1-3}$-alkylamino, acétylamino, propionylamino, phénylcarbonyl-amino, phénylcarbonylméthylamino, hydroxyalkylaminocarbonyle, (4-morpholinyl)carbonyle, (1-pyrrolidinyl)-carbonyle, (1-pipéridinyl)carbonyle, (hexahydro-1-azépinyl)carbonyle, (4-méthyl-1-pipérazinyl)carbonyle, méthylène-dioxy, aminocarbonylamino ou alkylaminocarbonylamino,
où, dans les groupes et groupements cités précédemment, en particulier dans A, B, W, X, Y, Z, $R^1$ à $R^4$, $R^{6a}$, $R^{6b}$, $R^{7a}$ , $R^{7b}$, $R^{7c}$, $R^{7d}$, $R^8$, $R^{10}$ à $R^{22}$, dans chaque cas un

ou plusieurs atomes C peuvent être substitués en outre une ou plusieurs fois avec F et/ou dans chaque cas un ou deux atomes C peuvent être substitués indépendamment l'un de l'autre en outre une fois avec Cl ou Br et/ou dans chaque cas un ou plusieurs cycles phényle peuvent comporter indépendamment les uns des autres en outre un, deux ou trois substituants choisis dans le groupe F, Cl, Br, I, $C_{1-4}$-alkyle, $C_{1-4}$-alcoxy, difluorométhyle, trifluorométhyle, hydroxy, amino, $C_{1-3}$-alkylamino, di-($C_{1-3}$-alkyl)-amino, acétylamino, aminocarbonyle, cyano, difluorométhoxy, trifluorométhoxy, amino-$C_{1-3}$-alkyle, $C_{1-3}$-alkylamino-$C_{1-3}$-alkyle et di-($C_{1-3}$-alkyl)-amino-$C_{1-3}$-alkyle et/ou peuvent être substitués une fois avec nitro, et
l'atome H d'un groupe carboxy présent ou un atome H lié à un atome N peut être remplacé dans chaque cas par un groupement clivable in vivo,
leurs tautomères, leurs stéréoisomères, leurs énantiomères, leurs mélanges et leurs sels,
avec les mesures (M2) et (M3) suivantes
(M2) au cas où W représente -CH=CH- et Y représente un groupe phénylène et Z est une simple liaison, les ponts X et Z sont situés en position para l'un par rapport à l'autre sur le cycle phénylène du groupe Y et au moins l'une des conditions suivantes est remplie :

(a) le groupe Y dans la signification phénylène est substitué au moins une fois,
(b) b possède la valeur 0 et le groupement A est substitué au moins deux fois,
(c) b possède la valeur 1 ;

(M3) que les composés individuels suivants ne soient pas inclus :

N-[4-(2-diéthylamino-éthoxy)-phényl]-3-phényl-propionamide,
N-[4-(2-morpholin-4-yléthoxy)-phényl]-3-phényl-propionamide,
3-(4-chloro-phényl)-N-{2-[4-(2-diéthylamino-éthoxy)-phényl]-éthyl}-acrylamide,
N-{2-[3-(4-{2-[2-(4-chloro-phénoxy)-acétylamino]-éthyl}-phénoxy)-2-hydroxypropylamino]-éthyl}-isobutyramide,
{2-[3-(4-{2-[2-(4-chloro-phénoxy)-acétylamino]-éthyl}-phénoxy)-2-hydroxypropylamino]-éthyl}-amide d'acide cyclopentanecarboxylique,
2-(4-chloro-phénoxy)-N-(2-{4-[2-hydroxy-3-(2-phénylacétylamino-éthylamino)-propoxy]-phényl}-éthyl)-acétamide.

**2.** Composés amides selon la revendication 1 **caractérisés en ce que** $R^1$, $R^2$ représentent indépendamment l'un de l'autre H, un groupe $C_{1-8}$-alkyle ou $C_{3-7}$-cycloalkyle éventuellement substitué avec le groupement $R^{11}$, ou un groupement phényle éventuellement substitué une ou plusieurs fois avec le groupement $R^{12}$ et/ou une fois avec nitro, avec la condition qu'au moins l'un des groupements $R^1$, $R^2$ présente une signification différente de H, ou
$R^1$ et $R^2$ forment un pont $C_{2-8}$-alkylène où

- un ou deux groupes -$CH_2$- peuvent être remplacés indépendamment l'un de l'autre par -CH=N- ou -CH=CH- et/ou
- un ou deux groupes -$CH_2$- peuvent être remplacés indépendamment l'un de l'autre par -O-, -S-, -CO-, -C(=$CH_2$)- ou -$NR^{13}$- de telle manière que les hétéroatomes ne sont pas liés entre eux directement,

où, dans le pont alkylène défini précédemment, un ou plusieurs atomes H peuvent être remplacés par $R^{14}$, et
où le pont alkylène défini précédemment peut être substitué avec un ou deux groupes carbo- ou hétérocycliques Cy identiques ou différents de telle manière que la liaison entre le pont alkylène et le groupe Cy a lieu

- via une simple ou double liaison,
- via un atome C commun avec formation d'un système cyclique spirocyclique,
- via deux atomes C et/ou N voisins communs avec formation d'un système cyclique bicyclique condensé ou
- via trois ou plusieurs atomes C et/ou N avec formation d'un système cyclique ponté,

X représente un pont $C_{1-4}$-alkylène non ramifié et
au cas où le groupe Y est lié à X via un atome C, représente aussi -$CH_2$-CH=CH-, -$CH_2$-C≡C-, $C_{2-4}$-alkylèneoxy ou $C_{2-4}$-alkylène-$NR^4$-,
où le pont X peut être lié à $R^1$ avec inclusion de l'atome N lié à $R^1$ et X avec formation d'un groupe hétérocyclique, et où un atome C peut être substitué avec $R^{10}$ et/ou un ou deux atomes C peuvent être substitués dans chaque cas avec un ou deux groupements $C_{1-6}$-alkyle identiques ou différents, avec la condition que le groupe X dans la signification $C_{2-4}$-alkylèneoxy ne présente aucun substituant hydroxy ; et

Z représente une simple liaison, $C_{1-4}$-alkylène, où deux atomes C voisins peuvent être liés l'un à l'autre avec un pont $C_{1-4}$-alkylène supplémentaire,
où un atome C du pont alkylène peut être substitué avec $R^{10}$ et/ou un ou deux atomes C peuvent être substitués indépendamment l'un de l'autre avec un ou deux groupements $C_{1-6}$-alkyle identiques ou différents, et
b a la valeur 0, $R^{10}$ représente hydroxy, ω-hydroxy-$C_{1-3}$-alkyle, $C_{1-4}$-alcoxy, ω-($C_{1-4}$-alcoxy)-$C_{1-3}$-alkyle, amino, $C_{1-4}$-alkyl-amino, di-($C_{1-4}$-alkyl)-amino, cyclo-$C_{3-6}$-alkylèneimino, amino-$C_{1-3}$-alkyle, $C_{1-4}$-alkyl-amino-$C_{1-3}$-alkyle, di-($C_{1-4}$-alkyl)-amino-$C_{1-3}$-alkyle, cyclo-$C_{3-6}$-alkylèneimino-$C_{1-3}$-alkyle, amino-$C_{1-3}$-alcoxy, $C_{1-4}$-alkyl-amino-$C_{1-3}$-alcoxy, di-($C_{1-4}$-alkyl)-amino-$C_{1-3}$-alcoxy ou cyclo-$C_{3-6}$-alkylèneimino-$C_{1-3}$-alcoxy,
$R^{14}$ représente halogène, $C_{1-6}$-alkyle, $R^{15}$-O-, $R^{15}$-O-CO-, $R^{15}$-CO-, $R^{15}$-CO-O-, $R^{16}R^{17}$N-, $R^{18}R^{19}$-CO-, $R^{15}$-O-$C_{1-3}$-alkyle, $R^{15}$-O-CO-$C_{1-3}$-alkyle, $R^{15}$-CO-$C_{1-3}$-alkyle, $R^{15}$-CO-O-$C_{1-3}$-alkyle, $R^{16}R^{17}$N-$C_{1-3}$-alkyle, $R^{18}R^{19}$N-CO-$C_{1-3}$-alkyle ou Cy-$C_{1-3}$-alkyle,
$R^{15}$ représente H, $C_{1-4}$-alkyle, $C_{3-7}$-cycloalkyle, $C_{3-7}$-cycloalkyl-$C_{1-3}$-alkyle, phényle ou phényl-$C_{1-3}$-alkyle,
$R^{17}$ présente l'une des significations indiquées pour $R^{16}$ ou phényle, phényl-$C_{1-3}$-alkyle, $C_{1-4}$-alkylcarbonyle, hydroxycarbonyl-$C_{1-3}$-alkyle, $C_{1-4}$-alcoxycarbonyl-amino-$C_{2-3}$-alkyle, N-($C_{1-4}$-alkylcarbonyl)-N-($C_{1-4}$-alkyl)-amino-$C_{2-3}$-alkyle, $C_{1-4}$-alkylsulfonyle, $C_{1-4}$-alkylsulfonylamino-$C_{2-3}$-alkyle ou N-($C_{1-4}$-alkylsulfonyl)-N-($C_{1-4}$-alkyl)-amino-$C_{2-3}$-alkyle,
$R^{20}$ représente halogène, hydroxy, cyano, $C_{1-6}$-alkyle, $C_{3-7}$-cycloalkyle, $C_{3-7}$-cycloalkyl-$C_{1-3}$-alkyle, hydroxy-$C_{1-4}$-alkyle, $R^{22}$-$C_{1-3}$-alkyle ou l'une des significations indiquées pour $R^{22}$,
$R^{21}$ représente $C_{1-4}$-alkyle, ω-hydroxy-$C_{2-3}$-alkyle, ω-$C_{1-4}$-alcoxy-$C_{2-6}$-alkyle, ω-$C_{1-4}$-alkyl-amino-$C_{2-6}$-alkyle, ω-di-($C_{1-4}$-alkyl)-amino-$C_{2-6}$-alkyle, ω-cyclo-$C_{3-6}$-alkylèneimino-$C_{2-6}$-alkyle, phényle, phényl-$C_{1-3}$-alkyle, $C_{1-4}$-alkylcarbonyle, carboxy, $C_{1-4}$-alcoxy-carbonyle ou $C_{1-4}$-alkylsulfonyle,
$R^{22}$ représente phényle, phényl-$C_{1-3}$-alcoxy, $C_{1-4}$-alcoxy, $C_{1-4}$-alkylthio, carboxy, $C_{1-4}$-alkylcarbonyle, $C_{1-4}$-alcoxycarbonyle, aminocarbonyle, $C_{1-4}$-alkylaminocarbonyle, di-($C_{1-4}$-alkyl)-aminocarbonyle, cyclo-$C_{3-6}$-alkylèneiminocarbonyle, $C_{1-4}$-alkyl-sulfonyle, $C_{1-4}$-alkyl-sulfinyle, $C_{1-4}$-alkylsulfonylamino, amino, $C_{1-4}$-alkylamino, di-($C_{1-4}$-alkyl)-amino, cyclo-$C_{3-6}$-alkylèneimino, phényl-$C_{1-3}$-alkylamino ou N-($C_{1-4}$-alkyl)-phényl-$C_{1-3}$-alkylamino, acétylamino, propionylamino, phénylcarbonylamino, phénylcarbonylméthylamino, hydroxyalkylaminocarbonyle, (4-morpholinyl) carbonyle, (1-pyrrolidinyl)-carbonyle, (1-pipéridinyl)carbonyle, (hexahydro-1-azépinyl)carbonyle, (4-méthyl-1-pipérazinyl)carbonyle, méthylènedioxy, aminocarbonylamino ou alkylaminocarbonylamino,
où les groupes et groupements $R^3$, $R^4$, $R^{6a}$, $R^{6b}$, $R^{7a}$, $R^{7b}$, $R^{7c}$, $R^{7d}$, $R^8$, $R^{11}$, $R^{12}$, $R^{16}$, $R^{18}$, $R^{19}$, W, Y, A, Cy possèdent la signification indiquée dans la revendication 1.

3. Composés amides selon la revendication 1 ou 2 **caractérisés en ce que** $R^1$, $R^2$ représentent indépendamment l'un de l'autre H, $C_{1-6}$-alkyle, $C_{3-7}$-cycloalkyle, $C_{3-7}$-cycloalkyl-$C_{1-3}$-alkyle, ω-hydroxy-$C_{2-3}$-alkyle, ω-($C_{1-4}$-alcoxy)-$C_{2-3}$-alkyle, $C_{1-4}$-alcoxy-carbonyl-$C_{1-4}$-alkyle, carboxyl-$C_{1-4}$-alkyle, amino-$C_{2-4}$-alkyle, $C_{1-4}$-alkyl-amino-$C_{2-4}$-alkyle, di-($C_{1-4}$-alkyl)-amino-$C_{2-4}$-alkyle, cyclo-$C_{3-6}$-alkylèneimino-$C_{2-4}$-alkyle, pyrrolidinyle, N-($C_{1-4}$-alkyl)-pyrrolidinyle, pyrrolidinyl-$C_{1-3}$-alkyle, N-($C_{1-4}$-alkyl)-pyrrolidinyl-$C_{1-3}$-alkyle, pipéridinyle, N-($C_{1-4}$-alkyl)-pipéridinyle, pipéridinyl-$C_{1-3}$-alkyle, N-($C_{1-4}$-alkyl)-pipéridinyl-$C_{1-3}$-alkyle, phényle, phényl-$C_{1-3}$-alkyle, pyridyle ou pyridyl-$C_{1-3}$-alkyle, où, dans les groupes et groupements cités précédemment, un ou plusieurs atomes C peuvent être substitués une ou plusieurs fois avec F et/ou un ou deux atomes C peuvent être substitués indépendamment l'un de l'autre une fois avec Cl ou Br et
où le groupement phényle ou pyridyle peut être substitué une ou plusieurs fois avec le groupement $R^{12}$ défini dans la revendication 1 et/ou une fois avec nitro, avec la condition qu'au moins l'un des groupements $R^1$, $R^2$ présente une signification différente de H.

4. Composés amides selon une ou plusieurs des revendications 1 à 3 **caractérisés en ce que** $R^1$ et $R^2$ forment un pont alkylène selon la revendication 1 de telle manière que $R^1R^2$N- forme un groupe choisi parmi azétidine, pyrrolidine, pipéridine, azépane, 2,5-dihydro-1H-pyrrole, 1,2,3,6-tétrahydropyridine, 2,3,4,7-tétrahydro-1H-azépine, 2,3,6,7-tétrahydro-1H-azépine, pipérazine, où la fonction imine libre est substituée avec $R^{13}$, pipéridin-4-one-oxime, pipérin-4-one-O-$C_{1-4}$-alkyl-oxime, morpholine et thiomorpholine,
où selon la revendication 1 un ou plusieurs atomes H peuvent être remplacés par $R^{14}$, et/ou peut être substitué de la manière indiquée dans la revendication 1 avec un ou deux groupes carbo- ou hétérocycliques Cy identiques ou différents, et/ou
où $R^{13}$, $R^{14}$ et Cy possèdent la signification indiquée dans la revendication 1 ou 2.

5. Composés amides selon une ou plusieurs des revendications précédentes **caractérisés en ce que** le groupe

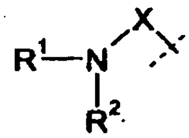

possède une signification selon l'une des formules partielles suivantes

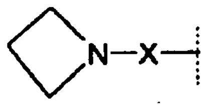 , 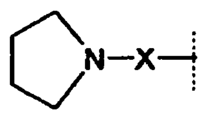 , 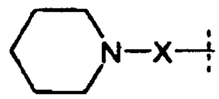 ,

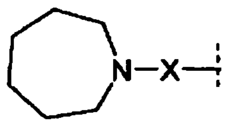 , 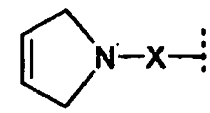 , 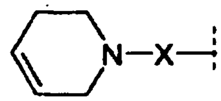 ,

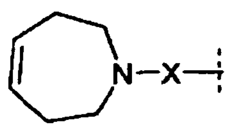 , 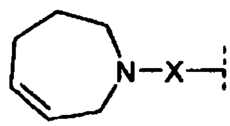 , 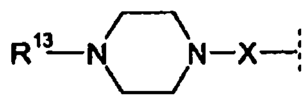 ,

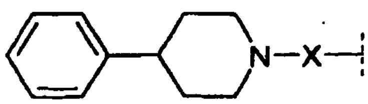 , 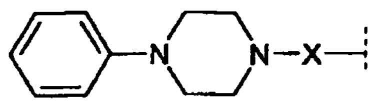 ,

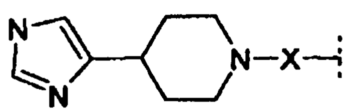 ,

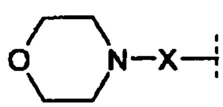 , 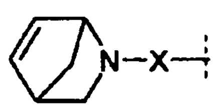 , 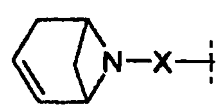 ,

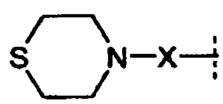 , 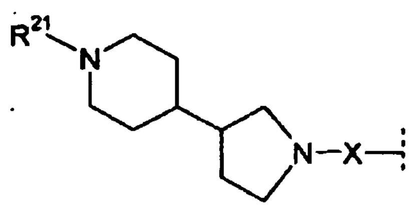 ,

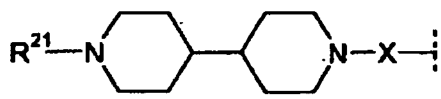 , 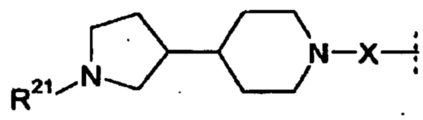 ,

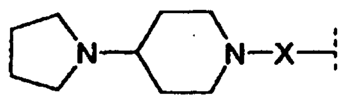 , 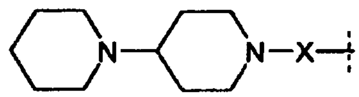 ,

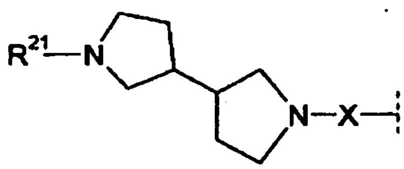 , 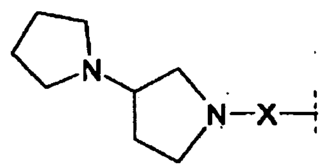 ,

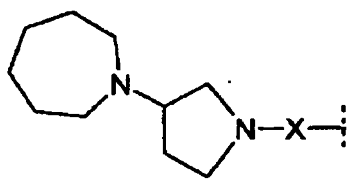 ,

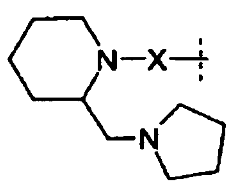 , 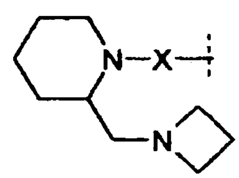 ,

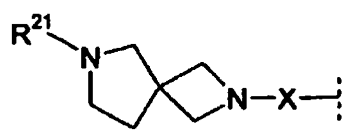 , ,

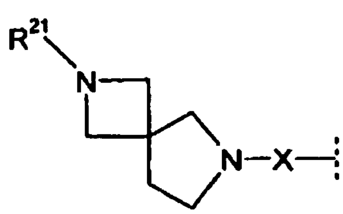 , 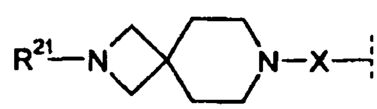 ,

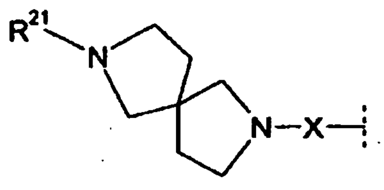 , 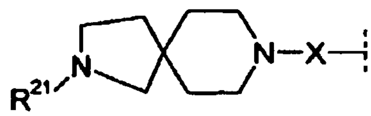 ,

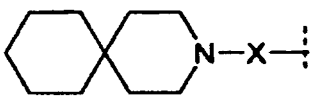 , 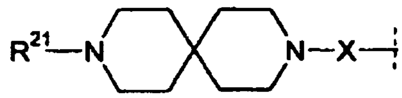 ,

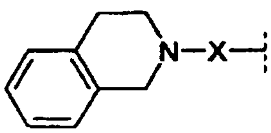 , 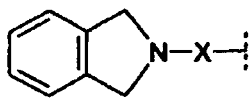 , 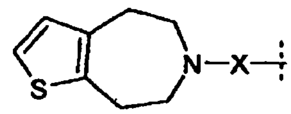 ,

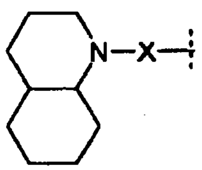 , 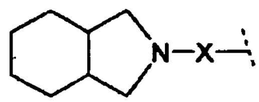 , 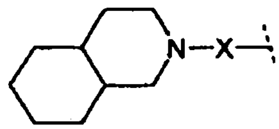 ,

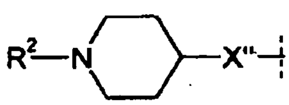 , 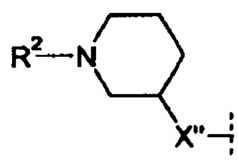 , 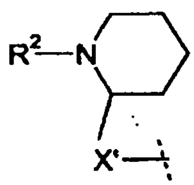 ,

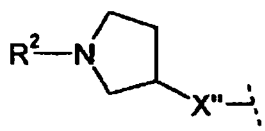 , 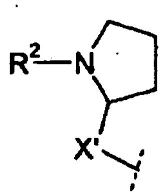 ,

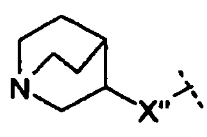 , 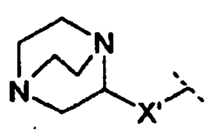 , 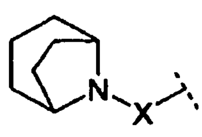 ,

où un ou plusieurs atomes H de l'hétérocycle formé par le groupe $R^1R^2N$- peuvent être remplacés par $R^{14}$ et le cycle lié à l'hétérocycle formé par le groupe $R^1R^2N$-peut être substitué une ou plusieurs fois sur un ou plusieurs atomes C avec $R^{20}$, dans le cas d'un cycle phényle également en outre une fois avec nitro et
X', X" représentent indépendamment l'un de l'autre une simple liaison ou $C_{1-3}$-alkylène et
au cas où le groupe Y est lié à X' ou X" via un atome C, également -$C_{1-3}$-alkylène-O-, -$C_{1-3}$-alkylène-NH- ou

$-C_{1-3}$-alkylène-N($C_{1-3}$-alkyle)-, et
où dans les significations citées précédemment pour X', X" dans chaque cas un atome C peut être substitué avec $R^{10}$ et/ou un ou deux atomes C peuvent être substitués chacun avec un ou deux substituants identiques ou différents choisis parmi $C_{1-6}$-alkyle, $C_{2-6}$-alcényle, $C_{2-6}$-alcynyle, $C_{3-7}$-cycloalkyle, $C_{3-7}$-cycloalkyl-$C_{1-3}$-alkyle, $C_{4-7}$-cycloalcényle et $C_{4-7}$-cycloalcényl-$C_{1-3}$-alkyle, où deux substituants alkyle et/ou alcényle peuvent être liés l'un à l'autre avec formation d'un système cyclique carbocyclique, et
où dans X', X" indépendamment l'un de l'autre dans chaque cas un ou plusieurs atomes C peuvent être substitués une ou plusieurs fois avec F et/ou dans chaque cas un ou deux atomes C peuvent être substitués indépendamment l'un de l'autre une fois avec Cl ou Br et
où $R^2$, $R^{10}$, $R^{13}$, $R^{14}$, $R^{20}$, $R^{21}$ et X possèdent les significations indiquées dans la revendication 1 ou 2.

**6.** Composés amides selon une ou plusieurs des revendications précédentes **caractérisés en ce que** X représente $-CH_2-$, $-CH_2-CH_2-$ ou $-CH_2-CH_2-CH_2-$ et
au cas où le groupe Y est lié à X via un atome C, également $-CH_2-CH=CH-$, $-CH_2-C\equiv C-$, $-CH_2-CH_2-O-$, $-CH_2-CH_2-CH_2-O-$, $-CH_2-CH_2-NR^4-$ ou $-CH_2-CH_2-CH_2-NR^4-$,
où le pont X peut être lié à $R^1$ avec inclusion de l'atome N lié à $R^1$ et X avec formation d'un groupe hétérocyclique, et où dans X un atome C peut être substitué avec un groupement hydroxy, ω-hydroxy-$C_{1-3}$-alkyle, ω-($C_{1-4}$-alcoxy)-$C_{1-3}$-alkyle et/ou $C_{1-4}$-alcoxy, et/ou un ou deux atomes C peuvent être substitués chacun avec un ou deux substituants identiques ou différents choisis parmi $C_{1-6}$-alkyle, $C_{3-7}$-cycloalkyle et $C_{3-7}$-cycloalkyl-$C_{1-3}$-alkyle, où deux substituants alkyle peuvent être liés l'un à l'autre avec formation d'un système cyclique carbocyclique, et
où dans chaque cas un ou plusieurs atomes C peuvent être substitués une ou plusieurs fois avec F et/ou dans chaque cas un ou deux atomes C peuvent être substitués indépendamment l'un de l'autre une fois avec Cl ou Br et avec la condition que le groupe X dans la signification $C_{2-4}$-alkylèneoxy ne comporte aucun substituant hydroxy ;
où $R^1$, $R^4$ et $R^{10}$ possèdent l'une des significations indiquées dans la revendication 1 ou 2.

**7.** Composés amides selon une ou plusieurs des revendications précédentes **caractérisés en ce que** Z est une simple liaison, $-CH_2-$ ou $-CH_2-CH_2-$, où un ou deux atomes C peuvent être substitués indépendamment l'un de l'autre une ou deux fois avec F, $CH_3$ ou $CF_3$ et/ou une fois avec Cl.

**8.** Composés amides selon une ou plusieurs des revendications précédentes **caractérisés en ce que** W représente $-CH_2-O-$, $-CH_2-NR^8-$, $-CH_2-CH_2-$ ou $-CH=CH-$,
où dans chaque cas un ou deux atomes C peuvent être substitués indépendamment l'un de l'autre avec F, $CH_3$ ou $CF_3$,
où $R^8$ possède l'une des significations indiquées dans la revendication 1 ou 2.

**9.** Composés amides selon une ou plusieurs des revendications précédentes **caractérisés en ce que** le groupe A représente phényle, pyridyle ou naphtyle,
où les groupes cycliques cités précédemment peuvent être substitués une ou plusieurs fois sur un ou plusieurs atomes C avec $R^{20}$, dans le cas d'un cycle phényle également en outre une fois avec nitro, et/ou un ou plusieurs groupes NH peuvent être substitués avec $R^{21}$, et
$R^{20}$ et $R^{21}$ possèdent les significations indiquées dans la revendication 1 ou 2.

**10.** Composés amides selon une ou plusieurs des revendications précédentes **caractérisés en ce que** b possède la valeur 0.

**11.** Composés amides selon une ou plusieurs des revendications précédentes **caractérisés en ce que** b possède la valeur 1 et B possède une signification choisie dans le groupe consistant en phényle, furanyle, thiényle et pyridyle, où les groupes cycliques cités précédemment peuvent être substitués une ou plusieurs fois sur un ou plusieurs atomes C avec $R^{20}$, dans le cas d'un cycle phényle également en outre une fois avec nitro, et
$R^{20}$ possède la signification indiquée dans la revendication 1 ou 2.

**12.** Composés amides selon une ou plusieurs des revendications précédentes **caractérisés en ce que**
A possède une signification selon la revendication 9,
B possède une signification selon la revendication 11 et
b possède la valeur 0 ou 1.

**13.** Composés amides selon la revendication 12 **caractérisés en ce que**
$R^1$, $R^2$ sont définis comme dans la revendication 3 ou 4,

X est défini comme dans la revendication 6,
W est défini comme dans la revendication 8 et
Z est défini comme dans la revendication 7.

**14.** Composés amides selon une ou plusieurs des revendications précédentes **caractérisés en ce que** $R^{20}$ représente F, Cl, Br, I, OH, cyano, $C_{1-4}$-alkyle, $C_{1-4}$-alcoxy, difluorométhyle, trifluorométhyle, difluorométhoxy, trifluorométhoxy, amino, $C_{1-3}$-alkyl-amino, di-$C_{1-3}$-alkyl-amino, carboxy ou $C_{1-4}$-alcoxy-carbonyle, où des substituants $R^{20}$ apparaissant plusieurs fois peuvent présenter des significations identiques ou différentes et dans le cas d'un cycle phényle celui-ci peut en outre être substitué aussi une fois avec nitro.

**15.** Composés amides selon la revendication 1 choisis dans le groupe des formules

(1) N-(3-chloro-4-(2-pipéridin-1-yl-éthoxy)-phényl]-2-(2-chloro-4-trifluoro-méthyl-phénoxy)-acétamide
(2) 2-(2-chloro-4-trifluorométhyl-phénoxy)-N-[3-cyano-4-(2-diéthylamino-éthoxy)-phényl]-acétamide
(3) 2-(2-chloro-4-trifluorométhyl-phénoxy)-N-(1-(2-diéthylamino-éthyl)-2,3-dihydro-1H-indol-5-yl]-acétamide
(4) N-(3-chloro-4-(3-diéthylamino-prop-1-ynyl)-phényl]-2-(2-chloro-4-trifluoro-méthyl-phénoxy)-acétamide
(5) 2-(2-chloro-4-trifluorométhyl-phénoxy)-N-[1-(2-diéthylamino-éthyl)-2,3-diméthyl-1H-indol-5-yl]-acétamide
(6) 2-(2-chloro-4-trifluorométhyl-phénoxy)-N-[1-(2-diéthylamino-éthyl)-1H-indol-5-yl]-acétamide
(7) 2-(2-chloro-4-trifluorométhyl-phénoxy)-N-[4-(2-diéthylamino-éthoxy)-3-méthoxy-phényl]-acétamide
(8) 2-(3-chloro-biphényl-4-yloxy)-N-[3-chloro-4-(2-diéthylamino-éthoxy)-phényl]-acétamide
(9) N-[3-chloro-4-(2-diéthylamino-éthoxy)-phényl]-2-(2-chloro-4-trifluorométhyl-phénoxy)-acétamide
(10) 2-(4-tert.-butyl-2-chloro-phénoxy)-N-[3-chloro-4-(2-diéthylamino-éthoxy)-phényl]-acétamide
(11) 3-chloro-4-{[3-chloro-4-(2-diéthylamino-éthoxy)-phénylcarbamoyl]-méthoxy}-benzoate de méthyle
(12) N-[3-chloro-4-(2-diéthylamino-éthoxy)-phényl]-2-(2,4-dibromo-phénoxy)-acétamide
(13) 2-(4-bromo-2-chloro-phénoxy)-N-[3-chloro-4-(2-diéthylamino-éthoxy)-phényl]-acétamide
(14) N-[3-chloro-4-(2-diéthylamino-éthoxy)-phényl]-2-(4-iodo-2-méthyl-phénoxy)-acétamide
(15) (2-{2-chloro-4-[2-(2,4-dichloro-phénoxy)-acétylamino]-phénoxy}-éthylamino)-acétate de méthyle
(16) N-(3-chloro-4-(2-pyrrolidin-1-yl-éthoxy)-phényl]-2-(2-chloro-4-trifluorométhyl-phénoxy)-acétamide
(17) N-{3-chloro-4-[2-(éthyl-propyl-amino)-éthoxy]-phényl}-2-(2-chloro-4-trifluorométhyl-phénoxy)-acétamide
(18) N-{3-chloro-4-[2-(éthyl-méthyl-amino)-éthoxy]-phényl}-2-(2-chloro-4-trifluorométhyl-phénoxy)-acétamide
(19) N-[3-chloro-4-(2-diéthylamino-éthoxy)-phényl]-2-(2-chloro-4-diméthyl-amino-phénoxy)-acétamide
(20) (E)-N-[3-chloro-4-(2-diéthylamino-éthoxy)-phényl]-3-(2-chloro-4-trifluoro-méthyl-phényl)-acrylamide
(21) N-[3-chloro-4-(2-diéthylamino-éthoxy)-phényl]-2-(2-chloro-4-trifluoro-méthyl-phénylamino)-acétamide
(22) N-[3-chloro-4-(2-diéthylamino-éthoxy)-phényl]-2-(2-chloro-4-furan-2-yl-phénoxy)-acétamide
(23) N-[3-chloro-4-(2-diéthylamino-éthoxy)-phényl]-2-(2-chloro-4-thiophén-2-yl-phénoxy)-acétamide
(24) N-[3-chloro-4-(2-diéthylamino-éthoxy)-phényl]-2-(2-chloro-4-pyridin-3-yl-phénoxy)-acétamide
(25) 2-(2-bromo-4-trifluorométhyl-phénoxy)-N-(3-chloro-4-(2-diéthylamino-éthoxy)-phényl]-acétamide
(26) N-{3-chloro-4-[2-(2,5-dihydro-pyrrol-1-yl)-éthoxy]-phényl}-2-(2-chloro-4-trifluorométhyl-phénoxy)-acétamide
(27) 1-(2-{2-chloro-4-[2-(2-chloro-4-trifluorométhyl-phénoxy)-acétylamino]-phénoxy}-éthyl)-pipéridine-4-carboxylate d'éthyle
(28) N-[3-chloro-4-(3-diéthylamino-propoxy)-phényl]-2-(2-chloro-4-trifluoro-méthyl-phénoxy)-acétamide
(29) N-{4-[2-(2-Aminométhyl-pyrrolidin-1-yl)-éthoxy]-3-chloro-phényl}-2-(2-chloro-4-trifluorométhyl-phénoxy)-acétamide
(30) N-{3-chloro-4-[2-(2-diméthylaminométhyl-pyrrolidin-1-yl)-éthoxy]-phényl}-2-(2-chloro-4-trifluorométhyl-phénoxy)-acétamide
(31) N-[3-bromo-4-(2-diéthylamino-éthoxy)-phényl]-2-(2-chloro-4-trifluoro-méthyl-phénoxy)-acétamide
(32) N-{3-chloro-4-[2-(4-méthoxy-pipéridin-1-yl)-éthoxy]-phényl}-2-(2-chloro-4-trifluorométhyl-phénoxy)-acétamide
(33) N-{3-chloro-4-[2-(4-hydroxy-pipéridin-1-yl)-éthoxy]-phényl}-2-(2-chloro-4-trifluorométhyl-phénoxy)-acétamide
(34) 2-(2-chloro-4-trifluorométhyl-phénoxy)-N-[4-(2-diéthylamino-éthoxy)-3-nitro-phényl]-acétamide
(35) N-[3-chloro-4-(2-diéthylamino-éthoxy)-phényl]-2-(2-chloro-4-trifluorométhoxy-phénylamino)-acétamide
(36) N-[3-chloro-4-(2-diéthylamino-éthoxy)-phényl]-2-(2-fluoro-4-trifluoro-méthyl-phénylamino)-acétamide
(37) 2-(2-bromo-4-trifluorométhyl-phénylamino)-N-[3-chloro-4-(2-diéthylamino-éthoxy)-phényl]-acétamide
(38) (E)-3-(4'-chloro-biphényl-4-yl)-N-(4-pipéridin-1-ylméthyl-phényl)acrylamide
(39) N-[3-chloro-4-(2-diéthylamino-éthylamino)-phényl]-2-(2-chloro-4-trifluoro-méthyl-phénoxy)-acétamide
(40) N-{3-chloro-4-[2-(4-méthyl-pipéridin-1-yl)-éthylamino]-phényl}-2-(2-chloro-4-trifluorométhyl-phénoxy)-acétamide
(41) (E)-3-(4'-chloro-biphényl-4-yl)-N-(4-diméthylaminométhyl-phényl)-acrylamide
(42) (E)-3-[5-(4-chloro-phényl)-pyridin-2-yl]-N-(4-pipéridin-1-ylméthylphényl)-acrylamide
(43) (E)-N-{3-chloro-4-[2-(4-méthyl-pipéridin-1-yl)-éthylamino]-phényl}-3-(2-chloro-4-trifluorométhyl-phényl)-acrylamide
(44) (E)-N-[3-chloro-4-(4-méthyl-pipéridin-1-ylméthyl)-phényl]-3-(2-chloro-4-trifluorométhyl-phényl)-acrylamide
(45) 2-(2-chloro-4-trifluorométhyl-phénoxy)-N-[4-(2-diéthylamino-éthoxy)-3-méthyl-phényl]-acétamide
(46) (E)-3-(2-chloro-4-trifluorméthyl-phényl)-N-[4-(2-diéthylamino-éthoxy)-3-méthyl-phényl]-acrylamide
(47) (E)-3-(2-chloro-4-trifluorométhyl-phényl)-N-[4-(2-diéthylamino-éthoxy)-3-méthoxy-phényl]-acrylamide
(48) (E)-N-[3-chloro-4-(2-diéthylamino-éthyl)-phényl]-3-(2-chloro-4-trifluorométhyl-phényl)-acrylamide
(49) N-[3-chloro-4-(2-diéthylamino-éthyl)-phényl]-2-(2-chloro-4-trifluorométhyl-phénoxy)-acétamide
(50) N-{3-chloro-4-[2-(4-méthyl-pipéridin-1-yl)-éthyl]-phényl}-2-(2-chloro-4-trifluorométhyl-phénoxy)-acétamide

y compris leurs sels.

**16.** Sels physiologiquement acceptables des composés amides selon une ou plusieurs des revendications 1 à 15.

**17.** Composition contenant au moins un composé amide selon une ou plusieurs des revendications 1 à 15 et/ou un sel selon la revendication 16 outre éventuellement un ou plusieurs adjuvants physiologiquement acceptables.

**18.** Médicament contenant au moins un composé amide selon une ou plusieurs des revendications 1 à 15 et/ou un sel selon la revendication 16 outre éventuellement un ou plusieurs vecteurs et/ou diluants inertes.

**19.** Utilisation non thérapeutique d'au moins un composé amide selon une ou plusieurs des revendications 1 à 15 et/ou d'un sel selon la revendication 16, y compris les composés exclus par les mesures (M2) et (M3) selon la revendication 1, pour influencer le comportement d'alimentation d'un mammifère en réduisant la faim, réfrénant l'appétit, contrôlant le comportement d'alimentation et/ou provoquant une sensation de satiété.

**20.** Utilisation non thérapeutique d'au moins un composé amide selon une ou plusieurs des revendications 1 à 15 et/ou d'un sel selon la revendication 16, y compris les composés exclus par les mesures (M2) et (M3) selon la revendication 1, pour réduire le poids corporel et/ou pour éviter une augmentation du poids corporel d'un mammifère.

**21.** Utilisation d'au moins un composé amide selon une ou plusieurs des revendications 1 à 15 et/ou d'un sel selon la revendication 16, y compris les composés exclus par les mesures (M2) et (M3) selon la revendication 1, pour la production d'un médicament à activité antagoniste de récepteur de MCH.

**22.** Utilisation d'au moins un composé amide selon une ou plusieurs des revendications 1 à 15 et/ou d'un sel selon la revendication 16, y compris les composés exclus par les mesures (M2) et (M3) selon la revendication 1, pour la production d'un médicament qui convient pour la prophylaxie et/ou le traitement des symptômes et/ou maladies qui sont provoqués par MCH ou qui sont dans une autre relation causale avec MCH.

**23.** Utilisation d'au moins un composé amide selon une ou plusieurs des revendications 1 à 15 et/ou d'un sel selon la revendication 16, y compris les composés exclus par les mesures (M2) et (M3) selon la revendication 1, pour la production d'un médicament qui convient pour la prophylaxie et/ou le traitement des troubles métaboliques et/ou des troubles de l'alimentation, en particulier de l'obésité, de la boulimie, de la boulimie mentale, de la cachexie, de l'anorexie, de l'anorexie mentale et de l'hyperphagie.

**24.** Utilisation d'au moins un composé amide selon une ou plusieurs des revendications 1 à 15 et/ou d'un sel selon la revendication 16, y compris les composés exclus par les mesures (M2) et (M3) selon la revendication 1, pour la production d'un médicament qui convient pour la prophylaxie et/ou le traitement des maladies et/ou troubles qui accompagnent l'obésité, en particulier du diabète, en particulier du diabète de type II, des complications diabétiques, y compris de la rétinopathie diabétique, de la neuropathie diabétique, de la néphropathie diabétique, de la résistance à l'insuline, de la tolérance au glucose pathologique, de l'insuffisance cardiaque, des maladies de la circulation cardiaque, en particulier de l'artériosclérose et de l'hypertension, de l'arthrite et de la gonite.

**25.** Utilisation d'au moins un composé amide selon une ou plusieurs des revendications 1 à 15 et/ou d'un sel selon la revendication 16, y compris les composés exclus par les mesures (M2) et (M3) selon la revendication 1, pour la

revendication 16, y compris les composés exclus par les mesures (M2) et (M3) selon la revendication 1, pour la production d'un médicament qui convient pour la prophylaxie et/ou le traitement de l'hyperlipidémie, de l'accumulation des graisses, des troubles émotionnels, des troubles de l'affectivité, des dépressions et des états de peur.

**26.** Médicament contenant
un premier principe actif qui est choisi parmi les composés amides selon une ou plusieurs des revendications 1 à 15 et/ou un sel selon la revendication 16, y compris les composés exclus par les mesures (M2) et (M3) selon la revendication 1, ainsi que
un second principe actif qui est choisi dans le groupe consistant en les principes actifs pour le traitement du diabète, les principes actifs pour le traitement des complications diabétiques, les principes actifs pour le traitement de l'obésité, de préférence différents des antagonistes de MCH, les principes actifs pour le traitement de l'hypertension, les principes actifs pour le traitement de l'hyperlipidémie, y compris l'artériosclérose, les principes actifs pour le traitement de l'arthrite, les principes actifs pour le traitement des états de peur et les principes actifs pour le traitement des dépressions,
outre éventuellement un ou plusieurs vecteurs et/ou diluants inertes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0121577 A
- WO 0182925 A
- EP 073016 A1
- US 3994900 A
- DE 1088955
- WO 0006153 A
- FR 1176918
- DE 3016827
- WO 0206245 A
- US 4948812 A
- US 3551478 A
- US 4146637 A
- US 4294851 A
- WO 0127081 A
- WO 9952869 A


### In der Beschreibung aufgeführte Nicht-Patentliteratur


- **Qu, D. et al.** A role for melanin-concentrating hormone in the central regulation of feeding behaviour. *Nature,* 1996, vol. 380 (6571), 243-7
- **Shimada, M. et al.** Mice lacking melanin-concentrating hormone are hypophagic and lean. *Nature,* 1998, vol. 396 (6712), 670-4
- **Borowsky, B. et al.** Antidepressant, anxiolytic and anorectic effects of a melanin-concentrating hormone-1 receptor antagonist. *Nat Med,* 2002, vol. 8 (8), 825-30
- **Chen, Y. et al.** Targeted disruption of the melanin-concentrating hormone receptor-1 results in hyperphagia and resistance to diet-induced obesity. *Endocrinology,* 2002, vol. 143 (7), 2469-77
- **Marsh, D.J. et al.** Melanin-concentrating hormone 1 receptor-deficient mice are lean, hyperactive, and hyperphagic and have altered metabolism. *Proc Natl Acad Sci U S A,* 2002, vol. 99 (5), 3240-5
- **Takekawa, S. et al.** T-226296: a novel, orally active and selective melanin-concentrating hormone receptor antagonist. *Eur J Pharmacol,* 2002, vol. 438 (3), 129-35
- **A. P. Tamiz et al.** *J. Med. Chem.,* 1999, vol. 42 (17), 3412-3420
- **von Fahmy et al.** *Archives of Pharmacal Research,* 2001, vol. 24 (3), 171-179
- **von Ariesan et al.** *Farmacia (Bucharest, Romania),* 1975, vol. 23 (3), 135-40
- **von Khlaponina.** *Khimiko-Farmatsevticheskii Zhurnal,* 1987, vol. 21 (8), 965-8
- **von Zheng et al.** *Zhongshan Daxue Xuebao, Ziran Kexueban,* 1989, vol. 28 (4), 124-7
- **von Baker et al.** *Journal of Medicinal Chemistry,* 1968, vol. 11 (5), 1054-9
- **von Guidicelli et al.** *Compt. Rend.,* 1955, vol. 241, 529-30
- *J. Org. Chem.,* 1962, vol. 27, 4660-4662
- **von Hoogduijn M et al.** Melanin-concentrating hormone and its receptor are expressed and functional in human skin. *Biochem. Biophys. Res Commun,* 2002, vol. 296, 698-701
- **von Karlsson OP ; Lofas S.** Flow-Mediated On-Surface Reconstitution of G-Protein Coupled Receptors for Applications in Surface Plasmon Resonance Biosensors. *Anal. Biochem.,* 2002, vol. 300, 132-138